(19) Europäisches Patentamt
European Patent Office
Office européen des brevets

(11) EP 2 647 628 A1

(12) **EUROPEAN PATENT APPLICATION**

(43) Date of publication:
**09.10.2013 Bulletin 2013/41**

(21) Application number: **12382132.4**

(22) Date of filing: **02.04.2012**

(51) Int Cl.:
***C07D 221/26*** (2006.01) ***C07D 401/04*** (2006.01)
***A61K 31/473*** (2006.01) *A61P 1/00* (2006.01)
*A61P 7/00* (2006.01) *A61P 13/00* (2006.01)
*A61P 19/00* (2006.01) *A61P 25/00* (2006.01)
*A61P 31/00* (2006.01) *A61P 35/00* (2006.01)
*A61P 37/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Almirall, S.A.**
  **08022 Barcelona (ES)**
- **Draconis Pharma, S.L.**
  **08225 Terrassa - Barcelona (ES)**
- **Laboratorios del. Dr. Esteve, S.A.**
  **08041 Barcelona (ES)**

(72) Inventors:
- **Aguilar, Nuria**
  **08005 BARCELONA (ES)**
- **Fernandez, Joan, Carles**
  **08004 BARCELONA (ES)**
- **Terricabras, Emma**
  **08173 SANT CUGAT DEL VALLES (Barcelona) (ES)**
- **Carceller Gonzalez, Elena**
  **08173 SANT CUGAT DEL VALLES (Barcelona) (ES)**
- **Salas Solana, Jordi**
  **08401 GRANOLLERS (Barcelona) (ES)**

(74) Representative: **Carpintero Lopez, Francisco et al**
**Herrero & Asociados, S.L.**
**Alcalá 35**
**28014 Madrid (ES)**

(54) **Substituted tricyclic compounds with activity towards ep1 receptors**

(57) The present invention belongs to the field of EP1 receptor ligands. More specifically it refers to compounds of general formula (I) having great affinity and selectivity for the EP1 receptor. The invention also refers to the process for their preparation, to their use as medicament for the treatment and/or prophylaxis of diseases or disorders mediated by the EP1 receptor as well as to pharmaceutical compositions comprising them.

EP 2 647 628 A1

Printed by Jouve, 75001 PARIS (FR)

## Description

### FIELD OF THE INVENTION

The present invention belongs to the field of EP1 receptor ligands. More specifically it refers to compounds of general formula (I) having great affinity and selectivity for the EP1 receptor. The invention also refers to the process for their preparation, to their use as medicament for the treatment and/or prophylaxis of diseases or disorders mediated by the EP1 receptor as well as to pharmaceutical compositions comprising them.

### BACKGROUND OF THE INVENTION

Prostanoids are a family of eicosanoids that comprise prostaglandins (PGs), prostacyclins (PGIs), and thromboxanes (Txs) . Their receptors belong to the G- protein coupled receptor (GPCR) superfamily of receptors and may be grouped into five classes, namely, prostaglandin D (DP), prostaglandin E (EP), prostaglandin F (FP), prostaglandin I (IP), and Thromboxane A (TP) based on their sensitivity to five naturally occurring prostanoids, PGD2, PGE2, PGF2 [alpha], PGI2, and TxA2, respectively (Coleman, R. A., 2000) .

Prostaglandins contribute to the sensitization of peripheral and central nociceptive neurons during peripheral inflammation (Dirig and Yaksh, 1999) and play an important role in the pathogenesis of neuropathic pain following nerve injury (Syriatowicz et al 1999; Kawahara et al, 2001; Samad et al, 2002; Ma and Eisenach, 2003; Durrenberger et al., 2006).

Prostaglandin E2 (PGE2) is considered to be the dominant pro-nociceptive prostanoid. Guay and colleagues, analyzing the concentrations of different prostaglandins in the cerebrospinal fluid, found that PGE2 was the most prevalent prostanoid and exhibited the highest increase after peripheral carrageenan-induced inflammation (Guay et al., 2004). PGE2 is generated in most cells in response to mechanical, thermal or chemical injury and inflammatory insult, resulting in sensitization or direct activation of nearby sensory nerve endings. Its production requires the activity of at least one of the two cyclooxygenase isoforms, COX-1 constitutively expressed or COX-2 which is inducible and particularly relevant for inflammation-induced PGE2 formation. Therefore, non-selective inhibitors of COX-1 and COX-2, and selective COX-2 inhibitors provide good pain relief. However, the long-term use is associated with gastrointestinal or cardiovascular side effects, respectively.

Downstream components of the inflammatory cascade could be an alternative approach for the treatment of the PGE2 associated pain. PGE2 binds to four different G-protein coupled receptors named EP1, EP2, EP3 and EP4 (Narumiya et al., 1999).

Studies employing antagonists suggest that blocking EP1, EP2, EP3 or EP4 receptors may reduce certain types of pain (Oka et al. 1997; Omote et al., 2002; Lin et al, 2006) and agonists increase nociceptive responses (Minami et al., 1994). Among these PGE2 receptor subtypes, most of drug discovery studies have focused on the EP1 receptors (Hall et al., 2007).

EP1 receptor stimulation mediates increases in intracellular calcium ions, facilitating neurotransmitter release (Asbóth et al., 1996). EP1 receptor is preferentially expressed in primary sensory neurons, including their spinal cord terminals (Oidda et al., 1995) although it is also distributed in other tissues (Breyer et al., 2000; Schlötzer-Schrehardt et al., 2002). In the brain, marked differences in the level of EP1 expression among the cerebral regions were found. The strongest levels of EP1 mRNA were found in parietal cortex and cerebellum, followed in descending order by frontal cortex and striatum. The hypothalamus, hippocampus and brain stem displayed a low-level EP1 mRNA signal (Candelario-Jalil et al., 2005). In the spinal cord, several studies have reported the effects of PGE2 on neuronal excitability or synaptic transmission (Baba et al., 2001) and pain transmission (Nakayama et al., 2004). Therefore, EP1 receptor antagonists, blocking the positive feedback cascade mediated by $PGE_2$, may result in analgesic efficacy. In this regard, using EP receptor deficient mice, a prominent contribution of EP1 receptors has been described (Minami et al., 2001). EP1 -/- knockout mice demonstrated a role of this receptor in mediating peripheral heat sensitization after subcutaneous PGE2 injection (Moriyama et al. 2005; Johansson et al. 2011), and EP1 receptor antagonism has been reported to reduce mechanical hyperalgesia in nerve injured rats (Kawahara et al., 2001), in the carrageenan model (Nakayama et al. 2002), or in the incisional model of postoperative pain (Omote et al 2002). Moreover, EP1 antagonists demonstrated analgesic activity in a complete Freund's adjuvant model of knee joint arthritis (Giblin et al, 2007; Hall et al, 2009). It has also been reported that the contribution of PGE2 in human visceral pain hypersensitivity is mediated through the EP1 receptor (Sarkar et al., 2003).

In addition to being useful for modulating pain, EP1 antagonists may also be useful for the treatment or prevention of other EP1 receptor-mediated diseases such as motility-related disorders including gastrointestinal disorders, urinary incontinence and other urinary tract diseases; dysmenorrhea; preterm labour; diabetic retinopathy; tumour angiogenesis; cancer; metastatic tumour growth; neurodegenerative diseases including senile dementia, Alzheimer's disease, Pick's disease, Huntington's chorea, Parkinson's disease, Creutzfeldt-Jakob disease, or amyotrophic lateral sclerosis; neuroprotection/stroke; glaucoma; osteoporosis; bone fractures; Paget's disease; hyperthermia including different types of

fever as rheumatic fever; symptoms associated with influenza or other viral infections; gastrointestinal disorders related with chemotherapy or irritable bowel syndrome; gastrointestinal bleeding; coagulation disorders including anaemia, hypoprothrombinemia, haemophilia or other bleeding problems; kidney diseases including nephritis, particularly mesangial proliferative glomerulonephritis and nephritic syndrome; thrombosis and occlusive vascular diseases.

In turn, EP1 receptor agonists also may have a number of utilities. These include, but are not limited to treatment of influenza, bone fracture healing, bone disease, glaucoma, ocular hypertension, dysmenorrhoea, pre-term labour, immune disorders, osteoporosis, asthma, allergy, fertility, male sexual dysfunction, female sexual dysfunction, periodontal disease, gastric ulcer, and renal disease. EP receptor agonists may also be useful for expansion of hematopoietic stem cell populations.

Based on the above mentioned results coming from animal and human studies, EP1 receptor has been identified as a selective target for the development of new potential therapies for the treatment of those disorders where PGE2 action is involved. In view of the potential therapeutic applications of agonists and antagonists of the EP1 receptor, a great effort is being directed to find selective ligands. Despite intense research efforts in this area, very few compounds with selective EP1 activity have been reported.

There is thus still a need to find compounds having pharmacological activity towards the EP1 receptor, being both effective and selective, and having good "drugability" properties, i.e. good pharmaceutical properties related to administration, distribution, metabolism and excretion.

The present invention hereby provide some novel compounds complying with the above mentioned properties.

## OBJECT OF THE INVENTION

The present invention discloses novel compounds with great affinity to EP1 receptors which might be used for the treatment of EP1-related disorders or diseases.

Specifically, it is an object of the invention a compound of general formula I:

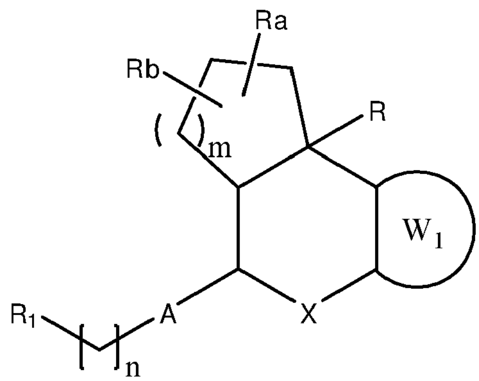

**(I)**

wherein:

**W1** is a benzene ring optionally substituted by one or more $R_2$ and optionally fused to a carbocyclic ring; or a 5 or 6-membered heterocyclic ring containing 1 or 2 heteroatoms selected from N, O or S, optionally substituted by one or more $R_2$;
**R** is a hydrogen; or $C_{1-6}$- alkyl;
**Ra** and **Rb** are independently selected from hydrogen; =O;- OH; $CH_2OH$- CO- $C_1$-$_6$- alkyl;- $SO_2$- $C_{1-6}$- alkyl; $C_{1-6}$- alkyl;- O- $C_{1-6}$- alkyl or- O- $C_{1-6}$- haloalkyl;
**$R_1$** is a- COOH; a tetrazol; a- $SO_2$- NH- C (=O)- $R_3$; a- C (=O) NH- $SO_2$- $R_4$; a- $SO_2$- OH or a- CN;
**$R_2$** is independently selected from hydrogen; halogen; $C_{1-6}$ alkyl; $C_{1-6}$- haloalkyl;- O- $C_{1-6}$- alkyl;- O- $C_{1-6}$- haloalkyl, $C_{3-6}$cycloalkyl;- O- $C_{3-6}$cycloalkyl;- $NR_5SO_2R_6$;
phenyl; -$CONH_2$ or -CN;
**$R_3$, $R_4$** are independently selected from a hydrogen; $C_{1-6}$- alkyl; an optionally substituted phenyl; or- N $(CH_3)_2$;
**$R_5$** and **$R_6$** are independently selected from a hydrogen; or $C_{1-6}$- alkyl;
**$R_7$** is selected from a hydrogen; $C_{1-6}$- alkyl; or a- $COR_8$

**$R_8$** is a $C_{1-6}$- alkyl;
**X** is selected from $NR_7$ or O;
**A** is a phenyl optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted by a $C_{1-6}$- alkyl; or an heteroaromatic ring or ring system optionally substituted by one or more substituents selected from $C_1$-$_6$- alkyl, halogen, $C_1$-$_6$- haloalkyl, -O- $C_{1-6}$- alkyl, -O- $C_{1-6}$- haloalkyl- NH- CO- $CH_3$, a- CN or a $C_{3-6}$cycloalkyl optionally substituted by a $C_{1-6}$- alkyl ;
**n** is 0, 1 or 2,
**m** is 1, 2, or 3;

and the salts, solvates and prodrugs thereof.

It is also an object of the invention the process for the preparation of compounds of general formula (I).

In another aspect, the invention relates to a compound of general formula (I) for use as a medicament.

Yet another object of the invention is a compound of general formula (I) for use in the treatment and/or prophylaxis of diseases or disorders mediated by the EP1 receptor. This includes but is not limited to diseases such as inflammatory related pain including low back and neck pain, skeletal pain, post-partum pain, toothache, sprains and straits, myositis, neuralgia, synovitis, arthritis, including rheumatoid arthritis, degenerative joint diseases, gout and ankylosing spondylitis, bursitis, burns including radiation and corrosive chemical injuries and sunburns; postoperative pain; neuropathic pain; visceral pain; tension headache; cluster headaches; migraine; motility-related disorders including gastrointestinal disorders, urinary incontinence and other urinary tract diseases; dysmenorrhea; preterm labour; diabetic retinopathy; tumour angiogenesis; cancer; metastatic tumour growth; neurodegenerative diseases including senile dementia, Alzheimer's disease, Pick's disease, Huntington's chorea, Parkinson's disease, Creutzfeldt-Jakob disease, or amyotrophic lateral sclerosis; neuroprotection/stroke; glaucoma; osteoporosis; bone fractures; Paget's disease; hyperthermia including different types of fever as rheumatic fever; symptoms associated with influenza or other viral infections; gastrointestinal disorders related with chemotherapy or irritable bowel syndrome; gastrointestinal bleeding; coagulation disorders including anaemia, hypoprothrombinemia, haemophilia or other bleeding problems; kidney diseases including nephritis, particularly mesangial proliferative glomerulonephritis and nephritic syndrome; thrombosis and occlusive vascular diseases.

It is another object of the invention a pharmaceutical composition comprising at least one compound of general formula (I) and at least one pharmaceutically acceptable carrier, additive, adjuvant or vehicle.

## DETAILED DESCRIPTION OF THE INVENTION

In a first aspect the invention relates to compounds of general formula (I):

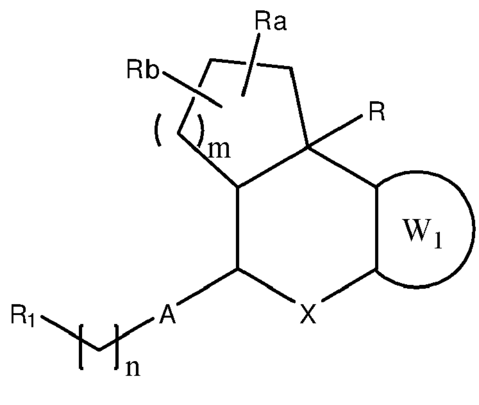

**(I)**

wherein:

**W1** is a benzene ring optionally substituted by one or more $R_2$ and optionally fused to a carbocyclic ring; or a 5 or 6-membered heterocyclic ring containing 1 or 2 heteroatoms selected from N, O or S, optionally substituted by one or more $R_2$;

**R** is a hydrogen; or $C_{1-6}$- alkyl;
**Ra** and **Rb** are independently selected from hydrogen; =O;- OH; $CH_2OH$- CO- $C_1$-$_6$- alkyl;- $SO_2$- $C_{1-6}$- alkyl; $C_{1-6}$-alkyl;- O- $C_{1-6}$- alkyl or- O- $C_{1-6}$- haloalkyl;
**$R_1$** is a- COOH; a tetrazol; a- $SO_2$- NH- C (=O)- $R_3$; a- C (=O) NH- $SO_2$- $R_4$; a- $SO_2$- OH or a- CN;
**$R_2$** is independently selected from hydrogen; halogen; $C_{1-6}$ alkyl; $C_{1-6}$- haloalkyl;- O- $C_{1-6}$- alkyl;- O- $C_{1-6}$- haloalkyl; $C_{3-6}$cycloalkyl;- O- $C_{3-6}$cycloalkyl;- $NR_5SO_2R_6$; phenyl;- $CONH_2$ or- CN;
**$R_3$** and **$R_4$** are independently selected from a hydrogen; $C_{1-6}$- alkyl; an optionally substituted phenyl; or- N $(CH_3)_2$
**$R_5$** and **$R_6$** are independently selected from a hydrogen; or $C_{1-6}$-alkyl
**$R_7$** is selected from a hydrogen; $C_{1-6}$- alkyl; or a- $COR_8$
**$R_8$** is a $C_{1-6}$- alkyl;
**X** is selected from $NR_7$ or O;
**A** is a phenyl optionally substituted by one or more substituents selected from $C_{1-6}$- alkyl, halogen, $C_1$-$_6$- haloalkyl, -O- $C_{1-6}$- alkyl, -O- $C_{1-6}$- haloalkyl, -O- $C_{1-6}$- haloalkyl, -NH- CO- $CH_3$, a- CN or a $C_{3-6}$cycloalkyl optionally substituted by a $C_{1-6}$- alkyl; or an heteroaromatic ring or ring system optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -O- $C_{1-6}$- haloalkyl, -NH- CO- $CH_3$, a- CN or a $C_{3-6}$cycloalkyl optionally substituted by a $C_{1-6}$- alkyl;
**n** is 0, 1 or 2,
**m** is 1,2 or 3

and the salts, solvates and prodrugs thereof.

Also included within the scope of the invention are the isomers, polymorphs, isotopes, salts, solvates and prodrugs of the compounds of formula (I). Any reference to a compound of formula (I) throughout the present specification includes a reference to any isomer, polymorph, isotope, salt, solvate or prodrug of such compound of formula I.

The compounds of formula I may exist in different physical forms, i.e. amorphous and crystalline forms. Moreover, the compounds of the invention may have the ability to crystallize in more than one form, a characteristic which is known as polymorphism. Polymorphs can be distinguished by various physical properties well known in the art such as X-ray diffraction pattern, melting point or solubility. All physical forms of the compounds of formula I, including all polymorphic forms ("polymorphs") thereof, are included within the scope of the invention.

Some of the compounds of the present invention may exist as several optical isomers and/or several diastereoisomers. Diastereoisomers can be separated by conventional techniques such as chromatography or fractional crystallization. Optical isomers can be resolved by conventional techniques of optical resolution to give optically pure isomers. This resolution can be carried out on any chiral synthetic intermediate or on the products of formula I. Optically pure isomers can also be individually obtained using enantiospecific synthesis. The present invention covers all individual isomers as well as mixtures thereof (for example racemic mixtures or mixtures of diastereomers), whether obtained by synthesis or by physically mixing them.

In addition, any formula given herein is intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes that can be incorporated into compounds of the invention include isotopes of hydrogen, carbon, nitrogen, oxygen, phosphorous, fluorine, chlorine, and iodine, such as $^{2}H$, $^{3}H$, $^{11}C$, $^{13}C$, $^{14}C$, $^{15}N$, $^{18}O$, $^{17}O$, $^{31}P$, $^{32}P$, $^{36}S$, $^{18}F$ , $^{36}Cl$ and $^{125}I$ , respectively, Such isotopically labelled compounds are useful in metabolic studies (preferably with 14C), reaction kinetic studies (with, for example $^{2}H$ or $^{3}H$), detection or imaging techniques [such as positron emission tomography (PET) or single- photon emission computed tomography (SPECT) ] including drug or substrate tissue distribution assays, or in radioactive treatment of patients. In particular, an $^{18}F$ or $^{11}C$ labeled compound may be particularly preferred for PET or SPECT studies. Further, substitution with heavier isotopes such as deuterium (i.e., $^{2}H$) may afford certain therapeutic advantages resulting from greater metabolic stability, for example increased in vivo half- life or reduced dosage requirements. In addition to the unlabeled form, all isotopically labeled forms of the compounds of formula I are included within the scope of the invention.

"Halogen" or "halo" as referred in the present invention represent fluorine, chlorine, bromine or iodine.

The term "alkyl, " alone or in combination, means an acyclic radical, linear or branched, preferably containing from 1 to about 6 carbon atoms. Examples of such radicals include methyl, ethyl, n- propyl, isopropyl, n- butyl, isobutyl, sec- butyl, tert- butyl, pentyl, iso- amyl, hexyl, heptyl, octyl, and the like. Where no specific substitution is specified, alkyl radicals may be optionally substituted with groups consisting of hydroxy, sulfhydryl, methoxy, ethoxy, amino, cyano, chloro, and fluoro. The carbon atom content of various hydrocarbon- containing moieties is indicated by suffix designating a lower and upper number of carbon atoms in the moiety. Thus, for example, '$C_{1-6}$- alkyl' refers to alkyl of 1 to 6 carbon atoms, inclusive.

An "alkylene" linking group preferably contains 1- 4 carbon atoms and represents for example methylene, ethylene, propylene, butylene. The carbon atom content of various hydrocarbon- containing moieties is indicated by

suffix designating a lower and upper number of carbon atoms in the moiety. Thus, for example, '$C_{1-4}$- alkylene' refers to an alkylene of 1 to 4 carbon atoms, inclusive.

An "alkenylene" linking group preferably contains 2 to 4 carbon atoms and represents for example ethenylene, 1, 3- propenylene, 1, 4- but- 1- enylene, 1, 4- but- 2- ethylene. The carbon atom content of various hydrocarbon- containing moieties is indicated by suffix designating a lower and upper number of carbon atoms in the moiety. Thus, for example, '$C_{2-4}$- alkenylene' refers to alkenylene of 2 to 4 carbon atoms, inclusive.

"Cycloalkyl" is preferably a monocyclic cycloalkyl containing from three to six carbon atoms. Examples include cyclopropyl, cyclobutyl, cyclopentyl, and cyclohexyl. The carbon atom content of various hydrocarbon- containing moieties is indicated by suffix designating a lower and upper number of carbon atoms in the moiety. Thus, for example, '$C_{3-6}$- cycloalkyl' refers to cycloalkyl of 3 to 6 carbon atoms, inclusive.

The term "carbocyclic", "carbocyclic ring" and "carbocyclyl" refer to a saturated, unsaturated or aromatic mono- or multi-ring cycloalkyl only formed from carbon atoms.

The terms "heterocycle", "heterocyclic ring" and "heterocyclyl" refer to a saturated, unsaturated or aromatic mono- or multi-ring cycloalkyl wherein one or more carbon atoms is replaced by N, S, or O. The terms "heterocycle", "heterocyclic ring system," and "heterocyclyl" include fully saturated ring structures such as piperazinyl, dioxanyl, tetrahydrofuranyl, oxiranyl, aziridinyl, morpholinyl, pyrrolidinyl, piperidinyl, thiazolidinyl, and others. The terms "heterocycle", "heterocyclic ring system," and "heterocyclyl" also include partially unsaturated ring structures such as dihydrofuranyl, dihydropyrrolyl, pyrazolinyl, imidazolinyl, pyrrolinyl, chromanyl, dihydrothienyl, and others. The term "heterocycle", "heterocyclic ring system," and "heterocyclyl" also include aromatic structures such as pyridyl, pyrimidinyl, pyridazinyl, pyrazinyl, thienyl, furanyl, pyrrolyl, pyrazolyl, imidazolyl, oxazolyl, isoxazolyl, thiazolyl, isothiazolyl, triazolyl, oxadiazolyl, thiadiazolyl, and tetrazolyl, optionally substituted.

The term "heteroaromatic ring" refers to an aromatic heterocyclic ring. Examples of "heteroaromatic ring" include pyridyl, pyrimidinyl, pyridazinyl, pyrazinyl, thionyl, furanyl, pyrrolyl, pyrazolyl, imidazolyl, oxazolyl, isoxazolyl, thiazolyl, isothiazolyl, triazolyl, oxadiazolyl, thiadiazolyl, and tetrazolyl, optionally substituted.

The term "ring" or "ring system" according to the present invention refers to ring systems comprising saturated, unsaturated or aromatic carbocyclic ring systems which contain optionally at least one heteroatom as ring member and which are optionally at least mono-substituted. Said ring systems may be condensed to other carbocyclic ring systems.

The term "monocyclic ring" refers to a ring system composed of a single ring.

The term "polycyclic ring" refers to a ring system composed of at least two rings.

The term "salt" must be understood as any form of an active compound used in accordance with this invention in which the said compound is in ionic form or is charged and coupled to a counter-ion (a cation or anion) or is in solution. This definition also includes quaternary ammonium salts and complexes of the active molecule with other molecules and ions, particularly complexes formed via ionic interactions. The definition particularly includes physiologically acceptable salts. This term must be understood as equivalent to "pharmaceutically acceptable salts".

The term "pharmaceutically acceptable salts" in the context of this invention means any salt that is tolerated physiologically (normally meaning that it is not toxic, particularly as a result of the counter- ion) when used in an appropriate manner for a treatment, particularly applied or used in humans and/or mammals. These pharmaceutically acceptable salts may be formed with cations or bases and, in the context of this invention, are understood to be salts formed by at least one compound used in accordance with the invention- normally an acid (deprotonated)- such as an anion and at least one physiologically tolerated cation, preferably inorganic, particularly when used on humans and/or mammals. Salts with alkali and alkali earth metals are particularly preferred, as well as those formed with ammonium cations ($NH_4^+$) . Preferred salts are those formed with (mono) or (di) sodium, (mono) or (di) potassium, magnesium or calcium. These physiologically acceptable salts may also be formed with anions or acids and, in the context of this invention, are understood as being salts formed by at least one compound used in accordance with the invention- normally protonated, for example in nitrogen- such as a cation and at least one physiologically tolerated anion, particularly when used on humans and/or mammals. This definition specifically includes in the context of this invention a salt formed by a physiologically tolerated acid, i.e. salts of a specific active compound with physiologically tolerated organic or inorganic acids- particularly when used on humans and/or mammals. Examples of this type of salts are those formed with: hydrochloric acid, hydrobromic acid, sulphuric acid, methanesulfonic acid, formic acid, acetic acid, oxalic acid, succinic acid, malic acid, tartaric acid, mandelic acid, fumaric acid, lactic acid or citric acid.

The term "solvate" in accordance with this invention should be understood as meaning any form of the active compound in accordance with the invention in which said compound is bonded by a non-covalent bond to another molecule (normally a polar solvent), especially including hydrates and alcoholates, for example methanolate.

The term "prodrug" is used in its broadest sense and encompasses those derivatives that are converted in vivo to the compounds of the invention. Examples of prodrugs include, but are not limited to, derivatives and metabolites of the compounds of formula (I) that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Preferably, prodrugs of compounds with carboxyl functional groups are the lower alkyl esters of the

carboxylic acid. The carboxylate esters are conveniently formed by esterifying any of the carboxylic acid moieties present on the molecule. Prodrugs can typically be prepared using well-known methods, such as those described by Burger "Medicinal Chemistry and Drug Discovery 6th ed. (Donald J. Abraham ed., 2001 , Wiley) and "Design and Applications of Prodrugs" (H. Bundgaard ed., 1985, Harwood Academic Publishers).

The terms "prevention", "preventing", "preventive" "prevent" and "prophylaxis" refer to the capacity of a therapeutic to avoid, minimize or difficult the onset or development of a disease or condition before its onset.

The terms "treating" or "treatment" is meant at least a suppression or an amelioration of the symptoms associated with the condition afflicting the subject, where suppression and amelioration are used in a broad sense to refer to at least a reduction in the magnitude of a parameter, e.g., symptom associated with the condition being treated, such as pain. As such, the method of the present invention also includes situations where the condition is completely inhibited, terminated, such that the subject no longer experiences the condition.

A particular embodiment of the invention compounds of formula (I) are represented by general formula (Ia):

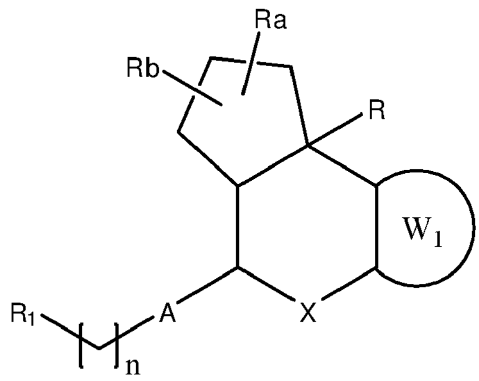

**(Ia)**

where **W1, R, Ra, Rb, $R_1$, A, X** and **n** have the same meaning as for compounds of general formula (I);

In another particular and preferred embodiment of the invention the compounds of formula (I) and more particularly those with formula (Ia) are represented by the more particular general formula (Ia'):

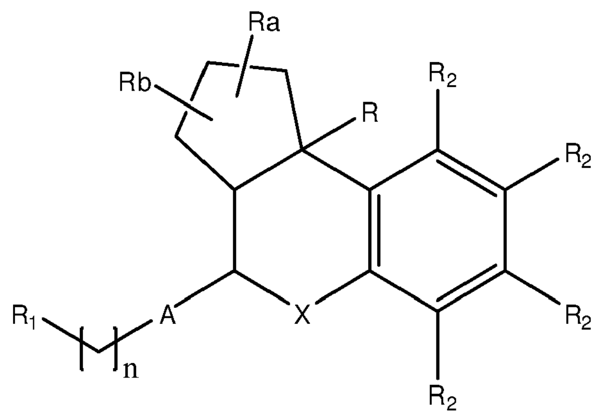

**(Ia')**

where **R, Ra, Rb, $R_1$, $R_2$, A, X** and **n** have the same meanings as for compounds of formula (Ia);

In another particular embodiment of the invention compounds of general formula (I) are represented by compounds of general formula (Ib):

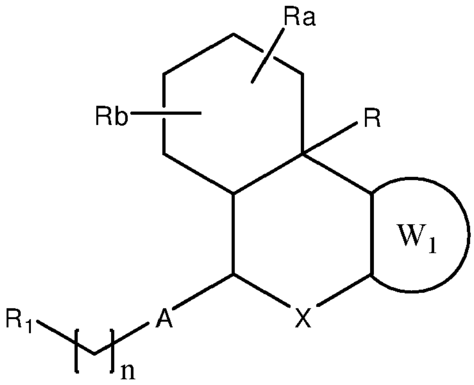

**(Ib)**

where **W1**, **R, Ra, Rb, $R_1$, A, X** and **n** have the same meaning as for general formula (I).

In another particular and preferred embodiment of the invention the compounds of formula (I) and more particularly those with formula (Ib) are represented by the more particular general formula (Ib'):

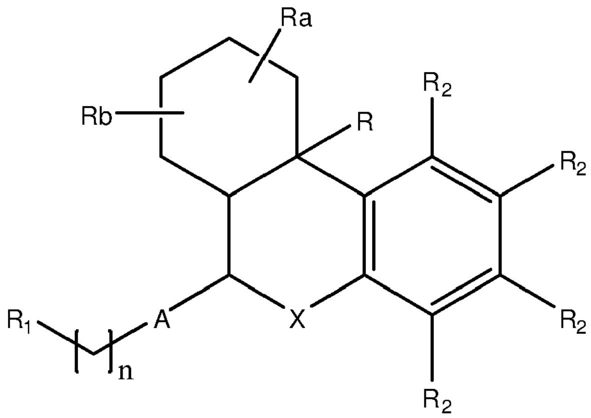

**(Ib')**

Where **R, Ra, Rb, $R_1$, $R_2$, A, X** and **n** have the same meanings as in general formula (I).

In another particular and preferred embodiment of the invention the compounds of formula (I) are represented by compounds of general formual (Ic):

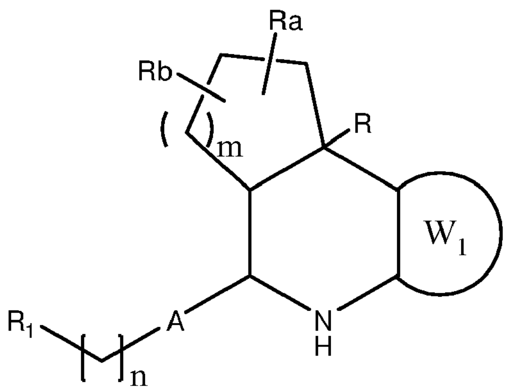

**(Ic)**

where **R, Ra, Rb, $R_1$, A, W1**, **m** and **n** have the same meanings as in general formula (I).

In another particular and preferred embodiment of the invention the compounds of formula (I) are represented by compounds of general formual (Id):

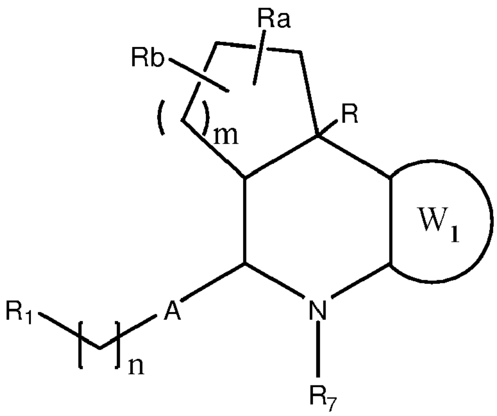

**(Id)**

where **R, Ra, Rb, $R_1$, $R_7$, A, W1**, **m** and **n** have the same meanings as in general formula (I).

In another particular and preferred embodiment of the invention the compounds of formula (I) are represented by compounds of general formual (Ie):

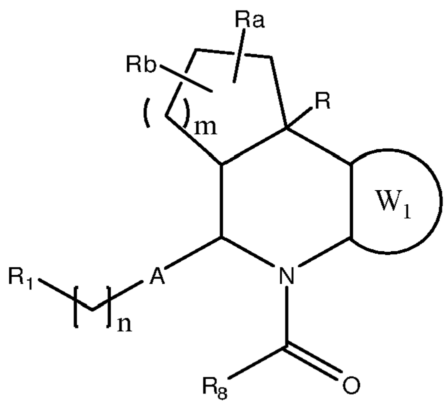

**(Ie)**

where **R, Ra, Rb, $R_1$, $R_8$, A, W1, m** and **n** have the same meanings as in general formula (I).

In a preferred embodiment of the invention **$R_1$** is a -COOH group in the compounds of general formula (I), (Ia),

(Ia'), (Ib), (Ib'), (Ic), (Id) and (Ie).

In another preferred embodiment of the invention **A** is a phenyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -O- $C_{1-6}$- haloalkyl, -NH- CO- $CH_3$, a- CN or a $C_{3-6}$cycloalkyl optionally substituted by a $C_{1-6}$- alkyl; a pyridinyl group optionally substituted by one or more substituents selected from $C_{1-6}$- alkyl, halogen, $C_{1-6}$- haloalkyl, -O- $C_{1-6}$- alky, -O- $C_{1-6}$- haloalkyl, -O- $C_{1-6}$- haloalkyl, -NH- CO- $CH_3$, a- CN or a $C_{3-6}$cycloalkyl optionally substituted by a $C_{1-6}$- alkyl l; a pyrazolyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -O- $C_{1-6}$- haloalkyl, -NH- CO- $CH_3$, a- CN or a $C_{3-6}$cycloalkyl optionally substituted by a $C_{1-6}$- alkyl; an imidazolyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -O- $C_{1-6}$- haloalkyl, -NH- CO- $CH_3$, a- CN or a $C_{3-6}$cycloalkyl optionally substituted by a $C_{1-6}$- alkyl; or a thiazolyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -O- $C_{1-6}$- haloalkyl, -NH- CO- $CH_3$, a- CN or a $C_{3-6}$cycloalkyl optionally substituted by a $C_{1-6}$- alkyl.

In another preferred embodiment in the compounds of the invention **X** is $NR_7$ where $R_7$ is a hydrogen .

In another preferred embodiment **n** is 0.

Another preferred embodiment is represented by compounds of formula (I) where **W1** is a benzene ring optionally substituted by one or more $\mathbf{R_2}$ where $\mathbf{R_2}$ has the same meaning as for general formula (I).

Among all the compounds encompassed by the general formula (I), (Ia), (Ia'), (Ib) (Ib'), (Ic), (Id) and/or (Ie) the following compounds are particularly preferred:

- 4- ((3aS, 4R, 9bR)- 2, 3, 3a, 4, 5, 9b- hexahydro- 1H- cyclopenta [c] quinolin- 4- yl) benzoic acid;
- 4- ((3aS, 4R, 9bR)- 8- bromo- 2, 3, 3a, 4, 5, 9b- hexahydro- 1H- cyclopenta [c] quinolin- 4- yl) benzoic acid;
- 4- ((3aS, 4R, 9bR)- 8- (trifluoromethyl)- 2, 3, 3a, 4, 5, 9b- hexahydro- 1H- cyclopenta [c] quinolin- 4- yl) benzoic acid;
- 4- ((3aS, 4R, 9bR)- 2, 3, 3a, 4, 5, 9b- hexahydro- 8- methyl- 1H- cyclopenta [c] quinolin- 4- yl) benzoic acid;
- 4- ((3aS, 4R, 9bR)- 2, 3, 3a, 4, 5, 9b- hexahydro- 8- methyl- 1H- cyclopenta [c] quinolin- 4- yl) benzoic acid (enantiomer 1) ;
- 4- ((3aS, 4R, 9bR)- 2, 3, 3a, 4, 5, 9b- hexahydro- 8- methyl- 1H- cyclopenta [c] quinolin- 4- yl) benzoic acid (enantiomer 2) ;
- 4- ((3aS, 4S, 9bR)- 2, 3, 3a, 4, 5, 9b- hexahydro- 8- methyl- 1H- cyclopenta [c] quinolin- 4- yl) benzoic acid;
- 4- ((3aS, 4R, 9bR)- 8- chloro- 2, 3, 3a, 4, 5, 9b- hexahydro- 1H- cyclopenta [c] quinolin- 4- yl) benzoic acid;
- 4- ((3aS, 4R, 9bR)- 8- chloro- 2, 3, 3a, 4, 5, 9b- hexahydro- 1H- cyclopenta [c] quinolin- 4- yl) benzoic acid (enantiomer 1) ;
- 4- ((3aS, 4R, 9bR)- 8- chloro- 2, 3, 3a, 4, 5, 9b- hexahydro- 1H- cyclopenta [c] quinolin- 4- yl) benzoic acid (enantiomer 2) ;
- 4- ((3aS, 4R, 9bR)- 2, 3, 3a, 4, 5, 9b- hexahydro- 8- methoxy- 1H- cyclopenta [c] quinolin- 4- yl) benzoic acid;
- 4- ((3aS, 4R, 9bR)- 2, 3, 3a, 4, 5, 9b- hexahydro- 8- methoxy- 1H- cyclopenta [c] quinolin- 4- yl) benzoic acid (enantiomer 1) ;
- 4- ((3aS, 4R, 9bR)- 2, 3, 3a, 4, 5, 9b- hexahydro- 8- methoxy- 1H- cyclopenta [c] quinolin- 4- yl) benzoic acid (enantiomer 2) ;
- 4- ((3aS, 4S, 9bR)- 2, 3, 3a, 4, 5, 9b- hexahydro- 8- methoxy- 1H- cyclopenta [c] quinolin- 4- yl) benzoic acid;
- 4- ((3aS, 4R, 9bR)- 6- cyclopropyl- 2, 3, 3a, 4, 5, 9b- hexahydro- 1H- cyclopenta [c] quinolin- 4- yl) benzoic acid;
- 4- ((3aS, 4S, 9bR)- 8- cyclopropyl- 2, 3, 3a, 4, 5, 9b- hexahydro- 1H- cyclopenta [c] quinolin- 4- yl) benzoic acid;

- 4- ((3aS, 4R, 9bR)- 8- cyclopropyl- 2, 3, 3a, 4, 5, 9b- hexahydro- 1H- cyclopenta [c] quinolin- 4- yl) benzoic acid;
- (+)- 4- ((3a*S*, 4*R*, 9b*R*)- 8- cyclopropyl- 2, 3, 3a, 4, 5, 9b- hexahydro- 1*H* cyclopenta [*c*] quinolin- 4- yl) benzoic acid;
- (- )- 4- ((3a*S*, 4*R*, 9b*R*)- 8- cyclopropyl- 2, 3, 3a, 4, 5, 9b- hexahydro- 1*H*- cyclopenta [*c*] quinolin- 4- yl) benzoic acid;
- 4- ((3aS, 4*R*, 9bR)- 2, 3, 3a, 4, 5, 9b- hexahydro- 8- propyl- 1H- cyclopenta [c] quinolin- 4- yl) benzoic acid;
- 4- ((3aS, 4R, 9bR)- 8- fluoro- 2, 3, 3a, 4, 5, 9b- hexahydro- 1H- cyclopenta [c] quinolin- 4- yl) benzoic acid;
- 4- ((3aS, 4R, 9bR)- 2, 3, 3a, 4, 5, 9b- hexahydro- 8- phenyl- 1H- cyclopenta [c] quinolin- 4- yl) benzoic acid;
- 4- ((3aS, 4R, 9bR)- 8- tert- butyl- 2, 3, 3a, 4, 5, 9b- hexahydro- 1H- cyclopenta [c] quinolin- 4- yl) benzoic acid;
- 4- ((3aS, 4R, 9bR)- 8- ethyl- 2, 3, 3a, 4, 5, 9b- hexahydro- 1H- cyclopenta [c] quinolin- 4- yl) benzoic acid;
- (+)- 4- ((3a*S*, 4*R*, 9*bR*)- 8- ethyl- 2, 3, 3a, 4, 5, 9b- hexahydro- 1H- cyclopenta [c] quinolin- 4- yl) benzoic acid;
- (- )- 4- ((3a*S*, 4*R*, 9b*R*)- 8- ethyl- 2, 3, 3a, 4, 5, 9b- hexahydro- 1H- cyclopenta [c] quinolin- 4- yl) benzoic acid;
- 4- ((3aS, 4R, 9bR)- 8- carbamoyl- 2, 3, 3a, 4, 5, 9b- hexahydro- 1H- cyclopenta [c] quinolin- 4- yl) benzoic acid;
- 4- ((3aS, 4R, 9bR)- 2, 3, 3a, 4, 5, 9b- hexahydro- 8- isopropyl- 1H- cyclopenta [c] quinolin- 4- yl) benzoic acid;
- 4- ((3aS, 4R, 9bR)- 8- cyano- 2, 3, 3a, 4, 5, 9b- hexahydro- 1H- cyclopenta [c] quinolin- 4- yl) benzoic acid;
- 4- ((3aS, 4R, 9bR)- 8- [(N- methylsulfonyl) amino]- )- 2, 3, 3a, 4, 5, 9b- hexahydro- 1H- cyclopenta [c] quinolin- 4- yl) benzoic acid;
- 4- ((3aS, 4R, 9bR)- 8- [(N- methyl- N- methylsulfonyl) amino]- 2, 3, 3a, 4, 5, 9b- hexahydro- 1H- cyclopenta [c] quinolin- 4- yl) benzoic acid;
- 3- ((3aS, 4R, 9bR)- 8- bromo- 2, 3, 3a, 4, 5, 9b- hexahydro- 1H- cyclopenta [c] quinolin- 4- yl) benzoic acid;
- 4- ((3aS, 4R, 9bR)- 2, 3, 3a, 4, 5, 9b- hexahydro- 7, 8- dimethoxy- 1H- cyclopenta [c] quinolin- 4- yl) benzoic acid;
- 4- ((3aS, 4R, 9bR)- 7- (trifluoromethyl)- 2, 3, 3a, 4, 5, 9b- hexahydro- 8- methyl- 1H- cyclopenta [c] quinolin- 4- yl) benzoic acid;
- 4- ((3aS, 4R, 9bR)- 9- (trifluoromethyl)- 2, 3, 3a, 4, 5, 9b- hexahydro- 8- methyl- 1H- cyclopenta [c] quinolin- 4- yl) benzoic acid;
- 4- ((3aS, 4R, 9bR)- 2, 3, 3a, 4, 5, 9b- hexahydro- 7- methoxy- 8- methyl- 1H- cyclopenta [c] quinolin- 4- yl) benzoic acid;
- 4- ((3aS, 4R, 9bR)- 2, 3, 3a, 4, 5, 9b- hexahydro- 6, 8- dimethyl- 1H- cyclopenta [c] quinolin- 4- yl) benzoic acid;
- 4- ((3aS, 4R, 9bR)- 9- chloro- 2, 3, 3a, 4, 5, 9b- hexahydro- 8- methyl- 1H- cyclopenta [c] quinolin- 4- yl) benzoic acid;
- 4- ((3aS, 4R, 9bR)- 7- chloro- 2, 3, 3a, 4, 5, 9b- hexahydro- 8- methyl- 1H- cyclopenta [c] quinolin- 4- yl) benzoic acid;
- 4- ((3aS, 4R, 9bR)- 2, 3, 3a, 4, 5, 9b- hexahydro- 5, 8- dimethyl- 1H- cyclopenta [c] quinolin- 4- yl) benzoic acid;
- 4- ((3aS, 4R, 9bR)- 5- acetyl- 2, 3, 3a, 4, 5, 9b- hexahydro- 8- methyl- 1H- cyclopenta [c] quinolin- 4- yl) benzoic acid;
- 4- ((6R, 6aS, 9aR)- 6, 6a, 7, 8, 9, 9a- hexahydro- 2- methyl- 5H- cyclopenta [c] [1, 5] naphthyridin- 6- yl) benzoic acid;
- 4- ((6R, 6aS, 9aR)- 6, 6a, 7, 8, 9, 9a- hexahydro- 2- methyl- 5H- cyclopenta [c] [1, 8] naphthyridin- 6- yl) benzoic acid;

● 2- (4- ((3aS, 4R, 9bR)- 2, 3, 3a, 4, 5, 9b- hexahydro- 8- methyl- 1H- cyclopenta [c] quinolin- 4- yl) phenyl) acetic acid;

● 3- (4- ((3aS, 4R, 9bR)- 2, 3, 3a, 4, 5, 9b- hexahydro- 8- methyl- 1H- cyclopenta [c] quinolin- 4- yl) phenyl) propanoic acid;

● 4- ((3aS, 4R, 9bR)- 2, 3, 3a, 4, 5, 9b- hexahydro- 8- methyl- 1H- cyclopenta [c] quinolin- 4- yl)- 2- methylbenzoic acid;

● 4- ((3aS, 4R, 9bR)- 2, 3, 3a, 4, 5, 9b- hexahydro- 8- methyl- 1H- cyclopenta [c] quinolin- 4- yl)- 3- methylbenzoic acid;

● (+)- 4- ((3aS, 4R, 9bR)- 2, 3, 3a, 4, 5, 9b- hexahydro- 8- methyl- 1H- cyclopenta [c] quinolin- 4- yl)- 3- methylbenzoic acid;

● (- )- 4- ((3aS, 4R, 9bR)- 2, 3, 3a, 4, 5, 9b- hexahydro- 8- methyl- 1H- cyclopenta [c] quinolin- 4- yl)- 3- methylbenzoic acid;

● 4- ((3aS, 4S, 9bR)- 2, 3, 3a, 4, 5, 9b- hexahydro- 8- methyl- 1H- cyclopenta [c] quinolin- 4- yl)- 3- methylbenzoic acid;

● 4- ((3aS, 4R, 9bR)- 8- chloro- 2, 3, 3a, 4, 5, 9b- hexahydro- 1H- cyclopenta [c] quinolin- 4- yl)- 3- methylbenzoic acid;

● 4- ((3aS, 4R, 9bR)- 8- fluoro- 2, 3, 3a, 4, 5, 9b- hexahydro- 1H- cyclopenta [c] quinolin- 4- yl)- 3- methylbenzoic acid;

● 4- ((3aS, 4R, 9bR)- 2, 3, 3a, 4, 5, 9b- hexahydro- 8- methoxy- 1H- cyclopenta [c] quinolin- 4- yl)- 3- methylbenzoic acid;

● 4- ((3aS, 4R, 9bR)- 2, 3, 3a, 4, 5, 9b- hexahydro- 8- methoxy- 5- methyl- 1H- cyclopenta [c] quinolin- 4- yl)- 3- methylbenzoic acid;

● 4- ((3aS, 4R, 9bR)- 8- cyclopropyl- 2, 3, 3a, 4, 5, 9b- hexahydro- 1H- cyclopenta [c] quinolin- 4- yl)- 3- methylbenzoic acid;

● 4- ((3aS, 4R, 9bR)- 8- (cyclopropylmethoxy)- 2, 3, 3a, 4, 5, 9b- hexahydro- 1H- cyclopenta [c] quinolin- 4- yl)- 3- methylbenzoic acid;

● 4- ((3aS, 4R, 9bR)- 2, 3, 3a, 4, 5, 9b- hexahydro- 8- isopropoxy- 1H- cyclopenta [c] quinolin- 4- yl)- 3- methylbenzoic acid;

● 4- ((3aS, 4S, 9bR)- 2, 3, 3a, 4, 5, 9b- hexahydro- 8- isopropoxy- 1 H- cyclopenta [c] quinolin- 4- yl)- 3- methylbenzoic acid;

● 3- chloro- 4- ((3aS, 4R, 9bR)- 2, 3, 3a, 4, 5, 9b- hexahydro- 8- methyl- 1H- cyclopenta [c] quinolin- 4- yl) benzoic acid;

● 4- ((3aS, 4R, 9bR)- 2, 3, 3a, 4, 5, 9b- hexahydro- 8- methoxy- 1H- cyclopenta [c] quinolin- 4- yl)- 3- methoxybenzoic acid;

● 4- ((3aS, 4R, 9bR)- 2, 3, 3a, 4, 5, 9b- hexahydro- 8- methyl- 1H- cyclopenta [c] quinolin- 4- yl)- 3- methoxybenzoic acid;

● 3- bromo- 4- ((3aS, 4R, 9bR)- 2, 3, 3a, 4, 5, 9b- hexahydro- 8- methyl- 1H- cyclopenta [c] quinolin- 4- yl) benzoic acid;

● 3- bromo- 4- ((3aS, 4R, 9bR)- 2, 3, 3a, 4, 5, 9b- hexahydro- 8- methoxy- 1H- cyclopenta [c] quinolin- 4- yl) benzoic acid;

● 3- ethyl- 4- ((3aS, 4R, 9bR)- 2, 3, 3a, 4, 5, 9b- hexahydro- 8- methyl- 1H- cyclopenta [c] quinolin- 4- yl) benzonitrile;

● 3- ethyl- 4- ((3aS, 4R, 9bR)- 2, 3, 3a, 4, 5, 9b- hexahydro- 8- methyl- 1H- cyclopenta [c] quinolin- 4- yl) benzoic acid;

● 3- ethyl- 4- ((3aS, 4R, 9bR)- 2, 3, 3a, 4, 5, 9b- hexahydro- 8- methoxy- 1H- cyclopenta [c] quinolin- 4- yl) benzoic acid;

- 3- ethyl- 4- ((3aS, 4S, 9bR)- 2, 3, 3a, 4, 5, 9b- hexahydro- 8- methoxy- 1H- cyclopenta [c] quinolin- 4- yl) benzoic acid;

- 4- ((3aS, 4S, 9bR)- 2, 3, 3a, 4, 5, 9b- hexahydro- 8- methyl- 1H- cyclopenta [c] quinolin- 4- yl)- 3, 5- dimethylbenzoic acid;

- 4- ((3aS, 4S, 9bR)- 2, 3, 3a, 4, 5, 9b- hexahydro- 8- methoxy- 1H- cyclopenta [c] quinolin- 4- yl)- 3, 5- dimethylbenzoic acid;

- 5- ((3aS, 4R, 9bR)- 2, 3, 3a, 4, 5, 9b- hexahydro- 8- methyl- 1H- cyclopenta [c] quinolin- 4- yl) pyridine- 2- carboxylic acid;

- 6- ((3aS, 4R, 9bR)- 2, 3, 3a, 4, 5, 9b- hexahydro- 8- methyl- 1H- cyclopenta [c] quinolin- 4- yl) pyridine- 3- carboxylic acid;

- 5- ((3aS, 4R, 9bR)- 2, 3, 3a, 4, 5, 9b- hexahydro- 8- methyl- 1H- cyclopenta [c] quinolin- 4- yl)- 6- methylpyridine- 2- carbonitrile;

- 5- ((3aS, 4R, 9bR)- 2, 3, 3a, 4, 5, 9b- hexahydro- 8- methyl- 1H- cyclopenta [c] quinolin- 4- yl)- 6- methylpyridine- 2- carboxylic acid;

- 5- ((3aS, 4R, 9bR)- 2, 3, 3a, 4, 5, 9b- hexahydro- 8- methyl- 1H- cyclopenta [c] quinolin- 4- yl)- 1- methyl- 1H- pyrazole- 3- carboxylic acid;

- 5- ((3aS, 4R, 9bR)- 2, 3, 3a, 4, 5, 9b- hexahydro- 8- methoxy- 1H- cyclopenta [c] quinolin- 4- yl)- 1- methyl- 1H- pyrazole- 3- carboxylic acid;

- 3- ((3aS, 4R, 9bR)- 2, 3, 3a, 4, 5, 9b- hexahydro- 8- methyl- 1H- cyclopenta [c] quinolin- 4- yl)- 1- methyl- 1H- pyrazole- 5- carboxylic acid;

- 3- ((3aS, 4R, 9bR)- 2, 3, 3a, 4, 5, 9b- hexahydro- 8- methoxy- 1H- cyclopenta [c] quinolin- 4- yl)- 1- methyl- 1H- pyrazole- 5- carboxylic acid;

- 4- ((3aS, 4S, 9bR)- 2, 3, 3a, 4, 5, 9b- hexahydro- 8, 9b- dimethyl- 1H- cyclopenta [c] quinolin- 4- yl) benzoic acid;

- 4- ((3aS, 4R, 9bR)- 2, 3, 3a, 4, 5, 9b- hexahydro- 8, 9b- dimethyl- 1H- cyclopenta [c] quinolin- 4- yl) benzoic acid;

- 4- ((3aS, 4S, 9bR)- 2, 3, 3a, 4, 5, 9b- hexahydro- 8- methoxy- 9b- methyl- 1H- cyclopenta [c] quinolin- 4- yl) benzoic acid;

- 4- ((3aS, 4R, 9bR)- 2, 3, 3a, 4, 5, 9b- hexahydro- 8- methoxy- 9b- methyl- 1H- cyclopenta [c] quinolin- 4- yl) benzoic acid;

- 4- ((3aS, 4S, 9bR)- 8- cyclopropyl- 2, 3, 3a, 4, 5, 9b- hexahydro- 9b- methyl- 1H- cyclopenta [c] quinolin- 4- yl) benzoic acid;

- 4- ((3aS, 4R, 9bR)- 8- cyclopropyl- 2, 3, 3a, 4, 5, 9b- hexahydro- 9b- methyl- 1H- cyclopenta [c] quinolin- 4- yl) benzoic acid;

- 4- ((3aS, 4S, 9bR)- 8- chloro- 2, 3, 3a, 4, 5, 9b- hexahydro- 9b- methyl- 1H- cyclopenta [c] quinolin- 4- yl) benzoic acid;

- 4- ((3aS, 4R, 9bR)- 8- chloro- 2, 3, 3a, 4, 5, 9b- hexahydro- 9b- methyl- 1H- cyclopenta [c] quinolin- 4- yl) benzoic acid;

- 4- ((3aS, 4S, 9bR)- 2, 3, 3a, 4, 5, 9b- hexahydro- 8, 9b- dimethyl- 1H- cyclopenta [c] quinolin- 4- yl)- 3- methylbenzoic acid;

- 4- ((3aS, 4R, 9bR)- 2, 3, 3a, 4, 5, 9b- hexahydro- 8, 9b- dimethyl- 1H- cyclopenta [c] quinolin- 4- yl)- 3- methylbenzoic

acid;

- 4- ((3aS, 4R, 9bR)- 2, 3, 3a, 4, 5, 9b- hexahydro- 8- isobutoxy- 1H- cyclopenta [c] quinolin- 4- yl)- 3- methylbenzoic acid;

- 4- ((3aS, 4S, 9bR)- 2, 3, 3a, 4, 5, 9b- hexahydro- 8- isobutoxy- 1H- cyclopenta [c] quinolin- 4- yl)- 3- methylbenzoic acid;

- 4- ((3aS, 4S, 9bR)- 2, 3, 3a, 4, 5, 9b- hexahydro- 8- methoxy- 5, 9b- dimethyl- 1H- cyclopenta [c] quinolin- 4- yl) benzoic acid;

- 4- ((3aS, 4R, 9bR)- 2, 3, 3a, 4, 5, 9b- hexahydro- 8- methoxy- 5, 9b- dimethyl- 1H- cyclopenta [c] quinolin- 4- yl) benzoic acid;

In another embodiment, the sodium salt of the previous compounds is preferred.

In another aspect the invention refers to a process for preparing the compounds of the invention.

The compounds of the invention may be made by a variety of methods, including standard chemistry. Any previously defined variable will continue to have the previously defined meaning unless otherwise indicated. Illustrative general synthetic methods are set out below and then the preparation of specific compounds of the invention is described in more detail in the Experimental Section.

Compounds of general formula (I) may be obtained following the synthetic scheme depicted in scheme 1.

## Scheme 1

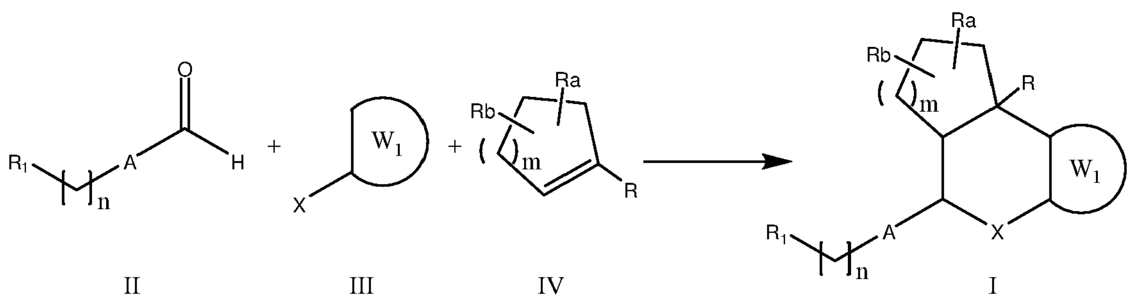

II III IV I

The reaction of an aldehyde of general formula II with an amine of general formula III in the presence of an alkene of general formula IV under acidic catalysis yield compounds of general formula I. Several Lewis acids such as scandium triflate ytterbium triflate, dysprosium triflate, copper (II) triflate, tin triflate, zinc triflate, zirconium triflate, borontrifluoroetherate, indium chloride, iron (III) chloride, ytterbium chloride, aluminium chloride, lanthanum (III) chloride, gadolinium (III) chloride, Samarium (III) chloride, titanium (II) chloride, titanium (IV) chloride, or organic acids such as trifluoroacetic acid trifluoromethane sulphonic acid, octane sulphonic acid, mixture of acetic acid with sulphuric acid can be used to induce this Povarov reaction. The reaction is carried out preferably in the presence of an organic solvent such as acetonitrile, dichloromethane, 1, 2- dichloroethane, toluene, chloroform, hexane, tetrahydrofurane, diethyl ether at a temperature between room temperature and the solvent boiling point.

Alternatively, the reaction can be performed in two steps as shown below in scheme 2.

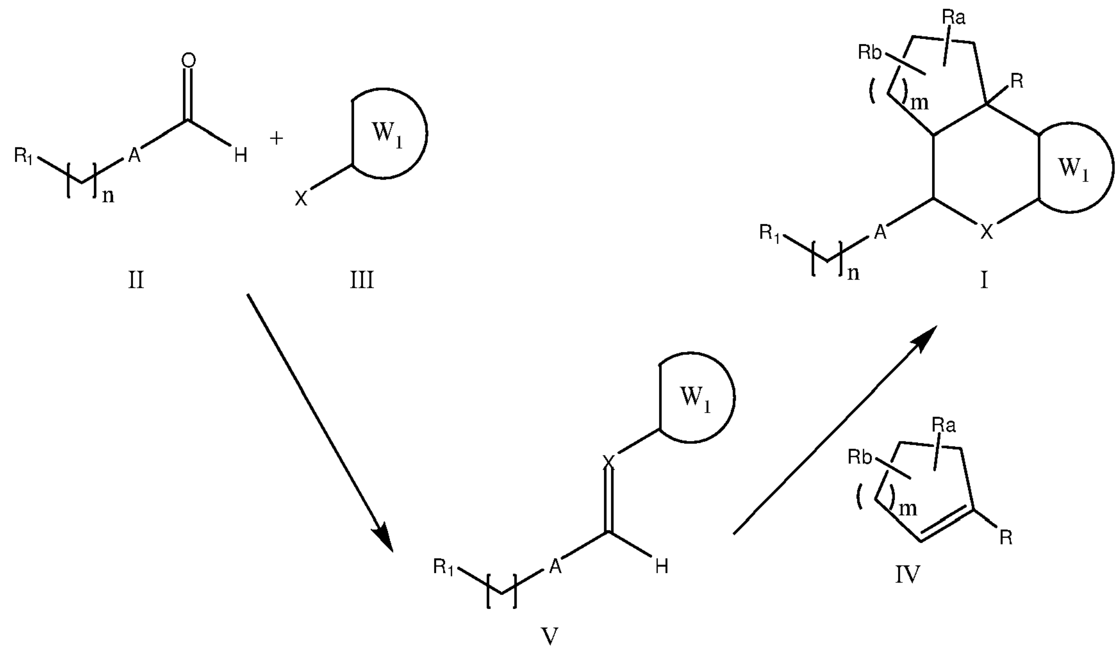

II III I IV V

In an initial step, reaction of aldehydes of formula II with amines of formula III in the presence of a dehydrating agent such as molecular sieves trimethyl orthoformate, acetic acid, titanium (IV) chloride, anhydrous magnesium sulphate or dehydration by Dean Stark system using p-toluenesulfonic acid in a solvent such as chloroform dichloromethane, toluene, methanol at a temperature between 20 and 150ºC to yield the corresponding imines V that upon isolation are allowed to further react with the corresponding alkenes of general formula IV in the presence of the same acids, solvents and conditions depicted above.

The reactions of scheme 1 and 2 are only applicable to the preparation of compounds of general formula I where X is $NR_7$.

Compounds of general formula II, III and IV are either commercially available or are synthesized according to methods known in the art.

In the particular case in which X is $NR_7$ with $R_7$ being $C_{1-6}$- alkyl, compounds of general formula (Id) can be obtained from compounds of general formula (Ic) by reaction with an alkylating agent such as VI wherein Y is an halogen atom such as chlorine, bromine or iodide or a sulphonate ester such as mesilate, tosilate or trifluoromethanesulphonate in basic media such as sodium hydride and in a solvent such as THF or DMF at a temperature from 0 to 150ºC (see scheme 3) .

Alternativelly, compounds of general formula (Id) can be obtained by reductive amination of aldehydes of general formual VII with amines of general formula (Ic) in acidic media such as acetic acid and in a protic solvent such as methanol or ethanol and with a reductive agent such as sodium borohydride, sodium triacetoxyborohydride or sodium cyanoborohydride at a temperature from 0ºC to the boiling point of the solvent. As an alternative to the acidic media a Lewis acid such as zinc chloride can be used.

## Scheme 3

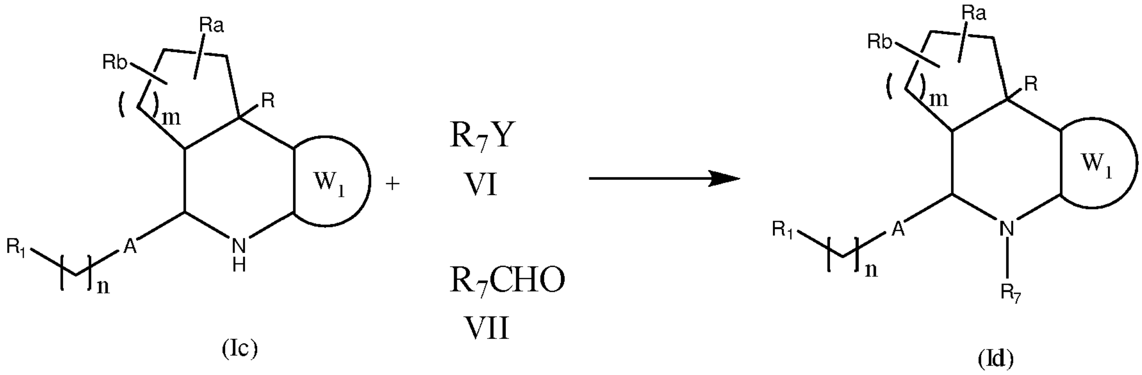

(Ic) VI VII (Id)

In the particular case in which X is $NR_7$ with $R_7$ being a $-COR_8$, compounds of general formual (Ie) (see scheme 4 below), can be obtained from compounds of general formula (Ic) by reaction of an acylating agent such as VIII in which R8 is a $C_{1-6}$-alkyl and Y is an halogen. The reaction takes place in basic media such as triethylamine, pyridine or diisopropylethylamine with a solvent such as dichloromethane, DMF, THF or pyridine at a temperature between 20 and 150ºC and in a standard reactor or in a microwave apparatus.

## Scheme 4

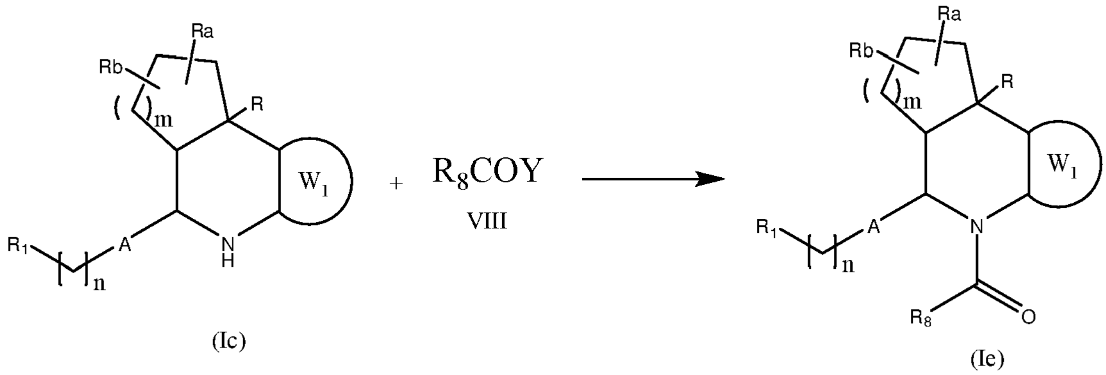


(Ic) VIII (Ie)

In the particular case in which m is 1, compounds of general formula (Ia) can be obtained as depicted in scheme 5. Aldehydes of general formula II react with amines of general formula III in the presence of cyclopentadiene under the conditions described above to yield compounds of general formula IX. Reaction of compounds of general formula IX under hydrogen using a catalyst such as Pd/C, Pd(OH)2/C, PtO2 in solvents such as methanol, ethanol, tetrahydrofurane, at atmospheric pressure or under hydrogen pressure at rt or higher yield the compounds of general formula (Ia).

## Scheme 5

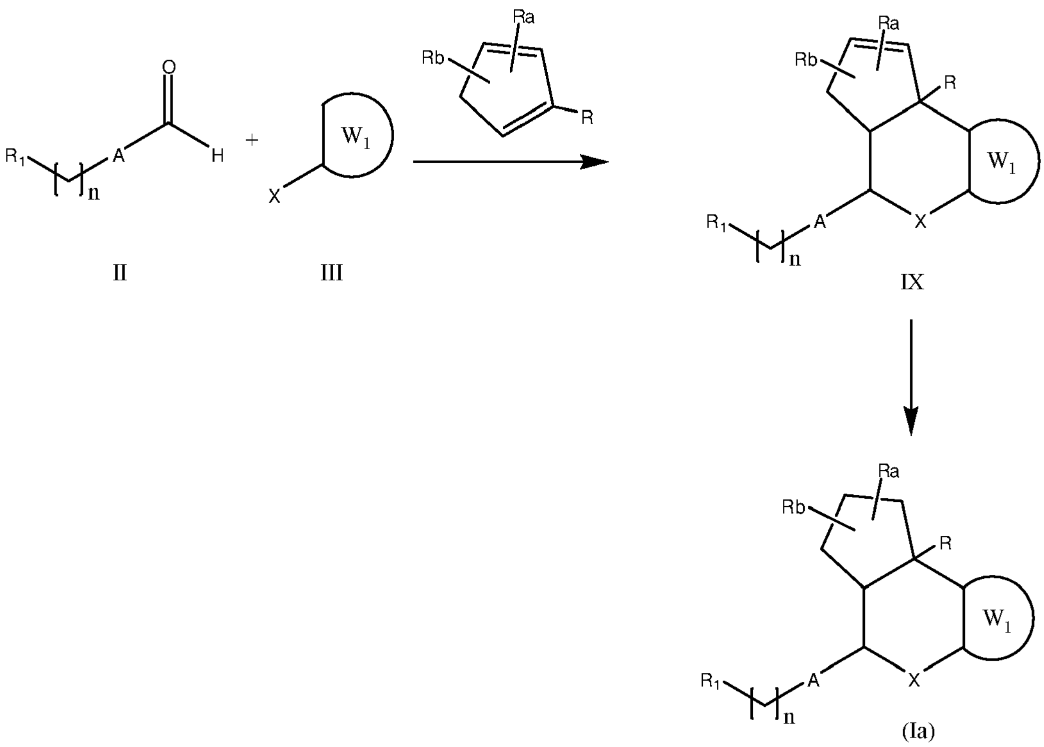

II III IX (Ia)

When the defined groups $R_1$ to $R_8$ are susceptible to chemical reaction under the conditions of the hereinbefore described processes or are incompatible with said processes, conventional protecting groups may be used in accordance with standard practice, for example see T.W. Greene and P.G.M. Wuts in "protective Groups in Organic Chemistry", 3rd Edition, John Wiley&Sons (1999). It may be that deprotection will form the last step in the synthesis of compounds of

formula (I).

An additional aspect of the invention relates to the therapeutic use of the compounds of general formula (I). As mentioned above, compounds of general formula (I) show a strong affinity to EP1 receptors. For this reason, they are suitable for the treatment and/or the prophylaxis of disorders and diseases mediated by EP1 receptors.

Compounds of the invention are particularly useful for modulating pain. The compounds of the present invention can treat or prevent the pain associated with several pathological conditions comprising, among others, inflammatory related pain (Hall et al. 2007) including low back and neck pain, skeletal pain, post- partum pain, toothache, sprains and straits, myositis, neuralgia, synovitis, arthritis, including rheumatoid arthritis, degenerative joint diseases (osteoarthritis), gout and ankylosing spondylitis, bursitis, burns including radiation and corrosive chemical injuries and sunburns; postoperative pain (Omote et al. 2001) including dental procedures; neuropathic pain (Kawahara et al. 2001) ; visceral pain (Sarkar et al. 2003) ; tension headache; cluster headaches; migraine and the like.

Moreover, by inhibition of prostanoid- induced smooth muscle contraction by antagonizing contractile prostanoids or mimicking relaxing prostanoids, EP1 modulators may be used in the treatment of motility- related disorders (with or without pain) such as gastroinstestinal disorders (Sarkar et al. 2003; Mizuguchi et al 2010) and urinary incontinence and other urinary tract diseases (Teramura et al. 2000; Lee et al. 2007; Okada et al., 2010; Wilbraham et al 2010; Miki et al 2010), dysmenorrhea and preterm labour.

The compounds of the invention can also be useful in prostaglandin-mediated proliferation disorders such as in diabetic retinopathy and tumour angiogenesis, cancer (Watanabe et al. 1999; Niho et al. 2005), the inhibition of cellular neoplasic transformations and metastatic tumour growth.

They can further be used in the treatment of neurodegenerative diseases (including senile dementia, Alzheimer's disease, Pick's disease, Huntington's chorea, Parkinson's disease, Creutzfeldt-Jakob disease, or Amyotrophic Lateral Sclerosis) (Li et al. 2011), neuroprotection/stroke (Abe et al 2009), glaucoma (Woodward et al 1997), bone loss (osteoporosis) and the proportion of bone formation (treatment of fractures) (Zhang et al 2011; Lee et al. 2007) and other bone diseases such as Paget's disease.

As PGE2-induced hyperthermia in the rat is mediated predominantly through the EP1 receptor (Hönemann et al. 2001; Oka et al. 2003) different kinds of fever as rheumatic fever, symptoms associated with influenza or other viral infections as well as common cold can be also target diseases for EP1 modulators.

The compounds of the invention can also have a cytoprotective activity in patients under different gastrointestinal disorders as related with chemotherapy, or irritable bowel disease. Other diseases that can be treated or prevented with the compounds of the invention include gastrointestinal bleeding, coagulation disorders including anaemia such as hypoprothrombinemia, haemophilia or other bleeding problems; kidney diseases (nephritis (Rahal et al. 2006), particularly mesangial proliferative glomerulonephritis and nephritic syndrome); thrombosis, and occlusive vascular diseases.

In this sense, compounds of formula (I) are suitable to treat or to prevent diseases or disorders comprising inflammatory related pain including low back and neck pain, skeletal pain, post-partum pain, toothache, sprains and straits, myositis, neuralgia, synovitis, arthritis, including rheumatoid arthritis, degenerative joint diseases (such as osteoarthritis), gout and ankylosing spondylitis, bursitis, burns including radiation and corrosive chemical injuries and sunburns; postoperative pain; neuropathic pain; visceral pain; tension headache; cluster headaches; migraine; motility-related disorders including gastrointestinal disorders, urinary incontinence and other urinary tract diseases; dysmenorrhea; preterm labour; diabetic retinopathy; tumour angiogenesis; cancer; metastatic tumour growth; neurodegenerative diseases including senile dementia, Alzheimer's disease, Pick's disease, Huntington's chorea, Parkinson's disease, Creutzfeldt-Jakob disease, or amyotrophic lateral sclerosis; neuroprotection/stroke; glaucoma; osteoporosis; bone fractures; Paget's disease; hyperthermia including different types of fever as rheumatic fever; symptoms associated with influenza or other viral infections; common cold, gastrointestinal disorders related with chemotherapy or irritable bowel syndrome; gastrointestinal bleeding; coagulation disorders including anaemia, hypoprothrombinemia, haemophilia or other bleeding problems; kidney diseases including nephritis, particularly mesangial proliferative glomerulonephritis and nephritic syndrome; thrombosis and occlusive vascular diseases.

The invention thus relates to a compound of formula (I) for use in the treatment and/or prophylaxis of an EP1-mediated disease or disorder. In one embodiment, the EP1-mediated disease or disorder is selected from the group consisting of pain, motility-related disorders, gastrointestinal disorders, urinary tract diseases, cancer, neurodegenerative diseases, stroke, glaucoma, bone diseases, fever, coagulation disorders and occlusive vascular diseases. In a preferred embodiment, the EP1-mediated disease or disorder is pain. In another embodiment, the EP1-mediated disease or disorder is selected from the group consisting of inflammatory related pain including low back and neck pain, skeletal pain, post-partum pain, toothache, sprains and straits, myositis, neuralgia, synovitis, arthritis, including rheumatoid arthritis, degenerative joint diseases (such as osteoarthritis), gout and ankylosing spondylitis, bursitis, burns including radiation and corrosive chemical injuries and sunburns; postoperative pain; neuropathic pain; visceral pain; tension headache; cluster headaches; migraine; motility-related disorders including gastrointestinal disorders, urinary incontinence and other urinary tract diseases; dysmenorrhea; preterm labour; diabetic retinopathy; tumour angiogenesis; cancer; metastatic tumour growth; neurodegenerative diseases including senile dementia, Alzheimer's disease, Pick's disease, Huntington's chorea, Parkinson's disease, Creutzfeldt-Jakob disease, or amyotrophic lateral sclerosis; neuroprotection/stroke; glaucoma; osteoporosis; bone fractures; Paget's disease; hyperthermia including different types of fever as rheumatic fever; symptoms associated with influenza or other viral infections; common cold, gastrointestinal disorders related with chemotherapy or irritable bowel syndrome; gastrointestinal bleeding; coagulation disorders including anaemia, hypoprothrombinemia, haemophilia or other bleeding problems; kidney diseases including nephritis, particularly mesangial proliferative glomerulonephritis and nephritic syndrome; thrombosis and occlusive vascular diseases. In a preferred embodiment, the EP1-mediated disease or disorder is pain comprising inflammatory related pain, including low back and neck pain, skeletal pain, post-partum pain, toothache, sprains and straits, myositis, neuralgia, synovitis, arthritis, including rheumatoid arthritis, degenerative joint diseases (such as osteoarthritis), gout and ankylosing spondylitis, bursitis, burns including radiation and corrosive chemical injuries and sunburns; postoperative pain; neuropathic pain; visceral pain; tension headache; cluster headaches; migraine.

A related aspect refers to the use of at least one compound of general formula (I) for the manufacture of a medicament for the treatment and/or prophylaxis diseases or disorders mediated by EP1 receptors or in which EP1 receptors are involved.

In one embodiment, the EP1-mediated disease or disorder is selected from the group consisting of pain, motility-related disorders, gastrointestinal disorders, urinary tract diseases, cancer, neurodegenerative diseases, stroke, glaucoma, bone diseases, fever, coagulation disorders and occlusive vascular diseases. In a preferred embodiment, the EP1-mediated disease or disorder is pain. In another embodiment, the EP1-mediated disease or disorder is selected from the group consisting of inflammatory related pain including low back and neck pain, skeletal pain, post-partum pain, toothache, sprains and straits, myositis, neuralgia, synovitis, arthritis, including rheumatoid arthritis, degenerative joint diseases (such as osteoarthritis), gout and ankylosing spondylitis, bursitis, burns including radiation and corrosive chemical injuries and sunburns; postoperative pain; neuropathic pain; visceral pain; tension headache; cluster headaches; migraine; motility-related disorders including gastrointestinal disorders, urinary incontinence and other urinary tract diseases; dysmenorrhea; preterm labour; diabetic retinopathy; tumour angiogenesis; cancer; metastatic tumour growth; neurodegenerative diseases including senile dementia, Alzheimer's disease, Pick's disease, Huntington's chorea, Parkinson's disease, Creutzfeldt-Jakob disease, or amyotrophic lateral sclerosis; neuroprotection/stroke; glaucoma; osteoporosis; bone fractures; Paget's disease; hypertermia including different types of fever as rheumatic fever; symptoms associated with influenza or other viral infections; common cold, gastrointestinal disorders related with chemotherapy or irritable bowel syndrome; gastrointestinal bleeding; coagulation disorders including anaemia, hypoprothrombinemia, haemophilia or other bleeding problems; kidney diseases including nephritis, particularly mesangial proliferative glomerulonephritis and nephritic syndrome; thrombosis and occlusive vascular diseases. In another embodiment, the EP1-mediated disease or disorder is selected from the group consisting of inflammatory related pain (including low back and neck pain, skeletal pain, post-partum pain, toothache, sprains and straits, myositis, neuralgia, synovitis, arthritis, including rheumatoid arthritis, degenerative joint diseases (such as osteoarthritis), gout and ankylosing spondylitis, bursitis, burns including radiation and corrosive chemical injuries and sunburns); postoperative pain; neuropathic pain; visceral pain; tension headache; cluster headaches; and migraine.

An aspect of the invention related to the therapeutic use of the compounds of general formula (I) is a method of treatment and/or prophylaxis of disorders and diseases mediated by EP1 receptors which comprises administering to a patient in need thereof a therapeutically effective amount of at least one compound of general formula (I). In one embodiment, the EP1-mediated disease or disorder is selected from the group consisting of pain, motility-related disorders, gastrointestinal disorders, urinary tract diseases, cancer, neurodegenerative diseases, stroke, glaucoma, bone diseases, fever, coagulation disorders and occlusive vascular diseases. In a preferred embodiment, the EP1-mediated disease or disorder is pain. In another embodiment, the EP1-mediated disease or disorder is selected from the group consisting of inflammatory related pain including low back and neck pain, skeletal pain, post-partum pain, toothache, sprains and straits, myositis, neuralgia, synovitis, arthritis, including rheumatoid arthritis, degenerative joint diseases (such as osteoarthritis), gout and ankylosing spondylitis, bursitis, burns including radiation and corrosive chemical injuries and sunburns; postoperative pain; neuropathic pain; visceral pain; tension headache; cluster headaches; migraine; motility-related disorders including gastrointestinal disorders, urinary incontinence and other urinary tract diseases; dysmenorrhea; preterm labour; diabetic retinopathy; tumour angiogenesis; cancer; metastatic tumour growth; neurodegenerative diseases including senile dementia, Alzheimer's disease, Pick's disease, Huntington's chorea, Parkinson's disease, Creutzfeldt-Jakob disease, or amyotrophic lateral sclerosis; neuroprotection/stroke; glaucoma; osteoporosis; bone fractures; Paget's disease; hyperthermia including different types of fever as rheumatic fever; symptoms associated with influenza or other viral infections; common cold, gastrointestinal disorders related with chemotherapy or irritable bowel syndrome; gastrointestinal bleeding; coagulation disorders including anaemia, hypoprothrombinemia, haemophilia or other bleeding problems; kidney diseases including nephritis, particularly mesangial proliferative glomerulonephritis and nephritic syndrome; thrombosis and occlusive vascular diseases. In another embodiment, the EP1-mediated disease or disorder is selected from the group consisting of inflammatory related pain (including low back and neck pain, skeletal pain, post-partum pain, toothache, sprains and straits, myositis, neuralgia, synovitis, arthritis, including rheumatoid

arthritis, degenerative joint diseases (such as osteoarthritis), gout and ankylosing spondylitis, bursitis, burns including radiation and corrosive chemical injuries and sunburns); postoperative pain; neuropathic pain; visceral pain; tension headache; cluster headaches; and migraine.

The amount of active ingredient that must be administered to the patient depends on the patient's weight, the type of application, the condition and severity of the disease. Normally, in human beings 1 to 1500 mg of the active compound is administered daily in one or several doses.

A further aspect of the invention regards a pharmaceutical composition which comprises a compound of general formula (I), and at least a pharmaceutically acceptable carrier, additive, adjuvant or vehicle.

The auxiliary materials or additives can be selected among carriers, excipients, support materials, lubricants, fillers, solvents, diluents, colorants, flavour conditioners such as sugars, antioxidants and/or agglutinants. In the case of suppositories, this may imply waxes or fatty acid esters or preservatives, emulsifiers and/or carriers for parenteral application. The selection of these auxiliary materials and/or additives and the amounts to be used will depend on the form of application of the pharmaceutical composition.

The pharmaceutical composition in accordance with the invention can be adapted to any form of administration, be it orally or parenterally, for example pulmonarily, nasally, rectally and/or intravenously. Therefore, the formulation in accordance with the invention may be adapted for topical or systemic application, particularly for dermal, subcutaneous, intramuscular, intra-articular, intraperitoneal, pulmonary, buccal, sublingual, nasal, percutaneous, vaginal, oral or parenteral application.

Suitable preparations for oral applications are tablets, pills, chewing gums, capsules, granules, drops or syrups. Suitable preparations for parenteral applications are solutions, suspensions, reconstitutable dry preparations or sprays.

The compounds of the invention are formulated as deposits in dissolved form or in patches, for percutaneous application.

Skin applications include ointments, gels, creams, lotions, suspensions or emulsions.

The preferred form of rectal application is by means of suppositories.

## EXPERIMENTAL SECTION

The following abbreviations are used along the experimental section:

DMF: dimethylformamide
BuLi: butyl lithium
EtAcO: ethyl acetate
EtOH: ethanol
DCM: dichloromethane
NMR: nuclear magnetic resonance
MeOH: methanol
NBS: N-bromosuccinimide
THF: tetrahydrofurane
$Et_2O$: diethyl ether
r.t.: room temperature
ACN: acetonitrile
TFA: trifluoroacetic acid
$^tBuONa$: sodium tert-butoxide
AIBN: 2, 2'- azobis (2- methylpropionitrile)
HCl: hydrochloride acid
NaOH: sodium hydroxide
LiOH: lithium hydroxide
$NH_4Cl$: ammonium chloride
$NaHCO_3$: sodium bicarbonate
$Na_2CO_3$: sodium carbonate
$Et_3N$: triethylamine
$MgSO_4$: magnesium sulphate
$AgNO_3$: silver nitrate
AgBr: silver bromide
$NaIO_4$: sodium periodate
$OsO_4$: osmium tetraoxide
IPA: Isopropanol
$^iPrMgCl$: isopropylmagnesium chloride

$^{i}PrMgCl.LiCl$: isopropylmagnesium chloride lithium chloride
$t_R$: retention time (min)
$POCl_3$: phosphorus oxychloride
$BF_3.OEt_2$: boron trifluoride diethyletherate
$Pd_2(dba)_3$: tris(dibenzylidineacetone)dipalladium
(+)- BINAP: (R)- (+)- 2, 2'- bis (diphenylphosphino)- 1, 1'- binaphthyl
$NiCl_2.6H_2O$: nickel (II) chloride hexahydrated.
$HNO_3$: nitric acid
$SnCl_2.2H_2O$: stannous chloride dihydrate
$NaBH_4$: sodium borohydride
$NaCNBH_3$: sodium cyanoborohydride
$Pt_2O$: platinum (iv) oxide

The following HPLC methods for LC-MS spectra have been used:

<u>Method 1:</u> X-Bridge C18, 3.5 μm 4.6 x 50 mm column; temperature: 25ºC; rate 2.0 mL/min; eluent: A = HCOOH 1 mM, B = ACN:MeOH:HCOOH (1:1:0.0005); gradient: 100% to 5% A in 12 min, 5% A 1 min, 5 to 100% A1 min.

<u>Method 2:</u> X-Bridge C18, 3.5 μm 4.6 x 50 mm column; temperature: 25ºC; rate 2.0 mL/min; eluent: A = HCOOH 1 mM, B = ACN:MeOH:HCOOH (1:1:0.0005); gradient: 95 to 0% A in 5.5 min, 0% A 3 min, 0 to 95% A 0.5 min.

<u>Method 3:</u> X-Bridge C18, 3.5 μm 4.6 x 50 mm column; temperature: 25ºC; rate 2.0 mL/min; eluent: A = HCOOH 1 mM, B = ACN:MeOH:HCOOH (1:1:0.0005); gradient: 95 to 0% A in 4.5 min, 0% A 1.5 min, 0 to 95% A 0.5 min.

Separation of enantiomers was carried out in a Preparative Waters HPLC (pump Waters 600; Manual injector Waters 2700; Detector Waters 2487; Software: MassLynx 3.5). Using preparative packed column with Chiralpak IA (20 μm) using a self-packing system (195 mm x 50 mm). Flux 55 mL/min and fraction collection λ = 254 nm.
Optical purity of the separated enantiomers were determinated by HPLC (Chiralpak IA 250 x 4.6 mm, 5 μm, 10 μL, 1 mL/min). Using the same eluents of the preparative chiral chromatography.
Optical rotation of the separated enantiomers were determined in a polarimeter JASCO P-2000 with a cell of 1 mL.

**<u>Intermediate compounds</u>**

***Intermediate compound 1: 1-cyclopropyl-4-nitrobenzene***

Method 1: To a solution of 1- cyclopropylbenzene (8.46 mmol, 1g) dissolved in anhydride acetic acid (5 mL) and cooled to 0ºC was added $HNO_3$ (0.63 mL) and the resulting solution was stirred at room temperature 4 h. The reaction mixture was carefully poured in ice and the resulting solution extracted with diethyl ether. The combined extracts were evaporated. The crude product was purified by flash chromatography on silica gel using an elution of 8% dichloromethane in hexanes to afford a mixture of 1- cyclopropyl- 2- nitrobenzene and 1- cyclopropyl- 4- nitrobenzene (ratio 3: 1) . (430 mg. Yield: 31%) .
LC- MS: $t_R$ = 3.10 $[M+H]^+$= 164 (method 3)
Method 2: To a solution of 1- bromo- 4- nitrobenzene (4.95 mmol, 1 g), cyclopropylboronic acid (6.43 mmol, 553 mg), palladium acetate (0.198 mmol, 45 mg), tricyclohexyl phosphine (0.445 mmol, 125 mg) and potassium carbonate (16.4 mmol, 3.5 g) was dissolved in Toluene (20mL) and $H_2O$ (2 mL) under Argon. The resulting solution was heated for 1h at 80ºC. After the reaction mixture was cooled and concentrated *in vacuum.* The crude product was purified by flash chromatography on silica gel using an elution of 7% ethylacetate in hexanes to afford 1- cyclopropyl- 4- nitrobenzene (800 mg. Yield: 99%) .
$^1H$ NMR (400 MHz, $CDCl_3$) δ 8.1 (2H, dd, *J* = 2 & 6.8 Hz), 7.15 (2H, dd, *J* = 2 & 6.8 Hz), 1.99 (1 H, m), 1.14-1.11 (2H, m), 0.83- 080 (2H, m)

***Intermediate compound 2: 2-cyclopropylbenzenamine***

A mixture of 1- cyclopropyl- 2- nitrobenzene and 4- cyclopropylbenzenamine (**intermediate compound 1**, method 1) (2.63 mmol, 430 mg) in dry MeOH was hydrogenated over Pd/C (2.5% w/w) .The resulting solution was stirred 16 h at room temperature. The catalyst was removed by filtration and the solvent was evaporated. The crude product was purified by flash chromatography on silica gel using an elution of dichloromethane in hexanes to afford only 2-cyclopropylbenzamine (250 mg. Yield: 71 %)

$^1$H NMR (400 MHz, $CDCl_3$) δ 7.07- 7.03 (2H, m), 6.73- 6.67 (2H, m), 1.72- 1.65 (1 H, m), 0.93- 0.88 (2H, m), 0.63- 0.6 (2H, m)

LC- MS: $t_R$ = 1.28 $[M+H]^+$= 134 (method 3)

***Intermediate compound 3: 4-cyclopropylbenzenamine***

Method 1: A solution of 1- cyclopropyl- 4- nitrobenzene (**intermediate compound 1**, method 2) (1.32 mmol, 215 mg) in dry MeOH was hydrogenated over Pd/C (2.5% w/w) .The resulting solution was stirred 16 h at room temperature. The catalyst was removed by filtration and the solvent was evaporated to give a mixture of 4- cyclopropylbenzenamine and 4- propylbenzenamine (8: 2) (Yield: 90%) .

LC- MS: $t_R$ = 1.78 $[M+H]^+$= 134 (method 3)

2.47 $[M+H]^+$= 136 (method 3)

Method 2: A solution of 1- cyclopropyl- 4- nitrobenzene (intermediate compound 1, method 2) (1.06 mmol, 176 mg) in EtOH (6 ml) and THF (6 mL) was hydrogenated over $Pt_2O$ (5% mol) .The resulting solution was stirred 16h at room temperature. The catalyst was removed by filtration and the solvent was evaporated to give the compound 4-cyclopropylbenzenamine (127 mg. Yield: 89%) .

$^1$H NMR (400 MHz, $CDCl_3$) δ 6.89 (2H, dd, *J* = 2.0 & 6.4Hz), 6.61 (2H, dd, *J*= 2.0 & 6.4Hz), 1.81- 1.79 (1 H, m), 0.85- 0.83 (2H, m), 0.58- 0.56 (2H, m) .

LC- MS: $t_R$ = 1.78 $[M+H]^+$= 134 (method 3)

***Intermediate compound 4: 1-isopropoxy-4-nitrobenzene***

4- Nitrophenol (1.79 mmol, 250 mg), propan- 2- ol (2.67 mmol, 0.20 mL), triphenylphosphine (2.67 mmol, 702 mg) were combined in tetrahydrofuran (7.2 mL) and cooled to 0º C. Diisopropyl azodicarboxylate (2.67 mmol, 0.53 mL) was added dropwise. Once the addition was complete, the reaction was warmed to room temperature and stirred for 16 h. Reaction was concentrated and purified on silica gel, eluting with a gradient from 10% to 50% ethyl acetate in hexane to give 1- isopropoxy- 4- nitrobenzene as an oil (320 mg, 99%) .

$^1$H NMR (400 MHz, $CDCl_3$) δ 8.18 (2H, d, *J* = 9.2 Hz), 6.91 (1 H, d, *J* = 9.2 Hz), 4.69- 4.64 (1 H, m), 1.39 (6H, d, *J* = 6.4 Hz)

LC- MS: $t_R$ =3.20 $[M+H]^+$= 338 (method 3)

***Intermediate compound 5: 4-isopropoxybenzenamine***

To a solution of the **intermediate compound 4** (1.76 mmol, 320 mg) in absolute EtOH (8 mL), stannous chloride dehydrate (7.92 mmol, 1.78 g) was added and the reaction mixture was heated to reflux for 18 h. Reaction mixture was diluted with aqueous 1 N NaOH solution and ethyl acetate. The aqueous layer was extracted with EtAcO. The combined extracts were washed with brine, dried over anhydrous magnesium sulfate and concentrated in *vacuum* to give intermediate 5 (239 mg, 90%) with no further purification.

LC- MS: $t_R$ =1.12 $[M+H]^+$= 152 (method 3)

***Intermediate compound 6: 1-(cyclopropylmethoxy)-4-nitrobenzene***

4- nitrophenol (9.3 mmol, 1.3g) was dissolved in DMF (20 mL) . Potassium carbonate (3 eq., 28mmol, 3.8 g) and bromomethyl cyclopropane (1.1 eq., 10.23 mmol, 1mL) were added and the resulting mixture was stirred at room temperature for 60h. Then bromomethyl cyclopropane (1.1.eq.) and potassium carbonate (1eq.) were added and left at room temperature for 16h. The mixture was diluted with ethyl acetate and the organic layer washed by saturated sodium carbonate solution, brine, dried over anhydrous sodium sulfate, filtered and evaporated to afford a residue which was purified by chromatography over silica using hexane/ ethyl acetate as eluents to give 1.67 g of the title compound (70%) .

$^1$H NMR (400 MHz, $CDCl_3$) δ 8.21- 8.17 (2H, m), 6.96- 6.93 (2H, m), 3.90 (2H, d, J = 7.2 Hz), 1.31- 1.28 (1 H, m), 0.71- 0.67 (2H, m), 0.40- 0.36 (2H, m) .

LC- MS: $t_R$ = 3.70 $[M+H]^+$= not ion (method 3)

***Intermediate compound 7: 4-(cyclopropylmethoxy)benzenamine***

1- (cyclopropylmethoxy)- 4- nitrobenzene **intermediate compound 6** (8.6 mmol, 1.67g) was dissolved in ethanol (40 mL), and tin (II) chloride dihydrate (4.5 eq, 40.68 mmol, 9.18g) was added and the resulting mixture was refluxed for 16h. After cooling, the mixture was concentrated in vacuum. The residue was dissolved in ethyl acetate and 1N

NaOH. This mixture was filtered through Celite®. The aqueous phase was extracted with ethyl acetate and the combine organic extracts were washed by brine, dried over anhydrous sodium sulphate, filtered and concentrated in vacuum to afford 1.18g as an oil which was used in the next step without further purification. (80%) .

$^1H$ NMR (400 MHz, $CDCl_3$) δ 6.76- 6.72 (2H, m), 6.65- 6.62 (2H, m), 3.72 (2H, d, J = 6.8 Hz), 3.40 (2H, brs), 1.36- 1.23 (1H, m), 0.63- 0.58 (2H, m), 0.33- 0.30 (2H, m) .

LC- MS: $t_R$ = 1.15 & 1.68 $[M+H]^+$= 164 (method 3) .

***Intermediate compound 8: N-(4-nitrophenyl)-methanesulfonamide***

To a solution of 4-nitroaniline (7.24 mmol, 1 g) in DCM (10 mL) at 0ºC in an icebath, triethylamine (23.7 mmol, 3.3 mL) has been added under stirring. To this mixture a solution of methanesulphonyl chloride (23.7 mmol, 1.8 mL) has been added dropwise. The mixture was left to stir at room temperature 16 h. The reaction mixture was neutralized with saturated aqueous solution of $NH_4Cl$. The organic phase was separated, and, after evaporation of the solvent, the residue was washed with ethanol and filtered to give an bissulphonamide intermediate which was dissolved in a mixture ethanol:water (2:1, 36 mL). LiOH (39.1 mmol, 0.93 mg) was added to the solution and the reaction mixture was refluxed for two hours. After cooling at room temperature, the reaction mixture was neutralized with aqueous solution of $NH_4Cl$ and the aqueous phase was extracted with DCM. The organic phase was separated and dried over anhydrous sodium sulfate, filtered and concentrated under vacuum to give the title compound which was used in the next step without further purification. (1.3 g. Yield= 83%)

$^1H$ NMR (400 MHz, $CDCl_3$) δ 8.26 (2H, d, J = 9.2 Hz), 7.31 (2H, d, J = 8.8 Hz), 3.15 (3H, s) .

LC- MS: $t_R$ =2.28 $[M+H]^+$= 215 (method 3)

***Intermediate compound 9: N-(4-aminophenyl)-methanesulfonamide***

To a solution of **intermediate compound 8** (3.23 mmol, 0.70 g) in MeOH (7 mL) Pd/C 10 % (10% w/w, 70 mg) was added and the mixture was hydrogenated at room temperature for 16 hours. After that, the reaction mixture was filtered through a pad of Celite© washing with THF. The solvent was evaporated under vacuum to give the title compound which was used at the next step without further purification. (0.4 g. Yield= 67%)

$^1H$ NMR (400 MHz, DMSO- $d_6$) δ 8.95 (1 H, brs), 6.94 (2H, d, J = 8.8 Hz), 6.56 (2H, d, *J*= 8.8 Hz), 5.06 (1 H, brs), 2.85 (3H, s) .

LC- MS: $t_R$ =0.36 $[M+H]^+$= 187 (method 3)

***Intermediate compound 10: N-methyl-N-(4-nitrophenyl)-methanesulfonamide***

To a solution of **intermediate compound 8** (2.31 mmol, 0.50 g) in anhydrous ACN (15 mL), anhydrous potassium carbonate (5.78 mmol, 0.79 g) and iodomethane (7.39 mmol, 0.46 mL) were added sequentially and the reaction mixture was stirred at room temperature for 18 h. The solvent was concentrated under vacuum and the crude residue was treated with water and EtAOc. The organic phase was separated and washed with saturated aqueous sodium carbonate solution and brine, dried over anhydrous magnesium sulfate, filtered and concentrated under vacuum to give the title compound which was used in the next step without further purification. (0.50 g. Yield=95%)

$^1H$ NMR (400 MHz, $CDCl_3$) δ 8.26 (2H, d, J = 9.2 Hz), 7.56 (2H, d, J = 9.2 Hz), 3.42 (3H, s), 2.92 (3H, s) .

LC- MS: $t_R$ =2.43 $[M+H]^+$= 231 (method 3)

***Intermediate compound* 11: *N-(4-aminophenyl)-N-methyl-methanesulfonamide***

$SnCl_2$. $2H_2O$ (10.8 mmol, 2.4 g) was added to a solution of **intermediate compound 10** (1.08 mmol, 0.25 g) in THF (15 ml) . The mixture was kept under stirring at 70ºC for 16 h. After cooling, the solvent was evaporated under vacuum and the residue was treated with water and DCM. The mixture was neutralized with saturated $NaHCO_3$ and filtered through a pad of Celite© washing with DCM and water. The organic phase was separated in a funnel, washed with brine, dried over anhydrous sodium sulfate and concentrated under vacuum to give the title compound which was used in the next step without further purification. (0.19 g. Yield=87%)

LC- MS: $t_R$ =0.93 $[M+H]^+$= 201 (method 3)

***Intermediate compound 12: 3-(4-(hydroxymethyl)phenyl)propanoic acid***

To a solution of 3- (4- formylphenyl) acrylic acid (5.68 mmol, 1g) in EtOH (20mL), was added DIPEA (6.1 mmol, 1mL) and was hydrogenated over Pd/C (12.5% w/w) .The resulting solution was stirred 2 h at room temperature. The catalyst was removed by filtration and the solvent was evaporated to give 3- (4- (hydroxymethyl) phenyl) propanoic acid

(1 g. Yield: 100%) .

LC- MS: $t_R$ = 2.12 $[M-H]^-$= 179 (method 3) .

***Intermediate compound 13: 3-(4-formylphenyl)propanoic acid***

To a solution of 3- (4- (hydroxymethyl) phenyl) propanoic acid (**intermediate compound 12**) (3.88 mmol, 700 mg) in dry chloroform (20 mL) was added Manganese (IV) oxide activated (19.4 mmol, 1.68 g) . The reaction mixture was heated 48 h at 80ºC. The reaction mixture was cooled, filtered and concentrated *in vacuum.* The crude product was purified by flash chromatography on silica gel using an elution of methanol in dichloromethane to afford 3- (4-formylphenyl) propanoic acid (74mg. Yield: 10%)

LC- MS: $t_R$ = 2.37 $[M-H]^-$= 177 (method 3) .

***Intermediate compound 14: methyl 2-(4-(hydroxymethyl)phenyl)acetate***

To a solution of 2- (4- (hydroxymethyl) phenyl) acetic acid (1.2 mmol, 200mg) in dry MeOH (7 mL) was added acetyl chloride (2.4 mmol, 0.171 mL) . The reaction mixture was stirred overnight at room temperature. The crude product was concentrated *in vacuum* to give the compound methyl 2- (4- (hydroxymethyl) phenyl) acetate. (216 mg. Yield: 100%)

LC- MS: $t_R$ = 2.28 $[M+H]^+$ = 181 (method 3) .

***Intermediate compound 15: methyl 2-(4-formylphenyl)acetate***

A solution of 2- (4- (hydroxymethyl) phenyl) acetate (**intermediate compound 14**) (1.2 mmol, 216 mg) was dissolved in DCM (4 mL) and treated with manganese (IV) oxide (10.8 mmol, 938 mg) stirred 16h at room temperature. Total conversion was not achieved, then the reaction mixture was heated at 55ºC for 6 h. The crude product was filtered and concentrated *in vacuum* to give the compound methyl 2- (4- formylphenyl) acetate (150mg. Yield: 70%) .

$^1H$ NMR (400 MHz, DMSO- $d_6$) δ 9.99 (1 H, s), 7.87 (2H, d, *J*=6Hz), 7.50 (2H, d, *J*=5.6Hz), 3.83 (2H, s), 3.63 (3H, s) .

LC- MS: $t_R$ = 2.47 $[M+H]^+$ = 179 (method 3) .

***Intermediate compound 16: 4-formyl-2-methylbenzoic acid***

A solution of 4- bromo- 2- methylbenzoic acid (2.32 mmol, 500 mg) in dry THF (12 mL) was purged with argon. The solution was cooled at- 78ºC, BuLi (2.5 M in hexane, 7.5 mmol, 3 ml), and DMF (5.16 mmol, 0.4 mL) was added and the reaction was stirred for 1h at- 78ºC and then warmed to 25ºC and allowed to react for an additional hour. The reaction was quenched with aqueous 1 N HCl and extracted with EtAcO. The organic layer was extracted with aqueous 1 N NaOH and the aqueous layer was separated and the pH adjusted with aqueous 1 N HCl. The aqueous was extracted with EtAcO, then washed with brine, dried over anhydrous sodium sulfate, filtered and concentrated. The resulting crude was purified by flash chromatography on silica gel using an elution of methanol in dichloromethane. (112 mg. Yield: 29%)

LC- MS: $t_R$ = 2.37 $[M-H]^-$= 163 (method 3) .

***Intermediate compound 17: 3-chloro-4-formylbenzoic acid***

To a solution of 4- bromo- 3- chlorobenzoic acid (2.12 mmol, 500 mg) in anhydrous THF (6 mL) was added *i*-PrMgCl·LiCl (1.3 M in THF, 6.36 mmol, 4.9 mL) and was cooled at- 78ºC under a $N_2$ atmosphere. After 10 minutes, the temperature was raised to 0ºC and the reaction was stirred for a 1 h at 0ºC. DMF (10.6 mmol, 0.825 ml) was added. The reaction was warmed to room temperature, stirred for 1.5 h and quenched by saturated $NH_4Cl$. The layers were separated and the aqueous layer was extracted twice with EtAcO. The aqueous layer was separated and the pH adjusted with aqueous 1 N HCl. The aqueous layer was extracted with EtAcO then washed with brine, dried over anhydrous sodium sulfate, filtered and concentrated to obtain the compound 3- chloro- 4- formylbenzoic acid (300 mg. Yield: 77%) .

$^1H$ NMR (400 MHz, DMSO- $d_6$) δ 12.7 (1 H, s), 10.37 (1 H, s), 8.03- 8.01 (2H, m), 7.97 (1 H, d, *J*= 8Hz) .

LC- MS: $t_R$ = 2.47 $[M-H]^-$= 183 (method 3) .

***Intermediate compound 18: 4-formyl-3-methylbenzoic acid***

A solution of 4- bromo- 3- methylbenzoic acid (2.32 mmol, 500 mg) in dry THF (12 mL) was purged with argon. The solution was cooled at- 78ºC, BuLi (2.5M in hexane, 7.5 mmol, 3 mL), and DMF (5.16 mmol, 0.4 mL) was added and the reaction was stirred for 1h at- 78ºC and then warmed to 25ºC and allowed to react for an additional hour. The reaction was quenched with aqueous 1 N HCl and extracted with EtAcO. The organic layer was extracted with aqueous

1 N NaOH and the aqueous layer was separated and the pH adjusted with aqueous 1 N HCl. The aqueous was extracted with EtAcO then washed with brine, dried over anhydrous sodium sulfate, filtered and concentrated. The resulting crude was purified by precipitation with hexane (90 mg. Yield: 23%) $^1$H NMR (400 MHz, DMSO- $d_6$) δ 13.25 (1 H, s), 10.32 (1 H, s), 7.92 (2H, m), 7.89 (1 H, s), 2.67 (3H, s) .

LC- MS: $t_R$ = 2.53 $[M-H]^-$= 163 (method 3) .

***Intermediate compound 19: methyl 4-formyl-3-methylbenzoate***

A solution of methyl 4- iodo- 3- methylbenzoate (21.7 mmol, 6 g) in dry THF (120 mL) was purged with argon. The solution was cooled to- 15ºC, *i*- PrMgCl (2M in THF, 108.5 mmol, 54.25 mL), and DMF (130.2 mmol, 10 mL) were added and the reaction was stirred for 2 h at- 15ºC and then warmed to 25ºC and allowed to react for an additional hour. The reaction was quenched with aqueous 1 N HCl and extracted with EtAcO. The organic layer was washed with brine, dried over anhydrous sodium sulfate, filtered and concentrated. The resulting crude was purified by flash chromatography on silica gel using an elution of 6% ethylacetate in hexanes to give methyl 4- formyl- 3- methylbenzoate (3.08g .Yield: 80%) .

$^1$H NMR (400 MHz, $CDCl_3$) δ 10.35 (1H, s), 8.00 (1H, d, *J*=8Hz), 7.99 (1H, s), 7.86 (1 H, d, *J*=8Hz), 3.95 (3H, s), 2.72 (3H, s)

LC- MS: $t_R$ = 2.85 $[M+H]^+$ = not ion (method 3) .

***Intermediate compound 20: methyl 4-formyl-3-methoxybenzoate***

To a solution of methyl 4- (bromomethyl)- 3- methoxybenzoate (1.93 mmol, 500 mg) in anhydrous DMSO (2.7 mL) was added $NaHCO_3$ (2.32 mmol, 195 mg) . The reaction mixture was stirred for 3 h at 50ºC. Water was added to the reaction mixture, and the mixture was extracted with EtAcO. The organic layer was dried over anhydrous $MgSO_4$, the solvent was filtered and evaporated. The crude product was purified by flash chromatography on silica gel using an elution of 8% ethylacetate in hexanes to give methyl 4- formyl- 3- methoxybenzoate (140 mg. Yield: 37%) .

$^1$H NMR (400 MHz, $CDCl_3$) δ 10.51 (1 H, s), 7.87 (1 H, d, J=8.Hz), 7.69- 7.67 (2H, m), 4.00 (3H, s), 3.95 (3H, s) .

LC- MS: $t_R$ = 2.75 $[M+H]^+$ = 195 (method 3) .

***Intermediate compound 21 and 22: methyl 3-bromo-4-(dibromomethyl)benzoate and methyl 3-bromo-4-(bromomethyl)benzoate***

Method 1: To a solution of methyl 3- bromo- 4- methylbenzoate (2.18 mmol, 500 mg) in $CCl_4$ (3 mL), NBS (2.18 mmol, 389 mg), and benzoyl peroxide (0.111 mmol, 27 mg) were added. The reaction mixture was stirred 16 h at 82 ºC. The resulting solution extracted with dichloromethane and $H_2O$. The organic layers were washed with brine, dried over anhydrous magnesium sulfate, filtered and concentrated. The crude product was purified by flash chromatography on silica gel using an elution of 6% ethylacetate in hexanes afforded methyl 3- bromo- 4- (bromomethyl) benzoate 22 (250 mg. Yield: 37%) and methyl 3- bromo- 4- (dibromomethyl) benzoate **21** which was again purified by flash chromatography on silica gel using an elution of 25% dichloromethane in hexane (110 mg. Yield: 17%) .

Method 2: To a solution of methyl 3- bromo- 4- methylbenzoate (2.18 mmol, 500 mg) in $CCl_4$ (3 mL), NBS (2.62 mmol, 466 mg), and AIBN (0.218 mmol, 36 mg) were added. The reaction mixture was stirred 16h at 90ºC. The resulting solution extracted with dichloromethane and $H_2O$. The organic layers were washed with saturated solution of $NaHCO_3$, dried over anhydrous magnesium sulfate, filtered and concentrated. The crude product was purified by flash chromatography on silica gel using an elution of 4% ethylacetate in hexanes afforded methyl 3- bromo- 4- (bromomethyl) benzoate **22** (250 mg. Yield: 37%) and methyl 3- bromo- 4- (dibromomethyl) benzoate **21** which was again purified by flash chromatography on silica gel using an elution of 25% dichloromethane in hexane (110 mg. Yield: 17%)

Method 3: To a solution of methyl 3- bromo- 4- methylbenzoate (2.18 mmol, 500 mg) in $CCl_4$ (5 mL), NBS (6.54mmol, 1170 mg), and benzoyl peroxide (0.218 mmol, 53 mg) were added. The reaction mixture was stirred 16h at 90 ºC. The reaction was cooled and the precipitate of succinimide and unreacted NBS was removed by filtration and washed with ethylacetate. The crude product was purified by flash chromatography on silica gel using an elution of 37% dichloromethane in hexanes afforded methyl 3- bromo- 4- (dibromomethyl) benzoate **21**. (840 mg. Yield: 100%) $^1$H NMR (400 MHz, $CDCl_3$) δ 8.18 (1 H, d, *J*=1.6Hz), 8.09 (1 H, d, *J*=8.8Hz), 8.04 (1 H, dd, *J*= 1.6 & 8.4Hz), 7.07 (1 H, s) 3.94 (3H, s) .

LC- MS: $t_R$ = 4.20 $[M+H]^+$ = not ion (method 3) .

Methyl 3- bromo- 4- (bromomethyl) benzoate **22**

$^1$H NMR (400 MHz, $CDCl_3$) δ 8.25 (1H, s), 7.95 (1H, d, *J*=8Hz), 7.53 (1H, d, *J*=8Hz), 4.61 (2H, s), 3.93 (3H, s) .

LC- MS: $t_R$ = 3.82 $[M+H]^+$ = not ion (method 3) .

***Intermediate compound 23:* methyl 3-bromo-4-formylbenzoate**

Method 1: To a solution of methyl 3- bromo- 4- (dibromomethyl) benzoate (**intermediate compound 21**) (056 mmol, 220 mg) in acetonitrile (1mL) was added a solution of $AgNO_3$ (1.68 mmol, 285 mg) in water (0.5 mL), and the mixture was heated for 20 min under reflux. After the solution was allowed to cool, AgBr was filtered off and washed with DCM, the combined filtrate was washed with water and dried over anhydrous sodium sulfate. The solvent was evaporated and the residue was purified by flash chromatography on silica gel using an elution of 4% ethylacetate in hexanes afforded methyl 3- bromo- 4- formylbenzoate **23** (59 mg. Yield: 43%) .

Method 2: To a solution of methyl 3- bromo- 4- (bromomethyl) benzoate **(intermediate compound 22)** (1.43 mmol, 442 mg) in dichloromethane at 0ºC was added a DMSO solution (3.2 mL) of trimethylamine oxide (5.72 mmol, 430 mg) . The reaction mixture was warmed at 28ºC for 8 h. To this mixture was added $Na_2CO_3$ solution, and the organic layer was extracted with DCM, and washed with brine, dried over $MgSO_4$ and concentrated to give methyl 3- bromo- 4- formylbenzoate **23** (173 mg. Yield: 49%) .

$^1$H NMR (400 MHz, $CDCl_3$) δ 10.41 (1 H, s), 8.32 (1 H, s), 8.07 (1 H, d, J = 8Hz), 7.96 (1 H, d, *J*= 8Hz), 3.97 (3H, s) .

LC- MS: $t_R$ = 3.25 $[M+H]^+$ = not ion (method 3) .

***Intermediate compound 24: 4-bromo-2-ethylbenzaldehyde***

Method 1: To a solution of 4- bromo- 2- ethyliodobenzene (1.61 mmol, 0.5 g) dissolved in anhydride THF (6 mL) at- 78ºC was added 2.5 M *n*- butyllithium solution in hexane (1.9 mmol, 0.77 mL) dropwise. The mixture was stirred 30 min at- 78 ºC before *N*- formylmorpholine (3.69 mmol, 0.37 mL) was added and the reaction stirred at this temperature for 1 h. The reaction was quenched with aqueous 1 N HCl and extracted with EtAcO. The combined organic extracts were washed with brine, dried over anhydrous sodium sulfate, filtered and concentrated. Purification of the crude material by flash chromatography on silica gel using an elution of 3% ethylacetate in hexanes afforded the title compound (211 mg, 62%) .

Method 2: The following compound was prepared using the same methodology as intermediate compound 19 using 4- bromo- 2- ethyliodobenzene (6.4 mmol, 2g) . Purification of the crude material by flash chromatography on silica gel using an elution of 3% ethylacetate in hexanes afforded the title compound (1.18 g, 86%) .

$^1$H (400 MHz, $CDCl_3$) δ 10.24 (1 H, s), 7.69 (1 H, d, J = 8.4 Hz), 7.51 (1 H, dd, *J* = 8.4 & 2 Hz), 7.48 (1H, d, J = 2 Hz), 3.05 (2H, q, J = 7.6 Hz), 1.28 (3H, t, J = 7.6 Hz) .

LC- MS: $t_R$ = 3.67 $[M+H]^+$= 213/215 (method 3)

***Intermediate compound 25: 3-ethyl-4-formylbenzonitrile***

Method 1: the aldehyde intermediate compound 24 (0.70 mmol, 150 mg), zinc cyanide (0.52 mmol, 61 mg) and tetrakis(triphenylphosphine)palladium (0) (0.02 mmol, 24 mg) were dissolved in anhydrous DMF (1.5 mL). The mixture was degassed with Argon for 5 min. The reaction was heated in a microwave at 220 ºC for 5 min. After that the reaction was diluted with water and extracted with toluene. The organic phase was separated and washed with brine, dried over and sodium sulfate, filtered and concentrated. Purification of the crude material by flash chromatography on silica gel using an elution of 3% ethylacetate in hexanes afforded the pure title compound (49 mg, 43%).

Method 2: the aldehyde **intermediate compound 24** (0.70 mmol, 150 mg) and copper (I) cyanide (0.91 mmol, 82 mg) were mixed in *N*- methyl- 2- pyrrolidinone (2 mL) . The mixture was heated in a microwave at 200 ºC for 15 min. The reaction was diluted with DCM and filtered through a pad of Celite® and concentrated to dryness. The crude material was purified by flash chromatography on silica gel using an elution of 3% ethylacetate in hexanes to afford the pure title compound (87 mg, 77%) .

$^1$H (400 MHz, $CDCl_3$) δ 10.00 (1 H, s), 7.93 (1 H, d, *J*= 8 Hz), 7.66 (1 H, dd, *J*= 8 & 1.2 Hz), 7.62 (1 H, s), 3.11 (2H, q, J= 7.6 Hz), 1.31 (3H, t, J= 7.6 Hz) .

LC- MS: $t_R$ = 2.88 $[M+H]^+$ not ion (method 3) .

***Intermediate compound 26: 3-ethyl-4-formylbenzoic acid***

A mixture of the 3- ethyl- 4- formylbenzonitrile **intermediate compound 25** (1.38 mmol, 220 mg) and concentrated hydrochloric acid (2 mL) was boiled under reflux for 16 h. The mixture was cooled and diluted with water. The reaction mixture was extracted with $Et_2O$, the combined organic extracts were washed with brine, dried over anhydrous magnesium sulfate and concentrated to dryness to afford the title compound which was used in the next step without further purification. (219 mg, 89%)

$^1$H (400 MHz, $CDCl_3$) δ 10.40 (1 H, s), 8.09 (1 H, dd, J = 8 & 1.2 Hz), 8.06 (1 H, s), 7.94 (1 H, d, *J*= 8.4 Hz), 3.15 (2H, q, *J*= 7.6 Hz), 1.33 (3H, t, *J*= 7.6 Hz) .

LC- MS: $t_R$ = 2.63 $[M+H]^-$= 177 (method 3)

***Intermediate compound 27: 4-bromo-2,6-dimethylbenzaldehyde***

The following compound was prepared using the same methodology as in intermediate compound 19 using the 5- bromo- 2- iodo- m- xylene (6.4 mmol, 2g) . Purification of the crude material by flash chromatography on silica gel using an elution of 3% ethylacetate in hexanes afforded the title compound (1.27 g, 92%) .

$^1H$ (400 MHz, $CDCl_3$) $\delta$ 10.56 (1 H, s), 7.28 (2H, brs), 2.59 (6H, s) .

LC- MS: $t_R$ = 3.73 $[M+H]^+$ not ion (method 3) .

***Intermediate compound 28: 4-formyl-3,5-dimethylbenzonitrile***

The following compound was prepared using the same methodology as in intermediate compound 25 (method 2) using the aldehyde i**ntermediate compound 27** (0.70 mmol, 250 mg). The reaction was diluted with DCM and fltered through a pad of Celite® and concentrated to dryness. The crude material was purified by flash chromatography on silica gel using an elution of 15% ethylacetate in hexanes afforded the pure title compound (138 mg, 74%). $^1H$ (400 MHz, $CDCl_3$) $\delta$ 10.63 (1 H, s), 7.40 (2H, brs), 2.63 (6H, s).

LC- MS: $t_R$ = 2.88 $[M+H]^+$ not ion (method 3) .

***Intermediate compound 29: 4-formyl-3,5-dimethylbenzoic acid***

The following compound was prepared using the same methodology as in intermediate compound 26 using the aldehyde intermediate compound 28 (0.94 mmol, 150 mg). The mixture was cooled and diluted with water. The reaction mixture was extracted with $Et_2O$. The combined organic extracts were washed with brine, dried over magnesium sulfate and concentrated to dryness to afford the title compound which was used in the next step without further purification. (165 mg, 98%)

$^1H$ (400 MHz, $CDCl_3$) $\delta$ 10.68 (1 H, s), 7.83 (2H, s), 2.66 (6H, s) .

LC- MS: $t_R$ = 2.60 $[M+H]^-$= 177 (method 3)

***Intermediate compound 30: methyl 4-formyl-3,5-dimethylbenzoate***

4- formyl- 3, 5- dimethylbenzoic acid **intermediate compound 29** (0.81 mmol, 145 mg) was dissolved in dry methanol (8 mL) and stirred for 10 min at 0ºC. Thionyl chloride (7.27 mmol, 0.53 mL) was added dropwise, the reaction mixture was slowly brought to room temperature and then stirred for 4 h. The methanol was evaporated and the resulting residue was extracted into DCM, washed with saturated sodium chloride, dried over anhydrous magnesium sulfate, filtered and evaporated to afford the title compound as a solid (141 mg, 91%) .

$^1H$ (400 MHz, $CDCl_3$) $\delta$ 10.66 (1 H, s), 7.76 (2H, s), 3.94 (3H, s), 2.65 (6H, s) .

LC- MS: $t_R$ = 3.22 $[M+H]^+$ not ion (method 3) .

***Intermediate compound 31: 5-formylpyridine-2-carboxylic acid***

To a solution of 6- bromopyridine- 3- carbaldehyde (2.69 mmol, 500mg), was dissolved in a mixture of MeOH (2.7 mL) and DMF (2.7 mL) and $Et_3N$ (5.38 mmol, 0.75 ml) was added. To this solution palladium (II) acetate (0.07 mmol, 15 mg), and 1, 1- bis (diphenylphosphino) ferrocene (0.14 mmol, 75 mg) were added. The mixture was degassed and was bubbled carbon monoxide gas. The resulting solution was heated at 55ºC for 48 h. Total conversion was not achieved, then $Et_3N$ (2.69 mmol, 0.38 mL), palladium (II) acetate (0.03mmol, 8 mg), and 1, 1- bis (diphenylphosphino) ferrocene (0.07 mmol, 38 mg) were added again. The mixture was degassed and was again bubbled carbon monoxide gas. The resulting solution was heated at 55ºC for 24 hours. The crude product was poured into water and extracted with EtAcO. The aqueous layer was concentrated *in vacuum,* then was dissolved in EtAcO and washed with $Na_2CO_3$ saturated. The organic layer was dried with anhydrous sodium sulfate, filtered and concentrated *in vacuum* to give the compound methyl 5- formylpyridine- 2- carboxylate (30 mg. Yield: 7%) . The aqueous layer was concentrated and the crude was purified using reverse $C_{18}$ chromatography using water (0.1% formic acid) and acetonitrile (0.1% formic acid) as eluents to give the title compound 5- formylpyridine- 2- carboxylic acid (84 mg. Yield: 19%) .

$^1H$ NMR (400 MHz, DMSO- $d_6$) $\delta$ 13.6 (1H, s), 10.19 (1H, s), 9.17 (1H, s), 8.42 (1 H, d, *J*=8Hz), 8.21 (1 H, d, J=8Hz)

LC- MS: $t_R$ = 1.8 $[M+H]^+$ = 166 (method 3) .

***Intermediate compound 32: 3-bromo-6-iodo-2-methylpyridine***

Sodium iodide (2 eq., 16 mmol, 2.40 g) and 2, 5- dibromo- 6- methylpyridine (2.0 g, 8.0 mmol) were combined in propionitrile (20 mL) and the resulting slurry was stirred under nitrogen for 5 min. Iodotrimethylsilane (0.2 eq., 1.6 mmol, 0.23 mL) was added and the reaction mixture was heated at 95ºC with stirring under nitrogen for 16 h. The slurry was cooled to room temperature, diluted with a 1: 1 mixture of ethylacetate and water. The mixture was stirred for 15 min and the aqueous and organic phases were then separated. The organic layer was washed sequentially with equal volume of saturated aqueous sodium bicarbonate solution, sodium thiosulfate (5% aqueous solution) and brine. The organic layer was dried over anhydrous sodium sulphate, filtered and concentrated under reduce pressure to afford the desired product which was purified by flash chromatography on silica gel using an elution of 4% ethylacetate in hexanes afforded the title compound as an oil (1.72 g, 72%) .

$^1H$ NMR (400 MHz, $CDCl_3$) $\delta$ 7.41 (2H, s), 2.63 (3H, s) .

LC- MS: $t_R$ = 3.53 $[M+H]^+$= 298/300 (method 3)

***Intermediate compound 33: 5-bromo-6-methylpyridine-2-carbonitrile***

Under an inert atmosphere, 3- bromo- 6- iodo- 2- methylpyridine (**intermediate compound 32**) (1.25 g, 4.19 mmol) and acetonitrile (15 mL) were combined and stirred for 30 min with copper cyanide (0.5 eq., 2.0 mmol, 185 mg), sodium cyanide (0.8 eq., 3.35 mmol, 165 mg) . The reaction mixture was heated at 80ºC with stirring under nitrogen for 16 h. The slurry was cooled to room temperature, diluted with a 0.5 M aqueous solution of ammonia and stirred for 15 min, filtered through Celite® and washed with ethylacetate. Organic layer was then separated. The organic layer was washed sequentially with 0.5 M aqueous solution of ammonia and brine. The organic layer was dried over anhydrous sodium sulphate, filtered and concentrated under reduce pressure. Purification by flash chromatography on silica gel using an elution of 7% ethylacetate in hexanes afforded the title compound as an oil (650 mg, 79%) .

$^1H$ NMR (400 MHz, $CDCl_3$) $\delta$ 7.95 (1 H, d, J = 8.4 Hz), 7.40 (1 H, d, J = 8.4 Hz), 2.17 (3H, s) .

LC- MS: $t_R$ = 2.75 $[M+H]^+$= 197/199 (method 3)

***Intermediate compound 34: 6-methyl-5-vinylpyridine-2-carbonitrile***

An argon degassed solution of 5- bromo- 6- methylpyridine- 2- carbonitrile **(intermediate compound 33**) (650 mg, 3.3 mmol), 4, 4, 5, 5, -tetramehyl- 2- vinyl- 1, 3, 2- dioxaborolane (3.3 mmol, 0.56 mL), tetrakis (triphenylphosphine) palladium (5 mol %, 0.16 mmol, 190 mg) and 2N aqueous sodium carbonate solution (3.4 eq., 11.22 mmol, 5.60 mL) in toluene/ ethanol (2: 1, 45 mL) was stirred at 95 ºC under argon for 16 h. The slurry was cooled to room temperature, diluted with ethylacetate and water. The organic layer was washed with brine and dried over anhydrous sodium sulphate, filtered and concentrated under reduce pressure to afford the desired product which was purified by flash chromatography on silica gel using an elution of 20% ethylacetate in hexanes afforded the title compound as an oil (246 mg, 52%) .

$^1H$ NMR (400 MHz, $CDCl_3$) $\delta$ 7.81 (1 H, d, J = 8.0 Hz), 7.52 (1 H, d, J = 8.0 Hz), 6.89 (1H, dd, *J* = 17.6 & 10.8 Hz), 5.79 (1 H, d, J = 17.2 Hz), 5.58 (1 H, d, J = 10.8 Hz), 2.62 (3H, s) .

LC- MS: $t_R$ = 2.58 $[M+H]^+$= 145 (method 3)

***Intermediate compound 35: 5-formyl-6-methylpyridine-2-carbonitrile***

6- Methyl- 5- vinylpyridine- 2- carbonitrile (**intermediate compound 34**) (245 mg, 1.7 mmol), was dissolved in acetone/ water (6: 1, 21 mL) and then $OsO_4$ (0.74 eq., 1.3 mmol, 330 mg) and $NaIO_4$ (3eq, 5.1 mmol, 1.09 g) were added thereto. The resulting mixture was kept at room temperature for 3 h. Filtration of the reaction mixture was carried out. Filtrate was diluted with ethylacetate. The organic layer was then separated and washed with a saturated aqueous sodium carbonate solution and brine, then dried over anhydrous sodium sulphate, filtered and concentrated under reduce pressure to afford the desired product which was purified by flash chromatography on silica gel using an elution of 40% ethylacetate in hexanes afforded the title compound as an solid (178 mg, 71 %) .

$^1H$ NMR (400 MHz, $CDCl_3$) $\delta$ 10.38 (1 H, s), 8.23 (1 H, d, *J* = 8.4 Hz), 7.73 (1 H, d, *J*= 8.0 Hz), 2.94 (3H, s) .

LC- MS: $t_R$ = 1.68 & 1.77 $[M+H]^+$= not ion (method 3)

***Intermediate compound 36: (E)-ethyl 2,4-dioxo-6-phenylhex-5-enoate***

Sodium pellets (1eq, 0.1 mol, 2.3 g) were slowly added in absolute ethyl alcohol (100 mL) . After the sodium had reacted, the solvent was removed under reduced pressure and anhydrous ether (130 mL) was added. The reaction mixture was cooled to- 5ºC, and a solution of 4- phenyl- 3- buten- 2- one (14.6 g, 0.1 mol) and diethyl oxalate (1.2 eq., 0.12 mol, 16 mL) in anhydrous ether (25 mL) was added over 30 min. After stirring 16h at room temperature the yellow

solid was filtered and washed with ether, after 1h of drying at room temperature the solid was partitioned between dichloromethane (600 mL) and 1 N sulfuric acid (200 mL) . The organic layer was then separated and dried over anhydrous sodium sulphate, filtered and concentrated under reduce pressure to afford the desired product (22.05 g, 89%) as a yellowish solid, which was used in the next step without further purification.

LC- MS: $t_R$ = 3.80 $[M+H]^+$= 247 (method 3)

***Intermediate compound 37 and 38: 1-methyl-5-styryl-1H-pyrazole-3-carboxylic acid ethyl ester and 2-methyl-5-styryl-2H-pyrazole-3-carboxylic acid ethyl ester***

A solution of (*E*)- ethyl 2, 4- dioxo- 6- phenylhex- 5- enoate (**intermediate compound 36**) (2.5 g, 0.01 mol) and methylhydrazine (1.2 eq., 0.012 mol, 0.65 mL) in ethanol was heated at reflux for 16h. The solvent was removed in vacuum and the residue was purified by flash chromatography on silica gel using hexane and ethylacetate as eluents. At 20% of ethylacetate in hexanes the isomer 2- methyl- 5- styryl- 2H- pyrazole- 3- carboxylic acid ethyl ester **38** elutes firstly (710 mg), later at 40% of ethylacetate in hexanes the title isomer elutes to afford 1.64 g of 1- methyl- 5- styryl- 1 H- pyrazole- 3- carboxylic acid ethyl ester **37** (overall yield=92%) .

1- methyl- 5- styryl- 1 H- pyrazole- 3- carboxylic acid ethyl ester **37**

$^1H$ NMR (400 MHz, $CDCl_3$) δ 7.48 (2H, dd, *J*= 8.8 & 1.6 Hz), 7.35 (2H, t, *J*= 7.2 Hz), 7.28- 7.24 (1 H, m), 7.14 & 7.11 (1 H, 2s), 7.07 & 7.03 (1 H, 2s), 7.01 (1 H, s), 4.36 (2H, q, J = 6.8 Hz), 4.18 (3H, s), 1.40 (3H, t, J = 6.8 Hz) .

LC- MS: $t_R$ = 3.58 $[M+H]^+$= 257 (method 2)

2- methyl- 5- styryl- 2H- pyrazole- 3- carboxylic acid ethyl ester **38**:

$^1H$ NMR (400 MHz, $CDCl_3$) δ 7.51- 7.48 (2H, m), 7.41- 7.36 (2H, m), 7.34- 7.28 (1 H, m), 7.10 & 7.06 (1 H, 2s), 7.02 (1 H, s), 6.92 & 6.87 (1 H, 2s), 4.41 (2H, q, *J*= 7.2 Hz), 4.00 (3H, s), 1.41 (3H, t, *J*= 7.2 Hz) .

LC- MS: $t_R$ = 3.98 $[M+H]^+$= 257 (method 3)

***Intermediate compound 39: 5-formyl-1-methyl-1H-pyrazole-3-carboxylic acid ethyl ester***

The following compound was prepared using the same methodology as in Intermediate compound 35 using 1- methyl- 5- styryl- 1H- pyrazole- 3- carboxylic acid ethyl ester (**intermediate compound 37**) (256 mg, 1.0 mmol) for 2h. Purification of the crude material using flash chromatography on silica gel using an elution of 25% ethylacetate in hexanes afforded the title compound as a solid (255 mg, 70%) .

$^1H$ NMR (400 MHz, $CDCl_3$) δ 9.88 (1 H, s), 7.41 (1 H, s), 4.43 (2H, q, J = 6.8 Hz), 4.25 (3H, s), 1.41 (3H, t, *J*= 7.6 Hz) .

LC- MS: $t_R$ = 1.78 & 2.05 $[M+H]^+$= not ion (method 3)

***Intermediate compound 40: 3-formyl-1-methyl-1H-pyrazole-5-carboxylic acid ethyl ester***

The following compound was prepared using the same methodology as in Intermediate compound 35 using 2- methyl- 5- styryl- 2H- pyrazole- 3- carboxylic acid ethyl ester (**intermediate compound 38**) (700 mg, 2.73 mmol) for 1.30 h. Purification of the crude material using flash chromatography on silica gel using an elution of 26% ethylacetate in hexanes afforded the title compound as a solid (340 mg, 68%) .

$^1H$ NMR (400 MHz, $CDCl_3$) δ 9.96 (1 H, s), 7.33 (1 H, s), 4.36 (2H, q, J = 6.8 Hz), 4.28 (3H, s), 1.39 (3H, t, *J*= 7.6 Hz) .

LC- MS: $t_R$ = 1.78 & 2.05 $[M+H]^+$= not ion (method 3)

***Intermediate compound 41: 4-((3aS,4R,9bR)-8-bromo-3a,4,5,9b-tetrahydro-3H-cyclopenta[c]quinolin-4-yl)benzoic acid***

A catalytic amount of scandium trifluoromethanesulfonate (0.1 eq., 0.1 mmol, 49 mg) in anhydrous acetonitrile (5 mL) was added to a mixture of 4-formylbenzoic acid (1 mmol, 150 mg), 4-aminobromobenzene (1 mmol, 172 mg), and fresh distilled ciclopentadiene (5 eq, 5 mmol, 0.40 mL). The reaction mixture was stirred at room temperature for 16 h. Solvent was evaporated and purification of the crude material (1:23 mixture of regioisomers exo/endo) by flash chromatography on silica gel using an elution of 40% ethylacetate in hexanes afforded the title endo-isomer as a white solid (64 mg, 17%).

$^1H$ NMR (400 MHz, DMSO- $d_6$) δ 7.93 (2H, d, *J* = 8 Hz), 7.54 (2H, d, *J* = 8.4 Hz), 7.16 (1 H, s), 7.02 (1 H, dd, *J* = 8.4 & 1.6 Hz), 6.69 (1 H, d, *J* = 8.4 Hz), 5.92 (1 H, brs), 5.86 (1 H, brs), 5.57 (1 H, d, *J* = 4 Hz), 4.58 (1 H, s), 4.02 (1 H, d, *J* = 6.8 Hz), 2.90- 2.96 (1 H, m), 2.39- 2.32 (1 H, m), 1.57 (1 H, dd, *J* = 15.6 & 8.4 Hz) .

LC- MS: $t_R$ = 3.57 $[M+H]^+$= 370/372 (method 2)

***Intermediate compound 42: 4-((3aS,4R,9bR)-8-(trifluoromethyl)-3a,4,5,9b-tetrahydro-3H-cyclopenta[c]quinolin-4-yl)benzoic acid***

The following compound was prepared using the same methodology as in intermediate compound 41 using 4-trifluoromethylaniline (1 mmol). Solvent was evaporated and purification of the crude material (1:30 mixture of exo/endo regioisomers) by flash chromatography on silica gel using an elution of 40% ethylacetate in hexanes afforded the endo-compound as a solid. (318 mg. Yield: 88%).

$^1$H NMR (400 MHz, DMSO- $d_6$) $\delta$ 12.90 (1 H, brs), 7.94 (2H, d, *J* = 8.4 Hz), 7.55 (2H, d, *J*= 8.4 Hz), 7.30 (1 H, s), 7.19 (1 H, dd, *J* = 8.4 & 1.6 Hz), 6.85 (1 H, d, *J* = 8.4 Hz), 6.41 (1 H, s), 5.94- 5.92 (1 H, m), 5.58 (1 H, d, *J* = 4.8 Hz), 4.70 (1 H, d, *J* = 2.8 Hz), 4.08 (1 H, d, *J* = 8.8 Hz), 2.97- 2.92 (1 H, m), 2.38- 2.30 (1 H, m), 1.59 (1 H, dd, *J*= 14.8 & 8.4 Hz) .

LC- MS: $t_R$ = 7.90 $[M+H]^+$= 360 (method 1)

***Intermediate compound 43: 4-((3aS,4R,9bR)-3a,4,5,9b-tetrahydro-8-methyl-3H-cyclopenta[c]quinolin-4-yl)benzoic acid***

The following compound was prepared using the same methodology as in intermediate compound 41 using 4-methylaniline (1 mmol). Solvent was evaporated and purification of the crude material (1:20 mixture of exo/endo regioisomers) by flash chromatography on silica gel using an elution of 50% ethylacetate in hexanes afforded the endo-compound as a white solid which was precipitated in acetonitrile, filtered and dried in vacuum to give the title compound (121 mg. Yield: 39%).

$^1$H NMR (400 MHz, DMSO- $d_6$) $\delta$ 7.92 (2H, d, J = 8.4 Hz), 7.55 (2H, d, J = 8.4 Hz), 6.79 (1 H, s), 6.68 (1 H, dd, J = 8.0 & 1.6 Hz), 6.62 (1 H, d, J = 8.4 Hz), 5.83- 5.81 (1 H, m), 5.53 (1 H, d, J = 4.8 Hz), 5.47 (1 H, brs), 4.52 (1 H, d, J = 2.8 Hz), 3.98 (1 H, d, *J* = 8.4 Hz), 2.91 (1 H, dq, *J*= 9, 2 & 4.5 Hz), 2.41- 2.34 (1 H, m), 2.13 (3H, s), 1.56 (1 H, dd, *J* = 15.2 & 8.8 Hz) .

LC- MS: $t_R$ = 7.47 $[M+H]^+$= 306 (method 1)

***Intermediate compound 44: 4-((3aS,4R,9bR)-8-chloro-3a,4,5,9b-tetrahydro-3H-cyclopenta[c]quinolin-4-yl)benzoic acid***

The following compound was prepared using the same methodology as in intermediate compound 41 using 4-chloroaniline (1mmol). Solvent was evaporated and purification of the crude material (1:20 mixture of exo/endo regioisomers) by flash chromatography on silica gel using an elution of 40% ethylacetate in hexanes afforded the endo-compound as a white solid which was precipitated in acetonitrile, filtered and dried in vacuum to give the title compound (250 mg. Yield: 77%).

$^1$H NMR (400 MHz, DMSO- $d_6$) $\delta$ 7.93 (2H, d, *J* = 8.4 Hz), 7.54 (2H, d, *J* = 8.4 Hz), 7.04 (1 H, d, *J* = 2 Hz), 6.90 (1 H, dd, *J* = 8.8 & 2.4 Hz), 6.73 (1 H, d, *J* = 8.4 Hz), 5.90 (1 H, s), 5.87- 5.86 (1 H, m), 5.56 (1 H, d, *J* = 4.8 Hz), 4.58 (1 H, d, *J* = 3.2 Hz), 4.03- 4.00 (1 H, m), 2.94- 2.90 (1 H, m), 2.40- 2.32 (1 H, m), 1.57 (1 H, dd, *J* = 15.6 & 9.2 Hz) .

LC- MS: $t_R$ = 7.77 $[M+H]^+$= 326/328 (method 1)

***Intermediate compound 45****: 4-((3aS,4R,9bR)-3a,4,5,9b-tetrahydro-8-methoxy-3H-cyclopenta[c] quinolin-4-yl)benzoic acid*

The following compound was prepared using the same methodology as in intermediate compound 41 using 4-formylbenzoic acid (3.33 mmol, 500 mg) and *p*-anisidine (3.33 mmol, 411.5 mg). Solvent was evaporated and purification of the crude material (1:8 mixture of exo/endo regioisomers) by flash chromatography on silica gel using an elution of 40% ethylacetate in hexanes afforded the endo-isomer. (550 mg. Yield:51%)

Endo- isomer: *4- ((3aS, 4R, 9bR)- 3a, 4, 5, 9b- tetrahydro- 8- methoxy- 3H- cyclopenta [c] quinolin- 4- yl) benzoic acid*

$^1$H NMR (400 MHz, DMSO- $d_6$) $\delta$ 7.94 (2H, d, *J*=8.4Hz), 7.57 (2H, d, *J*=8.4Hz), 6.69 (1 H, d, *J*=8.8Hz), 6.63 (1 H, d, *J*=2.8Hz), 6.54 (1 H, dd, *J*=2.8 & 8.8Hz), 5.87- 5.86 (1 H, m), 5.57 (1 H, da, *J*=4.4Hz), 5.36 (1 H, s), 4.51 (1 H, d, *J*=2.8Hz), 4.04- 4.01 (1 H, m), 3.65 (3H, s), 2.95- 2.92 (1 H, m), 2.44- 2.37 (1 H, m), 1.62- 1.56 (1 H, m) .

LC- MS: $t_R$ = 4.40 $[M+H]^+$ = 322 (method 2)

***Intermediate compound 46: 4-(6-cyclopropyl-3a,4,5,9b-tetrahydro-3H-cyclopenta[c]quinolin-4-yl)benzoic acid***

The following compound was prepared using the same methodology as in intermediate compound 41 using 4-formylbenzoic acid (1.88 mmol, 282 mg) and 2-cyclopropylbenzamine (**intermediate compound 2**) (1.88 mmol, 250 mg). Solvent was evaporated and purification of the crude material by flash chromatography on silica gel using an elution

of 30% ethylacetate in hexanes afforded the endo-isomer. (460 mg. Yield:73%)

$^1$H NMR (400 MHz, DMSO- $d_6$) δ 7.97 (2H, d, *J*=8.4Hz), 7.63 (2H, d, *J*= 8Hz), 6.9 (1 H, d, *J*=7.6Hz), 6.75 (1 H, d, *J*=7.2Hz), 6.59 (1 H, t, *J*=4.6Hz), 5.58 (1 H, m), 5.56 (1 H, m), 4.77 (1 H, s), 4.68 (1H, m), 4.08 (1 H, d, *J*= 8.4Hz), 3.01- 2.99 (1 H, m), 2.44- 2.38 (1 H, m), 1.79- 1.77 (1 H, m), 1.65- 1.59 (1 H, m), 0.86- 0.83 (2H, m), 0.59- 0.57 (1 H, m), 0.42- 0.40 (1 H, m) .

LC- MS: $t_R$ = 3.72 $[M+H]^+$ = 332 (method 3)

***Intermediate compound 47 and 48: 4-(8-cyclopropyl-3a, 4, 5, 9b-tetrahydro-3H-cyclopenta[c]quinolin-4-yl)benzoic acid and 4-(3a, 4, 5, 9b-tetrahydro-8-propyl-3H-cyclopenta[c]quinolin-4-yl)benzoic acid***

The following compound was prepared using the same methodology as in intermediate compound 41 using 4-formylbenzoic acid (1.2 mmol, 181 mg) and 4-cyclopropylbenzenamine (**intermediate compound 3,** method 1) (1.2 mmol, 159 mg). Solvent was evaporated and purification of the crude material (1:9 mixture of exo/endo regioisomers of **47**) by flash chromatography on silica gel using an elution of 29% ethylacetate in hexanes afforded a mixture of endo-exo isomer cyclopropyl compound **47** (80%) and propyl compound **48** (17%). (220 mg. Yield:55%).

4- (8- cyclopropyl- 3a, 4, 5, 9b- tetrahydro- 3H- cyclopenta [c] quinolin- 4- yl) benzoic acid (**47**)

LC- MS: $t_R$ = 5.17 $[M+H]^+$ = 332 (method 2)

4- (3a, 4, 5, 9b- tetrahydro- 8- propyl- 3H- cyclopenta [c] quinolin- 4- yl) benzoic acid (**48**)

C- MS: $t_R$ = 5.33 $[M+H]^+$ = 334 (method 2)

***Intermediate compound 49: 4-(8-fluoro-3a, 4, 5, 9b-tetrahydro-3H-cyclopenta[c]quinolin-4-yl)benzoic acid***

The following compound was prepared using the same methodology as in intermediate compound 41 using 4-formylbenzoic acid (1.35 mmol, 203 mg) and 4-fluoroaniline (1.35 mmol, 150 mg). Solvent was evaporated and purification of the crude material by flash chromatography on silica gel using an elution of 50% ethylacetate in hexanes afforded the title compound. (339 mg.Yield:81%).

LC- MS: $t_R$ = 4.80 $[M+H]^+$ = 310 (method 2)

***Intermediate compound 50: 4-((3aS,4R,9bR)-3a,4,5,9b-tetrahydro-8-phenyl-3H-cyclopenta[c]quinolin-4-yl)benzoic acid***

The following compound was prepared using the same methodology as in intermediate compound 41 using 4-phenylaniline (1 mmol). Solvent was evaporated and purification of the crude material by flash chromatography on silica gel using an elution of 80% ethylacetate in hexanes afforded the endo-isomer as a white solid which was precipitated in dichloromethane, filtered and dried in vacuum to give the title compound (113 mg, 30%).

$^1$H NMR (400 MHz, DMSO- $d_6$) δ 12.88 (1 H, brs), 7.94 (2H, d, *J* = 8.4 Hz), 7.59- 7.54 (4H, m), 7.37 (2H, t, *J* = 7.6 Hz), 7.31 (1 H, d, *J* = 2.0 Hz ), 7.24- 7.20 (2H, m), 6.82 (1 H, d, *J* = 8.0 Hz), 5.97- 5.96 (1 H, m), 5.87 (1 H, brs), 5.56 (1 H, d, *J* = 4.8 Hz), 4.64 (1 H, d, *J* = 2.8 Hz), 4.11 (1 H, d, *J* = 8.4 Hz), 2.97 (1 H, q, *J* = 8.8 Hz), 2.44- 2.37 (1 H, m), 1.60 (1 H, dd, J = 16 & 9.6 Hz) .

LC- MS: $t_R$ = 10.92 $[M+H]^+$= 368 (method 1)

***Intermediate compound 51: 4-(8-tert-butyl-3a,4,5,9b-tetrahydro-3H-cyclopenta[c]quinolin-4-yl)benzoic acid***

The following compound was prepared using the same methodology as in intermediate compound 41 using 4-formylbenzoic acid (1 mmol, 150 mg) and 4-tert-butylaniline (1 mmol, 150 mg). Solvent was evaporated and purification of the crude material by flash chromatography on silica gel using an elution of 50% ethylacetate in hexanes afforded the title compound. (325 mg. Yield: 93%).

LC- MS: $t_R$ = 5.37 $[M+H]^+$ = 348 (method 2)

***Intermediate compound 52: 4-((3aS,4R,9bR)-8-ethyl-3a,4,5,9b-tetrahydro-3H-cyclopenta[c]quinolin-4-yl)benzoic acid***

The following compound was prepared using the same methodology as in intermediate compound 41 using 4-ethylaniline (1 mmol). Solvent was evaporated and purification of the crude material by flash chromatography on silica gel using an elution of 40% ethylacetate in hexanes afforded the endo-isomer as a white solid which was precipitated in dichloromethane, filtered and dried in vacuum to give the title compound (230 mg, 53%).

LC- MS: $t_R$ = 4.18 $[M+H]^+$= 320 (method 2)

***Intermediate compound 53: 4-((3aS,4R,9bR)-8-carbamoyl-3a,4,5,9b-tetrahydro-3H-cyclopenta[c]quinolin-4-yl) benzoic acid***

The following compound was prepared using the same methodology as in intermediate compound 41 using 4-aminobenzamide (1 mmol). Solvent was evaporated and purification of the crude material by flash chromatography on silica gel using an elution of 10% methanol in ethylacetate afforded the endo-isomer as a white solid (210 mg, 90%).

LC- MS: $t_R$ = 2.33 $[M+H]^+$= 320 (method 2)

***Intermediate compound 54: 4-((3aS,4R,9bR)-3a,4,5,9b-tetrahydro-8-isopropyl-3H-cyclopenta[c]quinolin-4-yl) benzoic acid***

The following compound was prepared using the same methodology as in intermediate compound 41 using 4-isopropylaniline (1 mmol). Solvent was evaporated and purification of the crude material by flash chromatography on silica gel using an elution of 40% ethylacetate in hexanes afforded the endo-isomer as a white solid (236 mg, 70%).

LC- MS: $t_R$ = 4.40 $[M+H]^+$= 334 (method 2)

***Intermediate compound 55: 4-((3aS,4R,9bR)-8-cyano-3a,4,5,9b-tetrahydro-3H-cyclopenta[c]quinolin-4-yl)benzoic acid***

The following compound was prepared using the same methodology as in intermediate compound 41 using 4-cyanoaniline (1 mmol). Solvent was evaporated and purification of the crude material by flash chromatography on silica gel using an elution of 60% ethylacetate in hexanes afforded the endo-isomer as a white solid (180 mg, 57%).

LC- MS: $t_R$ = 3.33 $[M+H]^+$= 317 (method 2)

***Intermediate compound 56: 4-((3aS,4R,9bR)-8-[(N-methylsulfonyl)amino]-3a,4,5,9b-tetrahydro -3H-cyclopenta [c]quinolin-4-yl)benzoic acid***

The following compound was prepared using the same methodology as in intermediate compound 41 using 4-formylbenzoic acid (2.1 mmol, 0.32 g) and **intermediate compound 9** (2.1 mmol, 0.4 g). Solvent was evaporated and purification of the crude material by flash chromatography on silica gel using an elution of 40% ethylacetate in hexanes afforded the title compound as a solid slightly impurified, which was purified again by reverse $C_{18}$ chromatography using water (0.1% formic acid) and acetonitrile (0.1% formic acid) as eluents to give 297 mg of the pure title endo-isomer. (Yield= 43%)

$^1H$ NMR (400 MHz, DMSO- $d_6$) $\delta$ 9.02 (1 H, brs), 7.94 (2H, d, *J* = 8.4 Hz), 7.56 (2H, d, *J* = 8.4 Hz), 6.87 (1 H, d, *J* = 2.4 Hz), 6.79 (1 H, dd, *J* = 2.4 & 8.8 Hz), 6.71 (1 H, d, *J* = 8.8 Hz), 5.81- 5.77 (1 H, m), 5.73 (1 H, brs), 5.59 (1 H, d, *J* = 4.8 Hz), 4.59 (1 H, d, *J* = 3.2 Hz), 4.03 (1 H, d, *J*= 8.4 Hz), 3.00- 2.91 (1 H, m), 2.85 (3H, s), 2.43- 2.63 (1 H, m), 1.59 (1 H, dd, *J* = 15.6 & 6.4 Hz) .

LC- MS: $t_R$ =7.22 $[M+H]^+$= 385 (method 1)

***Intermediate compound 57: 4-((3aS,4R,9bR)-8-[(N-methyl-N-methylsulfonyl)amino]-3a,4,5,9b-tetrahydro-3H-cyclopenta[c]quinolin-4yl) benzoic acid***

The following compound was prepared using the same methodology as in intermediate compound 41 using 4-formylbenzoic acid (1.1 mmol, 0.17 g) and **intermediate compound 11** (1.1 mmol, 0.23 g). Solvent was evaporated and purification of the crude material by flash chromatography on silica gel using an elution of 9% methanol in dichloromethane afforded the pure title endo-isomer as a solid (327 mg. Yield=71 %).

$^1H$ NMR (400 MHz, DMSO- $d_6$) $\delta$ 7.95 (2H, d, *J* = 8 Hz), 7.57 (2H, d, *J* = 8 Hz), 7.05 (1 H, d, *J* = 2.4 Hz), 6.93 (1 H, dd, *J* = 2.4 & 8.4 Hz), 6.74 (1 H, d, *J* = 8.4 Hz), 5.91 (1 H, s), 5.89- 5.86 (1 H, m), 5.58 (1 H, d, *J* = 4.4 Hz), 4.63 (1 H, d, *J* = 2.8 Hz), 4.04 (1 H, d, *J* = 8.4 Hz), 3.14 (3H, s), 3.00- 2.91 (1 H, m), 2.88 (3H, s), 2.42- 2.32 (1 H, m), 1.63-1.56 (1 H, m) .

LC- MS: $t_R$ =3.47 $[M+H]^+$= 399 (method 3)

***Intermediate compound 58: 3-((3aS,4R,9bR)-8-bromo-3a,4,5,9b-tetrahydro-3H-cyclopenta[c]quinolin-4-yl)benzoic acid***

The following compound was prepared using the same methodology as in intermediate compound 41 using 3-formylbenzoic (1mmol) acid and 4-bromoaniline (1 mmol). Solvent was evaporated and purification of the crude material (1:17 mixture of exo/endo regioisomers) by flash chromatography on silica gel using an elution of 40% ethylacetate in

hexanes afforded the endo-isomer as a solid which was precipitated in acetonitrile/hexane, filtered and dried in vacuum to give the title compound (110 mg. Yield: 30%).

$^1$H NMR (400 MHz, DMSO- $d_6$) δ 12.95 (1 H, brs), 8.02 (1 H, s), 7.84 (1 H, d, *J* = 7.6 Hz), 7.66 (1 H, d, *J* = 7.6 Hz), 7.48 (1 H, t, *J* = 7.6 Hz), 7.15 (1H, d, *J* = 2 Hz), 7.01 (1 H, dd, *J* = 8.8 & 2.4 Hz), 6.69 (1 H, d, *J*= 8.8 Hz), 5.93 (1 H, s), 5.89- 5.85 (1 H, m), 5.57 (1 H, d, *J* = 4.8 Hz), 4.58 (1 H, d, *J* = 2.8 Hz), 4.03- 4.00 (1 H, m), 2.94- 2.88 (1 H, m), 2.39- 2.34 (1 H, m), 1.57 (1 H, dd, *J* = 15.6 & 8.8 Hz) .

LC- MS: $t_R$ = 7.88 $[M+H]^+$= 370/372 (method 1)

***Intermediate compound 59: 4-((3aS,4R,9bR)-3a,4,5,9b-tetrahydro-7,8-dimethoxy-3H-cyclopenta[c]quinolin-4-yl) benzoic acid***

The following compound was prepared using the same methodology as in intermediate compound 41 using 4-formylbenzoic acid (1.6 mmol, 250 mg) and 3,4-dimethoxyaniline (1.6 mmol). Solvent was evaporated and purification of the crude material by flash chromatography on silica gel using an elution of 70% EtAcO in hexanes afforded the endo-isomer as a white solid (243 mg, 43%).

$^1$H NMR (400 MHz, DMSO- $d_6$) : δ 7.93 (2H, d, *J* = 8.4 Hz), 7.54 (2H, d, *J* = 8.4 Hz), 6.62 (1 H, s), 6.44 (1 H, s), 5.88 (1 H, m), 5.54 (1 H, d, *J* = 4.8 Hz), 5.34 (1 H, s), 4.51 (1 H, d, *J*= 2.4 Hz), 3.95 (1 H, d, *J*= 8.8 Hz), 3.65 (6H, s), 2.95- 2.89 (1 H, m), 2.41- 2.32 (1 H, m), 1.57 (1 H, dd, *J*= 15.5 & 6.4 Hz) .

LC- MS: $t_R$ = 3.68 $[M+H]^+$= 352 (method 3)

***Intermediate compound 60 and 61: 4-(7-(trifluoromethyl)-3a,4,5,9b-tetrahydro-8-methyl-3H-cyclopenta[c]quinolin-4-yl)benzoic acid (60) & 4-(9-(trifluoromethyl)-3a,4,5,9b-tetrahydro-8-methyl-3H-cyclopenta[c]quinolin-4-yl) benzoic acid (61)***

The following compounds were prepared using the same methodology as in intermediate compound 41 using 4- formylbenzoic acid (1.42 mmol, 214 mg) and 5- amino- 2- methylbenzotrifluoride (1.42 mmol, 250mg) . Solvent was evaporated and purification of the crude material by flash chromatography on silica gel using an elution with a gradient from 30% to 100% of ethylacetate in hexanes afforded 475 mg of a 1: 1 mixture of 8, 9- disustituted (61) & 7, 8- disustituted (60) regioisomers as a solid. (89%)

LC- MS: $t_R$ = 4.50 $[M+H]^+$= 374 (method 3) (Both regiosiomers)

***Intermediate compound 62: 4-((3aS,4R,9bR)-3a,4,5,9b-tetrahydro-7-methoxy-8-methyl-3H-cyclopenta[c]quinolin-4-yl)benzoic acid***

The following compound was prepared using the same methodology as in intermediate compound 41 using 4-formylbenzoic acid (1.45 mmol, 220 mg) and 2- methoxy- 4- aminotoluene (1.45 mmol, 200 mg) . Solvent was evaporated and purification of the crude material by flash chromatography on silica gel using an elution of 35% ethylacetate in hexanes afforded the title compound as the major product of a 1: 8 mixture of endo- 8, 9- disustituted & endo- 7, 8- disustituted regioisomers as a solid (162 mg, 33%)

4- ((3aS, 4R, 9bR)- 3a, 4, 5, 9b- tetrahydro- 7- methoxy- 8- methyl- 3H- cyclopenta [c] quinolin- 4- yl) benzoic acid (7, 8- disustituted regioisomer)

LC- MS: $t_R$ = 4.32 $[M+H]^+$= 336 (method 3)

4- ((3aS, 4R, 9bR)- 3a, 4, 5, 9b- tetrahydro- 9- methoxy- 8- methyl- 3H- cyclopenta [c] quinolin- 4- yl) benzoic acid (8, 9- disutituted regioisomer)

LC- MS: $t_R$ = 4.17 $[M+H]^+$= 336 (method 3)

***Intermediate compound 63: 4-((3aS,4R,9bR)-3a,4,5,9b-tetrahydro-6,8-dimethyl-3H-cyclopenta[c]quinolin-4-yl) benzoic acid***

The following compound was prepared using the same methodology as in intermediate compound 41 using 4-formylbenzoic acid (2.06 mmol, 310 mg) and 2,4-dimethylaniline (2.06 mmol, 250 mg). Solvent was evaporated and purification of the crude material by flash chromatography on silica gel using an elution of 40% ethylacetate in hexanes afforded the title endo-isomer as a solid (467 mg, 71%).

$^1$H NMR (400 MHz, DMSO- $d_6$) : δ 7.95 (2H, d, *J*= 8 Hz), 7.60 (2H, d, *J*= 8.4 Hz), 6.69 (1 H, s), 6.63 (1 H, s), 5.85- 5.83 (1 H, m), 5.53 (1 H, d, *J* = 5.2 Hz), 4.57 (1 H, d, *J* = 2.8 Hz), 4.47 (1 H, s), 4.04- 4.01 (1 H, m), 2.96- 2.88 (1 H, m), 2.41- 2.34 (1 H, m), 2.13 (3H, s), 2.10 (3H, s), 1.60- 1.54 (1 H, m) .

LC- MS: $t_R$ = 4.55 $[M+H]^+$= 320 (method 3)

***Intermediate compound 64 and 65: 4-((3aS,4R,9bR)-9-chloro-3a,4,5,9b-tetrahydro-8-methyl-3H-cyclopenta[c]quinolin-4-yl)benzoic acid (64) and 4-((3aS,4R,9bR)-7-chloro-3a,4,5,9b-tetrahydro-8-methyl-3H-cyclopenta[c]quinolin-4-yl)benzoic acid (65)***

The following compound was prepared using the same methodology as in intermediate compound 41 using 4-formylbenzoic acid (1.41 mmol, 212 mg) and 3- chloro- 4- methylaniline (1.41 mmol, 200 mg) . Solvent was evaporated and the crude material (1: 1, 8 mixture of endo- 8, 9- disustituted & endo- 7, 8- disustituted regioisomers) was purified by flash chromatography on silica gel using an elution with a gradient from 35% to 60% of ethylacetate in hexanes. Only high purity fractions of each regioisomer were collected giving two enriched fractions with each regioisomer, which were purified again by precipitation in acetonitrile/ drops of water to give 58 mg of pure 4- ((3aS, 4R, 9bR)- 9- chloro- 3a, 4, 5, 9b- tetrahydro- 8- methyl- 3H- cyclopenta [c] quinolin- 4- yl) benzoic acid (**64**) and 88 mg of pure 4- ((3aS, 4R, 9bR)- 7- chloro- 3a, 4, 5, 9b- tetrahydro- 8- methyl- 3H- cyclopenta [c] quinolin- 4- yl) benzoic acid (**65**) . (Overall yield=30%) .

4- ((3aS, 4R, 9bR)- 9- chloro- 3a, 4, 5, 9b- tetrahydro- 8- methyl- 3H- cyclopenta [c] quinolin- 4- yl) benzoic acid (8, 9- disustituted isomer **64**)

$^1$H NMR (400 MHz, DMSO- $d_6$) $\delta$ 7.93 (2H, d, *J* = 8 Hz), 7.51 (2H, d, *J* = 8.4 Hz), 6.90 (1 H, d, *J* = 8 Hz), 6.70 (1 H, d, *J* = 8 Hz), 5.97 (1 H, d, *J* = 3.6 Hz), 5.77 (1 H, s), 5.62 (1 H, d, *J* = 3.6 Hz), 4.47 (1 H, d, *J* = 2.8 Hz), 4.23 (1 H, d, *J* = 8.4 Hz), 3.17- 3.10 (1 H, m), 2.50 (1 H, m), 2.19 (3H, s), 1.69- 1.63 (1 H, m) .

LC- MS: $t_R$ = 5.37 $[M+H]^+$= 340 (method 2)

4- ((3aS, 4R, 9bR)- 7- chloro- 3a, 4, 5, 9b- tetrahydro- 8- methyl- 3H- cyclopenta [c] quinolin- 4- yl) benzoic acid (7, 8- disustituted isomer **65**)

$^1$H NMR (400 MHz, DMSO- $d_6$) $\delta$ 7.92 (2H, d, *J* = 8.4 Hz), 7.49 (2H, d, *J* = 8.4 Hz, ), 6.95 (1 H, s), 6.79 (1 H, s), 5.86- 5.84 (1 H, m), 5.80 (1 H, s), 5.57 (1 H, d, *J* = 4.8 Hz), 4.55 (1 H, d, *J* = 2.8 Hz), 3.98 (1 H, d, *J* = 9.2 Hz), 2.96- 2.89 (1 H, m), 2.41- 2.32 (1 H, m), 2.16 (3H, s), 1.62- 1.56 (m, 1H) .

LC- MS: $t_R$ = 5.32 $[M+H]^+$= 340 (method 2)

***Intermediate compound 66: methyl 4-(3a,4,5,9b-tetrahydro-8-methyl-3H-cyclopenta[c]quinolin-4-yl)benzoate***

The following compound was prepared using the same methodology as in intermediate compound 41 using methyl 4-formylbenzoate (5 mmol) and 4-methylaniline (5 mmol). Solvent was evaporated and purification of the crude material by flash chromatography on silica gel using an elution of 33% ethylacetate in hexanes afforded a mixture of exo/endo regioisomers (1:17) of the title compound (1.09 mg, 68%).

Endo- Isomer: Methyl 4- ((3a*S*, 4*R*, 9b*R*)- 3a, 4, 5, 9b- tetrahydro- 8- methyl- 3H- cyclopenta [c] quinolin- 4- yl) benzoate $^1$H NMR (400 MHz, DMSO- $d_6$) $\delta$ 7.94 (2H, d, *J* = 8.4 Hz), 7.55 (2H, d, *J* = 8.0 Hz), 6.79 (1 H, s), 6.68 (1 H, dd, *J* = 8.0 & 1.6 Hz), 6.62 (1 H, d, *J* = 8.4 Hz), 5.83- 5.81 (1 H, m), 5.53 (1 H, d, *J* = 4.8 Hz), 5.49 (1 H, brs), 4.54 (1 H, d, *J* = 2.8 Hz), 4.00 (1 H, d, *J*= 8.4 Hz), 3.84 (3H, s), 2.90 (1 H, m), 2.41- 2.33 (1 H, m), 2.13 (3H, s), 1.56 (1 H, dd, *J* = 15.2 & 8.8 Hz) .

LC- MS: $t_R$ = 4.40 $[M+H]^+$= 320 (method 2)

***Intermediate compound 67: methyl 4-(2,3,3a,4,5,9b-hexahydro-8-methyl-1H-cyclopenta[c]quinolin-4-yl)benzoate***

**The intermediate compound 66** (1.09 g, 3.41 mmol) was dissolved in EtOH and THF (1:1, 50 mL) and platinum dioxide (5% w/w, 50 mg) was added. The solution was hydrogenated at r.t., for 1 h. HPLC analysis showed total conversion. Reaction mixture was filtered and filter washed with THF. Solvent was evaporated and purification of the crude material using reverse $C_{18}$ chromatography using water (0.1% formic acid) and acetonitrile (0.1% formic acid) as eluents to give 662 mg a mixture of exo/endo regioisomers (1:17) of the title compound (68%).

Exo- Isomer LC- MS: $t_R$ = 5.27 $[M+H]^+$= 322 (method 2)

Endo- Isomer LC- MS: $t_R$ = 5.45 $[M+H]^+$= 322 (method 2)

***Intermediate compound 68: methyl 4-((3aS,4R,9bR)-2,3,3a,4,5,9b-hexahydro-5,8-dimethyl-1H-cyclopenta[c]quinolin-4-yl)benzoate***

To a solution of **intermediate compound 67** (0.311 mmol, 100 mg) dissolved in DMF (5 mL) and cooled at 0ºC was added 95% NaH (0.33 mmol, 8.2 mg) and the resulting solution was stirred for 1 h at room temperature. Iodomethane (0.33 mmol, 0.022 mL) was added and the mixture was stirred 16 h at room temperature. Total conversion was not achieved, then 95% NaH (0.31 mmol, 8 mg) and iodomethane (0.622 mmol, 0.039 mL) were added and the mixture was stirred 24 h at room temperature. 95% NaH (0.46 mmol, 12 mg) and iodomethane (1.2 mmol, 0.078 mL) were added again and the mixture was stirred 5 days at room temperature. Then, 95% NaH (0.46 mmol, 12 mg) and

iodomethane (1.2 mmol, 0.078 mL) were added and the mixture was stirred 24 h at room temperature. Solvent was evaporated and purification of the crude material by flash chromatography on silica gel using an elution of 3% ethylacetate in hexanes afforded the title compound (50 mg. Yield:50%).

LC- MS: $t_R$ = 5.78 $[M+H]^+$= 336 (method 2)

***Intermediate compound 69: methyl 4-((3aS,4R,9bR)-5-acetyl-2,3,3a,4,5,9b-hexahydro-8-methyl-1H-cyclopenta [c]quinolin-4-yl)benzoate***

The **intermediate compound 67** (0.31 mmol, 100 mg) was dissolved in anhydrous tetrahydrofurane (2 mL), pyridine (3.6 eq., 1.11 mmol, 0.09 mL), acetyl chloride (3 eq, 0.93 mmol, 0.07 mL) and acetyl anhydride (2eq., 0.65 mmol, 0.07 mL) were added at room temperature and the resulting solution was stirred at r.t. for 5h. Solvent was evaporated and the crude residue was dissolved in ethyl acetate and saturated aqueous sodium carbonate solution. The organic layer was extracted and washed with saturated aqueous sodium carbonate solution, 1 N HCl and brine. Then it was dried over anhydrous sodium sulphate, filtered and solvent evaporated to give 110 mg (98%) of the endo-isomer, which was used in the next step without further purification.

$^1H$ NMR (400 MHz, $CDCl_3$) $\delta$ 7.81 (2H, d, *J* = 8.4 Hz), 7.14 (1 H, s), 7.03- 6.99 (4H, m), 6.14 (1 H, brs), 3.85 (3H, s), 3.15- 3.03 (2H, m), 2.38 (3H, s), 2.18 (3H, s), 2.19- 2.12 (1 H, m), 1.90- 1.86 (1 H, m), 1.60 (1 H, brs), 1.52- 1.40 (2H, m), 0.92- 0.86 (1 H, m) .

LC- MS: $t_R$ = 5.00 $[M+H]^+$= 364 (method 2)

***Intermediate compound 70: 4-((6-methylpyridin-3-ylimino)methyl)benzoic acid***

A solution of 4- formylbenzoic acid (1.39 mmol, 209 mg) and 6- methylpyridin- 3- amine (1.39 mmol, 150 mg) in dry chloroform (15 mL) with molecular sieves was stirred at room temperature 48 h. The resulting solution was filtered and concentrated in vacuum to give the compound 4- ((6- methylpyridin- 3- ylimino) methyl) benzoic acid. The product is unstable and was used without further purification in the next step.

LC- MS: $t_R$ = 2.20 $[M+H]^+$ = not ion (method 2)

***Intermediate compound 71: 4-(6, 6a, 7, 9a-tetrahydro-2-methyl-5H-cyclopenta[c][1,5]naphthyridin-6-yl)benzoic acid***

To a solution of cyclopenta- 1, 3- diene (6.9 mmol, 563 mL) and $BF_3{\cdot}Et_2O$ (2.76 mmol, 350mL) were added to a solution of 4- ((6- methylpyridin- 3- ylimino) methyl) benzoic acid (**intermediate compound 70**) (1.38 mmol, 334 mg) in dry ACN (15 mL) . The mixture was stirred 48 h at room temperature. The resulting solution was evaporated and the crude product was purified using reverse $C_{18}$ chromatography using water (0.1% formic acid) and acetonitrile (0.1% formic acid) as eluents to give the title compound. (42 mg. Yield: 10%)

Endo- isomer **71**:

$^1H$ NMR (400 MHz, DMSO- $d_6$) $\delta$ 7.95 (2H, d, *J*=8.4Hz), 7.58 (2H, d, *J*=8Hz), 6.98 (1 H, d, *J*=8.4Hz), 6.78 (1 H, d, *J*= 8.4Hz), 5.86- 5.85 (1 H, m), 5.71 (1 H, s), 5.61- 5.59 (1 H, m), 4.62 (1 H, d, *J*=2.8Hz), 4.04 (1 H, d, *J*=7.6Hz), 3.06- 2.99 (1 H, m), 2.44- 2.37 (1 H, m), 2.29 (3H, s), 1.67- 1.61 (1 H, m) .

LC- MS: $t_R$ = 2.60 $[M+H]^+$ = 307 (method 2)

***Intermediate compound 72: 4-(6,6a,7,9a-tetrahydro-2-methyl-5H-cyclopenta[c][1,8]naphthyridin-6-yl)benzoic acid***

The following compound was prepared using the same methodology as in intermediate compound 41 using 2-amino- 5- methylpyridine (1.8 mmol, 0.20 g) . Total conversion was not achieved, additional scandium trifluoromethanesulfonate (0.5 eq, 0.92 mmol, 0.45 g) was added and the mixture reaction was left to stir at 65ºC 16 h. HPLC analysis showed total conversion. Solvent was evaporated and purification of the crude material by flash chromatography on silica gel using an elution of 10% methanol in dichloromethane afforded the pure title compound. (323 mg. Yield= 57%)

LC- MS: $t_R$ =2.20 $[M+H]^+$= 307 (method 3)

***Intermediate compound 73: methyl 2-(4-(3a, 4, 5, 9b-tetrahydro-8-methyl-3H-cyclopenta[c]quinolin-4-yl)phenyl) acetate***

The following compound was prepared using the same methodology as in intermediate compound 41 using methyl 2- (4- formylphenyl) acetate (intermediate **compound 15)** (0.84 mmol, 150 mg) and *p*- toluidine (0.84 mmol, 91 mg) . Solvent was evaporated and purification of the crude material (2: 8 mixture of exo/ endo regioisomers) by flash

chromatography on silica gel using an elution of 20% ethylacetate in hexanes afforded the title compound. (115 mg. Yield: 41 %) .

LC- MS: $t_R$ = 5.07 $[M+H]^+$ = 334 (method 2)

***Intermediate compound 74: methyl 2-(4-(2, 3, 3a, 4, 5, 9b-hexahydro-8-methyl-1H-cyclopenta[c]quinolin-4-yl) phenyl)acetate***

The following compound was prepared using the same methodology as in intermediate compound 67 using **intermediate compound 73** (0.345 mmol, 115mg) for 1 h. Purification of the crude material using reverse $C_{18}$ chromatography using water (0.1% formic acid) and acetonitrile (0.1% formic acid) as eluents afforded methyl 2- (4- (2, 3, 3a, 4, 5, 9b- hexahydro- 8- methyl- 1*H*- cyclopenta [c] quinolin- 4- yl) phenyl) acetate (74 mg. Yield: 64%) .

LC- MS: $t_R$ = 5.25 $[M+H]^+$ = 336 (method 2)

***Intermediate compound 75: 3-(4-(3a, 4, 5, 9b-tetrahydro-8-methyl-3H-cyclopenta[c]quinolin-4-yl)phenyl)propanoic acid***

The following compound was prepared using the same methodology as in intermediate compound 41 using 3- (4- formylphenyl) propanoic acid **(intermediate compound 13)** (0.415 mmol, 74 mg) and *p*- toluidine (0.145 mmol, 45 mg) . Solvent was evaporated and purification of the crude material (1: 6 mixture of exo/ endo regioisomers) by flash chromatography on silica gel using an elution of 50% ethylacetate in hexanes afforded the title compound. (42 mg. Yield: 30%) .

LC- MS: $t_R$ = 4.98 $[M+H]^+$ = 334 (method 2)

***Intermediate compound 76: 4-(3a, 4, 5, 9b-tetrahydro-8-methyl-3H-cyclopenta[c]quinolin-4-yl)-2-methylbenzoic acid***

The following compound was prepared using the same methodology as in intermediate compound 41 using 4- formyl- 2- methylbenzoic acid **(intermediate compound 16)** (0.4 mmol, 65 mg) and *p*- toluidine (0.4 mmol, 43 mg) . Solvent was evaporated and purification of the crude material by flash chromatography on silica gel using an elution of 50% ethylacetate in hexanes afforded the title compound. (61 mg. Yield: 47%) .

LC- MS: $t_R$ = 5.12 $[M+H]^+$ = 320 (method 2)

***Intermediate compound 77: 4-(3a, 4, 5, 9b-tetrahydro-8-methyl-3H-cyclopenta[c]quinolin-4-yl)-3-methylbenzoic acid***

The following compound was prepared using the same methodology as in intermediate compound 41 using 4- formyl- 3- methylbenzoic acid **(intermediate compound 18)** (0.55 mmol, 90 mg) and *p*- toluidine (0.55 mmol, 59 mg) . Solvent was evaporated and purification of the crude material (2: 8 mixture of exo/ endo regioisomers) by flash chromatography on silica gel using an elution of 20% ethylacetate in hexanes afforded the title compound. (115 mg. Yield: 65%) .

LC- MS: $t_R$ = 5.15 $[M+H]^+$ = 320 (method 2)

***Intermediate compound 78: methyl 4-(3a, 4, 5, 9b-tetrahydro-8-methyl-3H-cyclopenta[c]quinolin-4-yl)-3-methylbenzoate***

The following compound was prepared using the same methodology as in intermediate compound 41 using methyl 4- formyl- 3- methylbenzoate **(intermediate compound 19)** (3.53 mmol, 630 mg) and *p*- toluidine (3.53 mmol, 379 mg) . Solvent was evaporated and purification of the crude material by flash chromatography on silica gel using an elution of 8% ethylacetate in hexanes afforded the title compound. (1 g. Yield: 85%) .

LC- MS: $t_R$ = 4.85 $[M+H]^+$ = 320 (method 2)

***Intermediate compound 79: methyl 4-(2,3,3a, 4, 5, 9b-hexahydro-8-methyl-3H-cyclopenta[c]quinolin-4-yl)-3-methylbenzoate***

The following compound was prepared using the same methodology as in intermediate compound 67 from **intermediate compound 78** for 1 h. Solvent was evaporated and purification of the crude material by precipitation with DMSO/ACN, filtration of the white solid which was washed with ACN, dried under vacuum to give 1:9 exo/endo regioisomers mixture. (746 mg: Yield: 74%).

LC- MS: $t_R$ = 5.15 $[M+H]^+$ = 320 (method 2)

***Intermediate compound 80: 4-(8-chloro-3a, 4, 5, 9b-tetrahydro-3H-cyclopenta[c]quinolin-4-yl)-3-methylbenzoic acid***

The following compound was prepared using the same methodology as in intermediate compound 41 using 4-formyl- 3- methylbenzoic acid **(intermediate compound 18)** (0.46 mmol, 75 mg) and 4- chloroaniline (0.46 mmol, 59 mg) . Solvent was evaporated and purification of the crude material by flash chromatography on silica gel using an elution of 30% ethylacetate in hexanes afforded the title compound. (64 mg. Yield: 41%) .

LC- MS: $t_R$ = 5.23 $[M+H]^+$ = 340 (method 2)

***Intermediate compound 81: 4-(8-fluoro-3a, 4, 5, 9b-tetrahydro-3H-cyclopenta[c]quinolin-4-yl)-3-methylbenzoic acid***

The following compound was prepared using the same methodology as in intermediate compound 41 using 4-formyl- 3- methylbenzoic acid **(intermediate compound 18)** (0.46 mmol, 75 mg) and 4- fluoroaniline (0.46 mmol, 52 mg) . Solvent was evaporated and purification of the crude material by flash chromatography on silica gel using an elution of 40% ethylacetate in hexanes afforded the title compound. (58 mg. Yield: 33%) .

LC- MS: $t_R$ = 4.97 $[M+H]^+$ = 324 (method 2)

***Intermediate compound 82: 4-(3a, 4, 5, 9b-tetrahydro-8-methoxy-3H-cyclopenta[c]quinolin-4-yl)-3-methylbenzoic acid***

The following compound was prepared using the same methodology as in intermediate compound 41 using 4-formyl- 3- methylbenzoic acid **(intermediate compound 18)** (0.91 mmol, 150 mg) and p- anisidine (0.91 mmol, 113 mg) . Total conversion was not achieved after 16 h, then Sc $(OTf)_3$ (0.091 mmol, 45 mg) and cyclopentadiene (4.55 mmol 0.37 mL) were added again and the reaction mixture was stirred 24 h at room temperature. Solvent was evaporated and purification of the crude material by flash chromatography on silica gel using an elution of 38% ethylacetate in hexanes afforded the title compound. (129 mg. Yield: 42%) .

LC- MS: $t_R$ = 4.67 $[M+H]^+$ = 336 (method 2)

***Intermediate compound 83: 4-(8-cyclopropyl-3a, 4, 5, 9b-tetrahydro-3H-cyclopenta[c]quinolin-4-yl)-3-methylbenzoic acid***

The following compound was prepared using the same methodology as in intermediate compound 41 using 4-formyl- 3- methylbenzoic acid **(intermediate compound 18)** (0.68 mmol, 112 mg) and 4- cyclopropylbenzenamine **(intermediate compound 3,** method 2) (0.68 mmol, 91 mg) . Solvent was evaporated and purification of the crude material by flash chromatography on silica gel using an elution of 35% ethylacetate in hexanes afforded the title compound. (90 mg. Yield: 38%) .

LC- MS: $t_R$ = 5.30 $[M+H]^+$ = 346 (method 2)

***Intermediate compound 84: methyl 4-(8-(cyclopropylmethoxy)-3a,4,5,9b-tetrahydro-3H-cyclopenta[c]quinolin-4-yl)-3-methylbenzoate***

The following compound was prepared using the same methodology as in intermediate compound 41 using methyl- 4- formyl- 3- methyl benzoate **(intermediate compound 19)** (1 eq.) and 4- (cyclopropylmethoxy) benzenamine (1 eq) **(intermediate compound 7)** (1 eq) . The reaction mixture was stirred at room temperature for 16h. Then, scandium trifluoromethanesulfonate (0.4 eq) was added and left stirring at room temperature for 60 h. Solvent was evaporated and purification of the crude material by flash chromatography on silica gel using hexane and ethylacetate as eluents to afford 128 mg of a mixture of isomers exo/ endo (1: 4) of the impure title- compound.

LC- MS: $t_R$ = 4.78 & 4.92 $[M+H]^+$= 390 (method 2) .

***Intermediate compound 85: methyl 4-((3aS, 4R, 9bR)-8-(cyclopropylmethoxy)-2,3,3a,4,5,9b-hexahydro-1H-cyclopenta[c]quinolin-4-yl)-3-methylbenzoate***

The following compound was prepared using the same methodology as in intermediate compound 67 from **intermediate compound 84** (128 mg, 0.32 mmol) for 16 h. Solvent was evaporated and purification of the crude material using reverse $C_{18}$ chromatography using water (0.1 % formic acid) and acetonitrile (0.1% formic acid) as eluents to give

34 mg of the endo-isomer (25%).

$^1H$ NMR (400 MHz, $CDCl_3$) $\delta$ 7.88 (1 H, dd, *J* = 8.0 & 1.6 Hz), 7.83 (1 H, s), 7.73 (1 H, d, *J* = 8.0 Hz), 6.73 (1 H, d, *J* = 2.8 Hz), 6.63 (1 H, dd, *J* = 8.4 & 2.8 Hz), 6.54 (1 H, d, *J* = 8.8 Hz), 4.69 (1 H, d, *J* = 3.2 Hz), 3.91 (3H, s), 3.74 (2H, d, *J* = 6.8 Hz), 3.47- 3.42 (1 H, m), 2.52- 2.46 (1 H, m), 2.39 (3H, s), 2.17- 2.14 (1 H, m) 1.79- 1.70 (2H, m), 1.44- 1.38 (1 H, m), 1.28- 1.23 (1 H, m), 1.14- 1.12 (1 H, m), 0.64- 0.60 (2H, m), 0.35- 0.31 (2H, m) .

LC- MS: $t_R$ = 5.03 $[M+H]^+$= 392 (method 2)

***Intermediate compound 86: methyl 4-(3a,4,5,9b-tetrahydro-8-isopropoxy-3H-cyclopenta[c]quinolin-4-yl)-3-methylbenzoate***

The following compound was prepared using the same methodology as in intermediate compound 41 using **intermediate compound 19** (1.32 mmol, 236 mg) and **intermediate compound 5** (1.32 mmol, 200 mg). Solvent was evaporated and purification of the crude material by flash chromatography on silica gel using an elution of 15% ethylacetate in hexanes afforded the title compound as a 1:9 mixture of exo/endo isomers. (322 mg. Yield:64%).

LC- MS: $t_R$ =4.13 $[M+H]^+$= 152 (method 3) (Both isomers)

***Intermediate compound 87 & 88: methyl 4-((3aS,4R,9bR)-2,3,3a,4,5,9b-hexahydro-8-isopropoxy-1H-cyclopenta [c]quinolin-4-yl)-3-methylbenzoate (87) & methyl 4-((3aS,4S,9bR)-2,3,3a,4,5,9b-hexahydro-8-isopropoxy-1H-cyclopenta[c]quinolin-4-yl)-3-methylbenzoate (88)***

The following compounds were prepared using the same methodology as in intermediate compound 67 from **intermediate compound 86** (0.85 mmol, 322 mg) for 1 h. Purification of the crude material (1: 6.5 mixture of exo/ endo isomers) by reverse $C_{18}$ chromatography using water (0.1% formic acid) and acetonitrile (0.1% formic acid) as eluents to give 132 mg of the pure endo- isomer methyl 4- ((3aS, 4R, 9bR)- 2, 3, 3a, 4, 5, 9b- hexahydro- 8- isopropoxy- 1H- cyclopenta [c] quinolin- 4- yl)- 3- methylbenzoate **(87)** and 16.9 mg of the pure exo- isomer methyl 4- ((3aS, 4S, 9bR)- 2, 3, 3a, 4, 5, 9b- hexahydro- 8- isopropoxy- 1H- cyclopenta [c] quinolin- 4- yl)- 3- methylbenzoate **(88)** (Overall yield= 46%) .

Methyl- 4- ((3aS, 4R, 9bR)- 2, 3, 3a, 4, 5, 9b- hexahydro- 8- isopropoxy- 1H- cyclopenta [c] quinolin- 4- yl)- 3- methylbenzoate (endo- isomer **87**) :

LC- MS: $t_R$ =5.05 $[M+H]^+$= 152 (method 2)

Methyl- 4- ((3aS, 4S, 9bR)- 2, 3, 3a, 4, 5, 9b- hexahydro- 8- isopropoxy- 1H- cyclopenta [c] quinolin- 4- yl)- 3- methylbenzoate (exo- isomer **88) :**

LC- MS: $t_R$ =4.82 $[M+H]^+$= 152 (method 2)

***Intermediate compound 89: 3-chloro-4-(3a, 4, 5, 9b-tetrahydro-8-methyl-3H-cyclopenta[c]quinolin-4-yl)benzoic acid***

The following compound was prepared using the same methodology as in intermediate compound 41 using 3-chloro- 4- formylbenzoic acid **(intermediate compound 17)** (1.62 mmol, 300 mg) and *p*- toluidine (1.62 mmol, 173 mg) . Solvent was evaporated and purification of the crude material (1: 9 mixture of exo/ endo regioisomers) by flash chromatography on silica gel using an elution of 35% ethylacetate in hexanes afforded the exo- endo isomer. (305 mg. Yield: 55%) .

LC- MS: $t_R$ = 5.40 $[M+H]^+$ = 340 (method 2)

***Intermediate compound 90: methyl 4-(3a, 4, 5, 9b-tetrahydro-8-methoxy-3H-cyclopenta[c]quinolin-4-yl)-3-methoxybenzoate***

The following compound was prepared using the same methodology as in intermediate compound 41 using methyl 4- formyl- 3- methoxybenzoate **(intermediate compound 20)** (0.72 mmol, 140 mg) and *p*- anisidine (0.72 mmol, 89 mg) . Solvent was evaporated and purification of the crude material (1: 4.5 mixture of exo/ endo regioisomers) by flash chromatography on silica gel using an elution of 2% ethylacetate in hexanes afforded the mixture exo- endo isomer. (193 mg. Yield: 73%) .

LC- MS: $t_R$ = 4.73 $[M+H]^+$ = 366 (method 2)

***Intermediate compound 91: methyl 4-((3aS, 4R, 9bR)-2, 3, 3a, 4, 5, 9b-hexahydro-8-methoxy-1H-cyclopenta[c] quinolin-4-yl)-3-methoxybenzoate***

The following compound was prepared using the same methodology as in intermediate compound 67 from

**intermediate compound 90** (0.53 mmol, 193 mg) . Solvent was evaporated and purification of the crude material using reverse $C_{18}$ chromatography using water (0.1% formic acid) and acetonitrile (0.1% formic acid) as eluents to give endo-isomer methyl 4- ((3a*S*, 4*R*, 9bR*)* - 2, 3, 3a, 4, 5, 9b- hexahydro- 8- methoxy- 1*H*- cyclopenta [*c*] quinolin- 4- yl)- 3-methoxybenzoate (80 mg. Yield: 41%) .

LC- MS: $t_R$ = 4.80 $[M+H]^+$ = 368 (method 2)

***Intermediate compound 92: methyl 4-(3a, 4, 5, 9b-tetrahydro-8-methyl-3H-cyclopenta[c]quinolin-4-yl)-3-methoxybenzoate***

The following compound was prepared using the same methodology as in intermediate compound 41 using methyl 4- formyl- 3- methoxybenzoate **(intermediate compound 20)** (0.72 mmol, 140 mg) and *p*- toluidine (0.72 mmol, 78 mg) . Solvent was evaporated and purification of the crude material (1: 3.6 mixture of exo/ endo regioisomers) by flash chromatography on silica gel using an elution of 8% ethylacetate in hexanes afforded the mixture regioisomer compound. (154 mg. Yield: 61%) .

LC- MS: $t_R$ = 5.18 $[M+H]^+$ = 350 (method 2)

***Intermediate compound 93: methyl 4-((3aS, 4R, 9bR)-2, 3, 3a, 4, 5, 9b-hexahydro-8-methyl-1H-cyclopenta[c] quinolin-4-yl)-3-methoxybenzoate***

The following compound was prepared using the same methodology as in intermediate compound 67 from **intermediate compound 92** (0.44 mmol, 154 mg) The crude material endo- isomer methyl 4- ((3a*S*, 4*R*, 9b*R*)- 2, 3, 3a, 4, 5, 9b- hexahydro- 8- methyl- 1*H*- cyclopenta [*c*] quinolin- 4- yl)- 3- methoxybenzoate was used in the next step without purification (135 mg. Yield: 87%) .

LC- MS: $t_R$ = 5.40 $[M+H]^+$ = 352 (method 2)

***Intermediate compound 94: methyl 3-bromo-4-(3a,4,5,9b-tetrahydro-8-methyl-3H-cyclopenta[c]quinolin-4-yl) benzoate***

The following compound was prepared using the same methodology as in intermediate compound 41 using methyl 3- bromo- 4- formylbenzoate **(intermediate compound 23)** (0.243 mmol, 59 mg) and *p*- toluidine (0.243 mmol, 26 mg) . Solvent was evaporated and purification of the crude material (1: 10 mixture of exo/ endo regioisomers) by flash chromatography on silica gel using an elution of 29% ethylacetate in hexanes afforded the exo- endo isomer. (75 mg. Yield: 40%) .

Endo- Isomer **94:**

$^1H$ NMR (400 MHz, $CDCl_3$) $\delta$ 8.3 (1 H, d, *J*=8Hz), 8.24 (1 H, d, *J*= 1.6Hz), 7.74 (1 H, d, *J*=8Hz), 6.91 (1 H, s), 6.82 (1 H, d, *J*=8Hz), 6.57 (1 H, d, *J*=8Hz), 5.86 (1 H, m), 5.65 (1 H, m), 4.97 (1 H, d, *J*=3.2Hz), 4.13- 4.10 (1 H, m), 3.93 (3H, s), 3.27- 3.24 (1 H, m), 2.65- 2.55 (1 H, m), 2.25 (3H, s), 1.72- 1.66 (1 H, m) .

LC- MS: $t_R$ = 5.53 $[M+H]^+$ = 398- 400 (method 2)

***Intermediate compound 95: methyl 3-bromo-4-((3aS,4R,9bR)-2,3,3a,4,5,9b-hexahydro-8-methyl-1H-cyclopenta [c]quinolin-4-yl)benzoate***

The following compound was prepared using the same methodology as in intermediate compound 67 from **intermediate compound 94** (0.188 mmol, 75 mg) but using platinum dioxide (2.5% w/w, 2 mg) . Purification of the crude material using reverse $C_{18}$ chromatography using water (0.1% formic acid) and acetonitrile (0.1% formic acid) as eluents to give endo- isomer methyl 3- bromo- 4- ((3a*S*, 4*R*, 9b*R*)- 2, 3, 3a, 4, 5, 9b- hexahydro- 8- methyl- 1*H*- cyclopenta [*c*] quinolin- 4- yl) benzoate (54 mg. Yield: 71%)

$^1H$ NMR (400 MHz, DMSO- $d_6$) $\delta$ 8.11 (1H, d, *J*=1.6Hz), 7.99 (1H, dd, *J*=1.6 & 7.6Hz), 7.81 (1 H, d, *J*=8Hz), 6.86 (1 H, s), 6.73 (1 H, d, *J*=7.6Hz), 6.58 (1 H, d, *J*=8.4Hz), 5.60 (1 H, s), 4.68 (1 H, d, *J*=2.8Hz), 3.87 (3H, s), 3.38- 3.35 (1 H, m), 2.61- 2.54 (1 H, m), 2.17 (3H, s), 2.11- 2.06 (1 H, m), 1.71- 1.69 (1 H, m), 1.56- 1.53 (1 H, m), 1.41- 1.37 (2H, m), 0.96- 0.94 (1 H, m) .

LC- MS: $t_R$ = 5.72 $[M+H]^+$ = 400- 402 (method 2)

***Intermediate compound 96: methyl 3-bromo-4-(3a, 4, 5, 9b-tetrahydro-8-methoxy-3H-cyclopenta[c]quinolin-4-yl) benzoate***

The following compound was prepared using the same methodology as in intermediate compound 41 using methyl 3- bromo- 4- formylbenzoate **(intermediate compound 23)** (0.71 mmol, 173 mg) and *p*- anisidine (0.71 mmol,

88 mg) . The crude product was purified by precipitation with acetonitrile to give a white solid methyl 3- bromo- 4- (3a, 4, 5, 9b- tetrahydro- 8- methoxy- 3*H*- cyclopenta [*c*] quinolin- 4- yl) benzoate (1: 6 exo: endo isomer) (161 mg. Yield: 54%)

Endo- Isomer **96:**

$^1$H NMR (400 MHz, DMSO- $d_6$) δ 8.12 (1 H, d, *J*=2Hz), 8.01 (1 H, dd, *J*= 1.6 & 8.4Hz), 7.81 (1 H, d, *J*=8Hz), 6.68- 6.65 (2H, m), 6.56 (1 H, dd, *J*=2.8 & 8.8Hz), 5.88 (1 H, m), 5.58 (1 H, d, *J*=5.2Hz), 5.40 (1 H, s), 4.69 (1 H, d, *J*=2.8Hz), 4.04- 4.02 (1 H, d, *J*=9.2Hz), 3.88 (3H, s), 3.65 (3H, s), 3.11- 3.08 (1 H, m), 2.46- 2.41 (1 H, m), 1.58- 1.54 (1 H, m)

LC- MS: $t_R$ = 5.22 $[M+H]^+$ = 414- 416 (method 2)

***Intermediate compound 97: methyl 3-bromo-4-((3aS, 4R, 9bR)-2, 3, 3a, 4, 5, 9b-hexahydro-8-methoxy-1H-cyclopenta[c]quinolin-4-yl)benzoate***

The following compound was prepared using the same methodology as in intermediate compound 95 from **intermediate compound 96** (0.39 mmol, 161 mg) Purification of the crude material by reverse $C_{18}$ chromatography using water (0.1% formic acid) and acetonitrile (0.1% formic acid) as eluents to give endo- isomer methyl 3- bromo- 4- ((3a*S*, 4*R*, 9b*R*)- 2, 3, 3a, 4, 5, 9b- hexahydro- 8- methoxy- 1*H*- cyclopenta [c] quinolin- 4- yl) benzoate (129 mg. Yield: 79%) .

$^1$H NMR (400 MHz, DMSO- $d_6$) δ 8.11 (1H, d, *J*=1.6Hz), 7.80 (1H, dd, *J*=1.6 & 7.6Hz), 7.82 (1H, d, *J*=8Hz), 6.65- 6.63 (2H, m), 6.57 (1H, dd, *J*=2.4 & 8.4Hz), 5.46 (1 H, s), 4.63 (1 H, d, *J*=3.2Hz), 3.87 (3H, s), 3.66 (3H, s), 3.41- 3.37 (1 H, m), 2.63- 2.60 (1 H, m), 2.12- 2.07 (1 H, m), 1.70- 1.66 (1 H, m), 1.60- 1.54 (1 H, m), 1.41- 1.38 (2H, m), 0.97- 0.94 (1 H, m) .

LC- MS: $t_R$ = 5.43 $[M+H]^+$ = 416- 418 (method 2)

***Intermediate compound 98: 3-ethyl-4-((3aS,9bR)-3a,4,5,9b-tetrahydro-8-methyl-3H-cyclopenta[c]quinolin-4-yl) benzonitrile***

The following compound was prepared using the same methodology as in intermediate compound 41 using the **intermediate compound 25** (0.30 mmol, 48 mg) and p-toluidine (0.30 mmol, 32 mg). Solvent was evaporated and purification of the crude material by flash chromatography on silica gel using an elution of 40% ethylacetate in hexanes afforded the title compound as a 7:1 mixture of endo/exo-isomers as a white solid (71 mg. Yield: 75%).

Endo isomer **98:**

$^1$H (400 MHz, $CDCl_3$) δ 7.79 (1 H, d, *J* = 8 Hz), 7.54 (1 H, dd, *J* = 8 & 1.6 Hz), 7.51 (1 H, d, *J* = 1.6 Hz), 6.91 (1 H, s), 6.84 (1 H, dd, *J* = 8 & 1.6 Hz), 6.57 (1 H, d, *J* = 8 Hz), 5.87 (1 H, m), 5.70 (1 H, m), 4.87 (1 H, d, *J* = 2.8 Hz), 4.11 (1 H, m), 3.01- 2.94 (1 H, m), 2.86- 2.78 (1 H, m), 2.74- 2.62 (2H, m), 2.26 (3H, s), 1.76- 1.69 (1 H, m), 1.29- 1.25 (3H, m) .

LC- MS: $t_R$ = 5.23 $[M+H]^+$= 315 (method 2)

***Intermediate compound 99: 3-ethyl-4-((3aS,9bR)-3a,4,5,9b-tetrahydro-8-methyl-3H-cyclopenta[c]quinolin-4-yl) benzoic acid***

The following compound was prepared using the same methodology as in intermediate compound 41 using the **intermediate compound 26** (0.46 mmol, 82 mg) and using p-toluidine (0.46 mmol, 49 mg). Purification of the crude material by flash chromatography on silica gel using an elution of 40% ethylacetate in hexanes afforded the title compound as a 5.4:1 mixture of endo/exo- isomers as a white solid (104 mg. Yiled: 68%).

Endo isomer **99:**

$^1$H (400 MHz, $CDCl_3$) δ 7.79 (2H, s), 7.69 (1 H, d, J = 8.8 Hz), 6.81 (1 H, s), 6.69 (1 H, dd, J = 8 & 1.6 Hz), 6.62 (1 H, d, J = 8 Hz), 5.85 (1 H, m), 5.57 (1 H, d, J = 4.8 Hz), 5.32 (1 H, s), 4.71 (1 H, d, J = 2.8 Hz), 4.02 (1 H, d, J = 8.8 Hz), 2.92- 2.78 (2H, m), 2.74- 2.64 (2H, m), 2.15 (3H, s), 1.59- 1.53 (1 H, m), 1.22- 1.18 (3H, m) .

LC- MS: $t_R$ = 5.05 $[M+H]^+$= 334 (method 2)

***Intermediate compound 100: 3-ethyl-4-((3aS,9bR)-3a,4,5,9b-tetrahydro-8-methoxy-3H-cyclopenta[c]quinolin-4-yl)benzoic acid***

The following compound was prepared using the same methodology as in intermediate compound 41 using the **intermediate compound 26** (0.67 mmol, 120 mg) and *p*-anisidine (0.67 mmol, 83 mg). Purification of the crude material by flash chromatography on silica gel using an elution of 40% ethylacetate in hexanes afforded the title compound as a 2:1 mixture of endo/exo-isomers as a solid (79 mg. Yield: 34%).

LC- MS (endo- isomer) : $t_R$ = 4.53 $[M+H]^+$= 350 (method 2)

LC- MS (exo- isomer) : $t_R$ = 4.40 $[M+H]^+$= 350 (method 2)

***Intermediate compound 101: methyl 4-((3aS,4S,9bR)-3a,4,5,9b-tetrahydro-8-methyl-3H-cyclopenta[c]quinolin-4-yl)-3,5-dimethylbenzoate***

A catalytic amount of scandium trifluoromethanesulfonate (0.5 eq., 0.31 mmol, 153 mg) in anhydrous acetonitrile (10 mL) was added to a mixture of **intermediate compound 30** (0.62 mmol, 120 mg), *p*-toluidine (0.62 mmol, 67 mg) and fresh distilled ciclopentadiene (5 eq., 3.12 mmol, 0.25 mL). The reaction mixture was stirred at 50°C for 16h. Solvent was evaporated and purification of the crude material by flash chromatography on silica gel using an elution of 10% ethylacetate in hexanes afforded the pure title exo-compound. (144 mg. Yield: 66%).

$^1$H NMR (400 MHz, DMSO- $d_6$) $\delta$ 7.70 (2H, brs), 7.10 (1 H, s), 6.86 (1 H, dd, *J* = 8 & 1.6 Hz), 6.53 (1 H, d, *J* = 7.6 Hz), 5.99- 5.96 (1 H, m), 5.74- 5.70 (1 H, m), 4.36 (1 H, d, *J* = 11.2 Hz), 4.02- 3.98 (1 H, m), 3.92 (3H, s), 3.22- 3.14 (1 H, m), 2.74- 2.68 (3H, brs), 2.50- 2.38 (2H, m), 2.34- 2.23 (3H, brs), 2.29 (3H, s) .

LC- MS: $t_R$ = 5.45 $[M+H]^+$= 348 (method 2)

***Intermediate compound 102: methyl 4-((3aS,4S,9bR)-2,3,3a,4,5,9b-hexahydro-8-methyl-1H-cyclopenta[c]quinolin-4-yl)-3,5-dimethylbenzoate***

The following compound was prepared using the same methodology as in intermediate compound 67 from **intermediate compound 101** (0.41 mmol, 144 mg) . Purification of the crude material using reverse $C_{18}$ chromatography using water (0.1% formic acid) and acetonitrile (0.1% formic acid) as eluents gave 83 mg of the pure exo- compound methyl 4- ((3aS, 4S, 9bR)- 2, 3, 3a, 4, 5, 9b- hexahydro- 8- methyl- 1 H- cyclopenta [c] quinolin- 4- yl)- 3, 5- dimethylbenzoate (Yield: 57%) .

$^1$H NMR (400 MHz, DMSO- $d_6$) $\delta$ 7.62 (2H, s), 6.69 (1 H, dd, *J* = 8 & 2 Hz), 6.52 (1 H, d, *J* = 7.6 Hz), 5.69 (1 H, s), 4.20 (1 H, d, *J* = 11.2 Hz), 3.83 (3H, s), 2.98- 2.91 (1 H, m), 2.68- 2.60 (1 H, m), 2.60- 2.52 (3H, brs), 2.45- 2.37 (3H, brs), 2.26- 2.20 (1 H, m), 2.15 (3H, s), 1.74- 1.50 (4H, m), 1.14- 1.08 (1 H, m) .

LC- MS: $t_R$ = 5.57 $[M+H]^+$= 350 (method 2)

***Intermediate compound 103: methyl-4-((3aS,4S,9bR)-3a,4,5,9b-tetrahydro-8-methoxy-3H-cyclopenta[c]quinolin-4-yl)-3,5-dimethylbenzoate***

The following compound was prepared using the same methodology as in intermediate compound 100 using *p-anisidine* (0.75 mmol, 93 mg). Purification of the crude material by flash chromatography on silica gel using an elution of 10% ethylacetate in hexanes afforded the exo-isomer as a solid. (152 mg. Yield: 55%)

LC- MS: $t_R$ = 5.13 $[M+H]^-$= 364 (method 3)

***Intermediate compound 104: methyl 4-((3aS,4S,9bR)-2,3,3a,4,5,9b-hexahydro-8-methoxy-1H-cyclopenta[c]quinolin-4-yl)-3,5-dimethylbenzoate***

The following compound was prepared using the same methodology as in intermediate compound 67 from **intermediate compound 103** (0.42 mmol, 152 mg) . Purification of the crude material using reverse $C_{18}$ chromatography using water (0.1% formic acid) and acetonitrile (0.1% formic acid) as eluents gave 59 mg of the pure exo- isomer methyl 4- ((3aS, 4S, 9bR)- 2, 3, 3a, 4, 5, 9b- hexahydro- 8- methoxy- 1H- cyclopenta [c] quinolin- 4- yl)- 3, 5- dimethylbenzoate as a solid (Yield: 39%)

$^1$H NMR (400 MHz, DMSO- $d_6$) $\delta$ 7.62 (2H, s), 6.68 (1 H, d, *J* = 2.4 Hz), 6.58- 6.53 (2H, m), 5.53 (1H, s), 4.16 (1H, d, *J* = 11.2 Hz), 3.83 (3H, s), 3.65 (3H, s), 2.99- 2.93 (1 H, m), 2.72- 2.52 (4H, m), 2.45- 2.38 (3H, brs), 2.30- 2.22 (1 H, m), 1.72- 1.50 (4H, m), 1.14- 1.06 (1 H, m) .

LC- MS: $t_R$ = 5.25 $[M+H]^+$= 366 (method 2)

***Intermediate compound 105: 5-((3aS, 4R, 9bR)-3a, 4, 5, 9b-tetrahydro-8-methyl-3H-cyclopenta[c]quinolin-4-yl) pyridine-2-carboxylic acid***

To a solution of 5- formylpyridine- 2- carboxylic acid **intermediate compound 31** (60 mg, 0.4 mmol) in anhydrous ACN (15 ml) was added *p*- toluidine (43 mg, 0.4 mmol), cyclopenta- 1, 3- diene (163 mLl, 2 mmol) and $BF_3{\cdot}Et_2O$ (101 mL, 0.8 mmol) . The reaction mixture was stirred 16h at room temperature. Solvent was evaporated and purification of the crude material by reverse $C_{18}$ chromatography using water (0.1% formic acid) and methanol (0.1% formic acid) as eluents to give 17 mg of the endo- isomer 5- ((3a*S*, 4*R*, 9b*R*)- 3a, 4, 5, 9b- tetrahydro- 8- methyl- 3*H*- cyclopenta [*c*] quinolin- 4- yl) pyridine- 2- carboxylic acid (Yield: 13%)

$^1$H NMR (400 MHz, DMSO- $d_6$) $\delta$ 8.77 (1 H, s), 8.06- 8.00 (2H, m), 6.83 (1 H, s), 6.72 (1 H, d, *J*=8Hz), 6.23 (1 H, d, *J*=8Hz), 5.85 (1 H, m), 5.61 (1 H, s), 5.56 (1 H, m), 4.63 (1 H, d, *J*=2.8Hz), 4.01 (1 H, d, *J*=8.8Hz), 3.00- 2.97 (1 H,

m), 2.42- 2.36 (1 H, m), 2.16 (3H, s), 1.66- 1.60 (1 H, m) .

LC- MS: $t_R$ = 8.07 $[M+H]^+$ = 307 (method 1)

***Intermediate compound 106: methyl-6-((3aS,4R,9bR)-3a,4,5,9b-tetrahydro-8-methyl-3H-cyclopenta[c]quinolin-4-yl)pyridine-3-carboxylate***

The following compound was prepared using the same methodology as in intermediate compound 41 using methyl-6-formylnicotinate (1 mmol) and 4-methylaniline (1 mmol). The reaction mixture was stirred at room temperature for 16h. Solvent was evaporated and purification of the crude material by flash chromatography on silica gel using hexane and ethylacetate as eluents to afford 249 mg of the impure title endo-isomer (Yield=78%).

$^1H$ NMR (400 MHz, $CDCl_3$) δ 9.19 (1 H, dd, *J* = 2.4 & 1.6 Hz), 8.31 (1 H, dd, *J* = 8.0 & 2.0 Hz), 7.54 (1 H, d, *J* = 8.0 Hz), 6.89 (1 H, s), 6.73 (1 H, dd, *J* = 8.0 & 2.0 Hz), 6.64 (1 H, d, *J* = 8.0 Hz), 5.84- 5.81 (1 H, m), 5.62- 5.61 (1H, m), 4.75 (1 H, d, *J* = 3.2 Hz), 4.13- 4.16 (1 H, m), 3.97 (3H, s), 3.33 (1H, dq, *J* = 8.8 & 3.2 Hz), 2.46- 2.39 (1 H, m), 2.25 (3H, s), 1.86- 1.79 (1 H, m) .

LC- MS: $t_R$ = 4.77 min $[M+H]^+$= 321 (method 2)

***Intermediate compound 107: 6-((3aS,4R,9bR)-3a,4,5,9b-tetrahydro-8-methyl-3H-cyclopenta[c]quinolin-4-yl)pyridine-3-carboxylic acid***

To a solution of **intermediate compound 106** (0.20 mmol, 65 mg) in THF (1.5 mL) was added 1M LiOH (0.30 mL, 0.30 mmol). The reaction mixture was stirred 16 h at room temperature. The solvent was evaporated and the crude was purified by reverse $C_{18}$ chromatography using water (0.1% formic acid) and acetonitrile (0.1 % formic acid) as eluents, to give 49 mg of the title endo-isomer. (Yield: 80%)

$^1H$ NMR (400 MHz, DMSO- $d_6$) δ 9.02 (1H, d, *J* = 1.6 Hz), 8.27 (1 H, dd, *J* = 8.0 & 2.0 Hz), 7.64 (1 H, d, *J* = 8.4 Hz), 6.80 (1 H, s), 6.70 (1 H, dd, *J* = 8.4 & 1.6 Hz), 6.64 (1 H, d, *J* = 8.4 Hz), 5.82- 5.80 (1 H, m), 5.60 (1 H, brs), 5.51 (1 H, d, *J* = 5.2 Hz), 4.56 (1 H, d, *J* = 3.2 Hz), 4.01 (1 H, d, *J* = 8.8 Hz), 3.20- 3.12 (1 H, m), 2.26- 2.22 (1 H, m), 2.13 (3H, s), 1.62- 1.56 (1 H, m) .

LC- MS: $t_R$ = 8.73 $[M+H]^+$= 307 (method 1)

***Intermediate compound 108: methyl 6-((3aS,4R,9bR)-2,3,3a,4,5,9b-hexahydro-8-methyl-1H-cyclopenta[c]quinolin-4-yl)pyridine-3-carboxylate***

The following compound was prepared using the same methodology as in intermediate compound 67 from **intermediate compound 106** (180 mg, 0.56 mmol) for 2 h. After filtration, solvents were evaporated to obtain 170 mg of the pure endo-isomer, which was used in the next step without further purification.

$^1H$ NMR (400 MHz, $CDCl_3$) δ 9.18 (1H, d, *J*= 1.2 Hz), 8.30 (1H, dd, *J*= 8.4 & 2.4 Hz), 7.54 (1 H, d, *J* = 8.0 Hz), 6.95 (1 H, s), 6.83 (1 H, dd, *J* = 8.4 & 1.6 Hz), 6.60 (1 H, d, *J* = 8.0 Hz), 4.69 (1 H, d, *J* = 3.2 Hz), 4.35 (1 H, brs), 3.96 (3H, s), 3.49 (1 H, dt, *J* = 7.6 & 2.8 Hz), 2.80 (1 H, dq, *J* = 8.8 & 3.2 Hz), 2.25 (3H, s), 2.15- 2.09 (1 H, m), 1.83- 1.80 (1 H, m), 1.60- 1.43 (3H, m), 1.25- 1.15 (1 H, m) .

LC- MS: $t_R$ = 4.95 $[M+H]^+$= 323 (method 2)

***Intermediate compound 109: 5-((3aS,4R,9bR)-3a,4,5,9b-tetrahydro-8-methyl-3H-cyclopenta[c]quinolin-4-yl)-6-methylpyridine-2-carbonitrile***

The following compound was prepared using the same methodology as in intermediate compound 41 using 5-formyl- 6- methylpyridine- 2- carbonitrile **(intermediate compound 35)** (1 mmol) and 4- methylaniline (1 mmol) . Purification of the crude material by flash chromatography on silica gel using hexane and ethylacetate as eluents to afford 281 mg of the title endo- isomer (Yield=93%) .

$^1H$ NMR (400 MHz, DMSO- $d_6$) δ 8.07 (1 H, d, *J* = 8.0 Hz), 7.89 (1 H, d, *J* = 8.0 Hz), 6.82 (1 H, s), 6.69 (1 H, dd, *J* = 8.0 & 1.6 Hz), 6.58 (1 H, d, *J* = 7.6 Hz), 5.86- 5.83 (1 H, m), 5.54 ( 1 H, d, *J* = 4.8 Hz), 5.40 (1 H, s), 4.67 (1 H, d, *J* = 2.8 Hz), 4.03- 4.00 (1 H, m), 3.29 (3H, s), 3.00- 2.95 (1 H, m), 2.40- 2.33 (1 H, m), 2.13 (3H, s), 1.57- 1.51 (1 H, m) .

LC- MS: $t_R$ = 4.72 $[M+H]^+$= 302 (method 2)

***Intermediate compound 110: ethyl 5-((3aS,4R,9bR)-3a,4,5,9b-tetrahydro-8-methyl-3H-cyclopenta[c]quinolin-4-yl)-1-methyl-1H-pyrazole-3-carboxylate***

The following compound was prepared using the same methodology as in intermediate compound 41 using 5-formyl- 1- methyl- 1*H*- pyrazole- 3- carboxylic acid ethyl ester **(intermediate compound 39)** (1 mmol.) and 4- methylaniline (1mmol) . Solvent was evaporated and purification of the crude material by flash chromatography on silica gel using hexane and ethylacetate as eluents to afford 229 mg of the title endo- isomer (Yield=91%) .

$^1$H NMR (400 MHz, $CDCl_3$) δ 6.88 (2H, m), 6.82 (1 H, dd, *J* = 8.4 & 2 Hz), 6.54 (1 H, d, *J* = 7.6 Hz), 5.82- 5.80 (1 H, m), 5.67- 5.66 (1 H, m), 4.65 (1 H, d, *J* = 3.2 Hz), 4.45- 4.34 (2H, m), 4.12- 4.07 (1 H, m), 3.96 (3H, s), 3.60 (1 H, s), 3.05- 2.98 (1 H, m), 2.73- 2.66 (1 H, m), 2.24 (3H, s), 2.00- 1.92 (1 H, m), 1.40 (3H, t, *J* = 7.6 Hz) .

LC- MS: $t_R$ = 4.48 $[M+H]^+$= 338 (method 2)

***Intermediate compound 111: ethyl 5-((3aS,4R,9bR)-2,3,3a,4,5,9b-hexahydro-8-methyl-1H-cyclopenta[c]quinolin-4-yl)-1-methyl-1H-pyrazole-3-carboxylate***

The following compound was prepared using the same methodology as in intermediate compound 67 from **intermediate compound 110** (225 mg, 0.67 mmol). Solvent was evaporated to give 209 mg of the title endo-isomer, which was used in the next step without further purification.

$^1$H NMR (400 MHz, $CDCl_3$) δ 6.93 (1 H, s), 6.88 (1 H, s), 6.82 (1 H, dd, *J* = 7.6 & 1.2 Hz), 6.51 (1 H, d, *J*= 8.0 Hz), 4.57 (1 H, d, *J* = 3.2 Hz), 4.41- 4.34 (2H, m), 3.93 (3H, s), 3.63 (1 H, s), 3.44- 3.39 (1 H, m), 2.57- 2.48 (1 H, m), 2.25 (3H, s), 2.18- 2.09 (1 H, m), 1.84- 1.74 (2H, m), 1.52- 1.39 (2H, m), 1.40 (3H, t, *J*= 7.6 Hz) .

LC- MS: $t_R$ = 4.78 $[M+H]^+$= 340 (method 2)

***Intermediate compound 112: ethyl 5-((3aS,4R,9bR)-3a,4,5,9b-tetrahydro-8-methoxy-3H-cyclopenta[c]quinolin-4-yl)-1-methyl-1H-pyrazole-3-carboxylate***

The following compound was prepared using the same methodology as in intermediate compound 41 using 5-formyl- 1- methyl- 1*H*- pyrazole- 3- carboxylic acid ethyl ester **(intermediate compound 39)** (1 mmol) and 4- methoxyaniline (1mmol) . Solvent was evaporated and purification of the crude material by flash chromatography on silica gel using hexane and ethylacetate as eluents to afford 190 mg of the title endo- isomer (Yield=91%) .

$^1$H NMR (400 MHz, $CDCl_3$) δ 6.88 (1 H, s), 6.65- 6.57 (3H, m), 5.82- 5.78 (1 H, m), 5.69- 5.66 (1 H, m), 4.62 (1 H, d, *J* = 2.8 Hz), 4.45- 4.36 (2H, m), 4.13- 4.01 (1 H, m), 3.96 (3H, s), 3.75 (3H, s), 3.52 (1 H, s), 3.03- 3.00 (1 H, m), 2.73- 2.66 (1 H, m), 2.04- 1.94 (1 H, m), 1.40 (3H, t, *J*= 7.2 Hz) .

LC- MS: $t_R$ = 4.02 $[M+H]^+$= 354 (method 2)

***Intermediate compound 113: ethyl 5-((3aS,4R,9bR)-2,3,3a,4,5,9b-hexahydro-8-methoxy-1H-cyclopenta[c]quinolin-4-yl)-1-methyl-1H-pyrazole-3-carboxylate***

The following compound was prepared using the same methodology as in intermediate compound 67 from **intermediate compound 112** (190 mg, 0.54 mmol). Solvent was evaporated to give 174 mg of the title endo-isomer, which was used in the next step without further purification.

$^1$H NMR (400 MHz, $CDCl_3$) δ 6.87 (1 H, s), 6.88 (1 H, s), 6.70 (1 H, d, *J* = 2.4 Hz), 6.63 (1 H, dd, *J* = 8.4 & 2.8 Hz), 6.55 (1 H, d, *J* = 8.4 Hz), 4.54 (1 H, d, *J* = 2.8 Hz), 4.44- 4.38 (2H, m), 3.93 (3H, s), 3.76 (3H, s), 3.53 (1 H, s), 3.45- 3.41 (1 H, m), 2.54- 2.49 (1 H, m), 2.17- 2.12 (1 H, m), 1.81- 1.71 (2H, m), 1.53- 1.33 (2H, m), 1.40 (3H, t, *J* = 7.6 Hz) .

LC- MS: $t_R$ = 4.30 $[M+H]^+$= 356 (method 2)

***Intermediate compound 114: ethyl 3-((3aS,4R,9bR)-3a,4,5,9b-tetrahydro-8-methyl-3H-cyclopenta[c]quinolin-4-yl)-1-methyl-1H-pyrazole-5-carboxylate***

The following compound was prepared using the same methodology as in intermediate compound 41 using 3-formyl- 1- methyl- 1*H*- pyrazole- 5- carboxylic acid ethyl ester **(intermediate compound 40)** (1 mmol) and 4- methylaniline (1 mmol) . Purification of the crude material by flash chromatography on silica gel using hexane and ethylacetate as eluents to afford 128 mg of the title endo- isomer (Yield: 46%) .

$^1$H NMR (400 MHz, $CDCl_3$) δ 6.87 (1 H, s), 6.82 (1 H, s), 6.80 (1 H, dd, *J* = 8.0 & 2.0 Hz), 6.60 (1 H, d, *J* = 8.4 Hz), 5.83- 5.79 (1 H, m), 5.67- 5.65 (1 H, m), 4.63 (1 H, d, *J* = 3.2 Hz), 4.37 (2H, q, *J* = 7.6 Hz ), 4.16 (3H, s), 4.07 (1 H, d, *J* = 7.6 Hz), 3.20- 3.12 (1 H, m), 2.64- 2.56 (1 H, m), 2.23 (3H, s), 2.14- 2.07 (1 H, m), 1.39 (3H, t, *J* = 7.6 Hz) .

LC- MS: $t_R$ = 4.93 $[M+H]^+$= 338 (method 2)

***Intermediate compound 115: ethyl 3-((3aS,4R,9bR)-2,3,3a,4,5,9b-hexahydro-8-methyl-1H-cyclopenta[c]quinolin-4-yl)-1-methyl-1H-pyrazole-5-carboxylate***

The following compound was prepared using the same methodology as in intermediate compound 67 from **intermediate compound 114** (120 mg, 0.36 mmol). Solvent was evaporated and purification of the crude material by

flash chromatography on silica gel using hexane and ethylacetate as eluents to afford 104 mg of the title endo-isomer (Yield: 86%).

$^1$H NMR (400 MHz, $CDCl_3$) δ 6.92 (1 H, s), 6.82 (1 H, s), 6.80 (1 H, dd, *J* = 7.6 & 1.6 Hz), 6.53 (1 H, d, *J* = 8.4 Hz), 4.56 (1 H, d, *J* = 3.2 Hz), 4.34 (2H, q, *J* = 7.2 Hz), 4.16 (3H, s), 3.44- 3.39 (1 H, m), 2.66- 2.60 (1 H, m), 2.24 (3H, s), 2.12- 2.07 (1 H, m), 1.80- 1.71 (1 H, m), 1.70- 1.65 (1 H, m), 1.54- 1.45 (2H, m), 1.40 (3H, t, *J* = 7.6 Hz) .

LC- MS: $t_R$ = 5.12 $[M+H]^+$= 339 (method 2)

***Intermediate compound 116: ethyl 3-((3aS,4R,9bR)-3a,4,5,9b-tetrahydro-8-methoxy-3H-cyclopenta[c]quinolin-4-yl)-1-methyl-1H-pyrazole-5-carboxylate***

The following compound was prepared using the same methodology as in intermediate compound 41 using 3-formyl- 1- methyl- 1H- pyrazole- 5- carboxylic acid ethyl ester **(intermediate compound 40)** (1 mmol) and 4- methoxyaniline (1 mmol) . Purification of the crude material by flash chromatography on silica gel using hexane and ethylacetate as eluents to afford 94 mg of the title endo- isomer (Yield: 34%) .

$^1$H NMR (400 MHz, $CDCl_3$) δ 6.82 (1 H, s), 6.64- 6.58 (3H, m), 5.82- 5.78 (1 H, m), 5.68- 5.65 (1 H, m), 4.59 (1 H, d, *J* = 3.6 Hz), 4.37 (2H, q, *J* = 7.6 Hz), 4.16 (3H, s), 4.07 (1 H, d, *J* = 7.6 Hz), 3.75 (3H, s), 3.20- 3.12 (1 H, m), 2.62- 2.54 (1 H, m), 2.15- 2.08 (1 H, m), 1.37 (3H, t, *J* = 7.6 Hz) .

LC- MS: $t_R$ = 4.42 $[M+H]^+$= 354 (method 2)

***Intermediate compound 117: ethyl 3-((3aS,4R,9bR)-2,3,3a,4,5,9b-hexahydro-8-methoxy-1H-cyclopenta[c]quinolin-4-yl)-1-methyl-1H-pyrazole-5-carboxylate***

The following compound was prepared using the same methodology as in intermediate compound 67 from **intermediate compound 116** (90 mg, 0.36 mmol). Solvent was evaporated and purification of the crude material by flash chromatography on silica gel using hexane and ethylacetate as eluents to afford 69 mg of the title endo-isomer (Yield: 76%).

$^1$H NMR (400 MHz, $CDCl_3$) δ 6.81 (1 H, s), 6.69 (1 H, d, *J* = 2.8 Hz), 6.63- 6- 51 (3H, m), 4.52 (1 H, d, *J* = 2.8 Hz), 4.41- 4.31 (3H, m), 4.16 (3H, s), 3.75 (3H, s), 3.45- 3.40 (1 H, m), 2.67- 2.64 (1 H, m), 2.13- 2.08 (1 H, m), 1.79- 1.75 (1 H, m), 1.70- 1.64 (1 H, m), 1.51- 1.44 (2H, m), 1.40 (3H, t, *J* = 7.6 Hz) .

LC- MS: $t_R$ = 4.48 $[M+H]^+$= 356 (method 2)

***Intermediate compound 118: 1-isobutoxy-4-nitrobenzene***

The following compound was prepared using the same methodology as in intermediate compound 4 from 2-methylpropan- 1- ol (2.67 mmol, 0.25 mL) . Solvent was evaporated and purification of the crude material by flash chromatography on silica gel, eluting with a gradient from 10% to 50% ethyl acetate in hexane gave 1- isobutoxy- 4-nitrobenzene as an oil (350 mg, 99%) .

$^1$H NMR (400 MHz, $CDCl_3$) δ 8.20 (2H, d, *J* = 9.2 Hz), 6.95 (2H, d, *J* = 9.2 Hz), 3.82 (2H, d, *J*= 6.8 Hz), 2.2-2.1 (1 H, m), 1.05 (2H, d, *J*= 6.4 Hz)

LC- MS: $t_R$ =3.58 $[M+H]^+$= 196 (method 3) .

***Intermediate compound 119: 4-isobutoxybenzenamine***

The following compound was prepared using the same methodology as in intermediate compound 5 from **intermediate compound 118** (1.79 mmol, 350 mg). Reaction mixture was diluted with aqueous 1 N NaOH solution and ethyl acetate. The aqueous layer was extracted with EtAcO. The combined extracts were washed with brine, dried over anhydrous magnesium sulfate and concentrated in *vacuum* to give intermediate 119 (205 mg, 69%) with no further purification.

LC- MS: $t_R$ =1.62 $[M+H]^+$= 166 (method 3)

***Intermediate compound 120: methyl 4-(3a,4,5,9b-tetrahydro-8-isobutoxy-3H-cyclopenta[c]quinolin-4-yl)-3-methylbenzoate***

The following compound was prepared using the same methodology as in intermediate compound 41 using **intermediate compound 19** (1.21 mmol, 215 mg) and **intermediate compound 119** (1.21 mmol, 200 mg). Solvent was evaporated and purification of the crude material by flash chromatography on silica gel using an elution of 15% ethylacetate in hexanes afforded the title compound as a 1:12 mixture of exo/endo isomers. (323 mg. Yield: 68%).

LC- MS: $t_R$ =5.17 $[M+H]^+$= 392 (method 2) (Both isomers)

***Intermediate compound 121 & 122: methyl 4-((3aS,4R,9bR)-2,3,3a,4,5,9b-hexahydro-8-isobutoxy-1H-cyclopenta [c]quinolin-4-yl)-3-methylbenzoate (121) and methyl 4-((3aS,4S,9bR)-2,3,3a,4,5,9b-hexahydro-8-isobutoxy-1H-cyclopenta[c]quinolin-4-yl)-3-methylbenzoate (122)***

The following compounds were prepared using the same methodology as in intermediate compound 67 from **intermediate compound 120** (0.82 mmol, 323 mg) for 1 h. Purification of the crude material (1: 6.5 mixture of exo/ endo isomers) by reverse $C_{18}$ chromatography using water (0.1% formic acid) and acetonitrile (0.1% formic acid) as eluents gave 187 mg of the pure endo- isomer methyl 4- ((3aS, 4R, 9bR)- 2, 3, 3a, 4, 5, 9b- hexahydro- 8- isobutoxy- 1*H*- cyclopenta [c] quinolin- 4- yl)- 3- methylbenzoate **(121)** and 13.2 mg of the pure exo- isomer methyl 4- ((3aS, 4S, 9bR)- 2, 3, 3a, 4, 5, 9b- hexahydro- 8- isobutoxy- 1*H*- cyclopenta [c] quinolin- 4- yl)- 3- methylbenzoate **(122)** (Overall yield= 61 %) .

Methyl- 4- ((3aS, 4R, 9bR)- 2, 3, 3a, 4, 5, 9b- hexahydro- 8- isobutoxy- 1H- cyclopenta [c] quinolin- 4- yl)- 3- methylbenzoate (endo- isomer **121) :**

LC- MS: $t_R$ =5.43 $[M+H]^+$= 394 (method 2)

Methyl- 4- ((3aS, 4S, 9bR)- 2, 3, 3a, 4, 5, 9b- hexahydro- 8- isobutoxy- 1H- cyclopenta [c] quinolin- 4- yl)- 3- methylbenzoate (exo- isomer **122) :**

LC- MS: $t_R$ =5.27 $[M+H]^+$= 394 (method 2)

The following examples illustrate the scope of the invention.

**Examples of compounds of  general formula**

***Example 1: 4-((3aS,4R,9bR)-2,3,3a,4,5,9b-hexahydro-1H-cyclopenta [c]quinolin-4-yl)benzoic acid***

The **Intermediate compound 41** (60 mg, 0.16 mmol, ) was dissolved in ethanol and tetrahydrofurane (1: 1, 2 mL) and platinum dioxide (10%w/w, 6 mg) was added. The solution was hydrogenated at r.t., for 6h. HPLC analysis showed total conversion. Reaction mixture was filtered and washed with THF. Solvent was evaporated and purification of the crude material by flash chromatography on silica gel using an elution of 30% ethylacetate in hexanes afforded the title compound as a 3: 1 mixture of compounds as a white solid (32 mg, 53%), which was purified using reverse $C_{18}$ chromatography using water (0.1% formic acid) and acetonitrile (0.1% formic acid) as eluents to give 5 mg of 4- ((3a*S*, 4*R*, 9b*R*)- 2, 3, 3a, 4, 5, 9b- hexahydro- 1*H*- cyclopenta [c] quinolin- 4- yl) benzoic acid **1** and 18 mg 4- ((3a*S*, 4*R*, 9b*R*)- 8- bromo- 2, 3, 3a, 4, 5, 9b- hexahydro- 1*H*- cyclopenta [*c*] quinolin- 4- yl) benzoic acid **2**.

4- ((3a*S*, 4*R*, 9b*R*)- 2, 3, 3a, 4, 5, 9b- hexahydro- 1*H*- cyclopenta [*c*] quinolin- 4- yl) benzoic **1:**

$^1$H NMR (400 MHz, $CDCl_3$) δ 8.10 (2H, d, *J* = 8.4 Hz), 7.55 (2H, d, *J* = 8.0 Hz), 7.13 (1 H, d, *J* = 7.6 Hz), 7.01 (1 H, dt, *J* = 7, 2 & 0.8 Hz), 6.78 (1 H, dt, *J* = 7, 6 & 1.2 Hz), 6.62 (1 H, dd, *J* = 7.6 & 0.8 Hz), 4.65 (1 H, d, *J* = 2.8 Hz), 3.52- 3.47 (1 H, m), 2.54- 2.47 (1 H, m), 2.21- 2.11 (1 H, m), 1.85- 1.80 (1 H, m), 1.78- 1.69 (1 H, m), 1.62- 1.52 (1 H, m), 1.50- 1.40 (1 H, m), 1.22- 1.14 (1 H, m) .

LC- MS: $t_R$ = 7.73 $[M+H]^+$= 294 (method 1)

***Example 2: 4-((3aS,4R,9bR)-8-bromo-2,3,3a,4,5,9b-hexahydro-1H cyclopenta[c]quinolin-4-yl)benzoic acid***

The following compound was prepared using the same methodology as in intermediate compound 67 using **Intermediate compound 41** (370 mg, 1 mmol, ) . The solution was hydrogenated at r.t., for 2 h. HPLC analysis showed total conversion. Reaction mixture was filtered and washed with THF. Solvent was evaporated and purification of the crude material using reverse $C_{18}$ chromatography using water (0.1% formic acid) and acetonitrile (0.1% formic acid) as eluents afforded 154 mg (41%) of 4- ((3a*S*, 4*R*, 9b*R*)- 8- bromo- 2, 3, 3a, 4, 5, 9b- hexahydro- 1*H*- cyclopenta [*c*] quinolin- 4- yl) benzoic acid as a white solid.

4- ((3a*S*, 4*R*, 9b*R*)- 8- bromo- 2, 3, 3a, 4, 5, 9b- hexahydro- 1*H*- cyclopenta [*c*] quinolin- 4- yl) benzoic acid **2**:

$^1$H NMR (400 MHz, DMSO- $d_6$) δ 7.91 (2H, d, *J*= 8.4 Hz), 7.52 (2H, d, *J*= 8.0 Hz), 7.14 (1 H, d, *J* = 1.6 Hz), 7.01 (1 H, dd, *J* = 8.4 & 2 Hz), 6.65 (1H, d, *J* = 8.4 Hz), 6.03 (1 H, s), 4.53 (1 H, d, *J* = 3.2 Hz), 3.38 (1 H, dt, *J* = 7.2 & 1.6 Hz), 2.48- 2.40 (1 H, m), 2.08- 2.01 (1 H, m), 1.72- 1.64 (1 H, m), 1.52- 1.44 (1 H, m), 1.40- 1.34 (1 H, m), 1.03- 0.98 (1 H, m) .

LC- MS: $t_R$ = 8.67 $[M+H]^+$= 372/374 (method 1)

***Example 3: 4-((3aS,4R,9bR)-8-(trifluoromethyl)-2,3,3a,4,5,9b-hexahydro-1H-cyclopenta[c]quinolin-4-yl)benzoic acid***

The following compound was prepared using the same methodology as in intermediate compound 67 from **intermediate compound 42** (270 mg, 0.75 mmol) for 1.30 h. Purification of the crude material using reverse $C_{18}$

chromatography using water (0.1% formic acid) and acetonitrile (0.1% formic acid) as eluents to give 140 mg of the title compound which was precipitated in hexane, filtered and dried in vacuum to obtain 128 mg (47%) of the pure endo-isomer.

$^{1}$H NMR (400 MHz, DMSO- $d_6$) δ 8.12 (2H, d, *J* = 8.4 Hz), 7.54 (2H, d, *J* = 8.4 Hz), 7.37 (1 H, s), 7.24 (H, dd, *J* = 10 & 2 Hz), 6.64 (1 H, d, *J* = 8.4 Hz), 4.72 (1 H, d, *J* = 2.8 Hz), 3.52- 3.48 (1 H, m), 2.54- 2.50 (1 H, m), 2.22- 2.16 (1 H, m), 1.89- 1.85 (1 H, m), 1.69- 1.47 (3H, m), 1.25- 1.18 (1 H, m) .

LC- MS: $t_R$ = 8.52 $[M+H]^+$= 362 (method 1)

***Example 4: 4-((3aS,4R,9bR)-2,3,3a,4,5,9b-hexahydro-8-methyl-1H-cyclopenta[c]quinolin-4-yl)benzoic acid***

The following compound was prepared using the same methodology as in intermediate compound 67 from **intermediate compound 43** (70 mg, 0.23 mmol) for 16 h. Purification of the crude material by precipitation in dichloromethane, filtration, solid washed by ether and dried to give 25 mg of the title compound (Yield: 35%).

$^{1}$H NMR (400 MHz, $CDCl_3$) δ 8.10 (2H, d, *J* = 8.4 Hz), 7.55 (2H, d, *J* = 8.0 Hz), 6.95 (1 H, s), 6.83 (1 H, d, *J* = 8.0 Hz), 6.54 (1 H, d, *J* = 8.0 Hz), 4.60 (1 H, d, *J* = 3.2 Hz), 3.46 (1 H, dt, *J* = 8.0 & 3.2 Hz), 2.49 (1 H, dq, *J* = 10.4 & 2.8 Hz), 2.26 (3H, s), 1.82- 1.76 (1 H, m), 1.74- 1.67 (1 H, m), 1.57- 1.53 (1 H, m), 1.46- 1.39 (3H, m), 1.21- 1.12 (1 H, m) .

LC- MS: $t_R$ = 8.13 $[M+H]^+$= 308 (method 1)

Separation of 100 mg of (+/- ) endo- isomer 4- ((3a*S*, 4*R*, 9b*R*)- 2, 3, 3a, 4, 5, 9*b*- hexahydro- 8- methyl- 1*H*- cyclopenta [*c*] quinolin- 4- yl) benzoic acid **(4)** by chiral preparative chromatography was carried out using Heptane/IPA/TFA (80: 20: 0.1) as eluents to yield:

34 mg of the enantiomer 1 **(4a):**

Preparative HPLC: $t_R$ = 13.65

28 mg of the enantiomer 2 **(4b):**

Preparative HPLC: $t_R$ = 23.72

***Example 5: 4-((3aS,4S,9bR)-2,3,3a,4,5,9b-hexahydro-8-methyl-1H-cyclopenta[c]quinolin-4-yl)benzoic acid***

The following compounds were prepared using the same methodology as in intermediate compound 67 from **a mixture endo/exo of intermediate compound 43** from (663 mg, 2.07 mmol) for 3 h. Purification of the crude material using reverse $C_{18}$ chromatography using water (0.1% formic acid) and acetonitrile (0.1% formic acid) as eluents to give 32 mg of the 4- ((3a*S*, 4*S*, 9b*R*)- 2, 3, 3a, 4, 5, 9b- hexahydro- 8- methyl- 1*H*- cyclopenta [*c*] quinolin- 4- yl) benzoic acid (exo- isomer **5)** and 455 mg of 4- ((3a*S*, 4*R*, 9b*R*)- 2, 3, 3*a*, 4, 5, 9*b*- hexahydro- 8- methyl- 1*H*- cyclopenta [*c*] quinolin- 4- yl) benzoic acid (endo- isomer **4) .** (yield 77%)

4- ((3a*S*, 4*S*, 9b*F*)- 2, 3, 3a, 4, 5, 9*b*- hexahydro- 8- methyl- 1*H*- cyclopenta [*c*] quinolin- 4- yl) benzoic acid (exo- isomer **5) :**

$^{1}$H NMR (400 MHz, DMSO- $d_6$) δ 7.89 (2H, d, *J*= 8.4 Hz), 7.45 (2H, d, *J*= 8.0 Hz), 6.84 (1 H, m), 6.68 (1 H, dd, *J* = 8.0 & 1.6 Hz), 6.54 (1 H, t, *J* = 8 Hz), 5.72 (1 H, s),

3.67 (1 H, d, *J* = 10 Hz), 2.87 (1 H, q, *J* = 7.6 Hz), 2.45-2.39 (2H, m), 2.20-2.17 (1 H, m), 2.14 (3H, s), 1.67-1.47 (3H, m), 1.31-1.26 (1 H, m).

LC- MS: $t_R$ = 9.47 $[M+H]^+$= 308 (method 1)

***Example 6: 4-((3aS,4R,9bR)-8-chloro-2,3,3a,4,5,9b-hexahydro-1H-cyclopenta[c]quinolin-4-yl)benzoic acid***

The following compound was prepared using the same methodology as in intermediate compound 67 from **intermediate compound 44** (228 mg, 0.7 mmol) for 4 h. Purification of the crude material using reverse $C_{18}$ chromatography using water (0.1% formic acid) and acetonitrile (0.1% formic acid) as eluents to give 96 mg of the title compound which was precipitated in ether, filtered and dried in vacuum to obtain 75 mg (33%) of the pure endo-isomer.

$^{1}$H NMR (400 MHz, DMSO- $d_6$) δ 7.91 (2H, d, *J* = 8 Hz), 7.52 (2H, d, *J* = 8.4 Hz), 7.02 (1 H, s), 6.90 (1 H, dd, *J* = 8.8 & 2.2 Hz), 6.69 (1 H, d, *J* = 8.4 Hz), 6.00 (1 H, s), 4.53 (1 H, s), 3.39- 3.34 (1 H, m), 2.49- 2.41 (1 H, m), 2.07- 2.00 (1 H, m), 1.70- 1.67 (1 H, m), 1.49- 1.40 (1 H, m), 1.38- 1.34 (1 H, m), 1.05- 0.98 (1 H, m) .

LC- MS: $t_R$ = 8.43 min $[M+H]^+$= 328/330 (method 1)

Separation of 200 mg of (+/- )- 4- ((3aS, 4R, 9bR)- 8- chloro- 2, 3, 3a, 4, 5, 9b- hexahydro- 1*H*- cyclopenta [*c*] quinolin- 4- yl) benzoic acid **(6)** by preparative chiral chromatography was carried out using Heptane/IPA/ HCOOH (95: 5: 0.1) as eluents to yield:

94 mg of the enantiomer 1 **(6a):**

Preparative HPLC: $t_R$ = 31

85 mg of the enantiomer 2 **(6b):**

Preparative HPLC: $t_R$ = 54

***Example 7: 4-((3aS,4R,9bR)-2,3,3a,4,5,9b-hexahydro-8-methoxy-1H-cyclopenta[c]quinolin-4-yl)benzoic acid***

The following compound was prepared using the same methodology as in intermediate compound 67 from **intermediate compound 45** (1.15g, 3.58 mmol) for 1 h. Purification of the crude material using reverse $C_{18}$ chromatography using water (0.1% formic acid) and acetonitrile (0.1% formic acid) as eluents to give 700 mg of the title compound.(Yield: 60%)

$^1$H NMR (400 MHz, DMSO- $d_6$) $\delta$ 7.90 (2H, d, *J*= 8.4 Hz), 7.53 (2H, d, *J*= 8.4 Hz), 6.63 (1 H, d, *J* = 8.4 Hz), 6.60 (1H, d, *J* = 2.4 Hz), 6.53 (1H, d, *J* = 8.8 & 2.8 Hz), 6.40 (1 H, s), 4.43 (1 H, d, *J* = 2.8 Hz), 3.63 (3H, s), 3.38- 3.34 (1 H, m), 2.45- 2.41 (1 H, m), 2.07- 2.01 (1 H, m), 1.70- 1.67 (1 H, m), 1.55- 1.48 (1 H, m), 1.38- 1.32 (2H, m), 1.02- 0.96 (1 H, m) .

LC- MS: $t_R$ = 6.68 $[M+H]^+$= 324 (method 1)

Separation of 213 mg of (+/- )- 4- ((3aS, 4R, 9bR)- 2, 3, 3a, 4, 5, 9b- hexahydro- 8- methoxy- 1H- cyclopenta [c] quinolin- 4- yl) benzoic acid **(7)** by preparative chiral chromatography was carried out using Heptane/IPA (90: 10) as eluents to yield:

45 mg of the enantiomer 1 **(7a):**

Preparative HPLC: $t_R$ = 18

83 mg of the enantiomer 2 **(7b):**

Preparative HPLC: $t_R$ = 39

***Example 8: 4-((3aS,4S,9bR)-2,3,3a,4,5,9b-hexahydro-8-methoxy-1H-cyclopenta[c]quinolin-4-yl)benzoic acid***

The following compound was prepared using the same methodology as in intermediate compound 67 from **intermediate compound 45** for 1 h. Solvent was evaporated and purification of the crude material by reverse $C_{18}$ chromatography using water (0.1% formic acid) and acetonitrile (0.1% formic acid) as eluents to give exo- isomer 4- ((3aS, 4S, 9bR)- 2, 3, 3a, 4, 5, 9b- hexahydro- 8- methoxy- 1- *H*- cyclopenta [c] quinolin- 4- yl) benzoic acid (23 mg. Yield: 4%) .

$^1$H NMR (400 MHz, DMSO- $d_6$) $\delta$ 7.9 (2H, d, *J*=8.4Hz), 7.5 (2H, d, *J*=8.4Hz), 6.66 (1 H, d, *J*=2.4Hz), 6.59 (1 H, d, *J*=8.8Hz), 6.53 (1 H, dd, *J*=2.8 & 8.8Hz), 5.57 (1 H, s), 3.66 (1 H, d, *J*=10Hz), 3.63 (3H, s), 2.92- 2.88 (1 H, m), 2.23- 2.19 (2H, m), 1.67- 1.63 (1 H, m), 1.58- 1.47 (2H, m), 1.30- 1.27 (1 H, m) .

LC- MS: $t_R$ = 8.17 $[M+H]^+$ = 324 (method 1)

***Example 9: 4-((3aS, 4R, 9bR)-6-cyclopropyl-2, 3, 3a, 4, 5, 9b-hexahydro-1H-cyclopenta[c]quinolin-4-yl)benzoic acid***

The following compound was prepared using the same methodology as in intermediate compound 67 from **intermediate compound 46** for 1 h. Solvent was evaporated and purification of the crude material by reverse $C_{18}$ chromatography using water (0.1% formic acid) and acetonitrile (0.1% formic acid) as eluents to give the endo- isomer 4- ((3a*S*, 4*R*, 9b*R*)- 6- cyclopropyl- 2, 3, 3a, 4, 5, 9b- hexahydro- 1*H*- cyclopenta [*c*] quinolin- 4- yl) benzoic acid (89 mg. Yield: 43%)

$^1$H NMR (400 MHz, DMSO- $d_6$) $\delta$ 7.96 (2H, d, *J*=8.4Hz), 7.61 (2H, d, *J*=8Hz), 6.94 (1 H, d, *J*=7.2Hz), 6.76 (1 H, d, *J*=7.2Hz), 6.59 (1 H, t, *J*=7.6Hz), 4.88 (1 H, s), 4.65 (1 H, d, *J*=2.8Hz), 3.46- 3.42 (1 H, m), 2.09- 2.04 (1 H, m), 1.78- 1.73 (2H, m), 1.53- 1.50 (1 H, m), 1.38- 1.35 (2H, m), 1.04- 1.03 (1 H, m), 0.87- 0.83 (2H, m), 0.60- 0.57 (1 H, m), 0.43- 0.40 (1 H, m) .

LC- MS: $t_R$ = 11.28 $[M+H]^+$ = 334 (method 1)

***Example 10, 11 and 12: 4-((3aS, 4S, 9bR)-8-cyclopropyl-2, 3, 3a, 4, 5, 9b-hexahydro-1H-cyclopenta[c]quinolin-4-yl)benzoic acid (10); (+)-4-((3aS, 4R, 9bR)-8-cyclopropyl-2, 3, 3a, 4, 5, 9b-hexahydro-1H-cyclopenta[c]quinolin-4-yl)benzoic acid (11a); (-)-4-((3aS, 4R, 9bR)-8-cyclopropyl-2, 3, 3a, 4, 5, 9b-hexahydro-1H-cyclopenta[c]quinolin-4-yl)benzoic acid (11b) and 4-((3aS, 4R, 9bR)-2, 3, 3a, 4, 5, 9b-hexahydro-8-propyl-1H-cyclopenta[c]quinolin-4-yl) benzoic acid (12)***

The following compounds were prepared using the same methodology as in intermediate compound 67 from **intermediate compound 47 & 48** for 1h. Solvent were evaporated and purification of the crude material by reverse $C_{18}$ chromatography using water (0.1% formic acid) and acetonitrile (0.1% formic acid) as eluents to give the exo- isomer 4- ((3a*S*, 4*S*, 9b*R*)- 8- cyclopropyl- 2, 3, 3a, 4, 5, 9b- hexahydro- 1*H*- cyclopenta [c] quinolin- 4- yl) benzoic acid (4 mg) **(10),** the endo isomer 4- ((3a*S*, 4*R*, 9b*R*)- 8- cyclopropyl- 2, 3, 3a, 4, 5, 9b- hexahydro- 1*H*- cyclopenta [*c*] quinolin- 4- yl) benzoic acid **(11)** (81 mg) and 4- ((3a*S*, 4*R*, 9b*R*)- 2, 3, 3a, 4, 5, 9b- hexahydro- 8- propyl- 1*H*- cyclopenta [*c*] quinolin- 4- yl) benzoic acid **(12)** (13 mg) (Overall Yield: 47%) .

Exo- isomer **10:** 4- ((3a*S*, 4*S*, 9b*R*)- 8- cyclopropyl- 2, 3, 3a, 4, 5, 9b- hexahydro- 1*H*- cyclopenta [*c*] quinolin- 4- yl) benzoic acid

$^1$H NMR (400 MHz, DMSO- $d_6$) $\delta$ 7.91 (2H, d, *J*=8Hz), 7.48 (2H, d, *J*=8Hz), 6.80 (1 H, s), 6.60 (1 H, dd, *J*=1.6 & 8Hz), 6.55 (1 H, d, *J*=8Hz), 5.75 (1 H, s), 3.69 (1 H, d, *J*=10Hz), 2.90- 2.88 (1H, m), 2.19- 2.17 (2H, m), 1.77- 1.73 (1H, m), 1.67- 1.65 (1 H, m), 1.59- 1.49 (3H, m), 1.32- 1.30 (1 H, m), 0.81- 0.77 (2H, m), 0.52- 0.49 (2H, m) .

LC- MS: $t_R$ = 10.35 $[M+H]^+$ = 334 (method 1)

Endo- isomer **11:** 4- ((3a*S*, 4*R*, 9b*R*)- 8- cyclopropyl- 2, 3, 3a, 4, 5, 9b- hexahydro- 1*H*- cyclopenta [*c*] quinolin- 4- yl) benzoic acid

$^1$H NMR (400 MHz, DMSO- $d_6$) $\delta$ 7.92 (2H, d, *J*=8Hz), 7.53 (2H, d, *J*=8Hz), 6.77 (1 H, s), 6.60 (2H, m), 5.57 (1 H, s), 4.48 (1 H, d, *J*=2Hz), 3.37- 3.34 (1 H, m), 2.44- 2.41 (1 H, m), 2.05- 2.02 (1 H, m), 1.77- 1.70 (2H, m), 1.53- 1.50 (1 H, m), 1.38- 1.34 (2H, m), 1.02- 0.99 (1 H, m), 0.81- 0.79 (2H, m), 0.53- 0.49 (2H, m) .

LC- MS: $t_R$ = 10.75 $[M+H]^+$ = 334 (method 1)

Separation of 592 mg of (+/- )- 4- ((3a*S*, 4*R*, 9b*R*)- 8- cyclopropyl- 2, 3, 3a, 4, 5, 9b- hexahydro- 1*H*- cyclopenta [c] quinolin- 4- yl) benzoic acid **(11)** by preparative chiral chromatography was carried out using Heptane/IPA (90: 10) as eluents to yield:

195 mg of the enantiomer 1: (+)- 4- ((3a*S*, 4*R*, 9b*R*)- 8- cyclopropyl- 2, 3, 3a, 4, 5, 9b- hexahydro- 1*H*- cyclopenta [c] quinolin- 4- yl) benzoic acid **(11a)**

Preparative HPLC: $t_R$ = 15

Optical Purity: 99.9% ee

$[\alpha]^{Na}_D$= + 34.8 (0.5, $CHCl_3$)

146 mg of the enantiomer 2: (- )- 4- ((3aS, 4*R*, 9b*R*)- 8- cyclopropyl- 2, 3, 3a, 4, 5, 9b- hexahydro- 1*H*- cyclopenta [c] quinolin- 4- yl) benzoic acid **(11b)**

Preparative HPLC: $t_R$ =49

Optical Purity: 99.9% ee

$[\alpha]^{Na}_D$=- 38.6 (0.5, $CHCl_3$)

Endo- isomer propyl **12:** 4- ((3a*S*, 4*R*, 9b*R*)- 2, 3, 3a, 4, 5, 9b- hexahydro- 8- propyl- 1*H*- cyclopenta [c] quinolin- 4- yl) benzoic acid.

$^1$H NMR (400 MHz, DMSO- $d_6$) $\delta$ 7.92 (2H, d, *J*=8Hz), 7.54 (2H, d, *J*=8Hz), 6.83 (1 H, s), 6.71 (1 H, d, *J*=8Hz), 6.61 (1 H, d, *J*= 8Hz), 5.58 (1 H, s), 4.50 (1 H, d, *J*=2Hz), 3.39- 3.36 (1 H, m), 2.44- 2.38 (3H, m), 2.08- 2.03 (1 H, m), 1.71- 1.68 (1 H, m), 1.55- 1.48 (3H, m), 1.40- 1.35 (2H, m), 1.03- 0.98 (1 H, m), 0.89- 0.85 (3H, m) .

LC- MS: $t_R$ = 11.17 $[M+H]^+$ = 336 (method 1)

***Example 13: 4-((3aS, 4R, 9bR)-8-fluoro-2, 3, 3a, 4, 5, 9b-hexahydro-1H-cyclopenta[c]quinolin-4-yl)benzoic acid***

The following compound was prepared using the same methodology as in intermediate compound 67 from **intermediate compound 49** for 1 h. Solvent was evaporated and purification of the crude material by reverse $C_{18}$ chromatography using water (0.1% formic acid) and acetonitrile (0.1% formic acid) as eluents afforded the endo- isomer 4- ((3a*S*, 4*R*, 9b*R*)- 8- fluoro- 2, 3, 3a, 4, 5, 9b- hexahydro- 1*H*- cyclopenta [c] quinolin- 4- yl) benzoic acid (121 mg. Yield: 35%) .

$^1$H NMR (400 MHz, DMSO- $d_6$) $\delta$ 7.93 (2H, d, *J*= 8.4Hz), 7.54 (2H, d, *J*=8Hz), 6.85 (1 H, dd, *J*=2.4 & 10Hz), 6.75- 6.68 (2H, m), 5.77 (1 H, s), 4.55 (1 H, d, *J*= 2.8Hz), 3.41- 3.38 (1 H, m), 2.47- 2.45 (1 H, m), 2.09- 2.04 (1 H, m), 1.70- 1.67 (1 H, m), 1.52- 1.49 (1 H, m), 1.41- 1.36 (2H, m), 1.03- 1.01 (1 H, m) .

LC- MS: $t_R$ = 10.07 $[M+H]^+$ = 312 (method 1)

***Example 14: 4-((3aS,4R,9bR)-2,3,3a,4,5,9b-hexahydro-8-phenyl-1H-cyclopenta[c]quinolin-4-yl)benzoic acid***

The following compound was prepared using the same methodology as in intermediate compound 67 from **intermediate compound 50** (70 mg, 0.19 mmol) for 4 h. Purification of the crude material using reverse $C_{18}$ chromatography using water (0.1% formic acid) and acetonitrile (0.1% formic acid) as eluents to give 45 mg of the title compound which was purified again by silica chromatography using dichloromethane and methanol (6%) to obtain 21 mg (30%) of the pure endo-isomer.

$^1$H NMR (400 MHz, DMSO- $d_6$) $\delta$ 7.93 (2H, d, *J* = 8.4 Hz), 7.58- 7.52 (4H, m), 7.38- 7.32 (3H, m), 7.22 (2H, dd, *J* = 8 & 0.8 Hz), 6.77 (1 H, d, *J* = 8.4 Hz), 5.97 (1H, s), 4.59 (1H, d, *J* = 2.4 Hz), 3.46 (1H, t, *J* = 7.2 Hz), 2.12- 2.05 (1 H, m), 1.85- 1.78 (1 H, m), 1.58- 1.48 (1 H, m), 1.50- 1.44 (1 H, m), 1.42- 1.35 (2H, m), 1.07- 1.00 (1 H, m) .

LC- MS: $t_R$ = 11.25 $[M+H]^+$= 370 (method 1)

***Example 15: 4-((3aS, 4R, 9bR)-8-tert-butyl2, 3, 3a, 4, 5, 9b-hexahydro-1H-cyclopenta[c]quinolin-4-yl)benzoic acid***

The following compound was prepared using the same methodology as in intermediate compound 67 from **intermediate compound 51** for 1 h. Solvent was evaporated and purification of the crude material by reverse $C_{18}$ chromatography using water (0.1% formic acid) and acetonitrile (0.1% formic acid) as eluents afforded the endo- isomer 4- ((3a*S*, 4*R*, 9b*R*)- 8- *tert*- butyl- 2, 3, 3a, 4, 5, 9b- hexahydro- 1*H*- cyclopenta [c] quinolin- 4- yl) benzoic acid (140 mg. Yield: 42%) .

$^1$H NMR (400 MHz, DMSO- $d_6$) $\delta$ 7.93 (2H, d, *J*=8Hz), 7.54 (2H, d, *J*=8Hz), 7.01 (1 H, d, *J*=2Hz), 6.93 (1 H, dd, *J*=2.4 & 8.4Hz), 6.62 (1 H, d, *J*= 8.4Hz), 5.58 (1 H, s), 4.50 (1 H, d, *J*= 2.8Hz), 3.41- 3.38 (1 H, m), 2.51- 2.43 (1 H, m), 2.11- 2.06 (1 H, m), 1.73- 1.69 (1 H, m), 1.55- 1.50 (1 H, m), 1.42- 1.36 (2H, m), 1.23 (9H, s), 1.04- 1.00 (1 H, m) .

LC- MS: $t_R$ = 11.25 $[M+H]^+$ = 350 (method 1)

***Example 16: (+)-4-((3aS,4R,9bR)-8-ethyl-2,3,3a,4,5,9b-hexahydro-1H-cyclopenta[c]quinolin-4-yl)benzoic acid (16a) acid & (-)-4-((3aS,4R,9bR)-8-ethyl-2,3,3a,4,5,9b-hexahydro-1H-cyclopenta[c]quinolin-4-yl)benzoic acid (16b)***

The following compound was prepared using the same methodology as in intermediate compound 67 from **intermediate compound 52** (230 mg, 0.72 mmol) for 1 h. Purification of the crude material using reverse $C_{18}$ chromatography using water (0.1% formic acid) and acetonitrile (0.1% formic acid) as eluents to give 90 mg (52%) of the racemic title compound.

$^1$H NMR (400 MHz, DMSO- $d_6$) $\delta$ 7.91 (2H, d, *J*= 8.4 Hz), 7.52 (2H, d, *J*= 8.4 Hz), 6, 83 (1 H, s), 6.72 (1 H, dd, *J* = 8.0 & 1.6 Hz), 6.60 (1 H, d, *J* = 8.4 Hz), 5.56 (1 H, s), 4.48 (1 H, d, *J* = 2.8 Hz), 3.37- 3.30 (1 H, m), 2.47- 2.40 (2H, q, *J* = 7.6 Hz), 2.07- 2.01 (1 H, m), 1.70- 1.66 (1 H, m), 1.53- 1.48 (1 H, m), 1.38- 1.33 (2H, m), 1.10 (1 H, t, *J*= 8.0 Hz), 1.02- 0.98 (1 H, m) .

LC- MS: $t_R$ = 8.67 $[M+H]^+$= 322 (method 1)

Separation of 900 mg of (+/- )- 4- ((3a*S*, 4*R*, 9*bR*)- 8- ethyl- 2, 3, 3a, 4, 5, 9b- hexahydro- 1H- cyclopenta [c] quinolin- 4- yl) benzoic acid **(16)** by preparative chiral chromatography was carried out using Heptane/IPA (90: 10) as eluents to yield:

369 mg of the enantiomer 1: (+)- 4- ((3a*S*, 4*R*, 9b*R*)- 8- ethyl- 2, 3, 3a, 4, 5, 9b- hexahydro- 1H- cyclopenta [c] quinolin- 4- yl) benzoic acid **(16a)**

Preparative HPLC: $t_R$ = 12

Optical Purity: 99.9% ee

$[\alpha]^{Na}_D$= + 51.0 (0.5, $CHCl_3$)

339 mg of the enantiomer 2: (- )- 4- ((3a*S*, 4*R*, 9b*R*)- 8- ethyl- 2, 3, 3a, 4, 5, 9b- hexahydro- 1H- cyclopenta [c] quinolin- 4- yl) benzoic acid **(16b)**

Preparative HPLC: $t_R$ =27

Optical Purity: 99.9% ee

$[\alpha]^{Na}_D$=- 49.9 (0.5, $CHCl_3$)

***Example 17: 4-((3aS,4R,9bR)-8-carbamoyl-2,3,3a,4,5,9b-hexahydro-1H-cyclopenta[c]quinolin-4-yl)benzoic acid***

The following compound was prepared using the same methodology as in intermediate compound 67 from **intermediate compound 53** (300 mg, 0.72 mmol) for 1 h. Purification of the crude material using precipitation in acetonitrile, filtration and dryness gave 200 mg (67%) of the title compound.

$^1$H NMR (400 MHz, DMSO- $d_6$) $\delta$ 7.93 (2H, d, *J*= 8.0 Hz), 7.61 (1 H, s), 7.53 (2H, d, *J* = 8.8 Hz), 7.44 (1 H, dd, *J* = 8.4 & 1.6 Hz), 6.85 (1 H, s), 6.66 (1 H, d, *J* = 8.4 Hz), 6, 34 (1 H, s), 4.64 (1 H, d, *J* = 3.2 Hz), 3.39 (1 H, t, *J* = 6.8

Hz), 2.44- 2.41 (1 H, m), 2.09- 1.98 (1 H, m), 1.89- 1.84 (1 H, m), 1.51- 1.32 (3H, m), 1.06- 0.99 (1 H, m) .

LC- MS: $t_R$ = 4.58 $[M+H]^+$= 337 (method 1)

*Example 18: 4-((3aS,4R,9bR)-2,3,3a,4,5,9b-hexahydro-8-isopropyl-1H-cyclopenta[c]quinolin-4-yl)benzoic acid*

The following compound was prepared using the same methodology as in intermediate compound 67 from **intermediate compound 54** (236 mg, 0.70 mmol) for 1 h. Purification of the crude material using reverse $C_{18}$ chromatography using water (0.1% formic acid) and acetonitrile (0.1% formic acid) as eluents afforded 130 mg (55%) of the title compound.

$^1$H NMR (400 MHz, DMSO- $d_6$) δ 7.91 (2H, d, *J* = 8.4 Hz), 7.53 (2H, d, *J* = 8.4 Hz), 6.85 (1H, d, *J* = 1.6 Hz), 6.76 (1H, dd, *J* = 8.0 & 2 Hz), 6.60 (1H, d, *J* = 8.4 Hz), 5.56 (1 H, s), 4.48 (1 H, d, *J* = 3.2 Hz), 3.40- 3.30 (1 H, m), 2.74- 2.67 (1 H, m), 2.44- 1.37 (1 H, m), 2.08- 2.01 (1 H, m), 1.72- 1.65 (1 H, m), 1.53- 1.45 (1 H, m), 1.40- 1.31 (2H, m), 1.13 ( 6H, dd, *J* = 6.8 & 2 Hz) .

LC- MS: $t_R$ = 9.07 $[M+H]^+$= 336 (method 1)

*Example 19: 4-((3aS,4R,9bR)-8-cyano-2,3,3a,4,5,9b-hexahydro-1H-cyclopenta[c]quinolin-4-yl)benzoic acid*

The following compound was prepared using the same methodology as in intermediate compound 67 from **intermediate compound 55** (180 mg, 0.57 mmol) for 1 h. Purification of the crude material using reverse $C_{18}$ chromatography using water (0.1% formic acid) and acetonitrile (0.1% formic acid) as eluents to give 104 mg (58%) of the title compound.

$^1$H NMR (400 MHz, DMSO- $d_6$) δ 7.93 (2H, d, *J*= 8.0 Hz), 7.51 (2H, d, *J*= 8.0 Hz), 7.42 (1 H, s), 7.27 (1 H, dd, *J* = 8.8 & 2.0 Hz), 6, 88 (1 H, s), 6.76 (1 H, d, *J* = 8.8 Hz), 4.71 (1 H, d, *J* = 3.2 Hz), 3.36 (1 H, t, *J* = 6.8 Hz), 2.44- 2.41 (1 H, m), 2.05- 1.99 (1 H, m), 1.87- 1.83 (1 H, m), 1.42- 1.34 (2H, m), 1.05- 1.00 (1 H, m) .

LC- MS: $t_R$ = 6.80 $[M+H]^+$= 319 (method 1)

*Example 20: 4-((3aS,4R,9bR)-8-[(N-methylsulfonyl)amino])-2,3,3a,4,5,9b-hexahydro-1H-cyclopenta[c]quinolin-4-yl)benzoic acid*

The following compound was prepared using the same methodology as in intermediate compound 67 from **intermediate compound 56** (239 mg, 0.62 mmol) for 1 h. Purification of the crude material by reverse C18 chromatography using water (0.1% formic acid) and acetonitrile (0.1% formic acid) as eluents afforded the title-compound slightly impure, which was purified again using reverse $C_{18}$ chromatography eluting with water (0.1% HCOOH) and acetonitrile (0.1% HCOOH) to give 99 mg of the pure title endo-isomer.

$^1$H NMR (400 MHz, DMSO- $d_6$) δ 7.93 (2H, d, *J*= 8.4 Hz), 7.53 (2H, d, *J*= 8.4 Hz), 6.91 (1 H, d, *J* = 2 Hz), 6.80 (1 H, dd, *J* = 2.4 & 8.4 Hz), 6.67 (1 H, d, *J* = 8.8 Hz), 5.82 (1 H, brs), 4.54 (1 H, d, *J* = 2.8 Hz), 3.41- 3.36 (1 H, m), 2.84 (3H, s), 2.50- 2.41 (1 H, m), 2.12- 2.02 (1 H, m), 1.71- 1.64 (1 H, m), 1.56- 1.46 (1 H, m), 1.42- 1.34 (2H, m), 1.06- 0.98 (1 H, m) .

LC- MS: $t_R$ =7.55 $[M+H]^+$= 387 (method 1)

*Example 21: 4-((3aS,4R,9bR)-8-[(N-methyl-N-methylsulfonyl)amino]-2,3,3a,4,5,9b-hexahydro-1H-cyclopenta[c]quinolin-4-yl)benzoic acid*

The following compound was prepared using the same methodology as in intermediate compound 67 from **intermediate compound 57** (250 mg, 0.62 mmol) for 1 h. Purification of the crude material by reverse C18 chromatography using water (0.1% formic acid) and acetonitrile (0.1% formic acid) as eluents gave 162 mg of the title compound, which was triturated in acetonitrile-drops of water, filtered and dried to obtain 83 mg the pure title endo-isomer as a solid (Yield: 33%)

1 H NMR (400 MHz, $CD_3OD$) δ 8.01 (2H, d, *J* = 8 Hz), 7.56 (2H, d, *J* = 8.4 Hz), 7.12 (1 H, d, *J* = 1.6Hz), 6.99 (1H, dd, *J* = 2.4 & 8.4 Hz), 6.70 (1H, d, *J* = 8.4 Hz), 4.62 (1 H, d, *J* = 3.2 Hz), 3.50- 3.45 (1 H, m), 3.24 (3H, s), 2.87 (3H, s), 2.57- 2.50 (1 H, m), 2.22- 2.12 (1 H, m), 1.85- 1.77 (1 H, m), 1.71- 1.61 (1 H, m), 1.57- 1.40 (2H, m), 1.19- 1.10 (1 H, m) .

LC- MS: $t_R$ =8.38 $[M+H]^+$= 401 (method 1)

*Example 22: 3-((3aS,4R,9bR)-8-bromo-2,3,3a,4,5,9b-hexahydro-1H cyclopenta[c]quinolin-4-yl)benzoic acid*

The following compound was prepared using the same methodology as in intermediate compound 67 from **intermediate compound 58** (110 mg, 0.29 mmol) for 1 h. Purification of the crude material using reverse $C_{18}$ chromatography using water (0.1% formic acid) and acetonitrile (0.1% formic acid) as eluents gave 48 mg of the title compound

which was precipitated in hexane, filtered and dried in vacuum to obtain 39 mg (35%) of the pure endo-isomer.

$^1$H NMR (400 MHz, DMSO- $d_6$) δ 12.95 (1 H, brs), 8.07 (1 H, s), 7.83 (1 H, d, *J* = 7.6 Hz), 7.63 (1 H, d, *J* = 7.6 Hz), 7.47 (1 H, t, *J* = 7.2 Hz), 7.14 (1 H, d, *J* = 1.6 Hz), 7.02 (1 H, d, *J* = 8.4 & 2.0 Hz), 6.65 (1 H, d, *J* = 8.4 Hz), 6.03 (1 H, s), 4.53 (1 H, d, *J* = 2.8 Hz), 3.39- 3.34 (1 H, m), 2.45- 2.39 (1 H, m), 2.08- 2.01 (1 H, m), 1.70- 1.66 (1 H, m), 1.50- 1.44 (1 H, m), 1.41- 1.36 (2H, m), 1.01- 0.97 (1 H, m) .

LC- MS: $t_R$ = 8.52 $[M+H]^+$= 372/374 (method 1)

***Example 23: 4-((3aS,4R,9bR)-2,3,3a,4,5,9b-hexahydro-7,8-dimethoxy-1H-cyclopenta[c]quinolin-4-yl)benzoic acid***

The following compound was prepared using the same methodology as in intermediate compound 67 from **intermediate compound 59** (0.69 mmol, 243 mg) for 1 h. Purification of the crude material using reverse $C_{18}$ chromatography using water (0.1% formic acid) and acetonitrile (0.1% formic acid) as eluents to give 63 mg (26%) of the title pure endo-isomer.

$^1$H NMR (400 MHz, DMSO- $d_6$) : δ 7.92 (2H, d, *J* = 8.4 Hz), 7.54 (2H, d, *J* = 8 Hz), 6.60 (1 H, s), 6.38 (1 H, s), 5.47 (1 H, s), 4.47 (1 H, d, *J* = 2.4 Hz), 3.66 (3H, s), 3.65 (3H, s), 3.35- 3.30 (1 H, m), 2.50- 2.41 (1 H, m), 2.05- 1.95 (1 H, m), 1.74- 1.68 (1 H, m), 1.55- 1.48 (1 H, m), 1.39- 1.32 (1 H, m), 1.04- 0.97 (1 H, m)

LC- MS: $t_R$ = 8.72 $[M+H]^+$= 354 (method 1)

***Example 24 and 25: 4-((3aS,4R,9bR)-7-(trifluoromethyl)-2,3,3a,4,5,9b-hexahydro-8-methyl-1H-cyclopenta[c]quinolin-4-yl)benzoic acid (24) and 4-((3aS,4R,9bR)-9-(trifluoromethyl)-2,3,3a,4,5,9b-hexahydro-8-methyl-1H-cyclopenta[c]quinolin-4-yl)benzoic acid (25)***

The following compounds were prepared using the same methodology as in intermediate compound 67 from the **intermediate mixture of compounds 60 and 61** (1.27 mmol, 475 mg) for 1 h. Solvent was evaporated and the crude material (a mixture of endo- 8, 9- disustituted and endo- 7, 8- disustituted regioisomers) was purified by reverse $C_{18}$ chromatography using water (0.1% formic acid) and acetonitrile (0.1% formic acid) as eluents. Only high purity fractions of each regioisomer were collected giving two enriched fractions with each regioisomer, which were purified again by precipitation in hexane to give 30.7 mg of 4- ((3aS, 4R, 9bR)- 7- (trifluoromethyl)- 2, 3, 3a, 4, 5, 9b- hexahydro- 8- methyl- 1H- cyclopenta [c] quinolin- 4- yl) benzoic acid **(24)** and 55.6 mg of 4- ((3aS, 4R, 9bR)- 9- (trifluoromethyl)- 2, 3, 3a, 4, 5, 9b- hexahydro- 8- methyl- 1 H- cyclopenta [c] quinolin- 4- yl) benzoic acid **(25)** . (Overall yield=18%) .

4- ((3aS, 4R, 9bR)- 7- (trifluoromethyl)- 2, 3, 3a, 4, 5, 9b- hexahydro- 8- methyl- 1H- cyclopenta [c] quinolin- 4- yl) benzoic acid (endo- 7, 8- disustituted regioisomer **24)**

$^1$H NMR (400 MHz, DMSO- $d_6$) : δ 7.93 (2H, d, *J* = 8.4 Hz), 7.53 (2H, d, *J* = 8.4 Hz), 6.96 (2H, s), 5.91 (1 H, s), 4.30 (1 H, d, *J* = 3.6 Hz), 3.83- 3.77 (1 H, m), 2.67- 2.63 (1 H, m), 2.31 (3H, d, J = 3.2 Hz), 2.21- 2.15 (1 H, m), 1.58- 1.42 (2H, m), 1.33- 1.15 (2H, m), 1.04- 0.98 (1 H, m) .

LC- MS: $t_R$ = 11.03 $[M+H]^+$= 376 (method 1)

4- ((3aS, 4R, 9bR)- 9- (trifluoromethyl)- 2, 3, 3a, 4, 5, 9b- hexahydro- 8- methyl- 1H- cyclopenta [c] quinolin- 4- yl) benzoic acid (endo- 8, 9- disustituted regioisomer **25)**

$^1$H NMR (400 MHz, DMSO- $d_6$) : δ 7.94 (2H, d, *J*= 8.4 Hz), 7.54 (2H, d, *J*= 8 Hz), 7.05 (2H, d, *J* = 9.2 Hz), 6.09 (1 H, s), 4.56 (1 H, d, *J* = 3.2 Hz), 3.42- 3.39 (1 H, m), 2.27 (3H, d, *J* = 1.2 Hz), 2.14- 2.04 (1 H, m), 1.79- 1.72 (1 H, m), 1.53- 1.34 (3H, m), 1.07- 0.99 (1 H, m) .

LC- MS: $t_R$ = 11.12 $[M+H]^+$= 376 (method 1)

***Example 26: 4-((3aS,4R,9bR)-2,3,3a,4,5,9b-hexahydro-7-methoxy-8-methyl-1H-cyclopenta[c]quinolin-4-yl)benzoic acid***

The following compound was prepared using the same methodology as in intermediate compound 67 from **intermediate compound 62** (0.48 mmol, 162 mg) for 1 h. Purification of the crude material (1: 6 mixture of endo- 8, 9- disustituted & endo- 7, 8- disustituted regioisomers) by reverse $C_{18}$ chromatography using water (0.1% formic acid) and acetonitrile (0.1% formic acid) as eluents only allowed to obtain 62 mg of the major title endo- 7, 8- disustituted regioisomer **(26)** (Yield: 38%) .

$^1$H NMR (400 MHz, DMSO- $d_6$) : δ 7.92 (2H, d, *J* = 8.4 Hz), 7.54 (2H, d, *J* = 8.4 Hz, ), 6.75 (1 H, s), 6.32 (1 H, s) , 5.59 (1 H, s), 4.51 (1 H, d, *J* = 2.4 Hz), 3.67 (3H, s), 2.50- 2.37 (m, 2H), 2.01 (3H, s), 1.99- 1.93 (1 H, m), 1.73- 1.67 (1 H, m), 1.57- 1.45 (1 H, m), 1.39- 1.31 (2H, m), 1.03- 0.95 (1 H, m) .

LC- MS: $t_R$ = 10.52 $[M+H]^+$= 338 (method 1)

***Example 27: 4-((3aS,4R,9bR)-2,3,3a,4,5,9b-hexahydro-6,8-dimethyl-1H-cyclopenta[c]quinolin-4-yl)benzoic acid***

The following compound was prepared using the same methodology as in intermediate compound 67 from **intermediate compound 63** (1.44 mmol, 460 mg) for 1 h. Purification of the crude material using reverse $C_{18}$ chromatography using water (0.1% formic acid) and acetonitrile (0.1% formic acid) as eluents to give 280 mg of the title compound which was precipitated in acetonitrile/drops of water, filtered and dried in vacuum to obtain 193 mg (42%) of the pure endo-isomer.

$^1$H NMR (400 MHz, DMSO- $d_6$) : δ 7.93 (2H, d, *J*= 8 Hz), 7.59 (2H, d, *J*= 8.4 Hz), 6.73 (1 H, s), 6.64 (1 H, s), 4.59 (1 H, s), 4.53 (1 H, d, *J* = 2.4 Hz), 3.41- 3.33 (1 H, m), 2.48- 2.40 (1H, m), 2.14 (3H, s), 2.11 (3H, s), 2.07- 1.99 (1H, m), 1.76- 1.69 (1 H, m), 1.56- 1.46 (1 H, m), 1.38- 1.30 (2H, m), 1.04- 0.96 (1 H, m) .

LC- MS: $t_R$ = 11.15 $[M+H]^+$= 322 (method 1)

***Example 28: 4-((3aS,4R,9bR)-9-chloro-2,3,3a,4,5,9b-hexahydro-8-methyl-1H-cyclopenta[c]quinolin-4-yl)benzoic acid***

The following compound was prepared using the same methodology as in intermediate compound 67 from **intermediate compound 64** (0.26 mmol, 88 mg) for 1 h. Purification of the crude material using reverse $C_{18}$ chromatography using water (0.1% formic acid) and acetonitrile (0.1% formic acid) as eluents gave 28 mg of the pure endo-isomer (Yield: 31%).

$^1$H NMR (400 MHz, DMSO- $d_6$) : δ 7.93 (2H, d, *J*= 8 Hz), 7.53 (2H, d, *J*= 8.4 Hz), 6.89 (1 H, d, *J* = 8.4 Hz), 6.69 (1 H, d, *J* = 8.4 Hz), 5.76 (1 H, s), 4.31 (1 H, d, *J* = 3.6 Hz), 3.57- 3.51 (1 H, m), 2.65- 2.57 (1 H, m), 2.47- 2.39 (1 H, m), 2.21 (3H, s), 1.60- 1.44 (2H, m), 1.34- 1.18 (2H, m), 1.06- 1.00 (m, 1H) .

LC- MS: $t_R$ = 11.15 min $[M+H]^+$= 342 (method 1)

***Example 29: 4-((3aS,4R,9bR)-7-chloro-2,3,3a,4,5,9b-hexahydro-8-methyl-1H-cyclopenta[c]quinolin-4-yl)benzoic acid***

The following compound was prepared using the same methodology as in intermediate compound 67 from **intermediate compound 65** (0.17 mmol, 58 mg) for 1 h. Purification of the crude material using reverse $C_{18}$ chromatography using water (0.1% formic acid) and acetonitrile (0.1% formic acid) as eluents afforded 28 mg of the pure endo-isomer (Yield: 47%).

$^1$H NMR (400 MHz, DMSO- $d_6$) : δ 7.93 (2H, d, *J* = 8.4 Hz), 7.53 (2H, d, *J* = 8.4 Hz), 6.98 (1 H, s), 6.75 (1 H, s), 5.92 (1 H, s), 4.53 (1 H, d, *J* = 3.2 Hz), 3.32 (1 H, m), 2.48- 2.40 (1 H, m), 2.17 (3H, s), 2.08- 1.99 (1 H, m), 1.76- 1.69 (1 H, m), 1.53- 1.44 (1 H, m), 1.41- 1.33 (2H, m), 1.05- 0.97 (1 H, m) .

LC- MS: $t_R$ = 11.12 $[M+H]^+$= 342 (method 1)

***Example 30 : 4-((3aS,4R,9bR)-2,3,3a,4,5,9b-hexahydro-5,8-dimethyl-1H-cyclopenta[c]quinolin-4-yl)benzoic acid***

To a solution of methyl 4- ((3aS, 4R, 9bR)- 2, 3, 3a, 4, 5, 9b- hexahydro- 5, 8- dimethyl- 1H- cyclopenta [c] quinolin- 4- yl) benzoate **(intermediate compound 68)** (50 mg, 0.15 mmol) in THF (5 mL) was added 1M LiOH (0.30 mL, 0.30 mmol) . The reaction mixture was stirred 16 h at room temperature. Total conversion was not achieved, then 1 N LiOH (0.3 mmol) was added and was stirred for 48 h at room temperature. Solvent was evaporated and purification of the crude material by flash chromatography on silica gel using an elution of 8% MeOH in DCM afforded the title compound (32 mg. Yield: 66%) .

$^1$H NMR (400 MHz, DMSO- $d_6$) δ 7.88 (2H, d, *J*= 8Hz), 7.37 (2H, d, *J*= 8Hz), 6.91 (1 H, s), 6.87 (1 H, d, *J*= 8Hz), 6.69 (1 H, d, *J*= 8.4Hz), 4.42 (1 H, d, *J*= 4.4Hz), 3.26- 3.24 (1 H, m), 2.63 (3H, s), 2.46- 2.38 (1 H, m), 2.19 (3H, s), 2.01- 1.96 (1 H, m), 1.79- 1.74 (1 H, m), 1.60- 1.55 (1 H, m), 1.38- 1.35 (1 H, m), 1.29- 1.21 (1 H, m), 1.19- 1.16 (1 H, m) .

LC- MS: $t_R$ = 11.20 $[M+H]^+$= 322 (method 1)

***Example 31: 4-((3aS,4R,9bR)-5-acetyl-2,3,3a,4,5,9b-hexahydro-8-methyl-1H-cyclopenta[c]quinolin-4-yl)benzoic acid***

The following compound was prepared using the same methodology as in example 30 from **intermediate compound 69** (110 mg, 0.30 mmol) using 1 N LiOH (3 eq.) for 16h. Solvent was evaporated and the crude residue was dissolved in ethyl acetate and water. Aqueous layer was separated and acidified using 1 N HCl until pH 2. Ethyl acetate was added and the organic layer was extracted, washed with brine and dried over anhydrous sodium sulphate, filtered and concentrated to obtain 113 mg of the title compound, which was triturated using hexane. The white solid was filtered and washed with hexane, dried over vacuum to afford 70 mg of the pure endo-isomer (67%).

$^1H$ NMR (400 MHz, $CDCl_3$) δ 7.85 (2H, d, *J* = 8.4 Hz), 7.15 (1 H, s), 7.05- 7.02 (4H, m), 6.16 (1 H, brs), 3.16- 3.03 (2H, m), 2.39 (3H, s), 2.20 (3H, s), 2.18- 2.10 (1 H, m), 1.94- 1.88 (1 H, m), 1.52- 1.37 (3H, m), 0.88- 0.81 (1 H, m) .
LC- MS: $t_R$ = 9.37 min $[M+H]^+$= 350 (method 1)

***Example 32: 4-((6R, 6aS, 9aR)-6, 6a, 7, 8, 9, 9a-hexahydro-2-methyl-5H-cyclopenta[c][1,5]naphthyridin-6-yl)benzoic acid***

The following compound was prepared using the same methodology as in intermediate compound 67 from **intermediate compound 71** for 1 h. Solvent was evaporated. Due to the insolubility of the crude material in all solvents it was purified by precipitation and filtration to give the endo- isomer 4- ((6R, 6aS, 9aR)- 6, 6a, 7, 8, 9, 9a- hexahydro- 2- methyl- 5*H*- cyclopenta [c] [1, 5] naphthyridin- 6- yl) benzoic acid (15 mg. Yield: 37%) .
$^1H$ NMR (400 MHz, DMSO- $d_6$) δ 7.93 (2H, d, *J*=8.4Hz*),* 7.56 (2H, d, *J*=8.4Hz), 6.93 (1 H, d, *J*=8Hz), 6.78 (1 H, d, *J*=8.4Hz), 5.81 (1 H, s), 4.60 (1 H, d, *J*=2.8Hz), 3.39- 3.31 (1 H, m), 2.49 (1 H, m), 2.30 (3H, s), 2.08- 1.97 (2H, m), 1.51- 1.44 (1 H, m), 1.40- 1.35 (2H, m), 1.08- 1.00 (1 H, m) .
LC- MS: $t_R$ = 5.13 $[M+H]^+$ = 309 (method 1)

***Example 33: 4-((6R,6aS,9aR)-6,6a,7,8,9,9a-hexahydro-2-methyl-5H-cyclopenta[c][1,8]naphthyridin-6-yl)benzoic acid***

To a solution of **intermediate compound 72** (3.23 mmol, 0.70 g) in EtOH : THF (1:1, 18 mL), 10 % Pd/C (10% w/w, 10 mg) was added and the mixture was hydrogenated at room temperature for 4 h. HPLC analysis showed total conversion. Reaction mixture was filtered and filter washed by THF. Solvent was evaporated and purification of the crude material using reverse $C_{18}$ chromatography using water (0.1% formic acid) and acetonitrile (0.1% formic acid) as eluents gave 11.7 mg of the title compound as a solid. (Yield= 6%)
1 H NMR (400 MHz, DMSO-$d_6$) δ 7.99 (1 H, brs), 7.89 (2H, d, *J* = 8.4 Hz), 7.72 (1 H, dd, *J* = 1.6 & 9.2 Hz), 7.39 (1 H, d, *J* = 8 Hz), 7.20 (1 H, d, *J* = 8.8 Hz), 5.03 (1 H, d, *J* = 3.6 Hz), 4.86-4.82 (1 H, m), 2.91-2.82 (1 H, m), 2.34-2.25 (1 H, m), 2.20 (3H, s), 2.21-2.12 (1H, m), 1.57-1.31 (3H, m), 1.31-1.22 (1H, m).
LC- MS: $t_R$ =4.68 $[M+H]^+$= 309 (method 1)

***Example 34: 2-(4-((3aS, 4R, 9bR)-2, 3, 3a, 4, 5, 9b-hexahydro-8-methyl-1H-cyclopenta[c]quinolin-4-yl)phenyl) acetic acid***

The following compound was prepared using the same methodology as in example 30 from **intermediate compound 74** (74 mg, 0.22 mmol) using 1M LiOH ( 0.33 mL, 0.33 mmol) for 16 h. Purification of the crude material by reverse $C_{18}$ chromatography using water (0.1% formic acid) and acetonitrile (0.1% formic acid) as eluents afforded the endo- isomer 2- (4- ((3a*S*, 4*R*, 9b*R*)- 2, 3, 3a, 4, 5, 9b- hexahydro- 8- methyl- 1*H*- cyclopenta [*c*] quinolin- 4- yl) phenyl) acetic acid (32 mg. Yield: 45%) .
$^1H$ NMR (400 MHz, DMSO- $d_6$) δ 7.35 (2H, d, *J*= 8Hz), 7.22 (2H, d, *J=8.4Hz),* 6.82 (1 H, s), 6.68 (1 H, d, *J*=8Hz*),* 6.57 (1 H, d, *J*=8Hz), 5.43 (1 H, s), 4.39 (1 H, d, *J*=2.4Hz), 3.53 (2H, s), 2.41- 2.38 (1H, m), 2.15 (3H, s), 2.06- 20.1 (1H, m), 1.71- 1.67 (1 H, m), 1.59- 1.53 (1 H, m), 1.40- 1.35 (2H, m), 1.09- 1.04 (1 H, m) .
LC- MS: $t_R$ = 9.85 $[M+H]^+$ = 322 (method 1)

***Example 35: 3-(4-((3aS, 4R, 9bR)-2, 3, 3a, 4, 5, 9b-hexahydro-8-methyl-1H-cyclopenta[c]quinolin-4-yl)phenyl) propanoic acid***

The following compound was prepared using the same methodology as in intermediate compound 67 from **intermediate compound 75** for 1 h. Solvent was evaporated and purification of the crude material by reverse $C_{18}$ chromatography using water (0.1% formic acid) and acetonitrile (0.1% formic acid) as eluents afforded the endo- isomer 3- (4- ((3a*S*, 4*R*, 9b*R*)- 2, 3, 3a, 4, 5, 9b- hexahydro- 8- methyl- 1*H*- cyclopenta [*c*] quinolin- 4- yl) phenyl) propanoic acid (10 mg. Yield: 25%)
$^1H$ NMR (400 MHz, DMSO- $d_6$) δ 7.32 (2H, d, *J*= 8Hz), 7.19 (2H, d, *J*=8Hz), 6.81 (1H, s), 6.68 (1 H, d, *J*= 8Hz), 6.57 (1 H, d, *J*=8Hz), 5.42 (1H, s), 4.37 (1H, d, *J*=2.4Hz), 2.81 (2H, t, *J*=7.6Hz), 2.38- 2.33 (1 H, m), 2.15 (3H, s), 2.06- 2.01 (1 H, m), 1.70- 1.66 (1 H, m), 1.58- 1.53 (1 H, m), 1.40- 1.34 (2H, m), 1.08- 1.05 (1 H, m) .
LC- MS: $t_R$ = 10.07 $[M+H]^+$ = 336 (method 1)

***Example 36: 4-((3aS, 4R, 9bR)-2, 3, 3a, 4, 5, 9b-hexahydro-8-methyl-1H-cyclopenta[c]quinolin-4-yl)-2-methylbenzoic acid***

The following compound was prepared using the same methodology as in intermediate compound 67 from **intermediate compound 76** for 1 h. Solvent was evaporated and purification of the crude material by reverse $C_{18}$ chromatography using water (0.1% formic acid) and acetonitrile (0.1% formic acid) as eluents to give the endo- isomer 4- ((3a*S*, 4*R*, 9b*R*)- 2, 3, 3a, 4, 5, 9b- hexahydro- 8- methyl- 1*H*- cyclopenta [c] quinolin- 4- yl)- 2- methylbenzoic acid (10 mg. Yield: 24%) .

$^1$H NMR (400 MHz, DMSO- $d_6$) $\delta$ 7.81 (1 H, d, *J*=7.6Hz), 7.34- 7.32 (2H, m), 6.83 (1 H, s), 6.70 (1 H, d, *J*=8Hz), 6.60 (1 H, d, *J*=8.4Hz), 5.51 (1 H, s), 4.42 (1 H, d, *J*=2.4Hz), 3.37- 3.33 (1 H, m), 2.53 (3H, s), 2.45- 2.42 (1 H, m), 2.15 (3H, s), 2.07- 2.05 (1 H, m), 1.71- 1.67 (1 H, m), 1.56- 1.50 (1 H, m), 1.40- 1.35 (2H, m), 1.06- 1.02 (1 H, m) .

LC- MS: $t_R$ = 10.63 $[M+H]^+$ = 322 (method 1)

***Example 37: (+/-)-4-((3aS, 4R, 9bR)-2, 3, 3a, 4, 5, 9b-hexahydro-8-methyl-1H-cyclopenta[c]quinolin-4-yl)-3-methylbenzoic acid***

The following compound was prepared using the same methodology as in intermediate compound 67 from **intermediate compound 77** for 1 h. Solvent was evaporated and purification of the crude material by reverse $C_{18}$ chromatography using water (0.1% formic acid) and acetonitrile (0.1% formic acid) as eluents (52% ACN) to give the endo- isomer (+/- )- 4- ((3a*S*, 4*R*, 9b*R*)- 2, 3, 3a, 4, 5, 9b- hexahydro- 8- methyl- 1*H*cyclopenta [*c*] quinolin- 4- yl)- 3- methylbenzoic acid (62 mg. Yield: 53%) .

$^1$H NMR (400 MHz, DMSO- $d_6$) $\delta$ 7.79 (1 H, d, *J*=8Hz), 7.75 (1 H, s), 7.68 (1 H, d, *J*=8.4Hz), 5.38 (1 H, s), 4.61 (1 H, d, *J*=2.8Hz), 3.39- 3.35 (1 H, m), 2.46- 2.43 (1 H, m), 2.37 (3H, s), 2.16 (3H, s), 2.10- 2.05 (1H, m), 1.69- 1.58 (2H, m), 1.41- 1.36 (2H, m), 0.10- 0.96 (1 H, m) .

LC- MS: $t_R$ = 10.72 $[M+H]^+$ = 322 (method 1)

Separation of 400 mg of (+/- )- 4- ((3a*S*, 4*R*, 9b*R*)- 2, 3, 3a, 4, 5, 9b- hexahydro- 8- methyl- 1*H*- cyclopenta [*c*] quinolin- 4- yl)- 3- methylbenzoic acid **(37)** by preparative chiral chromatography was carried out using Heptane/IPA/TFA (95: 5: 0.1) as eluents to yield:

155 mg of the enantiomer 1: (+)- 4- ((3a*S*, 4*R*, 9b*R*)- 2, 3, 3a, 4, 5, 9b- hexahydro- 8- methyl- 1*H*- cyclopenta [c] quinolin- 4- yl)- 3- methylbenzoic acid **(37a)**

Preparative HPLC: $t_R$ = 16

Optical Purity: 99.9% ee

$[\alpha]^{Na}{}_D$= + 23.4 (0.6, $CHCl_3$)

167 mg of the enantiomer 2 **(37b) :** (- )- 4- ((3a*S*, 4*R*, 9b*R*)- 2, 3, 3a, 4, 5, 9b- hexahydro- 8- methyl- 1*H*- cyclopenta [c] quinolin- 4- yl)- 3- methylbenzoic acid **(37b)**

Preparative HPLC: $t_R$ =27

Optical Purity: 99.9% ee

***Example 38: 4-((3aS, 4S, 9bR)-2, 3, 3a, 4, 5, 9b-hexahydro-8-methyl-1H-cyclopenta[c]quinolin-4-yl)-3-methylbenzoic acid***

The following compound was prepared using the same methodology as in example 30 from **intermediate compound 79** (2.22 mmol, 746 mg). It was necessary 72 h to complete the reaction. Solvent was evaporated and purification of the crude material by reverse $C_{18}$ chromatography using water (0.1% formic acid) and acetonitrile (0.1% formic acid) as eluents gave endo-isomer (513 mg) and exo-isomer title compound (10 mg) (Overall yield: 76%).

$^1$H NMR (400 MHz, DMSO- $d_6$) $\delta$ 7.74 (2H, m), 7.46 (1 H, d, *J* = 8.8Hz), 6.87 (1 H, s), 6.69 (1 H, dd, *J* = 8.4 & 1.6 Hz), 5.63 (1 H, s), 3.98 (1 H, d, *J* = 9.6 Hz), 2.92- 2.90 (1 H, m), 2.38 (3H, s), 2.33- 2.31 (1 H, m), 2.29- 2.18 (1 H, m), 2.15 (3H, s), 1.66- 1.54 (4H, m), 1.20- 1.18 (1 H, m) .

LC- MS: $t_R$ = 8.05 $[M+H]^+$ = 322 (method 1)

***Example 39: 4-((3aS, 4R, 9bR)-8-chloro-2, 3, 3a, 4, 5, 9b-hexahydro-1H-cyclopenta[c]quinolin-4-yl)-3-methylbenzoic acid***

The following compound was prepared using the same methodology as in intermediate compound 67 from **intermediate compound 80** for 1 h. Solvent was evaporated and purification of the crude material by reverse $C_{18}$ chromatography using water (0.1% formic acid) and acetonitrile (0.1% formic acid) as eluents afforded the endo- isomer 4- ((3a*S*, 4*R*, 9b*R*)- 8- chloro- 2, 3, 3a, 4, 5, 9b- hexahydro- 1*H*- cyclopenta [*c*] quinolin- 4- yl)- 3- methylbenzoic acid (21 mg. Yield: 33%) .

$^1$H NMR (400 MHz, DMSO- $d_6$) δ 7.81 (1 H, d, *J*=8.4Hz), 7.75 (1 H, s), 7.65 (1 H, d, *J*=8Hz), 7.04 (1H, d, *J*=2Hz), 6.91 (1 H, dd, *J*=2.4Hz & 8.8Hz), 6.70 (1H, d, *J*=8.4Hz), 5.84 (1 H, s), 4.67 (1 H, d, *J*=2.8Hz), 3.43- 3.40 (1 H, m), 2.49- 2.45 (1 H, m), 2.38 (3H, s), 2.12- 2.06 (1 H, m), 1.70- 1.67 (1 H, m), 1.59- 1.54 (1 H, m), 1.42- 1.38 (2H, m), 1.00- 0.98 (1 H, m) .

LC- MS: $t_R$ = 10.93 $[M+H]^+$ = 342 (method 1) .

***Example 40: 4-((3aS, 4R, 9bR)-8-fluoro-2, 3, 3a, 4, 5, 9b-hexahydro-1H cyclopenta[c]quinolin-4-yl)-3-methylbenzoic acid***

The following compound was prepared using the same methodology as in intermediate compound 67 from **intermediate compound 81** for 1 h. Solvent was evaporated and purification of the crude material by reverse $C_{18}$ chromatography using water (0.1% formic acid) and acetonitrile (0.1% formic acid) as eluents afforded the endo- isomer 4- ((3a*S*, 4*R*, 9b*R*)- 8- fluoro- 2, 3, 3a, 4, 5, 9b- hexahydro- 1*H*- cyclopenta [*c*] quinolin- 4- yl)- 3- methylbenzoic acid (4.7 mg. Yield: 8%)

$^1$H NMR (400 MHz, $CD_3OD$) δ 7.85 (1 H, d, *J*=8Hz) , 7.82 (1 H, s), 7.73 (1 H, d, *J*=8Hz), 6.80 (1H, dd, J= 2.8Hz & 10Hz), 6.68- 6.65 (2H, m), 4.70 (1H, d, *J*=2.4Hz) , 3.45- 3.43 (1 H, m), 2.58- 2.55 (1 H, m), 2.42 (3H, s), 2.20- 2.15 (1 H, m), 1.78- 1.69 (2H, m), 1.57- 1.45 (2H, m), 1.13- 1.10 (1H, m) .

LC- MS: $t_R$ = 10.38 $[M+H]^+$ = 326 (method 1)

***Example 41: 4-((3aS, 4R, 9bR)-2, 3, 3a, 4, 5, 9b-hexahydro-8-methoxy-1H cyclopenta[c]quinolin-4-yl)-3-methylbenzoic acid***

The following compound was prepared using the same methodology as in intermediate compound 67 from **intermediate compound 82** for 1 h. Solvent was evaporated and purification of the crude material by reverse $C_{18}$ chromatography using water (0.1% formic acid) and acetonitrile (0.1% formic acid) as eluents afforded the endo- isomer 4- ((3a*S*, 4*R*, 9b*R*)- 2, 3, 3a, 4, 5, 9b- hexahydro- 8- methoxy- 1*H*- cyclopenta [*c*] quinolin- 4- yl)- 3- methylbenzoic acid (71 mg. Yield: 55%) .

$^1$H NMR (400 MHz, DMSO- $d_6$) δ 7.78 (1 H, d, *J*=8Hz), 7.74 (1 H, s), 7.69 (1 H, d, *J=8Hz),* 6.65- 6.62 (2H, m), 6.54 (1H, dd, *J*=2.8Hz & 8.4Hz), 5.23 (1H, s), 4.57 (1 H, d, *J*=2.8Hz), 3.65 (3H, s), 3.41- 3.39 (1 H, m), 2.50- 2.49 (1 H, m), 2.37 (3H, s), 2.11- 2.06 (1 H, m), 1.68- 1.59 (2H, m), 1.41- 1.35 (2H, m), 0.99- 0.96 (1 H, m) .

LC- MS: $t_R$ = 9.35 $[M+H]^+$ = 338 (method 1)

***Example 42: 4-((3aS, 4R, 9bR)-2, 3, 3a, 4, 5, 9b-hexahydro-8-methoxy-5-methyl-1H-cyclopenta[c]quinolin-4-yl)-3-methylbenzoic acid***

To a solution of 4- ((3a*S*, 4*R*, 9b*R*)- 2, 3, 3a, 4, 5, 9b- hexahydro- 8- methoxy- 1*H*- cyclopenta [*c*] quinolin- 4- yl)- 3- methylbenzoic acid **Example 41** (32 mg, 0.098 mmol) in dry methanol (2 mL) anhydrous THF (0.5 mL) was added formaldehyde (0.04 mL, 0.49 mmol, 37 % in water) .The reaction mixture was stirred overnight at room temperature. Then, $NaBH_3CN$ (28 mg, 0.44 mmol) was added and the reaction was stirred for 3 h at room temperature. Solvent was evaporated and purification of the crude material by reverse $C_{18}$ chromatography using water (0.1% formic acid) and acetonitrile (0.1% formic acid) as eluents afforded the endo- isomer 4- ((3a*S*, 4*R*, 9b*R*)- 2, 3, 3a, 4, 5, 9b- hexahydro- 8- methoxy- 5- methyl- 1*H*- cyclopenta [*c*] quinolin- 4- yl)- 3- methylbenzoic acid (23 mg. Yield: 67%) .

$^1$H NMR (400 MHz, DMSO- $d_6$) δ 7.76- 7.74 (2H, m), 7.45 (1 H, d, *J*= 8.4Hz), 6.79 (1 H, d, *J*=9.2Hz), 6.69- 6.67 (2H, m), 4.55 (1 H, d, *J*=3.2Hz), 3.68 (3H, s), 2.57 (3H, s), 2.39 (3H, s), 2.36- 2.32 (1 H, m), 2.00- 1.94 (2H, m), 1.53- 1.50 (1 H, m), 1.38- 1.34 (2H, m), 1.07- 1.05 (1 H, m) .

LC- MS: $t_R$ = 10.62 $[M+H]^+$ = 352 (method 1)

***Example 43: 4-((3aS, 4R, 9bR)-8-cyclopropyl-2, 3, 3a, 4, 5, 9b-hexahydro-1H cyclopenta[c]quinolin-4-yl)-3-methylbenzoic acid***

The following compound was prepared using the same methodology as in intermediate compound 67 from **intermediate compound 83** for 1 h. Solvent was evaporated and purification of the crude material by reverse $C_{18}$ chromatography using water (0.1% formic acid) and acetonitrile (0.1% formic acid) as eluents afforded the endo isomer 4- ((3a*S*, 4*R*, 9b*R*)- 8- cyclopropyl- 2, 3, 3a, 4, 5, 9b- hexahydro- 1*H*- cyclopenta [*c*] quinolin- 4- yl)- 3- methylbenzoic acid (28 mg: Yield: 31%) .

$^1$H NMR (400 MHz, DMSO- $d_6$) δ 7.78 (1 H, d, *J*=7.2Hz), 7.74 (1 H, s), 7.66 (1 H, d, *J*=8.4Hz), 6.77 (1 H, s), 6.59 (2H, m), 5.38 (1 H, s), 4.61 (1 H, s), 2.37 (3H, s), 2.10- 2.05 (1 H, m), 1.80- 1.75 (1 H, m), 1.67- 1.58 (2H, m), 1.41- 1.37 (2H, m), 1.00- 0.97 (1 H, m), 0.81- 0.79 (2H, m), 0.51 (2H, m) .

LC- MS: $t_R$ = 11.07 $[M+H]^+$ = 348 (method 1)

***Example 44: 4-((3aS, 4R, 9bR)-8-(cyclopropylmethoxy)-2,3,3a,4,5,9b-hexahydro-1H-cyclopenta[c]quinolin-4-yl)-3-methylbenzoic acid***

The following compound was prepared using the same methodology as in example 30 from **intermediate compound 85** (0.08 mmol, 30 mg). Solvent was evaporated and the residue was purified by reverse $C_{18}$ chromatography using water (0.1% formic acid) and acetonitrile (0.1% formic acid) as eluents, to give 29 mg of the title compound, which was triturated in hexane, filtered and dried to obtain 18 mg the pure title compound as a solid (Yield: 60%) $^1$H NMR (400 MHz, DMSO-$d_6$) δ 7.77 (1 H, d, *J* = 8.0), 7.83 (1 H, s), 7.73 (1 H, s), 7.67 (1 H, d, *J* = 8.0 Hz), 6.62-6.59 (2H, m), 6.52 (1 H, dd, *J* = 8.8 & 2.4Hz), 5.21 (1 H, s), 4.55 (1 H, d, *J* = 2.4 Hz), 3.67 (2H, d, *J* = 6.8 Hz), 2.49-2.43 (1 H, m), 2.36 (3H, s), 2.09-2.04 (1H, m), 1.66-1.57 (2H, m) 1.40-1.34 (2H, m), 1.17-1.13 (1H, m), 0.98-0.95 (1 H, m), 0.54-0.50 (2H, m), 0.28-0.26 (2H, m).

LC- MS: $t_R$ = 8.18 $[M+H]^+$= 378 (method 1)

***Example 45: 4-((3aS,4R,9bR)-2,3,3a,4,5,9b-hexahydro-8-isopropoxy-1H cyclopenta[c]quinolin-4-yl)-3-methylbenzoic acid***

The following compound was prepared using the same methodology as in example 30 from **intermediate compound 87** (0.34 mmol, 132 mg). The mixture was stirred at r.t. until HPLC showed no starting material left. It was necessary 48 h and an additional 1.1 mL of 1 M LiOH to complete the reaction. After that, the solvent was removed in vacuum and purification of the crude material by reverse $C_{18}$ chromatography using water (0.1% formic acid) and acetonitrile (0.1% formic acid) as eluents gave 84.6 mg of the pure title endo-isomer (Yield: 66%).

$^1$H NMR (400 MHz, DMSO- $d_6$) δ 7.78 (1 H, d, *J* = 8 Hz), 7.74 (1 H, s), 7.67 (1 H, d, *J* = 8.4 Hz), 6.62- 6.59 (2H, m), 6.52 (1 H, dd, *J* = 8.8 & 2.8 Hz), 5.22 (1 H, s), 4.57 (1 H, d, *J* = 2.8 Hz), 4.34- 4.39 (1 H, m), 3.40- 3.36 (1 H, m), 2.50- 2.42 (1 H, m), 2.37 (3H, s), 2.13- 2.04 (1H, m), 1.67- 1.57 (2H, m), 1.44- 1.32 (2H, m), 1.21 (3H, d, *J* = 1.2 Hz), 1.19 (3H, d, *J*= 1.6 Hz), 1.02- 0.94 (1 H, m) .

LC- MS: $t_R$ = 8.15 $[M+H]^+$= 366 (method 1)

***Example 46: 4-((3aS,4S,9bR)-2,3,3a,4,5,9b-hexahydro-8-isopropoxy-1H cyclopenta[c]quinolin-4-yl)-3-methylbenzoic acid***

The following compound was prepared using the same methodology as in example 30 from **intermediate compound 88** (0.04 mmol, 17 mg,). It was necessary 40 h and an additional 0.13 mL of 1M LiOH to complete the reaction. Solvent was evaporated and purification of the crude material by reverse $C_{18}$ chromatography using water (0.1% formic acid) and acetonitrile (0.1% formic acid) as eluents gave 9.9 mg of the pure title exo-isomer (Yield: 61%).

$^1$H NMR (400 MHz, DMSO- $d_6$) δ 7.77- 7.75 (1 H, m), 7.51- 7.49 (1 H, m), 6.66 (1 H, d, *J* = 2 Hz), 6.57- 6.50 (2H, m), 5.46 (1 H, s), 4.39- 4.33 (1 H, m), 3.94 (1 H, d, *J* = 10 Hz), 2.96- 2.90 (1 H, m), 2.40 (3H, s), 2.35- 2.28 (1 H, m), 2.24- 2.20 (1 H, m), 1.66- 1.52 (4H, m), 1.20 (6H, d, *J* = 6 Hz), 1.2- 1.14 (1 H, m) .

LC- MS: $t_R$ = 7.58 $[M+H]^+$= 366 (method 1)

***Example 47: 3-chloro-4-((3aS, 4R, 9bR)-2, 3, 3a, 4, 5, 9b-hexahydro-8-methyl-1H-cyclopenta[c]quinolin-4-yl)benzoic acid***

The following compound was prepared using the same methodology as in intermediate compound 67 from **intermediate compound 89** for 1 h but using $Pt_2O$ (2.5 mmol%, 4 mg) . Solvent was evaporated and purification of the crude material by reverse $C_{18}$ chromatography using water (0.1% formic acid) and acetonitrile (0.1% formic acid) as eluents afforded the endo- isomer 3- chloro- 4- ((3a*S*, 4*R*, 9b*R*)- 2, 3, 3a, 4, 5, 9b- hexahydro- 8- methyl- 1*H*- cyclopenta [*c*] quinolin- 4- yl) benzoic acid (225mg. Yield: 75%) .

$^1$H NMR (400 MHz, DMSO- $d_6$) δ 7.93 (1 H, d, *J*=8Hz) , 7.91 (1 H, s), 7.81 (1 H, d, *J*=8Hz), 6.85 (1 H, s), 6.72 (1 H, d, *J*=8.4Hz), 6.59 (1 H, d, *J*=8Hz), 5.55 (1 H, s), 4.74 (1 H, d, *J*= 2.8Hz), 3.37- 3.32 (1 H, m), 2.60- 2.57 (1 H, m), 2.16 (3H, s), 2.10- 2.05 (1 H, m), 1.71- 1.68 (1 H, m), 1.58- 1.53 (1 H, m), 1.43- 1.37 (2H, m), 0.99- 0.95 (1 H, m) .

LC- MS: $t_R$ = 11.32 $[M+H]^+$ = 342 (method 1)

***Example 48: 4-((3aS, 4R, 9bR)-2, 3, 3a, 4, 5, 9b-hexahydro-8-methoxy-1H cyclopenta[c]quinolin-4-yl)-3-methoxybenzoic acid***

The following compound was prepared using the same methodology as in example 30 from **intermediate**

**compound 91** (80 mg, 0.22 mmol) . Total conversion was not achieved, 1 N LiOH (0.88 mmol) was added and was stirred for 48 h at room temperature. Solvent was evaporated and purification of the crude material using reverse $C_{18}$ chromatography using water (0.1% formic acid) and acetonitrile (0.1% formic acid) as eluents afforded the endo- isomer 4- ((3a*S*, 4R, 9bR)- 2, 3, 3a, 4, 5, 9b- hexahydro- 8- methoxy- 1*H*- cyclopenta [*c*] quinolin- 4- yl)- 3- methoxybenzoic acid (66 mg. Yield: 84%) .

$^1$H NMR (400 MHz, DMSO- $d_6$) δ 7.60 (1 H, d, *J*=8Hz), 7.56 (1 H, d, *J*=8Hz), 7.47 (1 H, s), 6.63- 6.59 (2H, m), 6.52 (1 H, dd, *J*=2.8 & 8.8Hz), 5.14 (1 H, s), 4.61 (1 H, d, *J*=2.8Hz), 3.84 (3H, s), 3.63 (3H, s), 3.33- 3.29 (1 H, m), 2.55- 2.53 (1 H, m), 2.06- 2.01 (1 H, m), 1.65- 1.61 (1 H, m), 1.55- 1.49 (1 H, m), 1.36- 1.34 (2H, m), 0.98- 0.94 (1 H, m) .

LC- MS: $t_R$ = 8.32 $[M+H]^+$ = 354 (method 1)

***Example 49: 4-((3aS, 4R, 9bR)-2, 3, 3a, 4, 5, 9b-hexahydro-8-methyl-1H cyclopenta[c]quinolin-4-yl)-3-methoxybenzoic acid***

The following compound was prepared using the same methodology as in example 30 from **intermediate compound 93** (135 mg, 0.39 mmol) . The reaction mixture was stirred for 48 hours at room temperature. Solvent was evaporated and purification of the crude material using reverse $C_{18}$ chromatography using water (0.1% formic acid) and acetonitrile (0.1% formic acid) as eluents afforded the endo- isomer 4- ((3a*S*, 4*R*, 9b*R*)- 2, 3, 3a, 4, 5, 9b- hexahydro- 8- methyl- 1*H*- cyclopenta [*c*] quinolin- 4- yl)- 3- methoxybenzoic acid (82 mg. Yield: 62%) .

$^1$H NMR (400 MHz, DMSO- $d_6$) δ 7.63 (1 H, d, *J*=8Hz), 7.59 (1 H, d, *J*=8.8Hz) , 7.49 (1H, s), 6.82 (1H, s), 6.69 (1H, d, *J*=7.6Hz) , 6.59 (1H, d, *J*=8Hz), 5.32 (1H, s), 4.68 (1H, d, *J*=2.8Hz), 3.86 (3H, s), 3.32- 3.29 (1H, m), 2.56- 2.53 (1H, m), 2.15 (3H, s), 2.06- 2.01 (1 H, m), 1.69- 1.65 (1 H, m), 1.53- 1.50 (1 H, m), 1.39- 1.34 (2H, m), 0.97- 0.95 (1 H, m) .

LC- MS: $t_R$ = 10.00 $[M+H]^+$ = 338 (method 1)

***Example 50: 3-bromo-4-((3aS, 4R, 9bR)-2, 3, 3a, 4, 5, 9b-hexahydro-8-methyl-1H-cyclopenta[c]quinolin-4-yl)benzoic acid***

The following compound was prepared using the same methodology as in example 30 from **intermediate compound 95** (54 mg, 0.135 mmol) . Total conversion was not achieved, 1 N LiOH (0.405 mmol) was added and was stirred for 6 hours at room temperature. Solvent was evaporated and purification of the crude material using reverse $C_{18}$ chromatography using water (0.1% formic acid) and acetonitrile (0.1 % formic acid) as eluents afforded the endo-isomer 3- bromo- 4- ((3a*S*, 4*R*, 9b*R*)- 2, 3, 3a, 4, 5, 9b- hexahydro- 8- methyl- 1*H*- cyclopenta [*c*] quinolin- 4- yl) benzoic acid (18 mg. Yield: 34%) .

$^1$H NMR (400 MHz, DMSO- $d_6$) δ 8.08 (1 H, s), 7.95 (1 H, d, *J*=7.2Hz), 7.75 (1 H, d, *J*=7.6Hz), 6.85 (1 H, s), 6.72 (1 H, d, *J*=7.6Hz), 6.58 (1 H, d, *J*=8.4Hz), 5.57 (1 H, s), 4.67 (1 H, s), 3.39 (1 H, m), 2.61- 2.56 (1 H, m), 2.16 (3H, s), 2.11- 2.06 (1 H, m), 1.71- 1.69 (1 H, m), 1.57- 1.54 (1 H, m), 1.41- 1.37 (2H, m), 0.98- 0.96 (1 H, m) .

LC- MS: $t_R$ = 10.87 $[M+H]^+$ = 386- 388 (method 1)

***Example 51: 3-bromo-4-((3aS, 4R, 9bR)-2, 3, 3a, 4, 5, 9b-hexahydro-8-methoxy-1H-cyclopenta[c]quinolin-4-yl) benzoic acid***

The following compound was prepared using the same methodology as in example 30 from **intermediate compound 97** (129 mg, 0.31 mmol) . Total conversion was not achieved, 1 N LiOH (0.3mmol) was added and it was stirred for 48 hours at room temperature. Solvent was evaporated and purification of the crude material using reverse $C_{18}$ chromatography using water (0.1% formic acid) and acetonitrile (0.1 % formic acid) as eluents afforded the endo-isomer 3- bromo- 4- ((3a*S*, 4*R*, 9b*R*)- 2, 3, 3a, 4, 5, 9b- hexahydro- 8- methoxy- 1*H*- cyclopenta [*c*] quinolin- 4- yl) benzoic acid (93 mg. Yield: 75%) .

$^1$H NMR (400 MHz, DMSO- $d_6$) δ 8.09 (1 H, s), 7.97 (1 H, dd, *J*= 2 & 8.4Hz), 7.79 (1 H, d, *J*=8Hz), 6.65- 6.62 (2H, m), 6.57 (1 H, dd, *J*=2.8 & 8.4Hz), 5.44 (1 H, s), 4.63 (1 H, d, *J*=3.2Hz), 3.66 (3H, s), 3.41- 3.36 (1 H, m), 2.61- 2.52 (1 H, m), 2.12- 2.09 (1 H, m), 1.70- 1.66 (1 H, m), 1.60- 1.55 (1 H, m), 1.44- 1.38 (2H, m), 0.99- 0.95 (1 H, m) .

LC- MS: $t_R$ = 9.87 $[M+H]^+$ = 402- 404 (method 1) .

***Example 52: 3-ethyl-4-((3aS,4R,9bR)-2,3,3a,4,5,9b-hexahydro-8-methyl-1H cyclopenta[c]quinolin-4-yl)benzonitrile***

The following compound was prepared using the same methodology as in intermediate compound 67 from **intermediate compound 98** (0.22 mmol, 71 mg). Purification of the crude material by flash chromatography on silica gel using an elution of 40% ethylacetate in hexanes afforded a mixture of exo/endo isomers (1:5.3) as a solid (45 mg, 65%), which was purified again by reverse $C_{18}$ chromatography using water (0.1% formic acid) and acetonitrile (0.1%

formic acid) as eluents gave 20.4 mg of the title pure endo-isomer (Yield: 29%).

1 H NMR (400 MHz, DMSO- d6) δ 7.77 (1 H, d, J = 8.8 Hz), 7.68 (2H, m), 6.83 (1 H, s), 6.71 (1 H, dd, J = 8 & 2 Hz), 6.57 (1 H, d, J = 7.6 Hz), 5.44 (1 H, s), 4.66 (1 H, d, J = 2.8 Hz), 3.32- 3.30 (1 H, m), 2.83- 2.73 (1 H, m), 2.70- 2.60 (1 H, m), 2.41- 2.32 (1 H, m), 2.16 (3H, s), 2.11- 2.02 (1 H, m), 1.72- 1.53 (2H, m), 1.45- 1.33 (2H, m), 1.18 (3H, t, J = 7.6 Hz), 1.01- 0.93 (1 H, m) .

LC- MS: $t_R$ = 10.98 [M+H] += 317 (method 1)

***Example 53: 3-ethyl-4-((3aS,4R,9bR)-2,3,3a,4,5,9b-hexahydro-8-methyl-1H-cyclopenta[c]quinolin-4-yl)benzoic acid***

The following compound was prepared using the same methodology as in intermediate compound 67 from **intermediate compound 99** (0.31 mmol, 104 mg). Purification of the crude material (1:6 mixture of regioisomers exo/ endo) by reverse $C_{18}$ chromatography using water (0.1% formic acid) and acetonitrile (0.1% formic acid) as eluents gave 55.5 mg of the pure endo-compound. (Yield: 55%)

1 H NMR (400 MHz, DMSO-d6) δ 7.78 (2H, m), 7.70 (1 H, d, J = 8.4 Hz), 6.83 (1 H, s), 6.70 (1 H, dd, J = 8 & 1.6 Hz), 6.58 (1 H, d, J = 8 Hz), 5.39 (1 H, s), 4.66 (1 H, d, J = 2.4 Hz), 3.32-3.30 (1 H, m), 2.83-2.73 (1 H, m), 2.70-2.60 (1 H, m), 2.42-2.35 (1 H, m), 2.19 (3H, s), 2.11-2.02 (1 H, m), 1.72-1.57 (2H, m), 1.44-1.32 (2H, m), 1.19 (3H, t, J = 7.6 Hz), 1.04-0.96 (1 H, m).

LC- MS: $t_R$ = 10.53 [M+H] += 336 (method 1)

***Example 54 and 55: 3-ethyl-4-((3aS,4R,9bR)-2,3,3a,4,5,9b-hexahydro-8-methoxy-1H-cyclopenta[c]quinolin-4-yl) benzoic acid (54) and 3-ethyl-4-((3aS,4S,9bR)-2,3,3a,4,5,9b-hexahydro-8-methoxy-1H-cyclopenta[c]quinolin-4-yl)benzoic acid (55)***

The following compound was prepared using the same methodology as in intermediate compound 67 from **intermediate mixture compound 100** (0.22 mmol, 79 mg) . Purification of the crude material (1: 3 mixture of exo/ endo isomers) by reverse $C_{18}$ chromatography using water (0.1% formic acid) and acetonitrile (0.1% formic acid) as eluents afforded 6.3 mg of the exo- isomer 3- ethyl- 4- ((3aS, 4S, 9bR)- 2, 3, 3a, 4, 5, 9b- hexahydro- 8- methoxy- 1 H- cyclopenta [c] quinolin- 4- yl) benzoic acid (**55**) and 22.3 mg of the pure endo- isomer 3- ethyl- 4- ((3aS, 4R, 9bR)- 2, 3, 3a, 4, 5, 9b- hexahydro- 8- methoxy- 1 H- cyclopenta [c] quinolin- 4- yl) benzoic acid (**54**) . (Overall yield=36%) .

3- Ethyl- 4- ((3aS, 4S, 9bR)- 2, 3, 3a, 4, 5, 9b- hexahydro- 8- methoxy- 1 H- cyclopenta [c] quinolin- 4- yl) benzoic acid (exo- isomer **55**) :

$^1$H NMR (400 MHz, DMSO- $d_6$) : δ 7.78 (2H, m), 7.53 (1 H, d, *J* = 7.6 Hz), 6.69 (1 H, d, *J* = 2.4 Hz), 6.60 (1 H, d, *J* = 8.8 Hz), 6.54 (1 H, dd, *J* = 8.8 & 2.4 Hz), 5.47 (1 H, s), 3.95 (1 H, d, *J* = 9.6 Hz), 3.65 (3H, s), 3.00- 2.93 (1 H, m), 2.83- 2.66 (2H, m), 2.38- 2.32 (1 H, m), 2.28- 2.22 (1 H, m), 1.66- 1.52 (4H, m), 1.224- 1.14 (4H, m) .

LC- MS: $t_R$ = 8.67 $[M+H]^+$= 352 (method 1)

3- Ethyl- 4- ((3aS, 4R, 9bR)- 2, 3, 3a, 4, 5, 9b- hexahydro- 8- methoxy- 1 H- cyclopenta [c] quinolin- 4- yl) benzoic acid (endo- isomer **54**) :

$^1$H NMR (400 MHz, DMSO- $d_6$) : δ 7.78 (2H, m), 7.71 (1 H, d, *J* = 8.8 Hz), 6.63 (2H, d, *J* = 8.8 Hz), 6.54 (1 H, dd, *J* = 8.8 & 2.4 Hz), 5.25 (1 H, s), 4.61 (1 H, d, *J* = 2 Hz), 3.65 (3H, s), 3.43- 3.31 (1 H, m), 2.83- 2.74 (1 H, m), 2.70- 2.61 (1 H, m), 2.46- 2.36 (1 H, m), 2.13- 2.04 (1 H, m), 1.68- 1.58 (2H, m), 1.45- 1.33 (2H, m), 1.23- 1.17 (3H, m), 1.05- 0.96 (1 H, m) .

LC- MS: $t_R$ = 9.22 $[M+H]^+$= 352 (method 1)

***Example 56: 4-((3aS,4S,9bR)-2,3,3a,4,5,9b-hexahydro-8-methyl-1H cyclopenta[c]quinolin-4-yl)-3,5-dimethylbenzoic acid***

The following compound was prepared using the same methodology as in example 30 from **intermediate compound 102** (0.24 mmol, 83 mg). It was necessary 22 h and an additional 0.75 mL of 1 M LiOH to complete the reaction. Solvent was evaporated and purification of the crude material by reverse $C_{18}$ chromatography using water (0.1% formic acid) and acetonitrile (0.1% formic acid) as eluents gave 53.4 mg of the pure title exo-compound (Yield: 67%).

$^1$H NMR (400 MHz, $CDCl_3$) : δ 7.76 (2H, m), 7.01 (1 H, d, *J* = 2 Hz), 6.85 (1 H, dd, *J* = 8.4 & 1.6 Hz), 6.52 (1H, d, *J* = 8 Hz), 4.36 (1H, d, *J* = 10.8 Hz), 3.11- 3.05 (1H, m), 2.92- 2.84 (1 H, m), 2.72- 2.61 (3H, brs), 2.50- 2.40 (3H, brs), 2.36- 2.30 (1 H, m), 2.28 (3H, s), 1.81- 1.61 (4H, m), 1.28- 1.21 (1H, m) .

LC- MS: $t_R$ = 10.52 $[M+H]^+$= 336 (method 1)

***Example 57: 4-((3aS,4S,9bR)-2,3,3a,4,5,9b-hexahydro-8-methoxy-1H-cyclopenta[c]quinolin-4-yl)-3,5-dimethylbenzoic acid***

The following compound was prepared using the same methodology as in example 30 from **intermediate compound 104** (0.16 mmol, 59 mg). It was necessary 18 h and an additional 0.32 mL of 1M LiOH to complete the reaction. Solvent was evaporated and purification of the crude material by reverse $C_{18}$ chromatography using water (0.1% formic acid) and acetonitrile (0.1% formic acid) as eluents gave 36 mg of the pure title exo-compound (Yield: 63%).

$^1$H NMR (400 MHz, $CDCl_3$) : δ 7.76 (2H, m), 7.79 (1 H, d, *J* = 2.4 Hz), 6.67 (1 H, dd, *J* = 8.4 & 2.8 Hz), 6.55 (1 H, d, *J* = 8.8 Hz), 4.34 (1 H, d, *J* = 11.6 Hz), 3.78 (3H, s), 3.13- 3.06 (1 H, m), 2.94- 2.86 (1 H, m), 2.73- 2.60 (3H, brs), 2.50- 2.40 (3H, brs), 2.37- 2.31 (1 H, m), 1.79- 1.60 (4H, m), 1.28- 1.21 (1 H, m) .

LC- MS: $t_R$ = 9.43 $[M+H]^+$= 352 (method 1)

***Example 58: 5-((3aS, 4R, 9bR)-2, 3, 3a, 4, 5, 9b-hexahydro-8-methyl-1H cyclopenta[c]quinolin-4-yl)pyridine-2-carboxylic acid***

The following compound was prepared using the same methodology as in intermediate compound 67 from **intermediate compound 105** (0.17 mmol, 54 mg) but using $Pt_2O$ (15 % w/w, 8 mg) for 18 h. Solvent was evaporated and purification of the crude material by reverse $C_{18}$ chromatography using water (0.1 % formic acid) and acetonitrile (0.1 % formic acid) as eluents gave the endo- isomer 5- ((3a*S*, 4*R*, 9b*R*)- 2, 3, 3a, 4, 5, 9b- hexahydro- 8- methyl- 1*H*-cyclopenta [*c*] quinolin- 4- yl) pyridine- 2- carboxylic acid (5 mg. Yield: 10%) .

$^1$H NMR (400 MHz, $CD_3OD$) δ 8.74 (1 H, s), 8.17- 8.09 (2H, m), 6.90 (1 H, s), 6.76 (1 H, d, *J*=8.4Hz) , 6.59 (1 H, d, *J*=8Hz), 4.62 (1 H, s), 3.46- 3.44 (1 H, m), 2.56- 2.54 (1 H, m), 2.21 (3H, s), 2.17- 2.12 (1 H, m), 1.78- 1.76 (1 H, m), 1.68- 1.65 (1 H, m), 1.51- 1.44 (2H, m), 1.14- 1.12 (1 H, m) .

LC- MS: $t_R$ = 8.65 $[M+H]^+$ = 309 (method 1)

***Example 59: 6-((3aS,4R,9bR)-2,3,3a,4,5,9b-hexahydro-8-methyl-1H cyclopenta[c]quinolin-4-yl)pyridine-3-carboxylic acid***

The following compound was prepared using the same methodology as in example 30 from **intermediate compound 108** (0.53 mmol). The crude residue was purified by reverse $C_{18}$ chromatography using water (0.1% formic acid) and acetonitrile (0.1 % formic acid) as eluents, to give 119 mg of the title endo-isomer. (Yield: 73%)

$^1$H NMR (400 MHz, DMSO- $d_6$) δ 9.01 (1 H, d, *J* = 1.6 Hz), 8.27 (1 H, dd, *J* = 8.4 & 2.4 Hz), 7.64 (1 H, d, *J* = 8.4 Hz), 6.83 (1 H, s), 6.70 (1 H, d, *J* = 8.0 Hz), 6.61 (1 H, d, *J* = 8.0 Hz), 5.69 (1 H, s), 4.51 (1 H, d, *J*= 3.2 Hz), 3.54- 3.50 (1 H, m), 2.71- 2.64 (1 H, m), 2.14 (3H, s), 2.07- 1.97 (1 H, m), 1.73- 1.67 (1 H, m), 1.45- 1.29 (3H, m), 1.00- 1.94 (1 H, m) .

LC- MS: $t_R$ = 9.05 $[M+H]^+$= 309 (method 1)

***Example 60: 5-((3aS,4R,9bR)-2,3,3a,4,5,9b-hexahydro-8-methyl-1H cyclopenta[c]quinolin-4-yl)-6-methylpyridine-2-carbonitrile***

The following compound was prepared using the same methodology as in intermediate compound 67 from **intermediate compound 109** (275 mg, 0.91 mmol). Purification of the crude material using flash chromatography on silica gel using an elution of 23% ethylacetate in hexanes afforded the title endo-isomer as a solid (245 mg. Yield: 89%).

$^1$H NMR (400 MHz, $CDCl_3$) δ 8.10 (1 H, d, *J* = 8.4 Hz), 7.58 (1 H, d, *J* = 8.0 Hz), 6.96 (1 H, s), 6.84 (1 H, dd, *J* = 7.6 & 1.6 Hz), 6.53 (1 H, d, *J* = 8.4 Hz), 4.73 (1 H, d, *J* = 3.2 Hz), 3.49- 3.44 (2H, m), 2.62 (3H, s), 2.61- 2.47 (1 H, m), 2.26 (3H, s), 2.23- 2.14 (1 H, m), 1.83- 1.76 (1 H, m), 1.70- 1.59 (1 H, m), 1.57- 1.41 (1 H, m), 1.12- 1.07 (1 H, s) .

LC- MS: $t_R$ = 9.85 $[M+H]^+$= 304 (method 1)

***Example 61: 5-((3aS,4R,9bR)-2,3,3a,4,5,9b-hexahydro-8-methyl-1H cyclopenta[c]quinolin-4-yl)-6-methylpyridine-2-carboxylic acid***

The pyridine-2-carbonitrile **Example 60** (30 mg, 0.1 mmol) was suspended in a mixture of acetic acid: water: concentrated hydrochloric acid (1:1:2, 1 mL) and the mixture was heated at 110ºC for 5h. Reaction mixture was cooled down and left at r.t. for 16 h, when the solvent was evaporated. Purification of the crude material using reverse $C_{18}$ chromatography using water (0.1% formic acid) and acetonitrile (0.1% formic acid) as eluents afforded 13.4 mg of the title endo-isomer. (Yield: 34%)

$^1$H NMR (400 MHz, DMSO- $d_6$) δ 8.04 (1 H, d, *J* = 8.0 Hz), 7.89 (1 H, d, *J* = 8.0 Hz), 6.83 (1 H, s), 6.70 (1 H, d, *J* = 7.6 Hz), 6.56 (1 H, d, *J* = 8.4 Hz), 5.44 (1 H, s), 4.61 (1 H, d, *J* = 2.8 Hz), 3.39- 3.31 (1 H, m), 2.56 (3H, s), 2.15

(3H, s), 2.11- 2.02 (1 H, m), 1.71- 1.64 (1 H, m), 1.56- 1.48 (1 H, m), 1.41- 1.35 (2H, m), 0.98- 0.92 (1 H, m) .

LC- MS: $t_R$ = 7.58 $[M+H]^+$= 323 (method 1)

***Example 62: 5-((3aS,4R,9bR)-2,3,3a,4,5,9b-hexahydro-8-methyl-1H-cyclopenta[c]quinolin-4-yl)-1-methyl-1H-pyrazole-3-carboxylic acid***

The following compound was prepared using the same methodology as in example 30 from **intermediate compound 111** (225 mg, 0.66 mmol) using 1M LiOH (4 eq.) for 16 h. Purification of the crude material was carried out by precipitation at pH 6 using 1M HCl. The white solid was filtered, washed by water and dried under vacuum at 40ºC to give 84 mg of the title endo-isomer. (Yield: 41%)

$^1H$ NMR (400 MHz, DMSO- $d_6$) δ 12.5 (1 H, brs), 6.82 (1 H, s), 6.68 (1 H, d, *J* = 8.0 Hz), 6.61 (1 H, s), 6.55 (1H, d, *J* = 8.0 Hz), 5.46 (1 H, s), 4.53 (1 H, s), 3.86 (3H, s), 2.52- 2.49 (1 H, m), 2.13 (3H, s), 2.04- 1.98 (1 H, m), 1.67 (1 H, m), 1.60- 1.53 (1 H, m), 1.39- 1.36 (2H, m), 1.22- 1.20 (1 H, m) .

LC- MS: $t_R$ = 8.08 $[M+H]^+$= 312 (method 1)

***Example 63: 5-((3aS,4R,9bR)-2,3,3a,4,5,9b-hexahydro-8-methoxy-1H-cyclopenta[c]quinolin-4-yl)-1-methyl-1H-pyrazole-3-carboxylic acid***

The following compound was prepared using the same methodology as in example 30 from **intermediate compound 113** (190 mg, 0.54 mmol) using 1M LiOH (4 eq.) for 16 h. After neutralising the reaction mixture with 1M HCl until pH 6 and evaporation of the solvents, purification of the crude material using reverse $C_{18}$ chromatography using water (0.1% formic acid) and acetonitrile (0.1% formic acid) as eluents gave the title compound which was precipitated in acetonitrile, filtered and dried to give 74 mg of the pure title endo-carboxylic acid.

$^1H$ NMR (400 MHz, DMSO- $d_6$) δ 12.5 (1 H, brs), 6.61- 6.60 (3H, m), 6.53 (1 H, dd, *J* = 8.8 & 2.8 Hz), 5.32 (1 H, s), 4.49 (1H, d, *J* = 2.8 Hz), 3.86 (3H, s), 3.63 (3H, s), 3.38- 3.36 (1 H, m), 2.55- 2.49 (1 H, m), 2.08- 1.96 (1 H, m), 1.69- 1.54 (2H, m), 1.46- 1.32 (1 H, m), 1.26- 1.13 (1 H, m) .

LC- MS: $t_R$ = 6.73 $[M+H]^+$= 328 (method 1)

***Example 64: 3-((3aS,4R,9bR)-2,3,3a,4,5,9b-hexahydro-8-methyl-1H cyclopenta[c]quinolin-4-yl)-1-methyl-1H-pyrazole-5-carboxylic acid***

The following compound was prepared using the same methodology as in example 30 from **intermediate compound 115** (100 mg, 0.29 mmol) using 1 M LiOH (3 eq.) for 16 h. Purification of the crude material using reverse $C_{18}$ chromatography using water (0.1% formic acid) and acetonitrile (0.1% formic acid) as eluents afforded 74 mg of the pure title endo-carboxylic acid (Yield: 70%).

$^1H$ NMR (400 MHz, DMSO- $d_6$) δ 6.78 (1 H, d, *J* = 8.4 Hz), 6.77 (1 H, s), 6.67 (1 H, dd, *J* = 8.0 & 2.0 Hz), 6.56 (1 H, d, *J* = 8.4 Hz), 5.36 (1 H, s), 4.36 (1 H, d, *J* = 2.8 Hz), 4.09 (3H, s), 3.30- 3.27 (1 H, m), 2.55- 2.49 (1 H, m), 2.13 (3H, s), 2.02- 1.97 (1 H, m), 1.68- 1.63 (1 H, m), 1.55- 1.50 (1 H, m), 1.41- 1.29 (2H, m) .

LC- MS: $t_R$ = 8.13 $[M+H]^+$= 312 (method 1)

***Example 65: 3-((3aS,4R,9bR)-2,3,3a,4,5,9b-hexahydro-8-methoxy-1H-cyclopenta[c]quinolin-4-yl)-1-methyl-1H-pyrazole-5-carboxylic acid***

The following compound was prepared using the same methodology as in example 30 from **intermediate compound 117** (65 mg, 0.18 mmol) using 1 M LiOH (3 eq.) for 16 h. After neutralising the reaction mixture with 1 M HCl until pH 6 and evaporation of the solvents, purification of the crude material using reverse $C_{18}$ chromatography using water (0.1% formic acid) and acetonitrile (0.1% formic acid) as eluents gave the title compound which was precipitated in acetonitrile, filtered and dried under vacuum to give 31 mg of the pure title endo-carboxylic acid (Yield: 52%).

$^1H$ NMR (400 MHz, DMSO- $d_6$) δ 6.77 (1 H, s), 6.62- 6.49 (3H, m), 5.23 (1 H, s), 4.32 (1 H, d, *J* = 2.8 Hz), 4.03 (3H, s), 3.62 (3H, s), 3.32- 3.26 (1 H, m), 2.58- 2.51 (1 H, m), 2.03- 1.96 (1 H, m), 1.68- 1.61 (1 H, m), 1.56- 1.49 (1 H, m), 1.43- 1.27 (2H, m) .

LC- MS: $t_R$ = 6.12 $[M+H]^+$= 328 (method 1)

***Example 66 and 67: 4-((3aS,4S,9bR)-2,3,3a,4,5,9b-hexahydro-8,9b-dimethyl-1H-cyclopenta[c]quinolin-4-yl)benzoic acid (*66*) and 4-((3aS,4R,9bR)-2,3,3a,4,5,9b-hexahydro-8,9b-dimethyl-1H-cyclopenta[c]quinolin-4-yl)benzoic acid (*67*)***

Boron trifluoride diethyletherate (2 eq., 2 mmol, 0.25 mL) in anhydrous acetonitrile (4 mL) was added to a

mixture of 4- formylbenzoic acid (150 mg, 1 mmol), 4- methylaniline (1 mmol, 107 mg), and 1- methyl- 1- cyclopentene (5 eq., 5 mmol, 0.53 mL) . The reaction mixture was stirred at 50ºC for 24 h. Reaction mixture was diluted with saturated aqueous sodium bicarbonate solution and ethyl acetate. The aqueous layer was extracted with ethylacetate, then acidified to pH 3 with 1 N HCl solution and extracted with ethylacetate. The last combined organic layers was washed with brine, dried over anhydrous MgSO4, filtered, the solvent evaporated under vacuum and purification of the crude material (1: 1.3 mixture of regioisomers) by flash chromatography on silica gel using an elution of 13% methanol in dichloromethane afforded 2 fractions of each regioisomer. These fractions were purified again using reverse $C_{18}$ chromatography using water (0.1% formic acid) and acetonitrile (0.1% formic acid) as eluents to give 18 mg of 4- ((3a*S*, 4*S*, 9*bR*)- 2, 3, 3a, 4, 5, 9*b*- hexahydro- 8, 9*b*- dimethyl- 1*H*- cyclopenta [*c*] quinolin- 4- yl) benzoic acid and 45 mg (30%) of the pure endo-compound (Overall yield= 19%) .

4- ((3*aS*, 4*S*, 9*bR*)- 2, 3, 3*a*, 4, 5, 9*b*- hexahydro- 8, 9*b*- dimethyl- 1*H*- cyclopenta [*c*] quinolin- 4- yl) benzoic acid (exo- isomer **66) :**

$^1$H NMR (400 MHz, DMSO- $d_6$) δ 7.91 (2H, d, *J*= 8.0 Hz), 7.51 (2H, d, *J*= 8.0 Hz), 6.94 (1 H, m), 6.70 (1 H, dd, *J* = 8.0 & 1.6 Hz), 6.58 (1 H, t, *J* = 8 Hz), 5.67 (1 H, s), 3.73 (1 H, d, *J*= 9.6 Hz), 2.16 (3H, s), 1.98- 1.94 (1 H, m), 1.90- 1.86 (1 H, m), 1.81- 1.78 (1 H, m), 1.71- 1.67 (1 H, m), 1.62- 1.56 (2H, m), 1.36- 1.32 (1 H, m), 1.10 (3H, s) .

LC- MS: $t_R$ = 9.88 $[M+H]^+$= 322 (method 1)

4- ((3a*S*, 4*R*, 9b*R*)- 2, 3, 3*a*, 4, 5, 9*b*- hexahydro- 8, 9*b*- dimethyl- 1*H*- cyclopenta [*c*] quinolin- 4- yl) benzoic acid (endo- isomer **67) :**

$^1$H NMR (400 MHz, DMSO- $d_6$) δ 7.91 (2H, d, *J*= 8.4 Hz), 7.52 (2H, d, *J*= 8.0 Hz), 6.92 (1 H, m), 6.68 (1 H, dd, *J* = 8.0 & 1.6 Hz), 6.57 (1 H, t, *J* = 8.4 Hz), 5.75 (1 H, s), 4.45 (1 H, d, *J* = 2.46 Hz), 2.14 (3H, s), 2.09- 2.03 (1 H, m), 1.93- 1.88 (1 H, ta, *J* = 8.4 Hz), 1.60- 1.68 (1 H, m), 1.52- 1.57 (1 H, m), 1.40 (3H, s), 1.39- 1.31 (1 H, m), 1.17- 1.12 (1 H, m), 1.08- 1.00 (1 H, m) .

LC- MS: $t_R$ = 10.70 $[M+H]^+$= 322 (method 1)

***Example 68 and 69 : 4-((3aS,4S,9bR)-2,3,3a,4,5,9b-hexahydro-8-methoxy-9b-methyl-1H-cyclopenta[c]quinolin-4-yl)benzoic acid (68) and 4-((3aS,4R,9bR)-2,3,3a,4,5,9b-hexahydro-8-methoxy-9b-methyl-1H-cyclopenta[c]quinolin-4-yl)benzoic acid (69)***

The following compounds were prepared using the same methodology as in example 66 and 67 using *p*-anisidine (1 mmol, 107 mg) . Solvent was evaporated and the crude material (1: 1.4 mixture of endo/exo isomers) was purified by reverse $C_{18}$ chromatography using water (0.1% formic acid) and acetonitrile (0.1 % formic acid) . Only high purity fractions of each isomer were collected giving two enriched fractions with each regioisomer 51 mg of the fraction enriched in the exo- isomer was precipitated in acetonitrile/ drops of water to give 29 mg of 4- ((3aS, 4S, 9bR)- 2, 3, 3a, 4, 5, 9b- hexahydro- 8- methoxy- 9b- methyl- 1 H- cyclopenta [c] quinolin- 4- yl) benzoic acid **(68) .** The fraction enriched in the endo- isomer was purified again by reverse $C_{18}$ chromatography using water (0.1% formic acid) and acetonitrile (0.1% formic acid) as eluents to give 24 mg of the pure endo- compound 4- ((3aS, 4R, 9bR)- 2, 3, 3a, 4, 5, 9b- hexahydro-8- methoxy- 9b- methyl- 1 Hcyclopenta [c] quinolin- 4- yl) benzoic acid **(69)** (overall yield= 16%) .

4- ((3aS, 4S, 9bR)- 2, 3, 3a, 4, 5, 9b- hexahydro- 8- methoxy- 9b- methyl- 1 H- cyclopenta [c] quinolin- 4- yl) benzoic acid (exo- isomer **68) :**

$^1$H NMR (400 MHz, DMSO- $d_6$) : δ 7.92 (2H, d, *J* = 8 Hz), 7.52 (2H, d, *J* = 8 Hz), 6.73 (1 H, d, *J* = 2.4 Hz), 6.63 (1 H, d, *J* = 8.4 Hz), 6.54 (1 H, dd, *J* = 2.8 & 8.8 Hz), 5.52 (1 H, s), 3.68 (1 H, d, *J* = 10 Hz), 3.65 (3H, s), 1.99- 1.89 (2H, m), 1.84- 1.76 (1 H, m), 1.73- 1.57 (3H, m), 1.37- 1.29 (1 H, m), 1.14 (3H, m) .

LC- MS: $t_R$ = 8.40 $[M+H]^+$= 338 (method 1) .

4- ((3aS, 4R, 9bR)- 2, 3, 3a, 4, 5, 9b- hexahydro- 8- methoxy- 9b- methyl- 1H- cyclopenta [c] quinolin- 4- yl) benzoic acid (endo- isomer **69**) :

$^1$H NMR (400 MHz, DMSO- $d_6$) : δ 7.93 (2H, d, *J* = 8.4 Hz), 7.55 (2H, d, *J* = 8.4 Hz), 6.71 (1 H, d, *J* = 2.4 Hz), 6.63 (1 H, d, *J* = 8.4 Hz), 6.56 (1 H, dd, *J* = 2.8 & 8.4 Hz), 5.62 (1 H, s), 4.44 (1 H, d, *J* = 1.6 Hz), 3.65 (3H, s), 2.09- 2.05 (1 H, m), 1.97- 1.90 (1 H, m), 1.71- 1.53 (2H, m), 1.44 (3H, s), 1.40- 1.34 (1 H, m), 1.21- 1.12 (1 H, m), 1.09- 1.00 (1 H, m) .

LC- MS: $t_R$ = 9.70 $[M+H]^+$= 338 (method 1) .

***Example 70 and 71: 4-((3aS,45,9bR)-8-cyclopropyl-2,3,3a,4,5,9b-hexahydro-9b-methyl-1H-cyclopenta[c]quinolin-4-yl)benzoic acid (70) and 4-((3aS,4R,9bR)-8-cyclopropyl-2,3,3a,4,5,9b-hexahydro-9b-methyl-1H-cyclopenta[c]quinolin-4-yl)benzoic acid (71)***

The following compounds were prepared using the same methodology as in example 66 and 67 using **the intermediate compound 3 (method 2)** (1.2 mmol, 160 mg) . Solvent was evaporated and purification of the crude material (1: 1, 1 mixture of exo/ endo isomers) by reverse $C_{18}$ chromatography using water (0.1% formic acid) and acetonitrile (0.1% formic acid) afforded 38.5 mg of 4- ((3aS, 4S, 9bR)- 8- cyclopropyl- 2, 3, 3a, 4, 5, 9b- hexahydro- 9b-methyl- 1H- cyclopenta [c] quinolin- 4- yl) benzoic (**70**) acid and 53.30 mg of 4- ((3aS, 4R, 9bR)- 8- cyclopropyl- 2, 3, 3a, 4, 5, 9b- hexahydro- 9b- methyl- 1H- cyclopenta [c] quinolin- 4- yl) benzoic acid (**71**) both as a white solids. (Overall yield: 22%) .

4- ((3aS, 4S, 9bR)- 8- cyclopropyl- 2, 3, 3a, 4, 5, 9b- hexahydro- 9b- methyl- 1H- cyclopenta [c] quinolin- 4- yl) benzoic acid (exo- isomer **70**) :

$^1$H NMR (400 MHz, DMSO- $d_6$) : δ 7.91 (2H, d, *J*= 8.4 Hz), 7.51 (2H, d, *J*= 8 Hz), 6.90 (1 H, s), 6.57 (2H, d, *J* =0.8 Hz), 5.69 (1 H, s), 3.74 (1 H, d, *J* = 9.6 Hz), 1.99- 1.87 (2H, m), 1.83- 1.66 (3H, m), 1.62- 1.55 (2H, m), 1.38- 1.29 (1 H, m), 1.11 (3H, s), 0.83- 0.78 (2H, m), 0.53- 0.49 (2H, m) .

LC- MS: $t_R$ = 10.47 $[M+H]^+$= 348 (method 1)

4- ((3aS, 4R, 9bR)- 8- cyclopropyl- 2, 3, 3a, 4, 5, 9b- hexahydro- 9b- methyl- 1 H- cyclopenta [c] quinolin- 4- yl) benzoic acid (endo- isomer **71**) :

$^1$H NMR (400 MHz, DMSO- $d_6$) δ 7.93 (2H, d, *J* = 8.4 Hz), 7.54 (2H, d, *J* = 8.4 Hz), 6.89 (1 H, s), 6.58 (2H, s), 5.78 (1 H, s), 4.47 (1 H, d, *J* = 2.4 Hz), 2.12- 2.07 (1 H, m), 1.95- 1.90 (1 H, m), 1.79- 1.72 (1 H, m), 1.70- 1.50 (2H, m), 1.42 (3H, s), 1.41- 1.32 (1 H, m), 1.21- 0.99 (2H, m), 0.83- 0.78 (2H, m), 0.53- 0.49 (m, 2H) .

LC- MS: $t_R$ = 11.10 $[M+H]^+$= 348 (method 1)

***Example 72 and 73: 4-((3aS,4S,9bR)-8-chloro-2,3,3a,4,5,9b-hexahydro-9b-methyl-1H-cyclopenta[c]quinolin-4-yl) benzoic acid (72) and 4-((3aS,4R,9bR)-8-chloro-2,3,3a,4,5,9b-hexahydro-9b-methyl-1H cyclopenta[c]quinolin-4-yl)benzoic acid (73)***

The following compounds were prepared using the same methodology as in example 66 and 67 using 4-chloroaniline (1 mmol, 127 mg) . Solvent was evaporated and purification of the crude material (1: 1, 3 mixture of exo/ endo regioisomers) by reverse $C_{18}$ chromatography using water (0.1% formic acid) and acetonitrile (0.1% formic acid) afforded two fractions of each regioisomer. These fractions were purified again by precipitation in hexane/ acetonitrile, filtered and dried in vacuum to give 76.5 mg of 4- ((3aS, 4S, 9bR)- 8- chloro- 2, 3, 3a, 4, 5, 9b- hexahydro- 9b- methyl-1 H- cyclopenta [c] quinolin- 4- yl) benzoic (**72**) acid and 37 mg of the pure endo- compound 4- ((3aS, 4R, 9bR)- 8-chloro- 2, 3, 3a, 4, 5, 9b- hexahydro- 9b- methyl- 1 H- cyclopenta [c] quinolin- 4- yl) benzoic acid (**73**) (Overall yield: 33%) .

4- ((3aS, 4S, 9bR)- 8- chloro- 2, 3, 3a, 4, 5, 9b- hexahydro- 9b- methyl- 1H- cyclopenta [c] quinolin- 4- yl) benzoic acid (exo- isomer **72**) :

$^1$H NMR (400 MHz, DMSO- $d_6$) : δ 7.92 (2H, d, *J*= 8 Hz), 7.52 (2H, d, *J*= 8.4 Hz), 7.15 (1 H, d, *J* = 2 Hz), 6.91 (1 H, dd, *J* = 2.4 & 8.8 Hz), 6.69 (1 H, d, *J* = 8.8 Hz), 6.15 (1 H, s), 3.80 (1 H, d, *J* = 9.2 Hz), 2.00- 1.87 (2H, m), 1.84-1.68 (2H, m), 1.63- 1.55 (2H, m), 1.39.1.30 (1 H, m), 1.15 (3H, m) .

LC- MS: $t_R$ = 10.65 $[M+H]^+$= 342 (method 1)

4- ((3aS, 4R, 9bR)- 8- chloro- 2, 3, 3a, 4, 5, 9b- hexahydro- 9b- methyl- 1H- cyclopenta [c] quinolin- 4- yl) benzoic acid (endo- isomer **73**) :

$^1$H NMR (400 MHz, DMSO- $d_6$) : δ 7.94 (2H d, *J* = 8 Hz), 7.54 (2H, d, *J* = 8.4 Hz), 7.15 (1 H, d, *J* = 2.4 Hz), 6.92 (1 H, dd, *J*= 2.4 & 8.4 Hz), 6.70 (1 H, d, *J* = 8.4 Hz), 6.23 (1 H, s), 4.52 (1 H, d, *J* = 2.8 Hz), 2.11- 2.05 (1 H, m), 1.97- 1.92 (1 H, m), 1.71- 1.63 (1 H, m), 1.56- 1.46 (1 H, m), 1.42 (3H, s), 1.42- 1.35 (1 H, m), 1.23- 1.01 (2H, m)

LC- MS: $t_R$ = 10.90 $[M+H]^+$= 342 ((method 1)

***Example 74 and 75: 4-((3aS, 4S, 9bR)-2, 3, 3a, 4, 5, 9b-hexahydro-8, 9b-dimethyl-1H-cyclopenta[c]quinolin-4-yl)-3-methylbenzoic acid (74) and 4-((3aS, 4R, 9bR)-2, 3, 3a, 4, 5, 9b-hexahydro-8, 9b-dimethyl-1H-cyclopenta[c] quinolin-4-yl)-3-methylbenzoic acid (75)***

The following compound was prepared using the same methodology as in example 66 and 67 from **intermediate compound 18** (120 mg, 0.73 mmol) and *p*- toluidine (79 mg, 0.73 mmol) . Solvent was evaporated and purification of the crude material by reverse $C_{18}$ chromatography using water (0.1% formic acid) and methanol (0.1% formic acid) as eluents afforded two products, the exo- isomer 4- ((3a*S*, 4*S*, 9b*R*)- 2, 3, 3a, 4, 5, 9b- hexahydro- 8, 9b- dimethyl- 1*H* cyclopenta [c] quinolin- 4- yl)- 3- methylbenzoic acid (**74**) (14.7 mg. Yield: 6%) and the endo- isomer 4- ((3a*S*, 4*R*, 9b*R*)- 2, 3, 3a, 4, 5, 9b- hexahydro- 8, 9b- dimethyl- 1*H*- cyclopenta [*c*] quinolin- 4- yl)- 3- methylbenzoic acid (**75**) (33.6 mg. Yield: 14%) .

4- ((3a*S*, 4S, 9b*R*)- 2, 3, 3a, 4, 5, 9b- hexahydro- 8, 9b- dimethyl- 1*H* cyclopenta [*c*] quinolin- 4- yl)- 3- methylbenzoic acid (exo- isomer **74**)

$^1$H NMR (400 MHz, DMSO- $d_6$) δ 7.76 (2H, m), 7.53 (1 H, m), 6.96 (1 H, s), 6.69 (1 H, d, *J*=8Hz), 6.55 (1 H, d, *J*=8Hz), 5.56 (1 H, s), 3.99 (1 H, d, *J*=9.2Hz), 2.39 (3h, s), 2.17 (3H, s), 2.06- 2.04 (1 H, m), 1.90- 1.87 (2H, m), 1.79-1.73 (1 H, m), 1.61- 1.59 (2H, m), 1.23 (1 H, m), 1.14 (3H, m) .

LC- MS: $t_R$ = 10.22 $[M+H]^+$ = 336 (method 1)

4- ((3a*S*, 4*R*, 9b*R*)- 2, 3, 3a, 4, 5, 9b- hexahydro- 8, 9b- dimethyl- 1*H* cyclopenta [*c*] quinolin- 4- yl)- 3- methylbenzoic acid (endo- isomer **75**)

$^1$H NMR (400 MHz, DMSO- $d_6$) δ 7.79 (1 H, d, J=8.4Hz), 7.75 (1 H, s), 7.68 (1 H, d, J=8Hz), 6.95 (1 H, s), 6.70 (1 H, dd, *J*= 1.2 & 8Hz), 6.57 (1 H, d, *J*=8Hz), 5.59 (1 H, s), 4.62 (1 H, d, *J*=2Hz), 2.38 (3H, s), 2.16 (3H, s), 2.09- 2.04 (1 H, m), 1.95- 1.92 (1 H, m), 1.69- 1.64 (2H, m), 1.43 (3H, s), 1.38- 1.37 (1 H, m), 1.23- 1.17 (1 H, m), 1.03- 1.01 (1 H, m) .

LC- MS: $t_R$ = 11.02 $[M+H]^+$ = 336 (method 1)

***Example 76: 4-((3aS,4R,9bR)-2,3,3a,4,5,9b-hexahydro-8-isobutoxy-1H-cyclopenta[c]quinolin-4-yl)-3-methylbenzoic acid***

The following compound was prepared using the same methodology as in example 30 from **intermediate compound 121** (0.47 mmol, 187 mg). The mixture was stirred at r.t. until HPLC showed no starting material left. It was necessary 48 h and an addicional 1.5 mL of 1M LiOH to complete the reaction. After that, the solvent was removed in vacuum and purification of the crude material by reverse $C_{18}$ chromatography using water (0.1% formic acid) and acetonitrile (0.1% formic acid) as eluents gave 135 mg of the pure title endo-isomer (Yield: 75%).

$^1$H NMR (400 MHz, DMSO- $d_6$) δ 7.78 (1 H, d, *J* = 8 Hz), 7.74 (1 H, s), 7.69 (1 H, d, *J* = 8 Hz), 6.63- 6.61 (2H, m), 6.53 (1 H, dd, *J* = 8.4 & 2.8 Hz), 5.22 (1 H, s), 4.57 (1 H, d, *J* = 2.4 Hz), 3.62 (2H, d, *J* = 6.8 Hz), 3.41- 3.36 (1 H, m), 2.50- 2.42 (1 H, m), 2.37 (3H, s), 2.13- 2.04 (1H, m), 2.00- 1.90 (2H, m), 1.69- 1.56 (2H, m), 1.44- 1.32 (2H, m), 1.02- 0.94 (1 H, m), 0.96 (6 H, d, *J*= 6.8 Hz)

LC- MS: $t_R$ = 9.18 $[M+H]^+$= 380 (method 1)

***Example 77: 4-((3aS,4S,9bR)-2,3,3a,4,5,9b-hexahydro-8-isobutoxy-1H-cyclopenta[c]quinolin-4-yl)-3-methylbenzoic acid***

The following compound was prepared using the same methodology as in example 30 from **intermediate compound 122** (0.03 mmol, 13 mg). It was necessary 40 h and an addicional 0.07 mL of 1M LiOH to complete the reaction. Solvent was evaporated and purification of the crude material by reverse $C_{18}$ chromatography using water (0.1% formic acid) and acetonitrile (0.1% formic acid) as eluents gave 6 mg of the pure title exo-isomer (Yield: 47%).

$^1$H NMR (400 MHz, DMSO- $d_6$) δ 7.77- 7.75 (2H, m), 7.52- 7.50 (1 H, m), 6.67 (1 H, d, *J* = 2.4 Hz), 6.58- 6.52 (2H, m), 5.46 (1 H, s), 3.95 (1 H, d, *J* = 10 Hz), 3.61 (2H, d, *J* = 6.8 Hz), 2.97- 2.90 (1 H, m), 2.40 (3H, s), 2.36- 2.28 (1 H, m), 2.27- 2.20 (1 H, m), 1.99- 1.90 (2H, m), 1.68- 1.50 (4H, m), 1.23- 1.12 (1 H, m), 0.96 (6 H, d, *J*= 6.8 Hz) .

LC- MS: $t_R$ = 8.62 $[M+H]^+$= 380 (method 1)

***Example 78: 4-((3aS, 4S, 9bR)-2, 3, 3a, 4, 5, 9b-hexahydro-8-methoxy-5,9b-dimethyl-1H-cyclopenta[c]quinolin-4-yl)benzoic acid***

The following compound was prepared using the same methodology as in example 42 using **example 68** 4-((3aS, 4S, 9bR)- 2, 3, 3a, 4, 5, 9b- hexahydro- 8- methoxy- 9b- methyl- 1H- cyclopenta [c] quinolin- 4- yl) benzoic acid (0.1 mmol, 33 mg) . The crude material was purified by reverse $C_{18}$ chromatography using water (0.1% formic acid) and acetonitrile (0.1% formic acid) as eluents to give the pure title exo- isomer (15 mg. Yield: 43%) .

$^1$H NMR (400 MHz, DMSO- $d_6$) δ 7.84 (2H, d, *J*= 8.4 Hz), 7.23 (2H, d, *J*= 8 Hz), 6.75 (1 H, d, *J*=2.8 Hz), 6.70 (1 H, dd, *J*= 2.8 & 8.8 Hz), 6.64 (1 H, d, *J*= 8.8 Hz), 4.51 (1 H, d, *J*= 4.8 Hz), 3.67 (3H, s), 2.75 (3H, s), 2.30- 2.24 (1 H, m), 1.99- 1.87 (2H, m), 1.79- 1.71 (1 H, m), 1.56- 1.46 (2H, m), 1.38- 1.30 (1 H, m) .

LC- MS: $t_R$ = 10.27 $[M+H]^+$ = 352 (method 1)

***Example 79: 4-((3aS, 4R, 9bR)-2,3,3a,4,5,9b-hexahydro-8-methoxy-5,9b-dimethyl-1H-cyclopenta[c]quinolin-4-yl) benzoic acid***

The following compound was prepared using the same methodology as in example 42 using **example 69** 4-((3aS, 4R, 9bR)- 2, 3, 3a, 4, 5, 9b- hexahydro- 8- methoxy- 9b- methyl- 1H- cyclopenta [c] quinolin- 4- yl) benzoic acid (0.37 mmol, 126 mg) . The crude material was purified by reverse $C_{18}$ chromatography using water (0.1% formic acid) and acetonitrile (0.1% formic acid) as eluents to give the pure title endo- isomer (46 mg. Yield: 35%) .

$^1$H NMR (400 MHz, DMSO- $d_6$) δ 7.93 (2H, d, *J*= 8.4 Hz), 7.45 (2H, d, *J*= 8.4 Hz), 6.80 (1 H, d, *J*= 9.2 Hz), 6.77 (1 H, d, *J*= 2.8 Hz), 6.69 (1 H, dd, *J*= 2.8 & 8.8 Hz), 4.30 (1 H, d, *J*= 3.6 Hz), 3.68 (3H, s), 2.65 (3H, s), 2.25- 2.19 (1 H, m), 1.89- 1.84 (1 H, m), 1.65- 1.52 (2H, m), 1.38- 1.30 (1 H, m), 1.19- 1.04 (2H, m)

LC- MS: $t_R$ = 10.63 $[M+H]^+$ = 352 (method 1)

## Examples of biological activity

In the following examples the biological activity of compounds of formula (I) towards EP1 receptors is shown.

### *Human EP1 receptor radioligand binding assay*

To investigate binding properties of EP1 receptor ligands to human EP1 receptor, transfected HEK- 293 cell membranes and [3H]- PGE2 (Perkin Elmer) were used. In 96- well plates the assay was carried out with a total reaction volume of 250 μl, containing 25 μl of membrane suspension (30 μg protein/ well), 25 μl of [3H]- PGE2 (10 nM) in either absence or presence of 25 μl of either buffer or PGE2 (10 μM) for total and non- specific binding, respectively. Binding buffer contained 10 mM MES, 1 mM MgCl2 and 1 mM EDTA at pH 6.0. Plates were incubated at 25 °C for 60 minutes. After the incubation period, 200 μl of incubate were transferred to MultiScreen HTS, FB plates (Millipore), filtered and plates were washed 6 times with ice- cold 10 mM MES, 1 mM MgCl2 and 1 mM EDTA at pH 6.0. Filters were dried and counted in a MicroBeta scintillation counter (Perkin- Elmer) using EcoScint liquid scintillation cocktail.

Percentage inhibition was calculated relating compounds activity to the 0% inhibition of the wells incubated with 10 nM [3H]-PGE2 alone (total binding) and 100% inhibition of the wells incubated with 10 nM [3H]-PGE2 plus 10 μM PGE2 (non-specific binding). Competition binding curves were carried out by assaying 6 different concentrations with duplicated points. The inhibition constant (Ki) of each compound was calculated by the Cheng-Prusoff equation:

$$K_i = IC_{50} / (1 + [L] / KD)$$

where $IC_{50}$ is the concentration of compound that displaces the binding of radioligand by 50%, [L] is the free concentration of radioligand and KD is the dissociation constant of each radioligand. $IC_{50}$ values were obtained by fitting the data with non-linear regression using XIFit software (IDBS).

The results obtained in the biological assays disclosed above with representative compounds of formula (I) are shown in the Table below in which A means a Ki < 100nM, B means a Ki value between 100nM and 500nM and C means a Ki > 500 nM.

| EXAMPLE | Ki Binding |
|---|---|
| EXAMPLE 02 | B |
| EXAMPLE 04 | B |
| EXAMPLE 06a | A |
| EXAMPLE 07 | A |
| EXAMPLE 12 | C |
| EXAMPLE 18 | B |
| EXAMPLE 19 | C |
| EXAMPLE 29 | C |
| EXAMPLE 30 | C |
| EXAMPLE 36 | C |
| EXAMPLE 37a | A |
| EXAMPLE 43 | B |
| EXAMPLE 47 | B |
| EXAMPLE 55 | B |
| EXAMPLE 56 | A |
| EXAMPLE 57 | A |
| EXAMPLE 62 | C |
| EXAMPLE 69 | B |
| EXAMPLE 75 | B |

**References**

Abe T, Kunz A, Shimamura M, Zhou P, Anrather J, Iadecola C. (2009) The neuroprotective effect of prostaglandin E2 EP1 receptor inhibition has a wide therapeutic window, is sustained in time and is not sexually dimorphic. J Cereb Blood Flow Metab. 29 (1) : 66- 72.

Asbóth G, Phaneuf S, Europe- Finner GN, Tóth M, Bernal AL. (1996) Prostaglandin E2 activates phospholipase C and elevates intracellular calcium in cultured myometrial cells: involvement of EP1 and EP3 receptor subtypes. Endocrinology. 137 (6) : 2572- 9.

Baba H, Kohno T, Moore KA, Woolf CJ. (2001) Direct activation of rat spinal dorsal horn neurons by prostaglandin E2 The Journal of Neuroscience, 21 (5):1750—1756.

Breyer MD, Breyer RM. (2000) Prostaglandin receptors: their role in regulating renal function. Curr Opin Nephrol Hypertens. 2000 Jan; 9 (1) : 23- 9.

Candelario- Jalil E, Slawik H, Ridelis I, Waschbisch A, Akundi RS, Hüll M, Fiebich BL. (2005) Regional distribution of the prostaglandin E2 receptor EP1 in the rat brain: accumulation in Purkinje cells of the cerebellum. J Mol Neurosci. 27 (3) : 303- 10.

Coleman, R. A., Prostanoid Receptors. IUPHAR compendium of receptor characterization and classification, 2nd edition, 338-353, 2000.

Dirig DM, Yaksh TL. (1999) In vitro prostanoid release from spinal cord following peripheral inflammation: effects of substance P, NMDA and capsaicin. Br J Pharmacol. 126 (6) : 1333- 40.

Durrenberger PF, Facer P, Casula MA, Yiangou Y, Gray RA, Chessell IP, Day NC, Collins SD, Bingham S, Wilson AW, Elliot D, Birch R, Anand P. (2006) Prostanoid receptor EP1 and Cox-2 in injured human nerves and a rat model of nerve injury: a time-course study. BMC Neurol. 4;6:1.

Giblin GM, Bit RA, Brown SH, Chaignot HM, Chowdhury A, Chessell IP, Clayton NM, Coleman T, Hall A, Hammond B, Hurst DN, Michel AD, Naylor A, Novelli R, Scoccitti T, Spalding D, Tang SP, Wilson AW, Wilson R. (2007) The discovery of 6- [2- (5- chloro- 2- { [ (2, 4- difluorophenyl) methyl] oxy} phenyl)- 1- cyclopenten- 1- yl]- 2- pyridinecarboxylic acid, GW848687X, a potent and selective prostaglandin EP1 receptor antagonist for the treatment of inflammatory pain. Bioorg Med Chem Lett. 17 (2) : 385- 9.

T.W. Greene and P.G.M. Wuts "Protective groups in organic synthesis" (John Wiley & sons 10 1999)

Guay J., Bateman, K., Gordon R., Mancini J., Riendeau D. (2004) Carrageenan-induced paw edema in rat elicits a predominant prostaglandin E2 (PGE2) response in the central nervous system associated with the induction of microsomal PGE2 synthase-1 J. Biol Chem 2004. 279, 24866-24872.

Hall, A., Billinton A., Giblin G.M. (2007) EP1 antagonists for the treatment of inflammatory pain. Curr Opin. Drug Discov. Devel. 10 (2007) 597-612.

Hall A, Brown SH, Budd C, Clayton NM, Giblin GM, Goldsmith P, Hayhow TG, Hurst DN, Naylor A, Anthony Rawlings D, Scoccitti T, Wilson AW, Winchester WJ. (2009) Discovery of GSK345931 A: An EP (1) receptor antagonist with efficacy in preclinical models of inflammatory pain. Bioorg Med Chem Lett. 19 (2) : 497- 501.

Hönemann CW, Heyse TJ, Möllhoff T, Hahnenkamp K, Berning S, Hinder F, Linck B, Schmitz W, van Aken H. (2001) The inhibitory effect of bupivacaine on prostaglandin E (2) (EP (1) ) receptor functioning: mechanism of action. Anesth Analg. 93 (3) : 628- 634.

Johansson T, Narumiya S, Zeilhofer HU. (2011) Contribution of peripheral versus central EP1 prostaglandin receptors to inflammatory pain. Neurosci Lett. 495 (2) : 98- 101.

Kawahara H, Sakamoto A, Takeda S, Onodera H, Imaki J, Ogawa R. (2001) A prostaglandin E2 receptor subtype EP1 receptor antagonist (ONO- 8711) reduces hyperalgesia, allodynia, and c- fos gene expression in rats with chronic nerve constriction. Anesth Analg. 93 (4) : 1012- 7.

Lee T, Hedlund P, Newgreen D, Andersson KE. (2007) Urodynamic effects of a novel EP (1) receptor antagonist in normal rats and rats with bladder outlet obstruction. J Urol. 177 (4) : 1562- 1567.

Lee C.M, Genetos DC, You Z, Yellowley CE. (2007b) Hypoxia regulates PGE (2) release and EP1 receptor expression in osteoblastic cells. J Cell Physiol. 212 (1) : 182- 188

Li X, Cudaback E, Keene CD, Breyer RM, Montine TJ. (2011) Suppressed microglial E prostanoid receptor 1 signaling selectively reduces tumor necrosis factor alpha and interleukin 6 secretion from toll- like receptor 3 activation. Glia. 59 (4) : 569- 576

Lin CR, Amaya F, Barrett L, Wang H, Takada J, Samad TA, Woolf CJ (2006) Prostaglandin E2 receptor EP4 contributes to inflammatory pain hypersensitivity. J Pharmacol Exp Ther. 319 (3) : 1096- 103.

Ma W, Eisenach JC. (2003) Four PGE2 EP receptors are up- regulated in injured nerve following partial sciatic nerve ligation. Exp Neurol. 183 (2) : 581- 92.

Miki, T.; Matsunami, M.; Okada, H.; Matsuya, H.; Kawabata, A (2010) ONO-8130, an EP1 antagonist, strongly attenuates cystitis-related bladder pain caused by cyclophosphamide in mice. J Pharmacol Sci 112(Suppl. 1): Abst P1J-1-2

Minami T, Uda R., Horiguchi S., Ito S. Hyodo M., Hayaishi O. (1994) Allodynia evoked by intrathecal administration fo prostaglandin E2 to conscious mice. Pain, 1994, 57: 217-223.

Minami T, Nakano H, Kobayashi T, Sugimoto Y, Ushikubi F, Ichikawa A, Narumiya S, Ito S. (2001) Characterization of EP receptor subtypes responsible for prostaglandin E2- induced pain responses by use of EP1 and EP3 receptor knockout mice. Br J Pharmacol. 133 (3) : 438- 44.

Mizuguchi S, Ohno T, Hattori Y, Ae T, Minamino T, Satoh T, Arai K, Saeki T, Hayashi I, Sugimoto Y, Narumiya S, Saigenji K, Majima M. (2010) Roles of prostaglandin E2- EP1 receptor signaling in regulation of gastric motor activity and emptying. Am J Physiol Gastrointest Liver Physiol. 299 (5) : G1078- 1086

Moriyama T, Higashi T, Togashi K, Iida T, Segi E, Sugimoto Y, Tominaga T, Narumiya S, Tominaga M. (2005) Sensitization of TRPV1 by EP1 and IP reveals peripheral nociceptive mechanism of prostaglandins. Mol Pain. 1: 3.

Nakayama Y, Omote K, Namiki A. (2002) Role of prostaglandin receptor EP1 in the spinal dorsal horn in carrageenan-induced inflammatory pain. Anesthesiology. 97 (5) : 1254- 62.

Nakayama Y, Omote K, Kawamata T, Namiki A. (2004) Role of prostaglandin receptor subtype EP1 in prostaglandin E2- induced nociceptive transmission in the rat spinal dorsal horn. Brain Res. 1010 (1- 2) : 62- 8.

Narumiya S., Sugimoto Y., Ushikubi F. (1999) Protanoid receptors: structures, properties, and functions. Physiol Rev. 79 (1999) 1193-1226.

Niho N, Mutoh M, Kitamura T, Takahashi M, Sato H, Yamamoto H, Maruyama T, Ohuchida S, Sugimura T, Wakabayashi K. (2005) Suppression of azoxymethane- induced colon cancer development in rats by a prostaglandin E receptor EP1- selective antagonist. Cancer Sci. 96 (5) : 260- 264.

Oidda H., Namba T., Sugimoto Y., Ushikubi F., Ohishi H., Ichikawa A. et al (1995) In situ hybridization studies of prostacyclin receptor mRNA expression in various mouse organs. Br J Pharmacol 1995, 116, 2828-2837.

Oka T, Oka K, Saper CB. (2003) Contrasting effects of E type prostaglandin (EP) receptor agonists on core body temperature in rats. Brain Res. 968 (2) : 256- 262.

Oka T, Hosoi M, Oka K, Hori T. (1997) Biphasic alteration in the trigeminal nociceptive neuronal responses after

intracerebroventricular injection of prostaglandin E2 in rats. Brain Res. 749 (2) : 354- 7. Erratum in: Brain Res 757 (2) : 299.

Okada, H., Konemura, T., Maruyama, T (2010) ONO-8539, a novel ep1 receptor antagonist, suppresses bladder hyperactivity via excessive production of prostaglandin e2 (pge2) induced by intravesical instillation of atp in urodynamic evaluation of cynomolgus monkeys. Eur Urol Suppl 9(2):72

Omote K, Yamamoto H, Kawamata T, Nakayama Y, Namiki A. (2002) The effects of intrathecal administration of an antagonist for prostaglandin E receptor subtype EP (1) on mechanical and thermal hyperalgesia in a rat model of postoperative pain. Anesth Analg. 95 (6) : 1708- 12.

Omote K, Kawamata T, Nakayama Y, Kawamata M, Hazama K, Namiki A. (2001) The effects of peripheral administration of a novel selective antagonist for prostaglandin E receptor subtype EP (1), ONO- 8711, in a rat model of postoperative pain. Anesth Analg. 92 (1) : 233- 238.

Rahal S, McVeigh LI, Zhang Y, Guan Y, Breyer MD, Kennedy CR. (2006) Increased severity of renal impairment in nephritic mice lacking the EP1 receptor. Can J Physiol Pharmacol. 84 (8- 9) : 877- 885.

Sarkar S, Hobson AR, Hughes A, Growcott J, Woolf CJ, Thompson DG, Aziz Q. (2003) The prostaglandin E2 receptor- 1 (EP- 1) mediates acid- induced visceral pain hypersensitivity in humans. Gastroenterology. 124 (1) : 18-25.

Samad TA, Sapirstein A, Woolf CJ. (2002) Prostanoids and pain: unraveling mechanisms and revealing therapeutic targets. Trends Mol Med. 2002 Aug; 8 (8) : 390- 6.

Schlötzer-Schrehardt U, Zenkel M, Nüsing R.M. (2002) Expression and Localization of FP and EP Prostanoid. Invest. Ophthalmol. Vis. Sci. 43(5) 1475-1487.

Syriatowicz JP, Hu D, Walker JS, Tracey DJ. (1999) Hyperalgesia due to nerve injury: role of prostaglandins. Neuroscience. 94 (2) : 587- 94.

Teramura, T.; Kawatani, M.; Maruyama, T. (2000) Prostaglandin E1 facilitate primary afferent activity from the urinary bladder in the rat using selective EP1- receptor antagonist (ONO- 8711) . BJU Int 86 (Suppl. 3) : Abst P6.3.19

Watanabe K, Kawamori T, Nakatsugi S, Ohta T, Ohuchida S, Yamamoto H, Maruyama T, Kondo K, Ushikubi F, Narumiya S, Sugimura T, Wakabayashi K. (1999) Role of the prostaglandin E receptor subtype EP1 in colon carcinogenesis. Cancer Res. 59 (20) : 5093- 5096.

Wilbraham D., Masuda T.,Deacon S.,Kuwayama T., Vincent S. (2010) Safety, tolerability and pharmacokinetic of multiple ascending doses of the ep-1 receptor antagonist ono-8539, a potential new and novel therapy to overactive bladder in healthy young and elderly subjects Eur Urol Suppl 9(2):250.

Woodward DF, Regan JW, Lake S, Ocklind A. (1997) The molecular biology and ocular distribution of prostanoid receptors. Surv Ophthalmol. 41 Suppl 2:S15-21.

Zhang M, Ho HC, Sheu TJ, Breyer MD, Flick LM, Jonason JH, Awad HA, Schwarz EM, O'Keefe RJ.J (2011) EP1 (- /- ) mice have enhanced osteoblast differentiation and accelerated fracture repair. Bone Miner Res. 26 (4) : 792- 802.

## Claims

**1.** A compound of general formula **(I):**

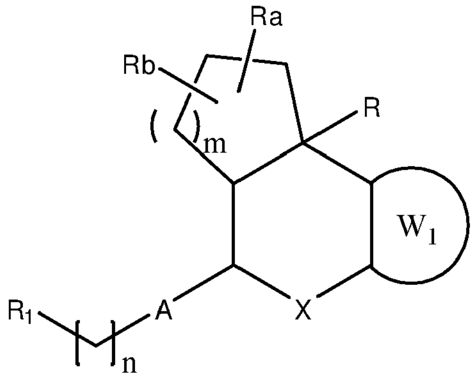

**(I)**

wherein:

**W1** is a benzene ring optionally substituted by one or more $R_2$ and optionally fused to a carbocyclic ring; or a 5 or 6-membered heterocyclic ring containing 1 or 2 heteroatoms selected from N, O or S, optionally substituted by one or more $R_2$;
**R** is a hydrogen; or $C_{1-6}$- alkyl;
**Ra** and **Rb** are independently selected from hydrogen;- OH;- $CH_2OH$; $C_{1-6}$- alkyl; —O—$C_{1-6}$- alkyl or —O—$C_{1-6}$- haloalkyl;
**$R_1$** is a —COOH; a tetrazol; a —$SO_2$- NH- C (=O)- $R_3$; a —C (=O) NH- $SO_2$- $R_4$; a —$SO_2$- OH or a —CN;
**$R_2$** is independently selected from hydrogen; halogen; $C_{1-6}$ alkyl; $C_{1-6}$- haloalkyl; — O—$C_{1-6}$- alkyl; —O—$C_{1-6}$- haloalkyl; $C_{3-6}$cycloalkyl; —O—$C_{3-6}$cycloalkyl;- $NR_5SO_2R_6$;
phenyl; -$CONH_2$ or —CN;
**$R_3$** and **$R_4$** are independently selected from a hydrogen; $C_{1-6}$- alkyl; an optionally substituted phenyl; or —N $(CH_3)_2$;
**$R_5$** and **$R_6$** are independently selected from a hydrogen; or $C_{1-6}$- alkyl **$R_7$** is selected from a hydrogen; $C_{1-6}$- alkyl; or a —$COR_8$
**$R_8$** is a $C_{1-6}$- alkyl;
**X** is selected from $NR_7$ or O;
**A** is a phenyl optionally substituted by one or more substituents selected from $C_{1-6}$- alkyl, halogen, $C_{1-6}$- haloalkyl, —O—$C_{1-6}$- alkyl, —O—$C_{1-6}$- haloalkyl, -NH- CO- $CH_3$, a —CN or a $C_{3-6}$cycloalkyl optionally substituted by a $C_{1-6}$- alkyl; or an heteroaromatic ring or ring system optionally substituted by one or more substituents selected from $C_{1-6}$- alkyl, halogen, $C_{1-6}$- haloalkyl, —O—$C_{1-6}$- alkyl, —O—$C_{1-6}$- haloalkyl, -NH- CO- $CH_3$, a —CN or a $C_{3-6}$cycloalkyl optionally substituted by a $C_{1-6}$- alkyl ;
**n** is 0, 1 or 2;
**m** is 1, or 2 or 3;

and the salts, solvates and prodrugs thereof.

**2.** A compound according to claim 1 having general formula **(Ia):**

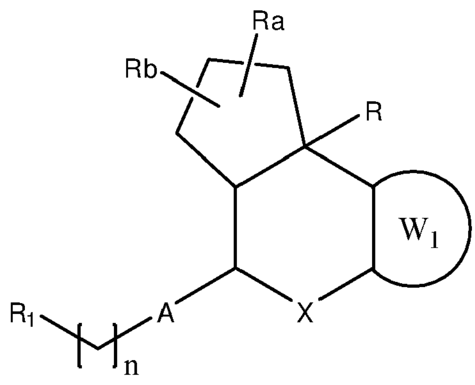

**(Ia)**

where **W1, R**, **Ra, Rb**, **$R_1$,** A, **X** and **n** have the same meanings as in claim 1.

**3.** A compound according to any of claim 1 or 2 having general formula **(Ia'):**

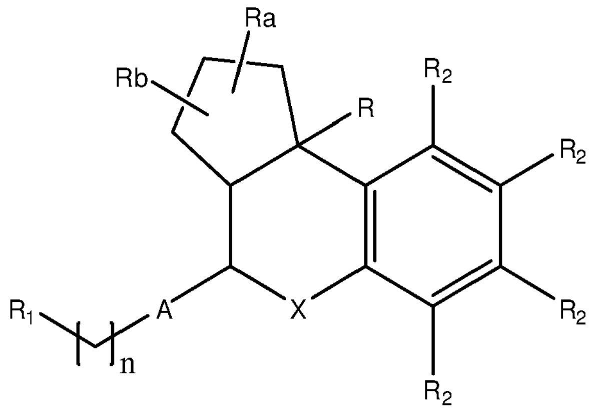

**(Ia')**

where **R**, **Ra, Rb**, **$R_1$, $R_2$, A**, X and n have the same meanings as in claim 1.

**4.** A compound according to claim 1 having general formula **(Ib):**

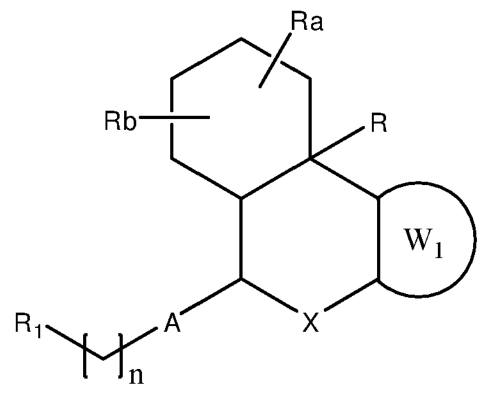

**(Ib)**

where **W1**, **R**, **Ra, Rb**, **$R_1$**, **A, X** and **n** have the same meanings as in claim 1.

**5.** A compound according to any of claims 1 or 4 having general formula **(Ib'):**

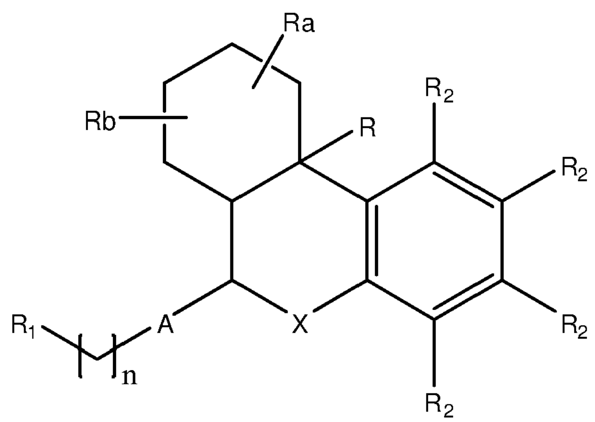

**(Ib')**

where **R**, **Ra, Rb**, **$R_1$, $R_2$**, **A, X** and **n** have the same meanings as in claim 1.

**6.** A compound according to claim 1 where **W1** is a benzene ring optionally substituted by one or more **$R_2$** where **$R_2$** has the same meaning as in claim 1.

**7.** A compound according to any of claims 1 to 6 where **$R_1$** is a -COOH.

**8.** A compound according to any of claims 1 to 7 where **A** is a phenyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted by a $C_{1-6}$- alkyl ; a pyridinyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted by a $C_{1-6}$- alkyl; a pyrazolyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted by a $C_{1-6}$- alkyl; an imidazolyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl,

-NH- CO- $CH_3$, a- CN or a $C_{3-6}$cycloalkyl optionally substituted by a $C_{1-6}$- alkyl; or a thiazolyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted by a $C_{1-6}$- alkyl.

**9.** A compound according to any of claims 1 to 8 where **X** is $NR_7$ where $R_7$ is a hydrogen.

**10.** A compound according to claim 1 to 9 where n=0.

**11.** A compound according to claim 1 where **W1** is a benzene ring optionally substituted by one or more $\mathbf{R_2}$;
$\mathbf{R_1}$ is a- COOH;
**A** is a phenyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted by a $C_{1-6}$- alkyl ; a pyridinyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted by a $C_{1-6}$- alkyl ; a pyrazolyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted by a $C_{1-6}$- alkyl ; an imidazolyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted by a $C_{1-6}$- alkyl ; or a thiazolyl group optionally substituted by one or more substituents selected from $C_{1-6}$- alkyl, halogen, $C_{1-6}$- haloalkyl, -O- $C_{1-6}$- alkyl, -O- $C_{1-6}$- haloalkyl, -NH- CO- $CH_3$, a- CN or a $C_{3-6}$cycloalkyl optionally substituted by a $C_{1-6}$- alkyl;
**X** is $NR_7$ where $R_7$ is a hydrogen ; and
**n** is 0.

**12.** A compound according to claim 1 selected from :

- 4- ((3aS, 4R, 9bR)- 2, 3, 3a, 4, 5, 9b- hexahydro- 1H- cyclopenta [c] quinolin- 4- yl) benzoic acid;
- 4- ((3aS, 4R, 9bR)- 8- bromo- 2, 3, 3a, 4, 5, 9b- hexahydro- 1H- cyclopenta [c] quinolin- 4- yl) benzoic acid;
- 4- ((3aS, 4R, 9bR)- 8- (trifluoromethyl)- 2, 3, 3a, 4, 5, 9b- hexahydro- 1H- cyclopenta [c] quinolin- 4- yl) benzoic acid;
- 4- ((3aS, 4R, 9bR)- 2, 3, 3a, 4, 5, 9b- hexahydro- 8- methyl- 1H- cyclopenta [c] quinolin- 4- yl) benzoic acid;
- 4- ((3aS, 4R, 9bR)- 2, 3, 3a, 4, 5, 9b- hexahydro- 8- methyl- 1H- cyclopenta [c] quinolin- 4- yl) benzoic acid (enantiomer 1) ;
- 4- ((3aS, 4R, 9bR)- 2, 3, 3a, 4, 5, 9b- hexahydro- 8- methyl- 1H- cyclopenta [c] quinolin- 4- yl) benzoic acid (enantiomer 2) ;
- 4- ((3aS, 4S, 9bR)- 2, 3, 3a, 4, 5, 9b- hexahydro- 8- methyl- 1H- cyclopenta [c] quinolin- 4- yl) benzoic acid;
- 4- ((3aS, 4R, 9bR)- 8- chloro- 2, 3, 3a, 4, 5, 9b- hexahydro- 1H- cyclopenta [c] quinolin- 4- yl) benzoic acid;
- 4- ((3aS, 4R, 9bR)- 8- chloro- 2, 3, 3a, 4, 5, 9b- hexahydro- 1H- cyclopenta [c] quinolin- 4- yl) benzoic acid (enantiomer 1) ;
- 4- ((3aS, 4R, 9bR)- 8- chloro- 2, 3, 3a, 4, 5, 9b- hexahydro- 1H- cyclopenta [c] quinolin- 4- yl) benzoic acid (enantiomer 2) ;
- 4- ((3aS, 4R, 9bR)- 2, 3, 3a, 4, 5, 9b- hexahydro- 8- methoxy- 1H- cyclopenta [c] quinolin- 4- yl) benzoic acid;
- 4- ((3aS, 4R, 9bR)- 2, 3, 3a, 4, 5, 9b- hexahydro- 8- methoxy- 1H- cyclopenta [c] quinolin- 4- yl) benzoic acid (enantiomer 1) ;
- 4- ((3aS, 4R, 9bR)- 2, 3, 3a, 4, 5, 9b- hexahydro- 8- methoxy- 1H- cyclopenta [c] quinolin- 4- yl) benzoic acid (enantiomer 2) ;
- 4- ((3aS, 4S, 9bR)- 2, 3, 3a, 4, 5, 9b- hexahydro- 8- methoxy- 1H- cyclopenta [c] quinolin- 4- yl) benzoic acid;
- 4- ((3aS, 4R, 9bR)- 6- cyclopropyl- 2, 3, 3a, 4, 5, 9b- hexahydro- 1H- cyclopenta [c] quinolin- 4- yl) benzoic acid;
- 4- ((3aS, 4S, 9bR)- 8- cyclopropyl- 2, 3, 3a, 4, 5, 9b- hexahydro- 1H- cyclopenta [c] quinolin- 4- yl) benzoic acid;
- 4- ((3aS, 4R, 9bR)- 8- cyclopropyl- 2, 3, 3a, 4, 5, 9b- hexahydro- 1H- cyclopenta [c] quinolin- 4- yl) benzoic acid;
- (+)- 4- ((3a*S*, 4*R*, 9b*R*)- 8- cyclopropyl- 2, 3, 3a, 4, 5, 9b- hexahydro- 1*H*- cyclopenta [*c*] quinolin- 4- yl) benzoic acid;
- (- )- 4- ((3a*S*, 4*R*, 9b*R*)- 8- cyclopropyl- 2, 3, 3a, 4, 5, 9b- hexahydro- 1*H*- cyclopenta [*c*] quinolin- 4- yl) benzoic acid;
- 4- ((3aS, 4R, 9bR)- 2, 3, 3a, 4, 5, 9b- hexahydro- 8- propyl- 1H- cyclopenta [c] quinolin- 4- yl) benzoic acid;
- 4- ((3aS, 4R, 9bR)- 8- fluoro- 2, 3, 3a, 4, 5, 9b- hexahydro- 1H- cyclopenta [c] quinolin- 4- yl) benzoic acid;
- 4- ((3aS, 4R, 9bR)- 2, 3, 3a, 4, 5, 9b- hexahydro- 8- phenyl- 1H- cyclopenta [c] quinolin- 4- yl) benzoic acid;
- 4- ((3aS, 4R, 9bR)- 8- tert- butyl- 2, 3, 3a, 4, 5, 9b- hexahydro- 1H- cyclopenta [c] quinolin- 4- yl) benzoic acid;

- 4- ((3aS, 4R, 9bR)- 8- ethyl- 2, 3, 3a, 4, 5, 9b- hexahydro- 1H- cyclopenta [c] quinolin- 4- yl) benzoic acid;
- (+)- 4- ((3a*S*, 4*R*, 9*bR*)- 8- ethyl- 2, 3, 3a, 4, 5, 9b- hexahydro- 1H- cyclopenta [c] quinolin- 4- yl) benzoic acid;
- (- )- 4- ((3a*S*, 4*R*, 9*bR*)- 8- ethyl- 2, 3, 3a, 4, 5, 9b- hexahydro- 1H- cyclopenta [c] quinolin- 4- yl) benzoic acid;
- 4- ((3aS, 4R, 9bR)- 8- carbamoyl- 2, 3, 3a, 4, 5, 9b- hexahydro- 1H- cyclopenta [c] quinolin- 4- yl) benzoic acid;
- 4- ((3aS, 4R, 9bR)- 2, 3, 3a, 4, 5, 9b- hexahydro- 8- isopropyl- 1H- cyclopenta [c] quinolin- 4- yl) benzoic acid;
- 4- ((3aS, 4R, 9bR)- 8- cyano- 2, 3, 3a, 4, 5, 9b- hexahydro- 1H- cyclopenta [c] quinolin- 4- yl) benzoic acid;
- 4- ((3aS, 4R, 9bR)- 8- [(N- methylsulfonyl) amino]- )- 2, 3, 3a, 4, 5, 9b- hexahydro- 1H- cyclopenta [c] quinolin- 4- yl) benzoic acid;
- 4- ((3aS, 4R, 9bR)- 8- [(N- methyl- N- methylsulfonyl) amino]- 2, 3, 3a, 4, 5, 9b- hexahydro- 1H- cyclopenta [c] quinolin- 4- yl) benzoic acid;
- 3- ((3aS, 4R, 9bR)- 8- bromo- 2, 3, 3a, 4, 5, 9b- hexahydro- 1H- cyclopenta [c] quinolin- 4- yl) benzoic acid;
- 4- ((3aS, 4R, 9bR)- 2, 3, 3a, 4, 5, 9b- hexahydro- 7, 8- dimethoxy- 1H- cyclopenta [c] quinolin- 4- yl) benzoic acid;
- 4- ((3aS, 4R, 9bR)- 7- (trifluoromethyl)- 2, 3, 3a, 4, 5, 9b- hexahydro- 8- methyl- 1H- cyclopenta [c] quinolin- 4- yl) benzoic acid;
- 4- ((3aS, 4R, 9bR)- 9- (trifluoromethyl)- 2, 3, 3a, 4, 5, 9b- hexahydro- 8- methyl- 1H- cyclopenta [c] quinolin- 4- yl) benzoic acid;
- 4- ((3aS, 4R, 9bR)- 2, 3, 3a, 4, 5, 9b- hexahydro- 7- methoxy- 8- methyl- 1H- cyclopenta [c] quinolin- 4- yl) benzoic acid;
- 4- ((3aS, 4R, 9bR)- 2, 3, 3a, 4, 5, 9b- hexahydro- 6, 8- dimethyl- 1H- cyclopenta [c] quinolin- 4- yl) benzoic acid;
- 4- ((3aS, 4R, 9bR)- 9- chloro- 2, 3, 3a, 4, 5, 9b- hexahydro- 8- methyl- 1H- cyclopenta [c] quinolin- 4- yl) benzoic acid;
- 4- ((3aS, 4R, 9bR)- 7- chloro- 2, 3, 3a, 4, 5, 9b- hexahydro- 8- methyl- 1H- cyclopenta [c] quinolin- 4- yl) benzoic acid;
- 4- ((3aS, 4R, 9bR)- 2, 3, 3a, 4, 5, 9b- hexahydro- 5, 8- dimethyl- 1H- cyclopenta [c] quinolin- 4- yl) benzoic acid;
- 4- ((3aS, 4R, 9bR)- 5- acetyl- 2, 3, 3a, 4, 5, 9b- hexahydro- 8- methyl- 1H- cyclopenta [c] quinolin- 4- yl) benzoic acid;
- 4- ((6R, 6aS, 9aR)- 6, 6a, 7, 8, 9, 9a- hexahydro- 2- methyl- 5H- cyclopenta [c] [1, 5] naphthyridin- 6- yl) benzoic acid;
- 4- ((6R, 6aS, 9aR)- 6, 6a, 7, 8, 9, 9a- hexahydro- 2- methyl- 5H- cyclopenta [c] [1, 8] naphthyridin- 6- yl) benzoic acid;
- 2- (4- ((3aS, 4R, 9bR)- 2, 3, 3a, 4, 5, 9b- hexahydro- 8- methyl- 1H- cyclopenta [c] quinolin- 4- yl) phenyl) acetic acid;
- 3- (4- ((3aS, 4R, 9bR)- 2, 3, 3a, 4, 5, 9b- hexahydro- 8- methyl- 1H- cyclopenta [c] quinolin- 4- yl) phenyl) propanoic acid;
- 4- ((3aS, 4R, 9bR)- 2, 3, 3a, 4, 5, 9b- hexahydro- 8- methyl- 1H- cyclopenta [c] quinolin- 4- yl)- 2- methylbenzoic acid;
- 4- ((3aS, 4R, 9bR)- 2, 3, 3a, 4, 5, 9b- hexahydro- 8- methyl- 1H- cyclopenta [c] quinolin- 4- yl)- 3- methylbenzoic acid;
- (+)- 4- ((3aS, 4R, 9bR)- 2, 3, 3a, 4, 5, 9b- hexahydro- 8- methyl- 1H- cyclopenta [c] quinolin- 4- yl)- 3- methylbenzoic acid;
- (- )- 4- ((3aS, 4R, 9bR)- 2, 3, 3a, 4, 5, 9b- hexahydro- 8- methyl- 1H- cyclopenta [c] quinolin- 4- yl)- 3- methylbenzoic acid;
- 4- ((3aS, 4S, 9bR)- 2, 3, 3a, 4, 5, 9b- hexahydro- 8- methyl- 1H- cyclopenta [c] quinolin- 4- yl)- 3- methylbenzoic acid;
- 4- ((3aS, 4R, 9bR)- 8- chloro- 2, 3, 3a, 4, 5, 9b- hexahydro- 1H- cyclopenta [c] quinolin- 4- yl)- 3- methylbenzoic acid;
- 4- ((3aS, 4R, 9bR)- 8- fluoro- 2, 3, 3a, 4, 5, 9b- hexahydro- 1H- cyclopenta [c] quinolin- 4- yl)- 3- methylbenzoic acid;
- 4- ((3aS, 4R, 9bR)- 2, 3, 3a, 4, 5, 9b- hexahydro- 8- methoxy- 1H- cyclopenta [c] quinolin- 4- yl)- 3- methylbenzoic acid;
- 4- ((3aS, 4R, 9bR)- 2, 3, 3a, 4, 5, 9b- hexahydro- 8- methoxy- 5- methyl- 1H- cyclopenta [c] quinolin- 4- yl)- 3- methylbenzoic acid;
- 4- ((3aS, 4R, 9bR)- 8- cyclopropyl- 2, 3, 3a, 4, 5, 9b- hexahydro- 1H- cyclopenta [c] quinolin- 4- yl)- 3- methylbenzoic acid;
- 4- ((3aS, 4R, 9bR)- 8- (cyclopropylmethoxy)- 2, 3, 3a, 4, 5, 9b- hexahydro- 1H- cyclopenta [c] quinolin- 4- yl)- 3- methylbenzoic acid;
- 4- ((3aS, 4R, 9bR)- 2, 3, 3a, 4, 5, 9b- hexahydro- 8- isopropoxy- 1H- cyclopenta [c] quinolin- 4- yl)- 3- methylbenzoic acid;

● 4- ((3aS, 4S, 9bR)- 2, 3, 3a, 4, 5, 9b- hexahydro- 8- isopropoxy- 1H- cyclopenta [c] quinolin- 4- yl)- 3- methylbenzoic acid;
● 3- chloro- 4- ((3aS, 4R, 9bR)- 2, 3, 3a, 4, 5, 9b- hexahydro- 8- methyl- 1H- cyclopenta [c] quinolin- 4- yl) benzoic acid;
● 4- ((3aS, 4R, 9bR)- 2, 3, 3a, 4, 5, 9b- hexahydro- 8- methoxy- 1H- cyclopenta [c] quinolin- 4- yl)- 3- methoxybenzoic acid;
● 4- ((3aS, 4R, 9bR)- 2, 3, 3a, 4, 5, 9b- hexahydro- 8- methyl- 1H- cyclopenta [c] quinolin- 4- yl)- 3- methoxybenzoic acid;
● 3- bromo- 4- ((3aS, 4R, 9bR)- 2, 3, 3a, 4, 5, 9b- hexahydro- 8- methyl- 1H- cyclopenta [c] quinolin- 4- yl) benzoic acid;
● 3- bromo- 4- ((3aS, 4R, 9bR)- 2, 3, 3a, 4, 5, 9b- hexahydro- 8- methoxy- 1H- cyclopenta [c] quinolin- 4- yl) benzoic acid;
● 3- ethyl- 4- ((3aS, 4R, 9bR)- 2, 3, 3a, 4, 5, 9b- hexahydro- 8- methyl- 1H- cyclopenta [c] quinolin- 4- yl) benzonitrile;
● 3- ethyl- 4- ((3aS, 4R, 9bR)- 2, 3, 3a, 4, 5, 9b- hexahydro- 8- methyl- 1H- cyclopenta [c] quinolin- 4- yl) benzoic acid;
● 3- ethyl- 4- ((3aS, 4R, 9bR)- 2, 3, 3a, 4, 5, 9b- hexahydro- 8- methoxy- 1H- cyclopenta [c] quinolin- 4- yl) benzoic acid;
● 3- ethyl- 4- ((3aS, 4S, 9bR)- 2, 3, 3a, 4, 5, 9b- hexahydro- 8- methoxy- 1H- cyclopenta [c] quinolin- 4- yl) benzoic acid;
● 4- ((3aS, 4S, 9bR)- 2, 3, 3a, 4, 5, 9b- hexahydro- 8- methyl- 1H- cyclopenta [c] quinolin- 4- yl)- 3, 5- dimethylbenzoic acid;
● 4- ((3aS, 4S, 9bR)- 2, 3, 3a, 4, 5, 9b- hexahydro- 8- methoxy- 1H- cyclopenta [c] quinolin- 4- yl)- 3, 5- dimethylbenzoic acid;
● 5- ((3aS, 4R, 9bR)- 2, 3, 3a, 4, 5, 9b- hexahydro- 8- methyl- 1H- cyclopenta [c] quinolin- 4- yl) pyridine- 2- carboxylic acid;
● 6- ((3aS, 4R, 9bR)- 2, 3, 3a, 4, 5, 9b- hexahydro- 8- methyl- 1H- cyclopenta [c] quinolin- 4- yl) pyridine- 3- carboxylic acid;
● 5- ((3aS, 4R, 9bR)- 2, 3, 3a, 4, 5, 9b- hexahydro- 8- methyl- 1H- cyclopenta [c] quinolin- 4- yl)- 6- methylpyridine- 2- carbonitrile;
● 5- ((3aS, 4R, 9bR)- 2, 3, 3a, 4, 5, 9b- hexahydro- 8- methyl- 1H- cyclopenta [c] quinolin- 4- yl)- 6- methylpyridine- 2- carboxylic acid;
● 5- ((3aS, 4R, 9bR)- 2, 3, 3a, 4, 5, 9b- hexahydro- 8- methyl- 1H- cyclopenta [c] quinolin- 4- yl)- 1- methyl- 1H- pyrazole- 3- carboxylic acid;
● 5- ((3aS, 4R, 9bR)- 2, 3, 3a, 4, 5, 9b- hexahydro- 8- methoxy- 1H- cyclopenta [c] quinolin- 4- yl)- 1- methyl- 1H- pyrazole- 3- carboxylic acid;
● 3- ((3aS, 4R, 9bR)- 2, 3, 3a, 4, 5, 9b- hexahydro- 8- methyl- 1H- cyclopenta [c] quinolin- 4- yl)- 1- methyl- 1H- pyrazole- 5- carboxylic acid;
● 3- ((3aS, 4R, 9bR)- 2, 3, 3a, 4, 5, 9b- hexahydro- 8- methoxy- 1H- cyclopenta [c] quinolin- 4- yl)- 1- methyl- 1H- pyrazole- 5- carboxylic acid;
● 4- ((3aS, 4S, 9bR)- 2, 3, 3a, 4, 5, 9b- hexahydro- 8, 9b- dimethyl- 1H- cyclopenta [c] quinolin- 4- yl) benzoic acid;
● 4- ((3aS, 4R, 9bR)- 2, 3, 3a, 4, 5, 9b- hexahydro- 8, 9b- dimethyl- 1H- cyclopenta [c] quinolin- 4- yl) benzoic acid;
● 4- ((3aS, 4S, 9bR)- 2, 3, 3a, 4, 5, 9b- hexahydro- 8- methoxy- 9b- methyl- 1H- cyclopenta [c] quinolin- 4- yl) benzoic acid;
● 4- ((3aS, 4R, 9bR)- 2, 3, 3a, 4, 5, 9b- hexahydro- 8- methoxy- 9b- methyl- 1H- cyclopenta [c] quinolin- 4- yl) benzoic acid;
● 4- ((3aS, 4S, 9bR)- 8- cyclopropyl- 2, 3, 3a, 4, 5, 9b- hexahydro- 9b- methyl- 1H- cyclopenta [c] quinolin- 4- yl) benzoic acid;
● 4- ((3aS, 4R, 9bR)- 8- cyclopropyl- 2, 3, 3a, 4, 5, 9b- hexahydro- 9b- methyl- 1H- cyclopenta [c] quinolin- 4- yl) benzoic acid;
● 4- ((3aS, 4S, 9bR)- 8- chloro- 2, 3, 3a, 4, 5, 9b- hexahydro- 9b- methyl- 1H- cyclopenta [c] quinolin- 4- yl) benzoic acid;
● 4- ((3aS, 4R, 9bR)- 8- chloro- 2, 3, 3a, 4, 5, 9b- hexahydro- 9b- methyl- 1H- cyclopenta [c] quinolin- 4- yl) benzoic acid;
● 4- ((3aS, 4S, 9bR)- 2, 3, 3a, 4, 5, 9b- hexahydro- 8, 9b- dimethyl- 1H- cyclopenta [c] quinolin- 4- yl)- 3- methylbenzoic acid;
● 4- ((3aS, 4R, 9bR)- 2, 3, 3a, 4, 5, 9b- hexahydro- 8, 9b- dimethyl- 1H- cyclopenta [c] quinolin- 4- yl)- 3- methylbenzoic acid;

- 4- ((3aS, 4R, 9bR)- 2, 3, 3a, 4, 5, 9b- hexahydro- 8- isobutoxy- 1H- cyclopenta [c] quinolin- 4- yl)- 3- methylbenzoic acid;
- 4- ((3aS, 4S, 9bR)- 2, 3, 3a, 4, 5, 9b- hexahydro- 8- isobutoxy- 1H- cyclopenta [c] quinolin- 4- yl)- 3- methylbenzoic acid;
- 4- ((3aS, 4S, 9bR)- 2, 3, 3a, 4, 5, 9b- hexahydro- 8- methoxy- 5, 9b- dimethyl- 1H- cyclopenta [c] quinolin-4- yl) benzoic acid;
- 4- ((3aS, 4R, 9bR)- 2, 3, 3a, 4, 5, 9b- hexahydro- 8- methoxy- 5, 9b- dimethyl- 1H- cyclopenta [c] quinolin-4- yl) benzoic acid;

**13.** A compound according to any of the claims 1 to 12 for use as a medicament.

**14.** A compound according to any of claims 1 to 12 for use in the treatment and/or prophylaxis of diseases or disorders mediated by the EP1 receptor.

**15.** A compound according to claim 14 where the disease or disorders comprises inflammatory related pain including low back and neck pain, skeletal pain, post-partum pain, toothache, sprains and straits, myositis, neuralgia, synovitis, arthritis, including rheumatoid arthritis, degenerative joint diseases, gout and ankylosing spondylitis, bursitis, burns including radiation and corrosive chemical injuries and sunburns; postoperative pain; neuropathic pain; visceral pain; tension headache; cluster headaches; migraine; motility-related disorders including gastrointestinal disorders, urinary incontinence and other urinary tract diseases; dysmenorrhea; preterm labour; diabetic retinopathy; tumour angiogenesis; cancer; metastatic tumour growth; neurodegenerative diseases including senile dementia, Alzheimer's disease, Pick's disease, Huntington's chorea, Parkinson's disease, Creutzfeldt-Jakob disease, or amyotrophic lateral sclerosis; neuroprotection/stroke; glaucoma; osteoporosis; bone fractures; Paget's disease; hyperthermia including different types of fever as rheumatic fever; symptoms associated with influenza or other viral infections; gastrointestinal disorders related with chemotherapy or irritable bowel syndrome; gastrointestinal bleeding; coagulation disorders including anaemia, hypoprothrombinemia, haemophilia or other bleeding problems; kidney diseases including nephritis, particularly mesangial proliferative glomerulonephritis and nephritic syndrome; thrombosis and occlusive vascular diseases.

**16.** A compound according to claim 14 where the disease or disorders comprises pain, inflammatory related pain including low back and neck pain, skeletal pain, post-partum pain, toothache, sprains and straits, myositis, neuralgia, synovitis, arthritis, including rheumatoid arthritis, degenerative joint diseases, gout and ankylosing spondylitis, bursitis, burns including radiation and corrosive chemical injuries and sunburns; postoperative pain; neuropathic pain; visceral pain; tension headache; cluster headaches; migraine.

**17.** Pharmaceutical composition comprising at least one compound according to any of claims 1-12 and at least one pharmaceutically acceptable carrier, additive, adjuvant or vehicle.

Europäisches Patentamt
European Patent Office
Office européen des brevets

# EUROPEAN SEARCH REPORT

Application Number
EP 12 38 2132

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/098600 A1 (ASTRAZENECA AB [SE]; ASTRAZENECA UK LTD [GB]; BECKER CHRISTOPHER [US];) 18 November 2004 (2004-11-18) * Formula (II); claims 1,2,3,5-8 * ----- | 1-3, 6-11, 13-17 | INV. C07D221/26 C07D401/04 A61K31/473 ADD. A61P1/00 A61P7/00 A61P13/00 A61P19/00 A61P25/00 A61P31/00 A61P35/00 A61P37/00 |
| A | WO 2005/016255 A2 (LIGAND PHARM INC [US]; MICHELLYS PIERRE [US]; CHEN JYUN-HUNG [US]; MEY) 24 February 2005 (2005-02-24) * claims 1-19 * ----- | 1-3, 6-15,17 | |
| A | WO 2004/072046 A2 (CAREX S A [FR]; KOUTNIKOVA HANA [FR]; SIERRA MICHAEL [FR]; BRAUN-EGLES) 26 August 2004 (2004-08-26) * abstract; page 99 - page 103; claims 1,3,5-8 * ----- | 1-3,6, 8-10, 13-17 | |
| | | | TECHNICAL FIELDS SEARCHED (IPC) |
| | | | C07D A61K A61P |

The present search report has been drawn up for all claims

1

| Place of search | Date of completion of the search | Examiner |
|---|---|---|
| Berlin | 12 September 2012 | Rufet, Jacques |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 12 38 2132

This annex lists the patent family members relating to the patent documents cited in the above-mentioned European search report.
The members are as contained in the European Patent Office EDP file on
The European Patent Office is in no way liable for these particulars which are merely given for the purpose of information.

12-09-2012

| Patent document cited in search report | Publication date | Patent family member(s) | Publication date |
|---|---|---|---|
| WO 2004098600 A1 | 18-11-2004 | AU 2004237130 A1 | 18-11-2004 |
| | | BR PI0410050 A | 25-04-2006 |
| | | CA 2524019 A1 | 18-11-2004 |
| | | CN 1784230 A | 07-06-2006 |
| | | EP 1631288 A1 | 08-03-2006 |
| | | JP 2006525302 A | 09-11-2006 |
| | | KR 20060009899 A | 01-02-2006 |
| | | MX PA05011785 A | 26-01-2006 |
| | | US 2007179172 A1 | 02-08-2007 |
| | | WO 2004098600 A1 | 18-11-2004 |
| | | ZA 200508860 A | 28-03-2007 |
| WO 2005016255 A2 | 24-02-2005 | NONE | |
| WO 2004072046 A2 | 26-08-2004 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BURGER.** Medicinal Chemistry and Drug Discovery. Wiley, 2001
- Design and Applications of Prodrugs. Harwood Academic Publishers, 1985
- **T.W. GREENE ; P.G.M. WUTS.** protective Groups in Organic Chemistry. John Wiley&Sons, 1999
- **ABE T ; KUNZ A ; SHIMAMURA M ; ZHOU P ; ANRATHER J ; LADECOLA C.** The neuroprotective effect of prostaglandin E2 EP1 receptor inhibition has a wide therapeutic window, is sustained in time and is not sexually dimorphic. *J Cereb Blood Flow Metab.,* 2009, vol. 29 (1), 66-72
- **ASBÓTH G ; PHANEUF S ; EUROPE-FINNER GN ; TÓTH M ; BERNAL AL.** Prostaglandin E2 activates phospholipase C and elevates intracellular calcium in cultured myometrial cells: involvement of EP1 and EP3 receptor subtypes. *Endocrinology,* 1996, vol. 137 (6), 2572-9
- **BABA H ; KOHNO T ; MOORE KA ; WOOLF CJ.** Direct activation of rat spinal dorsal horn neurons by prostaglandin E2. *The Journal of Neuroscience,* 2001, vol. 21 (5), 1750-1756
- **BREYER MD ; BREYER RM.** Prostaglandin receptors: their role in regulating renal function. *Curr Opin Nephrol Hypertens,* January 2000, vol. 9 (1), 23-9
- **CANDELARIO-JALIL E ; SLAWIK H ; RIDELIS I ; WASCHBISCH A ; AKUNDI RS ; HÜLL M ; FIEBICH BL.** Regional distribution of the prostaglandin E2 receptor EP1 in the rat brain: accumulation in Purkinje cells of the cerebellum. *J Mol Neurosci.,* 2005, 303-10
- **COLEMAN, R. A.** Prostanoid Receptors. IUPHAR compendium of receptor characterization and classification. 2000, 338-353
- **DIRIG DM ; YAKSH TL.** In vitro prostanoid release from spinal cord following peripheral inflammation: effects of substance P, NMDA and capsaicin. *Br J Pharmacol.,* 1999, vol. 126 (6), 1333-40
- **DURRENBERGER PF ; FACER P ; CASULA MA ; YIANGOU Y ; GRAY RA ; CHESSELL IP ; DAY NC ; COLLINS SD ; BINGHAM S ; WILSON AW.** Prostanoid receptor EP1 and Cox-2 in injured human nerves and a rat model of nerve injury: a time-course study. *BMC Neurol.,* 2006, vol. 4 (6), 1
- **GIBLIN GM ; BIT RA ; BROWN SH ; CHAIGNOT HM ; CHOWDHURY A ; CHESSELL IP ; CLAYTON NM ; COLEMAN T ; HALL A ; HAMMOND B.** The discovery of 6-[2-(5-chloro-2-{[(2,4-difluorophenyl)methyl]oxy}phenyl)-1-cyclopenten-1-yl]-2-pyridinecarboxylic acid, GW848687X, a potent and selective prostaglandin EP1 receptor antagonist for the treatment of inflammatory pain. *Bioorg Med Chem Lett.,* 2007, vol. 17 (2), 385-9
- **T.W. GREENE ; P.G.M. WUTS.** Protective groups in organic synthesis. John Wiley & sons, 1999, 10
- **GUAY J. ; BATEMAN, K. ; GORDON R. ; MANCINI J. ; RIENDEAU D.** Carrageenan-induced paw edema in rat elicits a predominant prostaglandin E2 (PGE2) response in the central nervous system associated with the induction of microsomal PGE2 synthase-1. *J. Biol Chem,* 2004, vol. 279, 24866-24872
- **HALL, A. ; BILLINTON A. ; GIBLIN G.M.** EP1 antagonists for the treatment of inflammatory pain. *Curr Opin. Drug Discov. Devel.,* 2007, vol. 10, 597-612
- **HALL A ; BROWN SH ; BUDD C ; CLAYTON NM ; GIBLIN GM ; GOLDSMITH P ; HAYHOW TG ; HURST DN ; NAYLOR A ; ANTHONY RAWLINGS D.** Discovery of GSK345931 A: An EP(1) receptor antagonist with efficacy in preclinical models of inflammatory pain. *Bioorg Med Chem Lett.,* 2009, vol. 19 (2), 497-501
- **HÖNEMANN CW ; HEYSE TJ ; MÖLLHOFF T ; HAHNENKAMP K ; BERNING S ; HINDER F ; LINCK B ; SCHMITZ W ; VAN AKEN H.** The inhibitory effect of bupivacaine on prostaglandin E(2) (EP(1)) receptor functioning: mechanism of action. *Anesth Analg.,* 2001, vol. 93 (3), 628-634
- **JOHANSSON T ; NARUMIYA S ; ZEILHOFER HU.** Contribution of peripheral versus central EP1 prostaglandin receptors to inflammatory pain. *Neurosci Lett.,* 2011, vol. 495 (2), 98-101
- **KAWAHARA H ; SAKAMOTO A ; TAKEDA S ; ONODERA H ; IMAKI J ; OGAWA R.** A prostaglandin E2 receptor subtype EP1 receptor antagonist (ONO-8711) reduces hyperalgesia, allodynia, and c-fos gene expression in rats with chronic nerve constriction. *Anesth Analg.,* 2001, vol. 93 (4), 1012-7

- **LEE T ; HEDLUND P ; NEWGREEN D ; ANDERSSON KE.** Urodynamic effects of a novel EP(1) receptor antagonist in normal rats and rats with bladder outlet obstruction. *J Urol.,* 2007, vol. 177 (4), 1562-1567
- **LEE C.M ; GENETOS DC ; YOU Z ; YELLOWLEY CE.** Hypoxia regulates PGE(2) release and EP1 receptor expression in osteoblastic cells. *J Cell Physiol.,* 2007, vol. 212 (1), 182-188
- **LI X ; CUDABACK E ; KEENE CD ; BREYER RM ; MONTINE TJ.** Suppressed microglial E prostanoid receptor 1 signaling selectively reduces tumor necrosis factor alpha and interleukin 6 secretion from toll-like receptor 3 activation. *Glia,* 2011, vol. 59 (4), 569-576
- **LIN CR ; AMAYA F ; BARRETT L ; WANG H ; TAKADA J ; SAMAD TA ; WOOLF CJ.** Prostaglandin E2 receptor EP4 contributes to inflammatory pain hypersensitivity. *J Pharmacol Exp Ther.,* 2006, vol. 319 (3), 1096-103
- **MA W ; EISENACH JC.** Four PGE2 EP receptors are up-regulated in injured nerve following partial sciatic nerve ligation. *Exp Neurol.,* 2003, vol. 183 (2), 581-92
- **MIKI, T. ; MATSUNAMI, M. ; OKADA, H. ; MATSUYA, H. ; KAWABATA, A.** ONO-8130, an EP1 antagonist, strongly attenuates cystitis-related bladder pain caused by cyclophosphamide in mice. *J Pharmacol Sci,* 2010, vol. 112 (1), 1J-12
- **MINAMI T ; UDA R. ; HORIGUCHI S. ; ITO S. ; HYODO M. ; HAYAISHI O.** Allodynia evoked by intrathecal administration fo prostaglandin E2 to conscious mice. *Pain,* 1994, vol. 57, 217-223
- **MINAMI T ; NAKANO H ; KOBAYASHI T ; SUGIMOTO Y ; USHIKUBI F ; ICHIKAWA A ; NARUMIYA S ; ITO S.** Characterization of EP receptor subtypes responsible for prostaglandin E2-induced pain responses by use of EP1 and EP3 receptor knockout mice. *Br J Pharmacol.,* 2001, vol. 133 (3), 438-44
- **MIZUGUCHI S ; OHNO T ; HATTORI Y ; AE T ; MINAMINO T ; SATOH T ; ARAI K ; SAEKI T ; HAYASHI I ; SUGIMOTO Y.** Roles of prostaglandin E2-EP1 receptor signaling in regulation of gastric motor activity and emptying. *Am J Physiol Gastrointest Liver Physiol.,* 2010, vol. 299 (5), G1078-1086
- **MORIYAMA T ; HIGASHI T ; TOGASHI K ; LIDA T ; SEGI E ; SUGIMOTO Y ; TOMINAGA T ; NARUMIYA S ; TOMINAGA M.** Sensitization of TRPV1 by EP1 and IP reveals peripheral nociceptive mechanism of prostaglandins. *Mol Pain.,* 2005, vol. 1, 3
- **NAKAYAMA Y ; OMOTE K ; NAMIKI A.** Role of prostaglandin receptor EP1 in the spinal dorsal horn in carrageenan-induced inflammatory pain. *Anesthesiology,* 2002, vol. 97 (5), 1254-62
- **NAKAYAMA Y ; OMOTE K ; KAWAMATA T ; NAMIKI A.** Role of prostaglandin receptor subtype EP1 in prostaglandin E2-induced nociceptive transmission in the rat spinal dorsal horn. *Brain Res.,* 2004, vol. 1010 (1-2), 62-8
- **NARUMIYA S. ; SUGIMOTO Y. ; USHIKUBI F.** Prostanoid receptors: structures, properties, and functions. *Physiol Rev.,* 1999, vol. 79, 1193-1226
- **NIHO N ; MUTOH M ; KITAMURA T ; TAKAHASHI M ; SATO H ; YAMAMOTO H ; MARUYAMA T ; OHUCHIDA S ; SUGIMURA T ; WAKABAYASHI K.** Suppression of azoxymethane-induced colon cancer development in rats by a prostaglandin E receptor EP1-selective antagonist. *Cancer Sci.,* 2005, vol. 96 (5), 260-264
- **OIDDA H. ; NAMBA T. ; SUGIMOTO Y. ; USHIKUBI F. ; OHISHI H. ; ICHIKAWA A. et al.** In situ hybridization studies of prostacyclin receptor mRNA expression in various mouse organs. *Br J Pharmacol,* 1995, vol. 116, 2828-2837
- **OKA T ; OKA K ; SAPER CB.** Contrasting effects of E type prostaglandin (EP) receptor agonists on core body temperature in rats. *Brain Res.,* 2003, vol. 968 (2), 256-262
- **OKA T ; HOSOI M ; OKA K ; HORI T.** Biphasic alteration in the trigeminal nociceptive neuronal responses after intracerebroventricular injection of prostaglandin E2 in rats. *Brain Res.,* 1997, vol. 749 (2), 354-7
- *Brain Res,* vol. 757 (2), 299
- **OKADA, H. ; KONEMURA, T. ; MARUYAMA, T.** ONO-8539, a novel ep1 receptor antagonist, suppresses bladder hyperactivity via excessive production of prostaglandin e2 (pge2) induced by intravesical instillation of atp in urodynamic evaluation of cynomolgus monkeys. *Eur Urol,* 2010, vol. 9 (2), 72
- **OMOTE K ; YAMAMOTO H ; KAWAMATA T ; NAKAYAMA Y ; NAMIKI A.** The effects of intrathecal administration of an antagonist for prostaglandin E receptor subtype EP(1) on mechanical and thermal hyperalgesia in a rat model of postoperative pain. *Anesth Analg,* 2002, vol. 95 (6), 1708-12
- **OMOTE K ; KAWAMATA T ; NAKAYAMA Y ; KAWAMATA M ; HAZAMA K ; NAMIKI A.** The effects of peripheral administration of a novel selective antagonist for prostaglandin E receptor subtype EP(1), ONO-8711, in a rat model of postoperative pain. *Anesth Analg.,* 2001, vol. 92 (1), 233-238
- **RAHAL S ; MCVEIGH LI ; ZHANG Y ; GUAN Y ; BREYER MD ; KENNEDY CR.** Increased severity of renal impairment in nephritic mice lacking the EP1 receptor. *Can J Physiol Pharmacol.,* 2006, vol. 84 (8-9), 877-885

- **SARKAR S ; HOBSON AR ; HUGHES A ; GROWCOTT J ; WOOLF CJ ; THOMPSON DG ; AZIZ Q.** The prostaglandin E2 receptor-1 (EP-1) mediates acid-induced visceral pain hypersensitivity in humans. *Gastroenterology,* 2003, vol. 124 (1), 18-25
- **SAMAD TA ; SAPIRSTEIN A ; WOOLF CJ.** Prostanoids and pain: unraveling mechanisms and revealing therapeutic targets. *Trends Mol Med.,* August 2002, vol. 8 (8), 390-6
- **SCHLÖTZER-SCHREHARDT U ; ZENKEL M ; NÜSING R.M.** Expression and Localization of FP and EP Prostanoid. *Invest. Ophthalmol. Vis. Sci.,* 2002, vol. 43 (5), 1475-1487
- **SYRIATOWICZ JP ; HU D ; WALKER JS ; TRACEY DJ.** Hyperalgesia due to nerve injury: role of prostaglandins. *Neuroscience,* 1999, vol. 94 (2), 587-94
- **TERAMURA, T. ; KAWATANI, M. ; MARUYAMA, T.** Prostaglandin E1 facilitate primary afferent activity from the urinary bladder in the rat using selective EP1-receptor antagonist (ONO-8711. *BJU Int,* 2000, vol. 86 (3), 6.3.19
- **WATANABE K ; KAWAMORI T ; NAKATSUGI S ; OHTA T ; OHUCHIDA S ; YAMAMOTO H ; MARUYAMA T ; KONDO K ; USHIKUBI F ; NARUMIYA S.** Role of the prostaglandin E receptor subtype EP1 in colon carcinogenesis. *Cancer Res.,* 1999, vol. 59 (20), 5093-5096
- **WILBRAHAM D. ; MASUDA T. ; DEACON S. ; KUWAYAMA T. ; VINCENT S.** Safety, tolerability and pharmacokinetic of multiple ascending doses of the ep-1 receptor antagonist ono-8539, a potential new and novel therapy to overactive bladder in healthy young and elderly subjects. *Eur Urol,* 2010, vol. 9 (2), 250
- **WOODWARD DF ; REGAN JW ; LAKE S ; OCKLIND A.** The molecular biology and ocular distribution of prostanoid receptors. *Surv Ophthalmol.,* 1997, vol. 41 (2), S15-21
- **ZHANG M ; HO HC ; SHEU TJ ; BREYER MD ; FLICK LM ; JONASON JH ; AWAD HA ; SCHWARZ EM ; O'KEEFE RJ.J.** EP1(-/-) mice have enhanced osteoblast differentiation and accelerated fracture repair. *Bone Miner Res.,* 2011, vol. 26 (4), 792-802